# EUROPEAN PATENT APPLICATION

(11) **EP 2 258 697 A1**
(43) Date of publication of application: **08.12.2010**
(21) Application number: 09723683.0
(22) Date of filing: 24.03.2009
(51) Int. Cl.: C07D 401/12, A61K 31/498, A61K 31/501, A61K 31/506, A61K 31/5377, A61P 7/02, A61P 9/00, C07D 401/14, C07D 417/14

(54) **HYDROXYQUINOXALINECARBOXAMIDE DERIVATIVE**

(30) Priority: 26.03.2008 JP 2008079919; 26.12.2008 JP 2008332106
(71) Applicant: Daiichi Sankyo Company, Limited, Chuo-ku Tokyo 103-8426 (JP)
(72) Inventor: MOTOKI, Kayoko, Tokyo 140-8710 (JP); NISHINAKA, Shigeyuki, Tokyo 140-8710 (JP); MASUDA, Sachie, Tokyo 140-8710 (JP); SAKAMOTO, Atsunobu, Tokyo 140-8710 (JP); KANEKO, Satoru, Tokyo 140-8710 (JP); ARITA, Tsuyoshi, Tokyo 140-8710 (JP); KAWAGOE, Keiichi, Tokyo 140-8710 (JP); YOKOMIZO, Aki, Tokyo 134-8630 (JP)
(74) Representative: Wallace, Sheila Jane
(86) International application number: PCT/JP2009/055741
(87) International publication number: WO 2009/119537

(57) **Abstract**

The present invention provides a novel hydroxyquinoxaline carboxamide derivative that is useful for preventing and/or treating blood coagulation disorders. A compound represented by formula (i), or a pharmacologically acceptable salt thereof: 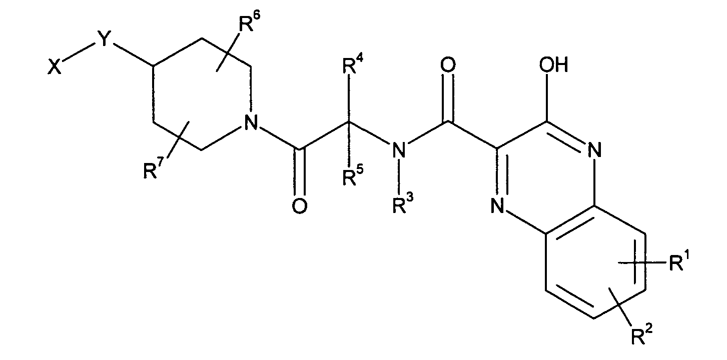 wherein, each of R¹ and R² independently represents a group such as a hydrogen atom or a halogen atom; R³ represents a group such as a hydrogen atom; each of R⁴ and R⁵ independently represents a group such as a hydrogen atom, a halogen atom or a C₁₋₄ alkyl group; each of R⁶ and R⁷ independently represents a hydrogen atom or a C₁₋₄ alkyl group; X represents a group such as a C₃₋₁₀ cycloalkyl group, C₆₋₁₀ aryl group or a 5- to 10-membered heterocyclic group, which may be substituted with substituent(s) selected from substituent group α; Y represents a group such as -CO-, -O- or -NRa-, and Ra represents a group such as a hydrogen atom or a C₁₋₄ alkyl group.

## Description

### TECHNICAL FIELD

The present invention relates to a hydroxyquinoxalinecarboxamide derivative, and pharmacologically acceptable salts thereof, that are useful as pharmaceuticals. More particularly, the present invention relates to a hydroxyquinoxalinecarboxamide derivative, and pharmacologically acceptable salts thereof, that demonstrate anticoagulant action.

### BACKGROUND ART

In recent years, average life span has become longer and the size of the elderly population has increased due to progress in medical care. In addition, the proportion of persons suffering from cardiovascular diseases has increased due to changes in eating habits and lifestyle. In particular, not only do thrombotic diseases such as cerebral infarction, myocardial infarction or peripheral circulatory disturbance demonstrate high mortality rates, but serious living restrictions caused by sequelae place a huge burden on patients. Inhibiting thrombus formation is important for preventing and treating these diseases.

Increased blood coagulation has conventionally been one of the causes of unstable angina, cerebral infarction, cerebral embolism, myocardial infarction, pulmonary infarction, pulmonary embolism, Buerger's disease, deep vein thrombosis, generalized intravascular coagulation syndrome, thrombosis following prosthetic valve replacement, reocclusion following vascular reconstruction and thrombosis during extracorporeal circulation. Accordingly, there is a need for an oral anticoagulant that has superior dose response, substantivity, low risk of hemorrhage and few side effects (Non-Patent Document 1).

Oral anticoagulants currently on the market are coumarin-based drugs as represented by warfarin (Patent Document 1). This drug inhibits the supply of reduced vitamin K by inhibiting the vitamin K metabolic cycle, and inhibits γ-carboxylation modification (Gla modification) of glutamic acid residues by γ-carboxylase. This leads the drug to inhibit activation of vitamin K-dependent blood coagulation factors (such as FII, FVII, FIX or FX) and demonstrate potent anticoagulant action. However, while warfarin demonstrates potent and stable sustained pharmacological effects, it has a narrow effective therapeutic range and requires monitoring by frequent blood sampling in order to accommodate individual differences in the effective dose. In addition, the risk of deviating from the therapeutic range due to drug interactions and the influence of vitamin K in the diet has also been pointed out. Thus, there is a desire for an oral anticoagulant that allows pharmacological effects to be easily controlled with low-frequency monitoring, has superior safety and has high general versatility.

In recent years, a coumarin derivative ATI-5923 (Patent Document 2) has been reported that is designed to avoid the drug interaction of warfarin and is currently undergoing clinical trials. In addition, sulfaquinoxaline has been reported as a compound other than a coumarin derivative (Non-Patent Document 2). However, the hydroxyquinoxaline structure possessed by the compounds of the present application as a partial structure thereof is not disclosed or suggested in these prior art documents.
[Patent Document 1] U.S. Patent No. 2642441
[Patent Document 2] WO 2005/100336
[Non-Patent Document 1] Thrombosis Research, Vol. 68, pp. 507-512, 1992
[Non-Patent Document 2] Archive of Biochemistry and Biophysics, Vol. 269, pp. 18-24, 1989

### DISCLOSURE OF THE INVENTION

### [Problems to be Solved by the Invention]

As a result of conducting studies on the synthesis of compounds which are able to inhibit activation of blood coagulation factors as described above along with the pharmacological action thereof, the inventors of the present invention found that a group of compounds having for the basic structure thereof a hydroxyquinoxalinecarboxamide derivative has superior anticoagulant action, thereby leading to completion of the present invention.

### [Means for Solving the Problems]

The present invention is directed to:
(1) a compound represented by the following general formula (i): 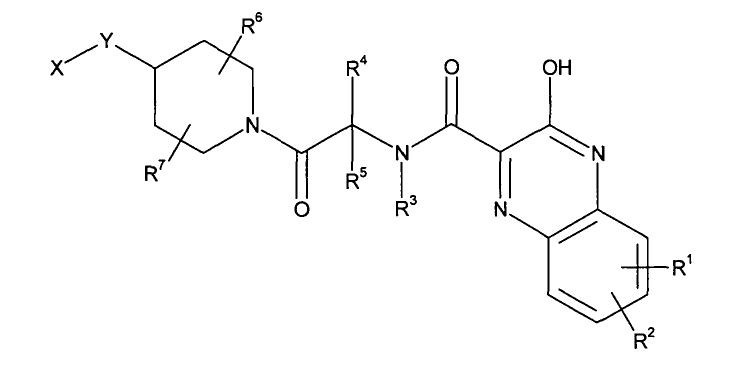 wherein,
   each of R¹ and R² independently represents a hydrogen atom, a halogen atom, a cyano group, a C₁₋₄ alkyl group or a C₁₋₄ alkoxy group,
   R³ represents a hydrogen atom or a C₁₋₄ alkyl group,
   each of R⁴ and R⁵ independently represents a hydrogen atom, a halogen atom, a C₁₋₄ alkyl group, a C₂₋₄ alkenyl group, a C₂₋₄ alkynyl group, a C₃₋₅ cycloalkyl group or a hydroxy C₁₋₄ alkyl group,
   each of R⁶ and R⁷ independently represents a hydrogen atom or a C₁₋₄ alkyl group,
   X represents a monocyclic or bicyclic C₃₋₁₀ cycloalkyl group, a monocyclic or bicyclic C₆₋₁₀ aryl group or a monocyclic or bicyclic 5-to 10-membered heterocyclic group (in which the heterocyclic group includes aromatic heterocycles and non-aromatic heterocycles and contains 1 to 3 atoms selected from the group consisting of a nitrogen atom, a sulfur atom and an oxygen atom), which may be substituted with 1 to 3 substituents selected from substituent group α,
   Y represents -CHRa-, -C(OH)Ra-, -CO-, -O-, -NRa-, -S-, -SO- or - SO₂-,
   Ra represents a hydrogen atom, a C₁₋₄ alkyl group, a halogeno C₁₋₄ alkyl group or a C₁₋₄ alkanoyl group, or
   in the case where Ra in Y is a C₁₋₄ alkyl group, a halogeno C₁₋₄ alkyl group or a C₁₋₄ alkanoyl group, Ra may form a 5- or 6-membered ring by bonding with an atom that is a component of X,
   substituent group α represents:
   a halogen atom, a cyano group, a nitro group, a hydroxy group;
   a C₁₋₄ alkyl group, a C₂₋₄ alkenyl group, a C₂₋₄ alkynyl group, a C₃₋₆ cycloalkyl group, a C₁₋₄ alkoxy group or a C₃₋₆ cycloalkoxy group, which may be substituted with substituent(s) selected from substituent group β;
   -OCORb, -CORb, -COORb, -CONRbRc, -NRbRc, -NRbCORc, -NRbSORc,-NRbSO₂Rc, -SRb, -SORb, -SO₂Rb;
   a phenyl group, and
   a 5- or 6-membered heterocyclic group, which may be substituted with a C₁₋₄ alkyl group(s) (in which the heterocyclic group includes aromatic heterocycles and non-aromatic heterocycles and contains 1 to 3 atoms selected from the group consisting of a nitrogen atom, a sulfur atom and an oxygen atom),
   each of Rb and Rc independently represents a hydrogen atom, a C₁₋₄ alkyl group or a halogeno C₁₋₄ alkyl group, and
   substituent group β represents a halogen atom, a hydroxy group, a C₁₋₄ alkoxy group and a C₁₋₄ alkanoyloxy group,
   or a pharmacologically acceptable salt thereof,
(2) a compound or a pharmacologically acceptable salt thereof, described in (1) above, wherein each of R¹ and R² independently represents a hydrogen atom or a halogen atom,
(3) a compound or a pharmacologically acceptable salt thereof, described in (1) above, wherein each of R¹ and R² independently represents a hydrogen atom or a fluorine atom,
(4) a compound or a pharmacologically acceptable salt thereof, described in (1) above, wherein R¹ and R² both represent a hydrogen atom,
(5) a compound or a pharmacologically acceptable salt thereof, described in any one of (1) to (4) above, wherein R³ represents a hydrogen atom or a methyl group,
(6) a compound or a pharmacologically acceptable salt thereof, described in any one of (1) to (4) above, wherein R³ represents a hydrogen atom,
(7) a compound or a pharmacologically acceptable salt thereof, described in any one of (1) to (6) above, wherein R⁴ represents a hydrogen atom, a fluorine atom, a C₁₋₄ alkyl group, a C₂₋₄ alkenyl group, a C₂₋₄ alkynyl group, a C₃₋₅ cycloalkyl group or a hydroxy C₁₋₄ alkyl group, and R⁵ represents a hydrogen atom,
(8) a compound or a pharmacologically acceptable salt thereof, described in any one of (1) to (6) above, wherein R⁴ represents a methyl group, an ethyl group, an isopropyl group, an isobutyl group, a t-butyl group, a cyclopropyl group or a 1-hydroxy-1-methylethyl group, and R⁵ represents a hydrogen atom,
(9) a compound or a pharmacologically acceptable salt thereof, described in any one of (1) to (6) above, wherein R⁴ represents a methyl group, an ethyl group, an isopropyl group, a t-butyl group, a cyclopropyl group or a 1-hydroxy-1-methylethyl group, and R⁵ represents a hydrogen atom,
(10) a compound or a pharmacologically acceptable salt thereof, described in any one of (1) to (6) above, wherein R⁴ represents an ethyl group, an isopropyl group, a cyclopropyl group or a 1-hydroxy-1-methylethyl group, and R⁵ represents a hydrogen atom,
(11) a compound or a pharmacologically acceptable salt thereof, described in any one of (1) to (6) above, wherein each of R⁴ and R⁵ independently represents a hydrogen atom, a halogen atom, a C₁₋₄ alkyl group, a C₂₋₄ alkenyl group, a C₂₋₄ alkynyl group or a C₃₋₅ cycloalkyl group,
(12) a compound or a pharmacologically acceptable salt thereof, described in any one of (1) to (6) above, wherein R⁴ represents a hydrogen atom, a methyl group, an ethyl group, an isopropyl group, an isobutyl group, a t-butyl group or a cyclopropyl group, and R⁵ represents a hydrogen atom,
(13) a compound or a pharmacologically acceptable salt thereof, described in any one of (1) to (12) above, wherein each of R⁶ and R⁷ independently represents a hydrogen atom or methyl group,
(14) a compound or a pharmacologically acceptable salt thereof, described in any one of (1) to (12) above, wherein R⁶ and R⁷ both represent a hydrogen atom,
(15) a compound or a pharmacologically acceptable salt thereof, described in any one of (1) to (14) above, wherein X represents a phenyl group, a naphthyl group, a pyridyl group, a pyridazinyl group, a pyrimidinyl group, a pyrazinyl group, a quinolyl group, an isoquinolyl group, a benzimidazolyl group, a benzoxazolyl group or a benzothiazolyl group, which may be substituted with 1 or 2 substituents selected from substituent group α, and
   substituent group α represents a halogen atom, a cyano group, a nitro group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, an acetoxy group, a trifluoromethoxy group, an acetyl group, a methoxycarbonyl group, an ethoxycarbonyl group, a dimethylcarbamoyl group, a piperidyl group, a pyrrolidyl group, a morpholino group, a pyrazolyl group, a methylpyrazolyl group, an oxadiazolyl group, a methyloxadiazolyl group, a phenyl group and a pyridyl group,
(16) a compound or a pharmacologically acceptable salt thereof, described in any one of (1) to (14) above, wherein X represents a phenyl group, a naphthyl group, a pyridyl group, a pyridazinyl group, a pyrimidinyl group, a pyrazinyl group, a quinolyl group or an isoquinolyl group, which may be substituted with 1 or 2 substituents selected from substituent group α, and
   substituent group α represents a fluorine atom, a chlorine atom, a bromine atom, a cyano group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, a trifluoromethoxy group, a piperidyl group, a pyrrolidyl group, a phenyl group and a pyridyl group,
(17) a compound or a pharmacologically acceptable salt thereof, described in any one of (1) to (14) above, wherein X represents a phenyl group, a 2-pyridyl group, a 3-pyridyl group, a 3-pyridazinyl group, a 2-pyrimidinyl group or a 5-pyrimidinyl group, which may be substituted with 1 or 2 substituents selected from substituent group α, and
   substituent group α represents a fluorine atom, a chlorine atom, a bromine atom, a cyano group, a hydroxy group, a methyl group, an ethyl group, a t-butyl group, a trifluoromethyl group, a methoxy group, an ethoxy group, a trifluoromethoxy group, a phenyl group and a pyridyl group,
(18) a compound or a pharmacologically acceptable salt thereof, described in any one of (1) to (14) above, wherein X represents a phenyl group or a 2-pyridyl group, which may be substituted with 1 or 2 substituents selected from substituent group α, and substituent group α represents a fluorine atom, a chlorine atom, a trifluoromethyl group, a methoxy group and a trifluoromethoxy group,
(19) a compound or a pharmacologically acceptable salt thereof, described in any one of (1) to (14) above, wherein X represents 4-fluorophenyl, 2,4-difluorophenyl, 3,4-difluorophenyl, 4-chlorophenyl, 2,4-dichlorophenyl, 4-trifluoromethylphenyl, 4-trifluoromethoxyphenyl, 5-fluoropyridin-2-yl, 5-chloropyridin-2-yl, 5-trifluoromethylpyridin-2-yl or 6-methoxypyridin-3-yl,
(20) a compound or a pharmacologically acceptable salt thereof, described in any one of (1) to (14) above, wherein X represents a phenyl group, a naphthyl group, a pyridyl group, a pyridazinyl group, a pyrimidinyl group, a pyrazinyl group, a quinolyl group or an isoquinolyl group, which may be substituted with 1 or 2 substituents selected from substituent group α, and substituent group α represents a fluorine atom, a chlorine atom, a bromine atom, a cyano group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, a trifluoromethoxy group, a phenyl group and a pyridyl group,
(21) a compound or a pharmacologically acceptable salt thereof, described in any one of (1) to (14) above, wherein X represents a phenyl group, a naphthyl group, a pyridyl group, a pyridazinyl group or a pyrimidinyl group, which may be substituted with 1 or 2 substituents selected from substituent group α, and substituent group α represents a fluorine atom, a chlorine atom, a bromine atom, a cyano group, a hydroxy group, a methyl group, a trifluoromethyl group, a methoxy group, a trifluoromethoxy group and a phenyl group,
(22) a compound or a pharmacologically acceptable salt thereof, described in any one of (1) to (14) above, wherein X represents a phenyl group or a pyridyl group, which may be substituted with 1 or 2 substituents selected from substituent group α, and substituent group α represents a fluorine atom, a chlorine atom, a cyano group, a trifluoromethyl group and a trifluoromethoxy group,
(23) a compound or a pharmacologically acceptable salt thereof, described in any one of (1) to (22) above, wherein Y represents -CO-, -O- or -NRa-, Ra represents a hydrogen atom, a methyl group or an ethyl group, and Ra may form a 5-membered ring by bonding with an atom that is a component of X in the case where Ra is an ethyl group in Y,
(24) a compound or a pharmacologically acceptable salt thereof, described in any one of (1) to (22) above, wherein Y represents -O- or -NRa-, and Ra represents a hydrogen atom, a methyl group or an ethyl group,
(25) a compound or a pharmacologically acceptable salt thereof, described in any one of (1) to (22) above, wherein Y represents -O-,
(26) a compound or a pharmacologically acceptable salt thereof, described in (1) above, which is selected from the following:
   N-{(1S)-2-[4-(2-fluorophenoxy)piperidin-2-yl]-1-methyl-2-oxoethyl}-3-hydroxyquinoxaline-2-carboxamide,
   N-{(1S)-2-[4-(3-fluorophenoxy)piperidin-1-yl]-1-methyl-2-oxoethyl}-3-hydroxyquinoxaline-2-carboxamide,
   N-{(1S)-2-[4-(4-fluorophenoxy)piperidin-1-yl]-1-methyl-2-oxoethyl}-3-hydroxyquinoxaline-2-carboxamide,
   N-{(1S)-2-[4-(4-chlorophenoxy)piperidin-1-yl]-1-methyl-2-oxoethyl}-3-hydroxyquinoxaline-2-carboxamide,
   3-hydroxy-N-[(1S)-1-methyl-2-oxo-2-{4-[4-(trifluoromethoxy)phenoxy]piperidin-1-yl}ethyl]quinoxaline-2-carboxamide,
   N-{(1S)-2-[4-(4-cyanophenoxy)piperidin-1-yl]-1-methyl-2-oxoethyl}-3-hydroxyquinoxaline-2-carboxamide,
   N-{(1S)-2-[4-(2,4-difluorophenoxy)piperidin-1-yl]-1-methyl-2-oxoethyl}-3-hydroxyquinoxaline-2-carboxamide,
   N-{(1S)-2-[4-(3,4-difluorophenoxy)piperidin-1-yl]-1-methyl-2-oxoethyl}-3-hydroxyquinoxaline-2-carboxamide,
   N-[(1S)-2-{4-[(5-chloropyridin-2-yl)oxy]piperidin-1-yl}-1-methyl-2-oxoethyl]-3-hydroxyquinoxaline-2-carboxamide,
   N-[(1S)-2-{4-[(5-fluoropyridin-2-yl)oxy]piperidin-1-yl}-1-methyl-2-oxoethyl]-3-hydroxyquinoxaline-2-carboxamide,
   3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
   N-[(1S)-1-{[4-(2-fluorophenoxy)piperidin-1-yl]carbonyl}propyl]-3-hydroxyquinoxaline-2-carboxamide,
   N-[(1S)-1-{[4-(3-fluorophenoxy)piperidin-1-yl]carbonyl}propyl]-3-hydroxyquinoxaline-2-carboxamide,
   N-[(1S)-1-{[4-(4-fluorophenoxy)piperidin-1-yl]carbonyl}propyl]-3-hydroxyquinoxaline-2-carboxamide,
   N-[(1S)-1-{[4-(4-chlorophenoxy)piperidin-1-yl]carbonyl}propyl]-3-hydroxyquinoxaline-2-carboxamide,
   3-hydroxy-N-[(1S)-1-({4-[4-(trifluoromethoxy)phenoxy]piperidin-1-yl}carbonyl)propyl]quinoxaline-2-carboxamide,
   N-[(1S)-1-{[4-(4-cyanophenoxy)piperidin-1-yl]carbonyl}propyl]-3-hydroxyquinoxaline-2-carboxamide,
   N-[(1S)-1-{[4-(2,4-difluorophenoxy)piperidin-1-yl]carbonyl}propyl]-3-hydroxyquinoxaline-2-carboxamide,
   N-[(1S)-1-{[4-(3,4-difluorophenoxy)piperidin-1-yl]carbonyl}propyl]-3-hydroxyquinoxaline-2-carboxamide,
   N-[(1S)-1-({4-[(5-fluoropyridin-2-yl)oxy]piperidin-1-yl}carbonyl)propyl]-3-hydroxyquinoxaline-2-carboxamide,
   N-[(1S)-1-({4-[(5-chloropyridin-2-yl)oxy]piperidin-1-yl}carbonyl)propyl]-3-hydroxyquinoxaline-2-carboxamide,
   3-hydroxy-N-{(1S)-1-[(4-{[5-(trifluoromethyl)pyridin-2-yl]oxy}piperidin-1-yl)carbonyl]propyl}quinoxaline-2-carboxamide,
   N-[(1S)-1-{[4-(2-fluorophenoxy)piperidin-1-yl]carbonyl}-2-methylpropyl]-3-hydroxyquinoxaline-2-carboxamide,
   N-[(1S)-1-{[4-(3-fluorophenoxy)piperidin-1-yl]carbonyl}-2-methylpropyl]-3-hydroxyquinoxaline-2-carboxamide,
   N-[(1S)-1-{[4-(4-fluorophenoxy)piperidin-1-yl]carbonyl}-2-methylpropyl]-3-hydroxyquinoxaline-2-carboxamide,
   N-[(1S)-1-{[4-(4-chlorophenoxy)piperidin-1-yl]carbonyl}-2-methylpropyl]-3-hydroxyquinoxaline-2-carboxamide,
   3-hydroxy-N-[(1S)-2-methyl-1-({4-[4-(trifluoromethoxy)phenoxy]piperidin-1-yl}carbonyl)propyl]quinoxaline-2-carboxamide,
   N-[(1S)-1-{[4-(4-cyanophenoxy)piperidin-1-yl]carbonyl}-2-methylpropyl]-3-hydroxyquinoxaline-2-carboxamide,
   N-[(1S)-1-{[4-(2,4-difluorophenoxy)piperidin-1-yl]carbonyl}-2-methylpropyl]-3-hydroxyquinoxaline-2-carboxamide,
   N-[(1S)-1-{[4-(3,4-difluorophenoxy)piperidin-1-yl]carbonyl}-2-methylpropyl]-3-hydroxyquinoxaline-2-carboxamide,
   N-[(1S)-1-({4-[(5-chloropyridin-2-yl)oxy]piperidin-1-yl}carbonyl)-2-methylpropyl]-3-hydroxyquinoxaline-2-carboxamide,
   N-[(1S)-1-({4-[(5-fluoropyridin-2-yl)oxy]piperidin-1-yl}carbonyl)-2-methylpropyl]-3-hydroxyquinoxaline-2-carboxamide,
   3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide,
   3-hydroxy-N-[(1S)-1-({4-[(6-methoxypyridin-3-yl)oxy]piperidin-1-yl}carbonyl)-2-methylpropyl]quinoxaline-2-carboxamide,
   N-[(1S)-1-{[4-(2,4-difluorobenzoyl)piperidin-1-yl]carbonyl}-2-methylpropyl]-3-hydroxyquinoxaline-2-carboxamide,
   N-[(1S)-1-({4-[(2,4-difluorophenyl)(hydroxy)methyl]piperidin-1-yl}carbonyl)-2-methylpropyl]-3-hydroxyquinoxaline-2-carboxamide,
   N-[(1S)-1-{[4-(4-fluorophenoxy)piperidin-1-yl]carbonyl}-2-hydroxy-2-methylpropyl]-3-hydroxyquinoxaline-2-carboxamide,
   N-{(1S)-1-cyclopropyl-2-[4-(2-fluorophenoxy)piperidin-1-yl]-2-oxoethyl}-3-hydroxyquinoxaline-2-carboxamide,
   N-{(1S)-1-cyclopropyl-2-[4-(3-fluorophenoxy)piperidin-1-yl]-2-oxoethyl}-3-hydroxyquinoxaline-2-carboxamide,
   N-{(1S)-1-cyclopropyl-2-[4-(4-fluorophenoxy)piperidin-1-yl]-2-oxoethyl}-3-hydroxyquinoxaline-2-carboxamide,
   N-{(1S)-2-[4-(4-chlorophenoxy)piperidin-1-yl]-1-cyclopropyl-2-oxoethyl}-3-hydroxyquinoxaline-2-carboxamide,
   N-[(1S)-1-cyclopropyl-2-oxo-2-{4-[4-(trifluoromethoxy)phenoxy]piperidin-1-yl}ethyl]-3-hydroxyquinoxaline-2-carboxamide,
   N-{(1S)-2-[4-(4-cyanophenoxy)piperidin-1-yl]-1-cyclopropyl-2-oxoethyl}-3-hydroxyquinoxaline-2-carboxamide,
   N-{(1S)-1-cyclopropyl-2-[4-(2,4-difluorophenoxy)piperidin-1-yl]-2-oxoethyl}-3-hydroxyquinoxaline-2-carboxamide,
   N-{(1S)-1-cyclopropyl-2-[4-(3,4-difluorophenoxy)piperidin-1-yl]-2-oxoethyl}-3-hydroxyquinoxaline-2-carboxamide,
   N-[(1S)-1-cyclopropyl-2-{4-[(5-fluoropyridin-2-yl)oxy]piperidin-1-yl}-2-oxoethyl]-3-hydroxyquinoxaline-2-carboxamide,
   N-[(1S)-2-{4-[(5-chloropyridin-2-yl)oxy]piperidin-1-yl}-1-cyclopropyl-2-oxoethyl]-3-hydroxyquinoxaline-2-carboxamide,
   N-[(1S)-1-cyclopropyl-2-oxo-2-(4-{[5-(trifluoromethyl)pyridin-2-yl]oxy}piperidin-1-yl)ethyl]-3-hydroxyquinoxaline-2-carboxamide,
   N-[(1S)-2,2-dimethyl-1-{[4-(2-fluorophenoxy)piperidin-1-yl]carbonyl}propyl]-3-hydroxyquinoxaline-2-carboxamide,
   N-[(1S)-2,2-dimethyl-1-{[4-(3-fluorophenoxy)piperidin-1-yl]carbonyl}propyl]-3-hydroxyquinoxaline-2-carboxamide,
   N-[(1S)-2,2-dimethyl-1-{[4-(4-fluorophenoxy)piperidin-1-yl]carbonyl}propyl]-3-hydroxyquinoxaline-2-carboxamide,
   N-[(1S)-1-{[4-(4-chlorophenoxy)piperidin-1-yl]carbonyl}-2,2-dimethylpropyl]-3-hydroxyquinoxaline-2-carboxamide,
   N-[(1S)-2,2-dimethyl-1-({4-[4-(trifluoromethoxy)phenoxy]piperidin-1-yl}carbonyl)propyl]-3-hydroxyquinoxaline-2-carboxamide,
   N-[(1S)-1-{[4-(4-cyanophenoxy)piperidin-1-yl]carbonyl}-2,2-dimethylpropyl]-3-hydroxyquinoxaline-2-carboxamide,
   N-[(1S)-1-{[4-(2,4-difluorophenoxy)piperidin-1-yl]carbonyl}-2,2-dimethylpropyl]-3-hydroxyquinoxaline-2-carboxamide,
   N-[(1S)-1-{[4-(3,4-difluorophenoxy)piperidin-1-yl]carbonyl}-2,2-dimethylpropyl]-3-hydroxyquinoxaline-2-carboxamide,
   N-[(1S)-2,2-dimethyl-1-({4-[(5-fluoropyridin-2-yl)oxy]piperidin-1-yl}carbonyl)propyl]-3-hydroxyquinoxaline-2-carboxamide,
   N-[(1S)-1-({4-[(5-chloropyridin-2-yl)oxy]piperidin-1-yl}carbonyl)-2,2-dimethylpropyl]-3-hydroxyquinoxaline-2-carboxamide, and
   N-{(1S)-2,2-dimethyl-1-[(4-{[5-(trifluoromethyl)pyridin-2-yl]oxy}piperidin-1-yl)carbonyl]propyl}-3-hydroxyquinoxaline-2-carboxamide,
(27) a compound or a pharmacologically acceptable salt thereof, described in (1) above, which is selected from the following:
   N-{(1S)-2-[4-(2-fluorophenoxy)piperidin-1-yl]-1-methyl-2-oxoethyl}-3-hydroxyquinoxaline-2-carboxamide,
   N-{(1S)-2-[4-(3-fluorophenoxy)piperidin-1-yl]-1-methyl-2-oxoethyl}-3-hydroxyquinoxaline-2-carboxamide,
   N-{(1S)-2-[4-(4-fluorophenoxy)piperidin-1-yl]-1-methyl-2-oxoethyl}-3-hydroxyquinoxaline-2-carboxamide,
   N-{(1S)-2-[4-(4-chlorophenoxy)piperidin-1-yl]-1-methyl-2-oxoethyl}-3-hydroxyquinoxaline-2-carboxamide,
   3-hydroxy-N-[(1S)-1-methyl-2-oxo-2-{4-[4-(trifluoromethoxy)phenoxy]piperidin-1-yl}ethyl]quinoxaline-2-carboxamide,
   N-{(1S)-2-[4-(4-cyanophenoxy)piperidin-1-yl]-1-methyl-2-oxoethyl}-3-hydroxyquinoxaline-2-carboxamide,
   N-{(1S)-2-[4-(2,4-difluorophenoxy)piperidin-1-yl]-1-methyl-2-oxoethyl}-3-hydroxyquinoxaline-2-carboxamide,
   N-{(1S)-2-[4-(3,4-difluorophenoxy)piperidin-1-yl]-1-methyl-2-oxoethyl}-3-hydroxyquinoxaline-2-carboxamide,
   N-[(1S)-2-{4-[(5-chloropyridin-2-yl)oxy]piperidin-1-yl}-1-methyl-2-oxoethyl]-3-hydroxyquinoxaline-2-carboxamide,
   N-[(1S)-2-{4-[(5-fluoropyridin-2-yl)oxy]piperidin-1-yl}-1-methyl-2-oxoethyl]-3-hydroxyquinoxaline-2-carboxamide,
   3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
   N-[(1S)-1-{[4-(2-fluorophenoxy)piperidin-1-yl]carbonyl}propyl]-3-hydroxyquinoxaline-2-carboxamide,
   N-[(1S)-1-{[4-(3-fluorophenoxy)piperidin-1-yl]carbonyl}propyl]-3-hydroxyquinoxaline-2-carboxamide,
   N-[(1S)-1-{[4-(4-fluorophenoxy)piperidin-1-yl]carbonyl}propyl]-3-hydroxyquinoxaline-2-carboxamide,
   N-[(1S)-1-{[4-(4-chlorophenoxy)piperidin-1-yl]carbonyl}propyl]-3-hydroxyquinoxaline-2-carboxamide,
   3-hydroxy-N-[(1S)-1-({4-[4-(trifluoromethoxy)phenoxy]piperidin-1-yl}carbonyl)propyl]quinoxaline-2-carboxamide,
   N-[(1S)-1-{[4-(4-cyanophenoxy)piperidin-1-yl]carbonyl}propyl]-3-hydroxyquinoxaline-2-carboxamide,
   N-[(1S)-1-{[4-(2,4-difluorophenoxy)piperidin-1-yl]carbonyl}propyl]-3-hydroxyquinoxaline-2-carboxamide,
   N-[(1S)-1-{[4-(3,4-difluorophenoxy)piperidin-1-yl]carbonyl}propyl]-3-hydroxyquinoxaline-2-carboxamide,
   N-[(1S)-1-({4-[(5-fluoropyridin-2-yl)oxy]piperidin-1-yl}carbonyl)propyl]-3-hydroxyquinoxaline-2-carboxamide,
   N-[(1S)-1-({4-[(5-chloropyridin-2-yl)oxy]piperidin-1-yl}carbonyl)propyl]-3-hydroxyquinoxaline-2-carboxamide,
   3-hydroxy-N-{(1S)-1-[(4-{[5-(trifluoromethyl)pyridin-2-yl]oxy}piperidin-1-yl)carbonyl]propyl}quinoxaline-2-carboxamide,
   N-[(1S)-1-{[4-(2-fluorophenoxy)piperidin-1-yl]carbonyl}-2-methylpropyl]-3-hydroxyquinoxaline-2-carboxamide,
   N-[(1S)-1-{[4-(3-fluorophenoxy)piperidin-1-yl]carbonyl}-2-methylpropyl]-3-hydroxyquinoxaline-2-carboxamide,
   N-[(1S)-1-{[4-(4-fluorophenoxy)piperidin-1-yl]carbonyl}-2-methylpropyl]-3-hydroxyquinoxaline-2-carboxamide,
   N-[(1S)-1-{[4-(4-chlorophenoxy)piperidin-1-yl]carbonyl}-2-methylpropyl]-3-hydroxyquinoxaline-2-carboxamide,
   3-hydroxy-N-[(1S)-2-methyl-1-({4-[4-(trifluoromethoxy)phenoxy]piperidin-1-yl}carbonyl)propyl]quinoxaline-2-carboxamide,
   N-[(1S)-1-{[4-(4-cyanophenoxy)piperidin-1-yl]carbonyl}-2-methylpropyl]-3-hydroxyquinoxaline-2-carboxamide,
   N-[(1S)-1-{[4-(2,4-difluorophenoxy)piperidin-1-yl]carbonyl}-2-methylpropyl]-3-hydroxyquinoxaline-2-carboxamide,
   N-[(1S)-1-{[4-(3,4-difluorophenoxy)piperidin-1-yl]carbonyl}-2-methylpropyl]-3-hydroxyquinoxaline-2-carboxamide,
   N-[(1S)-1-({4-[(5-chloropyridin-2-yl)oxy]piperidin-1-yl}carbonyl)-2-methylpropyl]-3-hydroxyquinoxaline-2-carboxamide,
   N-[(1S)-1-({4-[(5-fluoropyridin-2-yl)oxy]piperidin-1-yl}carbonyl)-2-methylpropyl]-3-hydroxyquinoxaline-2-carboxamide,
   3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide,
   N-{(1S)-1-cyclopropyl-2-[4-(2-fluorophenoxy)piperidin-1-yl]-2-oxoethyl}-3-hydroxyquinoxaline-2-carboxamide,
   N-{(1S)-1-cyclopropyl-2-[4-(3-fluorophenoxy)piperidin-1-yl]-2-oxoethyl}-3-hydroxyquinoxaline-2-carboxamide,
   N-{(1S)-1-cyclopropyl-2-[4-(4-fluorophenoxy)piperidin-1-yl]-2-oxoethyl}-3-hydroxyquinoxaline-2-carboxamide,
   N-{(1S)-2-[4-(4-chlorophenoxy)piperidin-1-yl]-1-cyclopropyl-2-oxoethyl}-3-hydroxyquinoxaline-2-carboxamide,
   N-[(1S)-1-cyclopropyl-2-oxo-2-{4-[4-(trifluoromethoxy)phenoxy]piperidin-1-yl}ethyl]-3-hydroxyquinoxaline-2-carboxamide,
   N-{(1S)-2-[4-(4-cyanophenoxy)piperidin-1-yl]-1-cyclopropyl-2-oxoethyl}-3-hydroxyquinoxaline-2-carboxamide,
   N-{(1S)-1-cyclopropyl-2-[4-(2,4-difluorophenoxy)piperidin-1-yl]-2-oxoethyl}-3-hydroxyquinoxaline-2-carboxamide,
   N-{(1S)-1-cyclopropyl-2-[4-(3,4-difluorophenoxy)piperidin-1-yl]-2-oxoethyl}-3-hydroxyquinoxaline-2-carboxamide,
   N-[(1S)-1-cyclopropyl-2-{4-[(5-fluoropyridin-2-yl)oxy]piperidin-1-yl}-2-oxoethyl]-3-hydroxyquinoxaline-2-carboxamide,
   N-[(1S)-2-{4-[(5-chloropyridin-2-yl)oxy]piperidin-1-yl}-1-cyclopropyl-2-oxoethyl]-3-hydroxyquinoxaline-2-carboxamide,
   N-[(1S)-1-cyclopropyl-2-oxo-2-(4-{[5-(trifluoromethyl)pyridin-2-yl]oxy}piperidin-1-yl)ethyl]-3-hydroxyquinoxaline-2-carboxamide,
   N-[(1S)-2,2-dimethyl-1-{[4-(2-fluorophenoxy)piperidin-1-yl]carbonyl}propyl]-3-hydroxyquinoxaline-2-carboxamide,
   N-[(1S)-2,2-dimethyl-1-{[4-(3-fluorophenoxy)piperidin-1-yl]carbonyl}propyl]-3-hydroxyquinoxaline-2-carboxamide,
   N-[(1S)-2,2-dimethyl-1-{[4-(4-fluorophenoxy)piperidin-1-yl]carbonyl}propyl]-3-hydroxyquinoxaline-2-carboxamide,
   N-[(1S)-1-{[4-(4-chlorophenoxy)piperidin-1-yl]carbonyl}-2,2-dimethylpropyl]-3-hydroxyquinoxaline-2-carboxamide,
   N-[(1S)-2,2-dimethyl-1-({4-[4-(trifluoromethoxy)phenoxy]piperidin-1-yl}carbonyl)propyl]-3-hydroxyquinoxaline-2-carboxamide,
   N-[(1S)-1-{[4-(4-cyanophenoxy)piperidin-1-yl]carbonyl}-2,2-dimethylpropyl]-3-hydroxyquinoxaline-2-carboxamide,
   N-[(1S)-1-{[4-(2,4-difluorophenoxy)piperidin-1-yl]carbonyl}-2,2-dimethylpropyl]-3-hydroxyquinoxaline-2-carboxamide,
   N-[(1S)-1-{[4-(3,4-difluorophenoxy)piperidin-1-yl]carbonyl}-2,2-dimethylpropyl]-3-hydroxyquinoxaline-2-carboxamide,
   N-[(1S)-2,2-dimethyl-1-({4-[(5-fluoropyridin-2-yl)oxy]piperidin-1-yl}carbonyl)propyl]-3-hydroxyquinoxaline-2-carboxamide,
   N-[(1S)-1-({4-[(5-chloropyridin-2-yl)oxy]piperidin-1-yl}carbonyl)-2,2-dimethylpropyl]-3-hydroxyquinoxaline-2-carboxamide, and
   N-{(1S)-2,2-dimethyl-1-[(4-{[5-(trifluoromethyl)pyridin-2-yl]oxy}piperidin-1-yl)carbonyl]propyl}-3-hydroxyquinoxaline-2-carboxamide,
(28) a compound or a pharmacologically acceptable salt thereof, described in (1) above, which is selected from the following:
   N-{(1S)-2-[4-(4-chlorophenoxy)piperidin-1-yl]-1-methyl-2-oxoethyl}-3-hydroxyquinoxaline-2-carboxamide,
   N-{(1S)-2-[4-(2,4-difluorophenoxy)piperidin-1-yl]-1-methyl-2-oxoethyl}-3-hydroxyquinoxaline-2-carboxamide,
   N-{(1S)-2-[4-(3,4-difluorophenoxy)piperidin-1-yl]-1-methyl-2-oxoethyl}-3-hydroxyquinoxaline-2-carboxamide,
   N-[(1S)-2-{4-[(5-chloropyridin-2-yl)oxy]piperidin-1-yl}-1-methyl-2-oxoethyl]-3-hydroxyquinoxaline-2-carboxamide,
   N-[(1S)-2-{4-[(5-fluoropyridin-2-yl)oxy]piperidin-1-yl}-1-methyl-2-oxoethyl]-3-hydroxyquinoxaline-2-carboxamide,
   3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
   N-[(1S)-1-{[4-(4-chlorophenoxy)piperidin-1-yl]carbonyl}propyl]-3-hydroxyquinoxaline-2-carboxamide,
   N-[(1S)-1-{[4-(4-fluorophenoxy)piperidin-1-yl]carbonyl}propyl]-3-hydroxyquinoxaline-2-carboxamide,
   N-[(1S)-1-({4-[(5-fluoropyridin-2-yl)oxy]piperidin-1-yl}carbonyl)propyl]-3-hydroxyquinoxaline-2-carboxamide,
   N-[(1S)-1-({4-[(5-chloropyridin-2-yl)oxy]piperidin-1-yl}carbonyl)propyl]-3-hydroxyquinoxaline-2-carboxamide,
   3-hydroxy-N-{(1S)-1-[(4-{[5-(trifluoromethyl)pyridin-2-yl]oxy}piperidin-1-yl)carbonyl]propyl}quinoxaline-2-carboxamide,
   N-[(1S)-1-{[4-(4-fluorophenoxy)piperidin-1-yl]carbonyl}-2-methylpropyl]-3-hydroxyquinoxaline-2-carboxamide,
   3-hydroxy-N-[(1S)-2-methyl-1-({4-[4-(trifluoromethoxy)phenoxy]piperidin-1-yl}carbonyl)propyl]quinoxaline-2-carboxamide,
   N-[(1S)-1-({4-[(5-fluoropyridin-2-yl)oxy]piperidin-1-yl}carbonyl)-2-methylpropyl]-3-hydroxyquinoxaline-2-carboxamide,
   N-[(1S)-1-({4-[(5-chloropyridin-2-yl)oxy]piperidin-1-yl}carbonyl)-2-methylpropyl]-3-hydroxyquinoxaline-2-carboxamide,
   3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide, and
   N-{(1S)-1-cyclopropyl-2-[4-(4-fluorophenoxy)piperidin-1-yl]-2-oxoethyl}-3-hydroxyquinoxaline-2-carboxamide.
(29) a pharmaceutical composition comprising as an active ingredient a compound or a pharmacologically acceptable salt thereof, selected from those described in any one of (1) to (28) above,
(30) a pharmaceutical composition for preventing and/or treating a blood coagulation disorder, comprising as an active ingredient a compound or a pharmacologically acceptable salt thereof, selected from those described in any one of (1) to (28) above,
(31) a pharmaceutical composition for preventing and/or treating thromboembolism, congenital anticoagulant factor deficiency or plasminogen abnormality, comprising as an active ingredient a compound or a pharmacologically acceptable salt thereof, selected from those described in any one of (1) to (28) above, and,
(32) a pharmaceutical composition for preventing thrombogenesis following artificial valve replacement surgery, or thrombogenesis caused by nonvalvular atrial fibrillation or atrial fibrillation accompanying valvular heart disease, comprising as an active ingredient a compound or a pharmacologically acceptable salt thereof, selected from those described in any of (1) to (28) above.

In addition, the present invention provides a method for preventing and/or treating blood coagulation disorders, comprising administering an effective amount of a compound or a pharmacologically acceptable salt thereof, selected from those described in any one of (1) to (28) above to a warm-blooded animal (and preferably a human) (such as a method for preventing and/or treating thromboembolism, congenital anticoagulant factor deficiency or plasminogen abnormality, or a method for preventing thrombogenesis following artificial valve replacement surgery or thrombogenesis caused by nonvalvular atrial fibrillation or atrial fibrillation accompanying valvular heart disease).

In the aforementioned general formula (i), a "halogen atom" in the definitions of R¹, R², R⁴, R⁵, α and β refers to a fluorine atom, a chlorine atom, a bromine atom or an iodine atom. It is preferably a fluorine atom or a chlorine atom, and more preferably a fluorine atom.

A "C₁₋₄ alkyl group" in the definitions of R¹, R², R³, R⁴, R⁵, R⁶, R⁷, Ra, Rb, Rc and α refers to a linear or branched alkyl group having 1 to 4 carbon atoms, for example, methyl, ethyl, propyl, isopropyl, n-butyl, isobutyl, s-butyl or t-butyl.

A "halogeno C₁₋₄ alkyl group" in the definitions of Ra, Rb and Rc refers to a group in which one or two or more hydrogen atoms of the aforementioned "C₁₋₄ alkyl group" are substituted by the aforementioned "halogen atom(s)". Examples are fluoromethyl, difluoromethyl, trifluoromethyl and 2,2,2-trifluoroethyl, and preferably trifluoromethyl.

A "hydroxy C₁₋₄ alkyl group" in the definitions of R⁴ and R⁵ refers to a group in which one or two or more hydrogen atoms of the aforementioned "C₁₋₄ alkyl group" are substituted by hydroxy group(s). Examples are hydroxymethyl, 1-hydroxyethyl, 2-hydroxyethyl, 1-hydroxy-1-methylethyl, 1-hydroxy-2-methylpropyl and 3-hydroxy-butyl. It is preferably 1-hydroxy-1-methylethyl.

A "C₂₋₄ alkenyl group" in the definitions of R⁴, R⁵ and α refers to a linear or branched alkenyl group having 2 to 4 carbon atoms, for example, vinyl, 1-propenyl, 2-propenyl, isopropenyl, 1-methyl-2-propenyl, 2-methyl-2-propenyl, 2-butenyl, 3-butenyl or 1,3-butadienyl. It is preferably an alkenyl group having 2 or 3 carbon atoms.

A "C₂₋₄ alkynyl group" in the definitions of R⁴ and R⁵ refers to a linear or branched alkynyl group having 2 to 4 carbon atoms, for example, an ethynyl, 2-propynyl, 1-methyl-2-propynyl, 2-methyl-2-propynyl, 2-butynyl or 3-butynyl group. It is preferably an alkynyl group having 2 or 3 carbon atoms.

A "C₃₋₅ cycloalkyl group" in the definitions of R⁴ and R⁵ refers to a cycloalkyl group having 3 to 5 carbon atoms, for example, cyclopropyl, cyclobutyl or cyclopentyl.

A "C₃₋₆ cycloalkyl group" in the definition of α refers to a cycloalkyl group having 3 to 6 carbon atoms, for example, the aforementioned "C₃₋₅ cycloalkyl group" or cyclohexyl.

A "monocyclic or bicyclic C₃₋₁₀ cycloalkyl group" in the definition of X refers to a monocyclic or bicyclic saturated cyclic hydrocarbon group having 3 to 10 carbon atoms, for example, the aforementioned "C₃₋₆ cycloalkyl group" or cyclooctyl, cyclononyl or tetrahydronaphthyl.

A "monocyclic or bicyclic C₆₋₁₀ aryl group" in the definition of X refers to an aromatic hydrocarbon group having 6 to 10 carbon atoms, for example, phenyl or naphthyl, and preferably phenyl.

A "5- or 6-membered heterocyclic group" in the definition of α includes 5- or 6-membered monocyclic aromatic heterocycles and non-aromatic heterocycles, and the heterocyclic group contains 1 to 3 atoms selected from the group consisting of a nitrogen atom, a sulfur atom and an oxygen atom.

Aromatic heterocyclic groups which are a "5- or 6-membered heterocyclic group" include monocyclic aromatic heterocyclic groups containing 1 to 3 atoms selected from the group consisting of a nitrogen atom, a sulfur atom and an oxygen atom such as 5-membered ring groups such as pyrrolyl, thienyl, furyl, pyrazolyl, imidazolyl, oxazolyl, isoxazolyl, triazolyl, tetrazolyl or thiadiazolyl, and 6-membered ring groups such as pyranyl, pyridyl, pyridazinyl, pyrimidinyl or pyrazinyl group. Preferred examples include 2-pyridyl, 3-pyridyl, 3-pyridazinyl, 2-pyrimidinyl and 5-pyrimidinyl, while more preferred examples include 2-pyridyl, 3-pyridyl, 3-pyridazinyl and 2-pyrimidinyl.

Among "5- or 6-membered heterocyclic groups", a non-aromatic heterocyclic group refers to a group in which 1 to 3 carbon atoms of a monocyclic cycloalkyl group or cycloalkenyl group having 5 or 6 carbon atoms are substituted with an atom selected from the group consisting of a nitrogen atom, a sulfur atom and an oxygen atom.

Non-aromatic heterocyclic groups which are of "5- or 6-membered heterocyclic groups" include monocyclic non-aromatic heterocyclic groups like a 5-membered ring group such as pyrrolidinyl, pyrrolinyl, imidazolidinyl, imidazolinyl, pyrazolidinyl, pyrazolinyl, oxazolidinyl or thiazolidinyl; and a 6-membered ring group such as piperidyl, tetrahydropyridyl, dihydropyridyl, piperazinyl, morpholinyl or thiomorpholinyl.

A "monocyclic or bicyclic 5- to 10-membered heterocyclic group" in the definition of X includes 5- to 10-membered monocyclic or bicyclic aromatic heterocycles and non-aromatic heterocycles, and the heterocyclic group contains 1 to 3 atoms selected from the group consisting of a nitrogen atom, a sulfur atom and an oxygen atom.

5- to 10-membered monocyclic or bicyclic aromatic heterocycles include, for example, aromatic heterocyclic groups in the aforementioned "5- or 6-membered heterocyclic groups", and bicyclic aromatic heterocyclic groups containing 1 to 3 atoms selected from the group consisting of a nitrogen atom, a sulfur atom and an oxygen atom, such as indolyl, isoindolyl, benzothiophenyl, isobenzothiophenyl, benzofuranyl, isobenzofuranyl, indazolyl, benzimidazolyl, imidazopyridyl, benzoxazolyl, benzothiazolyl, quinolyl or isoquinolyl. Preferred examples include pyridyl, pyridazinyl, pyrimidinyl, pyrazinyl, quinolyl, isoquinolyl, benzimidazolyl, benzoxazolyl and benzothiazolyl, more preferred examples include pyridyl, pyridazinyl, pyrimidinyl, quinolyl and isoquinolyl, even more preferred examples include 2-pyridyl, 3-pyridyl, 3-pyridazinyl, 2-pyrimidinyl and 5-pyrimidinyl, with 2-pyridyl being even more preferred.

A 5- to 10-membered monocyclic or bicyclic non-aromatic heterocyclic group refers to a group in which 1 to 3 carbon atoms of a monocyclic cycloalkyl or cycloalkenyl group having 5 to 10 carbon atoms are substituted with an atom selected from the group consisting of a nitrogen atom, a sulfur atom and and an oxygen atom, or a group in which 1 to 3 carbon atoms of a bicycloalkyl or bicycloalkenyl group having 7 to 10 carbon atoms are substituted with an atom selected from the group consisting of a nitrogen atom, a sulfur atom or an oxygen atom. Examples include a non-aromatic heterocyclic group among the aforementioned "5- or 6-membered heterocyclic groups", and bicyclic non-aromatic heterocyclic groups such as indolinyl, isoindolinyl, thiochromanyl group or chromanyl.

A "C₁₋₄ alkoxy group" in the definitions of R¹, R², α and β refers to a group in which an oxygen atom is bonded to the aforementioned "C₁₋₄ alkyl group". Examples include methoxy, ethoxy, propoxy, isopropoxy, butoxy, isobutoxy and t-butoxy, while preferred examples include methoxy, ethoxy, propoxy and isopropoxy, with methoxy being more preferred.

A "C₃₋₁₀ cycloalkoxy group" in the definition of α refers to a group in which an oxygen atom is bonded to the aforementioned "C₃₋₆ cycloalkyl group". Examples include cyclopropoxy, cyclobutoxy, cyclopentoxy and cyclohexyloxy.

A "C₁₋₄ alkanoyl group" in the definition of Ra refers to, for example, formyl, acetyl, propionyl, butyryl or isobutyryl.

A "C₁₋₄ alkanoyloxy group" in the definition of β refers to a group in which an oxygen atom is bonded to the aforementioned "C₁₋₄ alkanoyl group". Examples include formyloxy, acetyloxy, propionyloxy, butyryloxy and isobutyryloxy.

R¹ and R² of compound (i) of the present invention are each independently, preferably a hydrogen atom or a halogen atom, more preferably a hydrogen atom or a fluorine atom, and even more preferably both R¹ and R² are hydrogen atoms.

The substitution positions of R¹ and R² are preferably the 6 or 7 positions, and are more preferably the 7 position.

R³ of compound (i) of the present invention is preferably a hydrogen atom or a methyl group and more preferably a hydrogen atom.

R⁴ and R⁵ in compound (i) of the present invention are preferably such that R⁴ is a hydrogen atom, a fluorine atom, a C₁₋₄ alkyl group, a C₂₋₄ alkenyl group, a C₂₋₄ alkynyl group, a C₃₋₅ cycloalkyl group or a hydroxy C₁₋₄ alkyl group and R⁵ is a hydrogen atom. More preferably, R⁴ is a methyl group, an ethyl group, an isopropyl group, an isobutyl group, a t-butyl group, a cyclopropyl group or a 1-hydroxy-1-methylethyl group and R⁵ is a hydrogen atom. Even more preferably, R⁴ is a methyl group, an ethyl group, an isopropyl group, a t-butyl group, a cyclopropyl group or a 1-hydroxy-1-methylethyl group and R⁵ is a hydrogen atom. Still more preferably, R⁴ is an ethyl group, an isopropyl group, a cyclopropyl group or a 1-hydroxy-1-methylethyl group and R⁵ is a hydrogen atom.

R⁶ and R⁷ of compound (i) of the present invention are each independently, preferably a hydrogen atom or a methyl group, and more preferably R⁶ and R⁷ are both hydrogen atoms.

The substitution positions of R⁶ and R⁷ are preferably the 3 position or the 5 position.

X of compound (i) of the present invention is preferably a phenyl group, a naphthyl group, a pyridyl group, a pyridazinyl group, a pyrimidinyl group, a pyrazinyl group, a quinolyl group, an isoquinolyl group, a benzimidazolyl group, a benzoxazolyl group or a benzothiazolyl group that may be substituted with 1 or 2 substituents selected from the substituent group α, and the substituent group α consists of a halogen atom, a cyano group, a nitro group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, an acetoxy group, a trifluoromethoxy group, an acetyl group, a methoxycarbonyl group, an ethoxycarbonyl group, a dimethylcarbamoyl group, a piperidyl group, a pyrrolidyl group, a morpholino group, a pyrazolyl group, a methylpyrazolyl group, an oxadiazolyl group, a methyloxadiazolyl group, a phenyl group and a pyridyl group.

More preferably, X is a phenyl group, a naphthyl group, a pyridyl group, a pyridazinyl group, a pyrimidinyl group, a pyrazinyl group, a quinolyl group or an isoquinolyl group that may be substituted with 1 or 2 substituents selected from the substituent group α, and the substituent group α consists of a fluorine atom, a chlorine atom, a bromine atom, a cyano group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, a trifluoromethoxy group, a piperidyl group, a pyrrolidyl group, a phenyl group and a pyridyl group.

Even more preferably, X is a phenyl group, a naphthyl group, a pyridyl group, a pyridazinyl group, a pyrimidinyl group, a pyrazinyl group, a quinolyl group or an isoquinolyl group that may be substituted with 1 or 2 substituents selected from the substituent group α, and the substituent group α consists of a fluorine atom, a chlorine atom, a bromine atom, a cyano group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, a trifluoromethoxy group, a phenyl group and a pyridyl group.

Still more preferably, X is a phenyl group, a 2-pyridyl group, a 3-pyridyl group, a 3-pyridazinyl group, a 2-pyrimidinyl group or a 5-pyrimidinyl group that may be substituted with 1 or 2 substituents selected from the substituent group α, and the substituent group α consists of a fluorine atom, a chlorine atom, a bromine atom, a cyano group, a hydroxy group, a methyl group, an ethyl group, a t-butyl group, a trifluoromethyl group, a methoxy group, an ethoxy group, a trifluoromethoxy group, a phenyl group and a pyridyl group.

Even more preferably, X is a phenyl group or a pyridyl group that may be substituted with 1 or 2 substituents selected from the substituent group α, and the substituent group α consists of a fluorine atom, a chlorine atom, a cyano group, a trifluoromethyl group, a methoxy group and a trifluoromethoxy group.

Even more preferably, X is a phenyl group or a 2-pyridyl group that may be substituted with 1 or 2 substituents selected from the substituent group α, and the substituent group α consists of a fluorine atom, a chlorine atom, a trifluoromethyl group,a methoxy group and a trifluoromethoxy group.

Preferable specific examples of X include 2-fluorophenyl, 4-fluorophenyl, 2,4-difluorophenyl, 3,4-difluorophenyl, 2-chlorophenyl, 4-chlorophenyl, 2,4-dichlorophenyl, 3,4-dichlorophenyl, 2-bromophenyl, 4-bromophenyl, 2-chloro-4-fluorophenyl, 4-chloro-2-fluorophenyl, 2-bromo-4-fluorophenyl, 4-bromo-2-fluorophenyl, 2-cyanophenyl, 4-cyanophenyl, 2-cyano-4-fluorophenyl, 4-cyano-2-fluorophenyl, 2-hydroxyphenyl, 4-hydroxyphenyl, 2-methylphenyl, 4-methylphenyl, 2-trifluoromethylphenyl, 4-trifluoromethylphenyl, 2-methoxyphenyl, 4-methoxyphenyl, 2-trifluoromethoxyphenyl, 4-trifluoromethoxyphenyl, 2-biphenyl, 4-biphenyl, 5-fluoropyridin-2-yl, 6-fluoropyridin-3-yl, 5-chloropyridin-2-yl, 6-chloropyridin-3-yl, 6-methoxypyridin-3-yl, 5-cyanopyridin-2-yl, 5-trifluoromethylpyridin-2-yl, 5-trifluoromethoxypyridin-2-yl, 3,5-difluoropyridin-2-yl, 6-methoxypyridazin-3-yl, 6-isopropoxypyridazin-3-yl, 5-fluoropyrimidin-2-yl, 5-chloropyrimidin-2-yl, 5-bromopyrimidin-2-yl, 5-trifluoromethylpyrimidin-2-yl, 5-trifluoromethoxypyrimidin-2-yl or 5-phenylpyrimidin-2-yl.

More preferable specific examples of X include 4-fluorophenyl, 2,4-difluorophenyl, 3,4-difluorophenyl, 4-chlorophenyl, 2,4-dichlorophenyl, 4-trifluoromethylphenyl, 4-trifluoromethoxyphenyl, 5-fluoropyridin-2-yl, 5-chloropyridin-2-yl, 5-trifluoromethylpyridin-2-yl or 6-methoxypyridin-3-yl.

Y of compound (i) of the present invention is preferably -CO-, - O- or -NRa-, and Ra is preferably a hydrogen atom, a methyl group or an ethyl group. In the case where Ra in Y is an ethyl group, Ra may also preferably form a 5-membered ring by bonding with atoms that are components of X. More preferably, Y is -O- or -NRa-, and Ra is a hydrogen atom,a methyl group or an ethyl group. Y is even more preferably -O-.

Since compound (i) of the present invention is able to be in the form of a salt by reacting with acid in the case of having a basic group such as an amino group or by reacting with base in the case of having an acidic group such as a sulfonamido group, a "pharmacologically acceptable salt thereof" refers to that salt.

Examples of salts based on basic groups include inorganic acid salts such as hydrogen halides such as hydrofluorides, hydrochlorides, hydrobromides or hydroiodides; nitrates, perchlorates, sulfates or phosphates; organic acid salts such as lower alkanesulfonic acid salts such as methanesulfonates, trifluoromethanesulfonates or ethanesulfonates; arylsulfonic acid salts such as benzenesulfonates or p-toluenesulfonates; acetates, malates, fumarates, succinates, citrates, ascorbates, tartrates, oxalates or maleates; and amino acid salts such as glycine salts, lysine salts, arginine salts, ornithine salts, glutamates or aspartates.

On the other hand, examples of salts based on acidic groups include metal salts such as alkali metal salts such as sodium salts, potassium salts or lithium salts, alkaline earth metal salts such as calcium salts or magnesium salts, aluminum salts or iron salts; inorganic salts such as ammonium salts, amine salts such as organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzylphenethylamine salts, piperazine salts, tetramethylammonium salts or tris(hydroxymethyl)aminomethane salts; and amino acid salts such as glycine salts, lysine salts, arginine salts, ornithine salts, glutamates or aspartates.

A compound represented by general formula (i) of the present invention or a pharmacologically acceptable salt thereof may absorb moisture and form a hydrate as a result of allowing it to stand in air, and such hydrates are also included in the present invention.

A compound represented by general formula (i) of the present invention or a pharmacologically acceptable salt thereof may form a solvate as a result of allowing it to stand in a solvent, and such solvates are also included in the present invention.

A compound represented by general formula (i) of the present invention or a pharmacologically acceptable salt thereof has optical isomers based on an asymmetric center in a molecule thereof. In compounds of the present invention, these isomers and mixtures of these isomers are all represented by a single formula, namely general formula (i). Thus, the present invention includes all of these isomers and mixtures of these isomers.

Although examples of typical compounds represented by general formula (i) of the present invention include the compounds listed below, the present invention is not limited to these compounds.

Examples of these compounds include:
N-{(1S)-2-[4-(2-fluorophenoxy)piperidin-1-yl]-1-methyl-2-oxoethyl}-3-hydroxyquinoxaline-2-carboxamide,
N-{(1S)-2-[4-(3-fluorophenoxy)piperidin-1-yl]-1-methyl-2-oxoethyl}-3-hydroxyquinoxaline-2-carboxamide,
N-{(1S)-2-[4-(4-fluorophenoxy)piperidin-1-yl]-1-methyl-2-oxoethyl}-3-hydroxyquinoxaline-2-carboxamide,
N-{(1S)-2-[4-(4-chlorophenoxy)piperidin-1-yl]-1-methyl-2-oxoethyl}-3-hydroxyquinoxaline-2-carboxamide,
3-hydroxy-N-[(1S)-1-methyl-2-oxo-2-{4-[4-(trifluoromethoxy)phenoxylpiperidin-1-yllethyllquinoxaline-2-carboxamide,
N-{(1S)-2-[4-(4-cyanophenoxy)piperidin-1-yl]-1-methyl-2-oxoethyl}-3-hydroxyquinoxaline-2-carboxamide,
N-{(1S)-2-[4-(2,4-difluorophenoxy)piperidin-1-yl]-1-methyl-2-oxoethyl}-3-hydroxyquinoxaline-2-carboxamide,
N-{(1S)-2-[4-(3,4-difluorophenoxy)piperidin-1-yl]-1-methyl-2-oxoethyl}-3-hydroxyquinoxaline-2-carboxamide,
N-[(1S)-2-{4-[(5-chloropyridin-2-yl)oxy]piperidin-1-yl}-1-methyl-2-oxoethyl]-3-hydroxyquinoxaline-2-carboxamide,
N-[(1S)-2-{4-[(5-fluoropyridin-2-yl)oxy]piperidin-1-yl}-1-methyl-2-oxoethyl]-3-hydroxyquinoxaline-2-carboxamide,
3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
N-[(1S)-1-{[4-(2-fluorophenoxy)piperidin-1-yl]carbonyl}propyl]-3-hydroxyquinoxaline-2-carboxamide,
N-[(1S)-1-{[4-(3-fluorophenoxy)piperidin-1-yl]carbonyl}propyl]-3-hydroxyquinoxaline-2-carboxamide,
N-[(1S)-1-{[4-(4-fluorophenoxy)piperidin-1-yl]carbonyl}propyl]-3-hydroxyquinoxaline-2-carboxamide,
N-[(1S)-1-{[4-(4-chlorophenoxy)piperidin-1-yl]carbonyl}propyl]-3-hydroxyquinoxaline-2-carboxamide,
3-hydroxy-N-[(1S)-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}propyl]-3-hydroxyquinoxaline-2-carboxamide,
N-[(1S)-1-{[4-(2,4-difluorophenoxy)piperidin-1-yl]carbonyl}propyl]-3-hydroxyquinoxaline-2-carboxamide,
N-[(1S)-1-{[4-(3,4-difluorophenoxy)piperidin-1-yl]carbonyl}propyl]-3-hydroxyquinoxaline-2-carboxamide,
N-[(1S)-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)propyl]-3-hydroxyquinoxaline-2-carboxamide,
3-hydroxy-N-{(1S)-1-[(4-{[5-(trifluoromethyl)pyridin-2-yl]oxy}piperidin-1-yl)carbonyl]propyl}quinoxaline-2-carboxamide,
N-[(1S)-1-{[4-(2-fluorophenoxy)piperidin-1-yl]carbonyl}-2-methylpropyl]-3-hydroxyquinoxaline-2-carboxamide,
N-[(1S)-1-{[4-(3-fluorophenoxy)piperidin-1-yl]carbonyl}-2-methylpropyl]-3-hydroxyquinoxaline-2-carboxamide,
N-[(1S)-1-{[4-(4-fluorophenoxy)piperidin-1-yl]carbonyl}-2-methylpropyl]-3-hydroxyquinoxaline-2-carboxamide,
N-[(1S)-1-{[4-(4-chlorophenoxy)piperidin-1-yl]carbonyl}-2-methylpropyl]-3-hydroxyquinoxaline-2-carboxamide,
3-hydroxy-N-[(1S)-2-methyl-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}-2-methylpropyl]-3-hydroxyquinoxaline-2-carboxamide,
N-[(1S)-1-{[4-(2,4-difluorophenoxy)piperidin-1-yl]carbonyl}-2-methylpropyl]-3-hydroxyquinoxaline-2-carboxamide,
N-[(1S)-1-{[4-(3,4-difluorophenoxy)piperidin-1-yl]carbonyl}-2-methylpropyl]-3-hydroxyquinoxaline-2-carboxamide,
N-[(1S)-1-({4-[(5-chloropyridin-2-yl)oxy]piperidin-1-yl}carbonyl)-2-methylpropyl]-3-hydroxyquinoxaline-2-carboxamide,
N-[(1S)-1-({4-[(5-fluoropyridin-2-yl)oxy]piperidin-1-yl}carbonyl)-2-methylpropyl]-3-hydroxyquinoxaline-2-carboxamide,
3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide,
3-hydroxy-N-[(1S)-1-({4-[(6-methoxypyridin-3-yl)oxy]piperidin-1-yl}carbonyl)-2-methylpropyl]quinoxaline-2-carboxamide,
N-[(1S)-1-{[4-(2,4-difluorobenzoyl)piperidin-1-yl]carbonyl}-2-methylpropyl]-3-hydroxyquinoxaline-2-carboxamide,
N-[(1S)-1-({4-[(2,4-difluorophenyl)(hydroxy)methyl]piperidin-1-yl}carbonyl)-2-methylpropyl]-3-hydroxyquinoxaline-2-carboxamide,
N-[(1S)-1-{[4-(4-fluorophenoxy)piperidin-1-yl]carbonyl}-2-hydroxy-2-methylpropyl]-3-hydroxyquinoxaline-2-carboxamide,
N-{(1S)-1-cyclopropyl-2-[4-(2-fluorophenoxy)piperidin-1-yl]-2-oxoethyl}-3-hydroxyquinoxaline-2-carboxamide,
N-{(1S)-1-cyclopropyl-2-[4-(3-fluorophenoxy)piperidin-1-yl]-2-oxoethyl}-3-hydroxyquinoxaline-2-carboxamide,
N-{(1S)-1-cyclopropyl-2-[4-(4-fluorophenoxy)piperidin-1-yl]-2-oxoethyl}-3-hydroxyquinoxaline-2-carboxamide,
N-{(1S)-2-[4-(4-chlorophenoxy)piperidin-1-yl]-1-cyclopropyl-2-oxoethyl}-3-hydroxyquinoxaline-2-carboxamide,
N-[(1S)-1-cyclopropyl-2-oxo-2-{4-[4-(trifluoromethoxy)phenoxy]piperidin-1-yl}ethyl]-3-hydroxyquinoxaline-2-carboxamide,
N-{(1S)-2-[4-(4-cyanophenoxy)piperidin-1-yl]-1-cyclopropyl-2-oxoethyl}-3-hydroxyquinoxaline-2-carboxamide,
N-{(1S)-1-cyclopropyl-2-[4-(2,4-difluorophenoxy)piperidin-1-yl]-2-oxoethyl}-3-hydroxyquinoxaline-2-carboxamide,
N-{(1S)-1-cyclopropyl-2-[4-(3,4-difluorophenoxy)piperidin-1-yl]-2-oxoethyl}-3-hydroxyquinoxaline-2-carboxamide,
N-[(1S)-1-cyclopropyl-2-{4-[(5-fluoropyridin-2-yl)oxy]piperidin-1-yl}-2-oxoethyl]-3-hydroxyquinoxaline-2-carboxamide,
N-[(1S)-2-{4-[(5-chloropyridin-2-yl)oxy]piperidin-1-yl}-1-cyclopropyl-2-oxoethyl]-3-hydroxyquinoxaline-2-carboxamide,
N-[(1S)-1-cyclopropyl-2-oxo-2-(4-{[5-(trifluoromethyl)pyridin-2-yl]oxy}piperidin-1-yl)ethyl]-3-hydroxyquinoxaline-2-carboxamide,
N-[(1S)-2,2-dimethyl-1-{[4-(2-fluorophenoxy)piperidin-1-yl]carbonyl}propyl]-3-hydroxyquinoxaline-2-carboxamide,
N-[(1S)-2,2-dimethyl-1-{[4-(3-fluorophenoxy)piperidin-1-yl]carbonyl}propyl]-3-hydroxyquinoxaline-2-carboxamide,
N-[(1S)-2,2-dimethyl-1-{[4-(4-fluorophenoxy)piperidin-1-yl]carbonyl}propyl]-3-hydroxyquinoxaline-2-carboxamide,
N-[(1S)-1-{[4-(4-chlorophenoxy)piperidin-1-yl]carbonyl}-2,2-dimethylpropyl]-3-hydroxyquinoxaline-2-carboxamide,
N-[(1S)-2,2-dimethyl-1-({4-[4-(trifluoromethoxy)phenoxy]piperidin-1-yl}carbonyl)propyl]-3-hydroxyquinoxaline-2-carboxamide,
N-[(1S)-1-{[4-(4-cyanophenoxy)piperidin-1-yl]carbonyl}-2,2-dimethylpropyl]-3-hydroxyquinoxaline-2-carboxamide,
N-[(1S)-1-{[4-(2,4-difluorophenoxy)piperidin-1-yl]carbonyl}-2,2-dimethylpropyl]-3-hydroxyquinoxaline-2-carboxamide,
N-[(1S)-1-{[4-(3,4-difluorophenoxy)piperidin-1-yl]carbonyl}-2,2-dimethylpropyl]-3-hydroxyquinoxaline-2-carboxamide,
N-[(1S)-2,2-dimethyl-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)-2,2-dimethylpropyl]-3-hydroxyquinoxaline-2-carboxamide, and
N-{(1S)-2,2-dimethyl-1-[(4-{[5-(trifluoromethyl)pyridin-2-yl]oxy}piperidin-1-yl)carbonyl]propyl}-3-hydroxyquinoxaline-2-carboxamide,

Among these compounds, preferred are:
N-{(1S)-2-[4-(2-fluorophenoxy)piperidin-1-yl]-1-methyl-2-oxoethyl}-3-hydroxyquinoxaline-2-carboxamide,
N-{(1S)-2-[4-(3-fluorophenoxy)piperidin-1-yl]-1-methyl-2-oxoethyl}-3-hydroxyquinoxaline-2-carboxamide,
N-{(1S)-2-[4-(4-fluorophenoxy)piperidin-1-yl]-1-methyl-2-oxoethyl}-3-hydroxyquinoxaline-2-carboxamide,
N-{(1S)-2-[4-(4-chlorophenoxy)piperidin-1-yl]-1-methyl-2-oxoethyl}-3-hydroxyquinoxaline-2-carboxamide,
3-hydroxy-N-[(1S)-1-methyl-2-oxo-2-{4-[4-(trifluoromethoxy)phenoxy]piperidin-1-yl}ethyl]quinoxaline-2-carboxamide,
N-{(1S)-2-[4-(4-cyanophenoxy)piperidin-1-yl]-1-methyl-2-oxoethyl}-3-hydroxyquinoxaline-2-carboxamide,
N-{(1S)-2-[4-(2,4-difluorophenoxy)piperidin-1-yl]-1-methyl-2-oxoethyl}-3-hydroxyquinoxaline-2-carboxamide,
N-{(1S)-2-[4-(3,4-difluorophenoxy)piperidin-1-yl]-1-methyl-2-oxoethyl}-3-hydroxyquinoxaline-2-carboxamide,
N-[(1S)-2-{4-[(5-chloropyridin-2-yl)oxy]piperidin-1-yl}-1-methyl-2-oxoethyl]-3-hydroxyquinoxaline-2-carboxamide,
N-[(1S)-2-{4-[(5-fluoropyridin-2-yl)oxy]piperidin-1-yl}-1-methyl-2-oxoethyl]-3-hydroxyquinoxaline-2-carboxamide,
3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
N-[(1S)-1-{[4-(2-fluorophenoxy)piperidin-1-yl]carbonyl}propyl]-3-hydroxyquinoxaline-2-carboxamide,
N-[(1S)-1-{[4-(3-fluorophenoxy)piperidin-1-yl]carbonyl}propyl]-3-hydroxyquinoxaline-2-carboxamide,
N-[(1S)-1-{[4-(4-fluorophenoxy)piperidin-1-yl]carbonyl}propyl]-3-hydroxyquinoxaline-2-carboxamide,
N-[(1S)-1-{[4-(4-chlorophenoxy)piperidin-1-yl]carbonyl}propyl]-3-hydroxyquinoxaline-2-carboxamide,
3-hydroxy-N-[(1S)-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}propyl]-3-hydroxyquinoxaline-2-carboxamide,
N-[(1S)-1-{[4-(2,4-difluorophenoxy)piperidin-1-yl]carbonyl}propyl]-3-hydroxyquinoxaline-2-carboxamide,
N-[(1S)-1-{[4-(3,4-difluorophenoxy)piperidin-1-yl]carbonyl}propyl]-3-hydroxyquinoxaline-2-carboxamide,
N-[(1S)-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)propyl]-3-hydroxyquinoxaline-2-carboxamide,
3-hydroxy-N-{(1S)-1-[(4-{[5-(trifluoromethyl)pyridin-2-yl]oxy}piperidin-1-yl)carbonyl]propyl}quinoxaline-2-carboxamide,
N-[(1S)-1-{[4-(2-fluorophenoxy)piperidin-1-yl]carbonyl}-2-methylpropyl]-3-hydroxyquinoxaline-2-carboxamide,
N-[(1S)-1-{[4-(3-fluorophenoxy)piperidin-1-yl]carbonyl}-2-methylpropyl]-3-hydroxyquinoxaline-2-carboxamide,
N-[(1S)-1-{[4-(4-fluorophenoxy)piperidin-1-yl]carbonyl}-2-methylpropyl]-3-hydroxyquinoxaline-2-carboxamide,
N-[(1S)-1-{[4-(4-chlorophenoxy)piperidin-1-yl]carbonyl}-2-methylpropyl]-3-hydroxyquinoxaline-2-carboxamide,
3-hydroxy-N-[(1S)-2-methyl-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}-2-methylpropyl]-3-hydroxyquinoxaline-2-carboxamide,
N-[(1S)-1-{[4-(2,4-difluorophenoxy)piperidin-1-yl]carbonyl}-2-methylpropyl]-3-hydroxyquinoxaline-2-carboxamide,
N-[(1S)-1-{[4-(3,4-difluorophenoxy)piperidin-1-yl]carbonyl}-2-methylpropyl]-3-hydroxyquinoxaline-2-carboxamide,
N-[(1S)-1-({4-[(5-chloropyridin-2-yl)oxy]piperidin-1-yl}carbonyl)-2-methylpropyl]-3-hydroxyquinoxaline-2-carboxamide,
N-[(1S)-1-({4-[(5-fluoropyridin-2-yl)oxy]piperidin-1-yl}carbonyl)-2-methylpropyl]-3-hydroxyquinoxaline-2-carboxamide,
3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide,
N-{(1S)-1-cyclopropyl-2-[4-(2-fluorophenoxy)piperidin-1-yl]-2-oxoethyl}-3-hydroxyquinoxaline-2-carboxamide,
N-{(1S)-1-cyclopropyl-2-[4-(3-fluorophenoxy)piperidin-1-yl]-2-oxoethyl}-3-hydroxyquinoxaline-2-carboxamide,
N-{(1S)-1-cyclopropyl-2-[4-(4-fluorophenoxy)piperidin-1-yl]-2-oxoethyl}-3-hydroxyquinoxaline-2-carboxamide, N-{(1S)-2-[4-(4-chlorophenoxy)piperidin-1-yl]-1-cyclopropyl-2-oxoethyl}-3-hydroxyquinoxaline-2-carboxamide, N-[(1S)-1-cyclopropyl-2-oxo-2-{4-[4-(trifluoromethoxy)phenoxy]piperidin-1-yl}ethyl]-3-hydroxyquinoxaline-2-carboxamide, N-{(1S)-2-[4-(4-cyanophenoxy)piperidin-1-yl]-1-cyclopropyl-2-oxoethyl}-3-hydroxyquinoxaline-2-carboxamide, N-{(1S)-1-cyclopropyl-2-[4-(2,4-difluorophenoxy)piperidin-1-yl]-2-oxoethyl}-3-hydroxyquinoxaline-2-carboxamide, N-{(1S)-1-cyclopropyl-2-[4-(3,4-difluorophenoxy)piperidin-1-yl]-2-oxoethyl}-3-hydroxyquinoxaline-2-carboxamide, N-[(1S)-1-cyclopropyl-2-{4-[(5-fluoropyridin-2-yl)oxy]piperidin-1-yl}-2-oxoethyl]-3-hydroxyquinoxaline-2-carboxamide, N-[(1S)-2-{4-[(5-chloropyridin-2-yl)oxy]piperidin-1-yl}-1-cyclopropyl-2-oxoethyl]-3-hydroxyquinoxaline-2-carboxamide, N-[(1S)-1-cyclopropyl-2-oxo-2-(4-{[5-(trifluoromethyl)pyridin-2-yl]oxy}piperidin-1-yl)ethyl]-3-hydroxyquinoxaline-2-carboxamide, N-[(1S)-2,2-dimethyl-1-{[4-(2-fluorophenoxy)piperidin-1-yl]carbonyl}propyl]-3-hydroxyquinoxaline-2-carboxamide, N-[(1S)-2,2-dimethyl-1-{[4-(3-fluorophenoxy)piperidin-1-yl]carbonyl}propyl]-3-hydroxyquinoxaline-2-carboxamide, N-[(1S)-2,2-dimethyl-1-{[4-(4-fluorophenoxy)piperidin-1-yl]carbonyl}propyl]-3-hydroxyquinoxaline-2-carboxamide, N-[(1S)-1-{[4-(4-chlorophenoxy)piperidin-1-yl]carbonyl}-2,2-dimethylpropyl]-3-hydroxyquinoxaline-2-carboxamide, N-[(1S)-2,2-dimethyl-1-({4-[4-(trifluoromethoxy)phenoxy]piperidin-1-yl}carbonyl)propyl]-3-hydroxyquinoxaline-2-carboxamide, N-[(1S)-1-{[4-(4-cyanophenoxy)piperidin-1-yl]carbonyl}-2,2-dimethylpropyl]-3-hydroxyquinoxaline-2-carboxamide, N-[(1S)-1-{[4-(2,4-difluorophenoxy)piperidin-1-yl]carbonyl}-2,2-dimethylpropyl]-3-hydroxyquinoxaline-2-carboxamide, N-[(1S)-1-{[4-(3,4-difluorophenoxy)piperidin-1-yl]carbonyl}-2,2-dimethylpropyl]-3-hydroxyquinoxaline-2-carboxamide, N-[(1S)-2,2-dimethyl-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)-2,2-dimethylpropyl]-3-hydroxyquinoxaline-2-carboxamide, and
N-{(1S)-2,2-dimethyl-1-[(4-{[5-(trifluoromethyl)pyridin-2-yl]oxy}piperidin-1-yl)carbonyl]propyl}-3-hydroxyquinoxaline-2-carboxamide,

Among these compounds, more preferred are:
N-{(1S)-2-[4-(4-chlorophenoxy)piperidin-1-yl]-1-methyl-2-oxoethyl}-3-hydroxyquinoxaline-2-carboxamide,
N-{(1S)-2-[4-(2,4-difluorophenoxy)piperidin-1-yl]-1-methyl-2-oxoethyl}-3-hydroxyquinoxaline-2-carboxamide,
N-{(1S)-2-[4-(3,4-difluorophenoxy)piperidin-1-yl]-1-methyl-2-oxoethyl}-3-hydroxyquinoxaline-2-carboxamide,
N-[(1S)-2-{4-[(5-chloropyridin-2-yl)oxy]piperidin-1-yl}-1-methyl-2-oxoethyl]-3-hydroxyquinoxaline-2-carboxamide,
N-[(1S)-2-{4-[(5-fluoropyridin-2-yl)oxy]piperidin-1-yl}-1-methyl-2-oxoethyl]-3-hydroxyquinoxaline-2-carboxamide,
3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
N-[(1S)-1-{[4-(4-chlorophenoxy)piperidin-1-yl]carbonyl}propyl]-3-hydroxyquinoxaline-2-carboxamide,
N-[(1S)-1-{[4-(4-fluorophenoxy)piperidin-1-yl]carbonyl}propyl]-3-hydroxyquinoxaline-2-carboxamide,
N-[(1S)-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)propyl]-3-hydroxyquinoxaline-2-carboxamide,
3-hydroxy-N-{(1S)-1-[(4-{[5-(trifluoromethyl)pyridin-2-yl]oxy}piperidin-1-yl)carbonyl]propyl}quinoxaline-2-carboxamide,
N-[(1S)-1-{[4-(4-fluorophenoxy)piperidin-1-yl]carbonyl}-2-methylpropyl]-3-hydroxyquinoxaline-2-carboxamide,
3-hydroxy-N-[(1S)-2-methyl-1-({4-[4-(trifluoromethoxy)phenoxy]piperidin-1-yl}carbonyl)propyl]quinoxaline-2-carboxamide,
N-[(1S)-1-({4-[(5-fluoropyridin-2-yl)oxy]piperidin-1-yl}carbonyl)-2-methylpropyl]-3-hydroxyquinoxaline-2-carboxamide,
N-[(1S)-1-({4-[(5-chloropyridin-2-yl)oxy]piperidin-1-yl}carbonyl)-2-methylpropyl]-3-hydroxyquinoxaline-2-carboxamide,
3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide, and
N-{(1S)-1-cyclopropyl-2-[4-(4-fluorophenoxy)piperidin-1-yl]-2-oxoethyl}-3-hydroxyquinoxaline-2-carboxamide.

### [Effect of the Invention]

A compound represented by general formula (i) of the present invention or a pharmacologically acceptable salt thereof has superior anticoagulant action. Accordingly, it is useful as an active ingredient of a preventive and/or therapeutic preparation for blood coagulation disorders (such as a preventive and/or therapeutic agent for thromboembolisms, congenital anticoagulant factor deficiencies or plasminogen abnormalities, or preventive agents for thrombogenesis following artificial valve replacement surgery, or thrombogenesis caused by nonvalvular atrial fibrillation or atrial fibrillation accompanying valvular heart disease.

### BEST MODE FOR CARRYING OUT THE INVENTION

A compound represented by general formula (i) of the present invention can be manufactured in accordance with a manufacturing method indicated in <Manufacturing Method 1> to <Manufacturing Method 5>.

### <Manufacturing Method 1>

This manufacturing method is a method for obtaining a compound of the present invention represented by general formula (i) from a compound represented by general formula (ii).

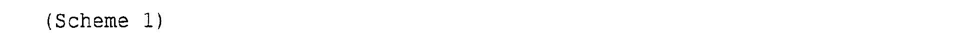

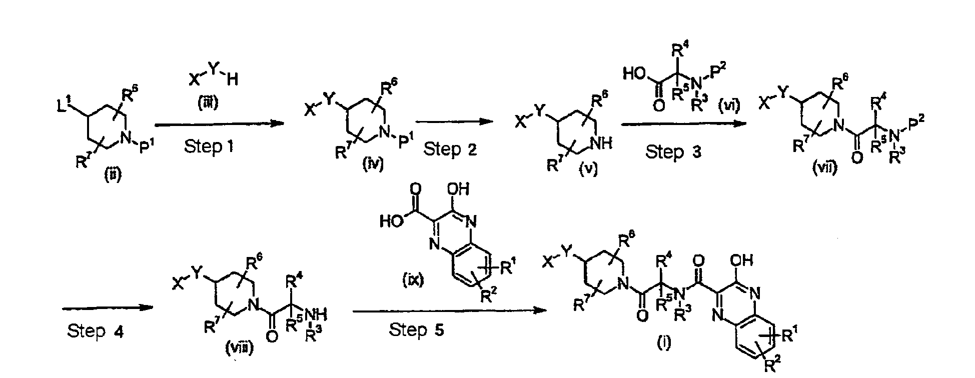

In the above formulae, R¹, R², R³, R⁴, R⁵, R⁶, R⁷, X and Y are the same as previously defined.

L¹ represents a leaving group. Examples of leaving groups (L¹) include halogen atoms such as a chloro group or a bromo group, and sulfonyloxy groups such as a methanesulfonyloxy group, a p-toluenesulfonyloxy group or a trifluoromethanesulfonyloxy group.

P¹ and P² represent protecting groups of an amino group. Examples of protecting groups (P¹ and P²) include alkoxycarbonyl groups such as a methoxycarbonyl group, an ethoxycarbonyl group, a t-butoxycarbonyl group or an allyloxycarbonyl group, an aryloxycarbonyl groups such as a benzyloxycarbonyl group or a para-methoxybenzyloxycarbonyl group; and arylmethyl groups such as a benzyl group or a triphenylmethyl group.

### Step 1

This step is a step for synthesizing a compound represented by general formula (iv) by reacting a piperidine derivative represented by general formula (ii), having a leaving group (L¹) at the 4 position, and in which the amino group is protected by a protecting group (P¹), and a compound represented by general formula (iii) in an inert solvent in the presence of a base.

Examples of inert solvents include halogen-based solvents such as methylene chloride, chloroform, dichloroethane or carbon tetrachloride, ether-based solvents such as tetrahydrofuran, 1,2-dimethoxyethane or dioxane, aromatic-based solvents such as benzene or toluene, and amide-based solvents such as N,N-dimethylformamide or N-methylpyrrolidone.

Examples of bases include organic bases such as pyridine, triethylamine, N,N-diisopropylethylamine, 4-(dimethylamino)pyridine, N-methylmorpholine, 2,6-lutidine or collidine, and carbonates, alkoxides and hydroxides of alkaline metals or alkaline earth metals such as sodium carbonate, potassium carbonate, cesium carbonate, cerium fluoride, sodium hydrogen carbonate, sodium ethoxide, potassium butoxide or sodium hydroxide.

The reaction temperature is from cooling to reflux heating (the temperature at which the solvent is heated and refluxed varies depending on the solvent), and specifically is 0 to 200°C and preferably 0 to 150°C.

The reaction time varies depending on the reaction temperature, raw material compounds, reaction reagents, solvent used in the reaction and the like. It is normally 30 minutes to 48 hours and preferably 1 to 8 hours.

A compound represented by general formula (iv) obtained in this step can be synthesized using a different method as indicated below.

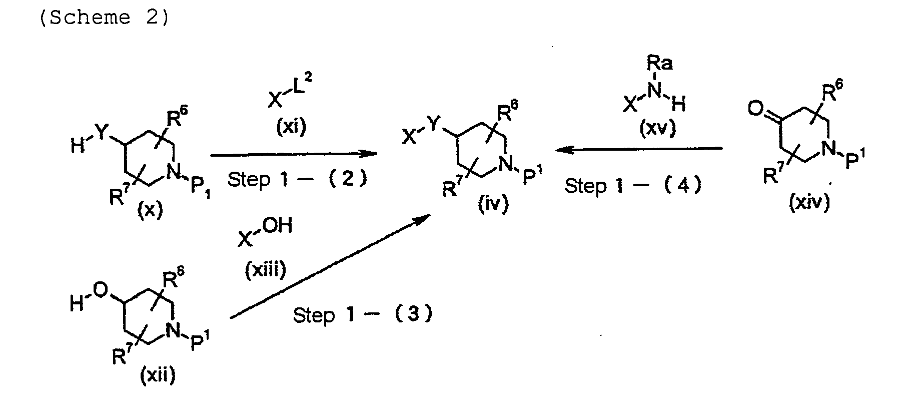

In the above formulae, R⁶, R⁷, Ra, P¹, X and Y are the same as previously defined.

L² represents a leaving group. Examples of leaving groups (L²) include halogen atoms such as a chloro group or a bromo group, and a methanesulfonyloxy group, a p-toluenesulfonyloxy group or a trifluoromethanesulfonyloxy group.

### Step 1-(2)

This step is a step for synthesizing a compound represented by the above formula (iv) from a compound represented by general formula (x) and a compound represented by general formula (xi).

This reaction can be achieved by a method that complies with the reaction conditions used in Step 1.

### Step 1-(3)

Among the compounds represented by formula (iv), a compound in which Y represents an oxygen atom can be synthesized from a compound represented by the above general formula (xii) using a Mitsunobu reaction carried out in a solvent.

Examples of solvents used in this reaction include solvents that are inert in the reaction, including aromatic-based solvents such as benzene, toluene or xylene, and ether-based solvents such as diethyl ether, tetrahydrofuran or dioxane.

Examples of reaction reagents include organic phosphorous reagents such as triphenylphosphine and diazo reagents such as diethyl azodicarboxylate, diisopropyl azodicarboxylate or dipiperidineamide azodicarboxylate, and preferably triphenylphosphine and diisopropyl azodicarboxylate are used.

The reaction temperature is from cooling to reflux heating (the temperature at which the solvent is heated and refluxed varies depending on the solvent), and specifically is 0 to 200°C and preferably 0 to 150°C.

The reaction time varies depending on the reaction temperature, raw material compounds, reaction reagents, solvent used in the reaction and the like. It is normally 30 minutes to 48 hours and preferably 1 to 8 hours.

### Step 1-(4)

Among the compounds represented by formula (iv), a compound in which Y represents a nitrogen atom can be synthesized from a 4-piperidinone derivative represented by the above formula (xiv), in which the amino group is protected with a protecting group (P¹), using a reductive amination method carried out in a solvent.

Examples of solvents used in this reaction include solvents that are inert in the reaction, including aromatic-based solvents such as benzene, toluene or xylene, ether-based solvents such as diethyl ether, tetrahydrofuran or dioxane, and alcohols such as methanol, ethanol or isopropanol.

As the reaction reagents, reducing agents such as sodium borohydride, sodium cyanoborohydride or sodium triacetoxyborohydride are used, and preferably sodium cyanoborohydride is used.

The reaction temperature is from cooling to reflux heating (the temperature at which the solvent is heated and refluxed varies depending on the solvent), and specifically is 0 to 200°C and preferably 0 to 150°C.

Although the reaction time varies depending on the reaction temperature, raw material compounds, reaction reagents, solvent used in the reaction and the like, it is normally 30 minutes to 48 hours and preferably 1 to 8 hours.

### Step 2

This step is a step for obtaining a compound represented by general formula (v), or a salt thereof, by removing a protecting group (P¹) of a compound represented by general formula (iv) by a suitable method.

The method used to remove the protecting group varies depending on the chemical properties of the protecting group used, and in the case of an acyl-type protecting group such as an alkanoyl group, alkoxycarbonyl group or aroyl group, the protecting group can be removed by hydrolysis by using a base such as an alkali metal hydroxide such as lithium hydroxide, sodium hydroxide or potassium hydroxide.

In addition, in the case of a substituted methoxycarbonyl-type protecting group such as a t-butylcarbonyl group or para-methoxybenzyloxycarbonyl group, the protecting group can be removed by a suitable acid such as acetic acid, hydrochloric acid, sulfuric acid, hydrobromic acid, phosphoric acid, trifluoroacetic acid, trifluoromethanesulfonic acid or a combination thereof.

In addition, an arylmethoxycarbonyl group such as a benzyloxycarbonyl group, a para-methoxybenzyloxycarbonyl group or a para-nitrobenzyloxycarbonyl group, and an arylmethyl group such as a benzyl group can be removed by hydrolysis using a palladium-carbon catalyst.

A triphenylmethyl group can be removed with, for example, formic acid, acetic acid, hydrochloric acid, hydrobromic acid, sulfuric acid, phosphoric acid, trifluoroacetic acid, trifluoromethanesulfonic acid or a combination thereof, and can be removed by hydrolysis using a palladium-carbon catalyst.

This step is preferably carried out in water, a suitable organic solvent or a mixed solvent thereof, and examples of suitable organic solvents used include solvents that are inert in the reaction including halogen-based solvents such as methylene chloride, chloroform, dichloroethane or carbon tetrachloride, ethers such as tetrahydrofuran, 1,2-dimethoxyethane or dioxane, and alcohols such as methanol or ethanol.

The reaction temperature is from cooling to reflux heating (the temperature at which the solvent is heated and refluxed varies depending on the solvent), and specifically is 0 to 200°C and preferably 0 to 150°C.

Although the reaction time varies depending on the reaction temperature, raw material compounds, reaction reagents, solvent used in the reaction and the like, it is normally 30 minutes to 48 hours and preferably 1 to 8 hours.

### Step 3

This step is a step for obtaining compounds represented by general formula (vii) by reacting compounds represented by general formula (v), or a salt thereof, with amino acid derivatives represented by general formula (vi).

In this reaction, after converting the amino acid derivative represented by general formula (vi) to an activating compound, the amino acid derivative is condensed with a compound represented by general formula (v). While examples of activating compounds include acid halides produced using an acid halide such as thionyl chloride, active esters obtained by reacting with a phenol such as para-nitrophenol, active amides obtained by reacting with, for example, 1,1-carbonyldiimidazole, and reaction products with 1-benzotriazolyloxy-pyrrolidino-phosphonium hexafluorophosphite or N,N-dicyclohexylcarbodiimide hydrochloride which are usually used in peptide synthesis from amino acids. Reaction products with N,N-dicyclohexylcarbodiimide hydrochloride are preferably used. In this reaction, 1-hydroxybenzotriazole and the like can also be added as necessary.

A compound represented by general formula (vii) can be obtained by reacting an activating compound of an amino acid derivative obtained in this manner and a compound represented by general formula (v), or a salt thereof, in an inert solvent, and normally in the presence of a base.

Examples of inert solvents used include solvents that are inert in the reaction, including halogen-based solvents such as methylene chloride, chloroform, dichloroethane or carbon tetrachloride, ether-based solvents such as tetrahydrofuran, 1,2-dimethoxyethane or dioxane, aromatic-based solvents such as benzene or toluene, and amide-based solvents such as N,N-dimethylformamide or N-methylpyrrolidone.

Examples of bases used include organic bases such as pyridine, triethylamine, N,N-diisopropylethylamine, 4-(dimethylamino)pyridine, N-methylmorpholine, 2,6-lutidine or collidine, and carbonates, alkoxides and hydroxides of alkaline metals or alkaline earth metals such as sodium carbonate, potassium carbonate, sodium hydrogen carbonate, sodium ethoxide, potassium butoxide, sodium hydroxide or potassium hydride. Triethylamine and N-methylmorpholine are preferably used.

The reaction temperature is from cooling to reflux heating (the temperature at which the solvent is heated and refluxed varies depending on the solvent), and specifically is 0 to 200°C and preferably 0 to 150°C.

The reaction time varies depending on the reaction temperature, raw material compounds, reaction reagents, solvent used in the reaction and the like. It is normally 30 minutes to 48 hours and preferably 1 to 8 hours.

### Step 4

This step is a step for obtaining a compound represented by general formula (viii), or a salt thereof, by removing a protecting group (P²) of an amino group of a compound represented by general formula (vii).

This reaction can be achieved by a method that complies with the reaction conditions used in Step 2.

### Step 5

This step is a step for obtaining a compound represented by general formula (i) by carrying out a condensation reaction between an amine represented by general formula (viii), or a salt thereof, and a carboxylic acid represented by general formula (ix).

This reaction can be achieved by a method that complies with the reaction conditions used in Step 3.

### <Manufacturing Method 2>

This manufacturing method is a method for obtaining a compound of the present invention represented by general formula (i) by synthesizing a compound represented by general formula (vii) from a compound represented by general formula (xvi).

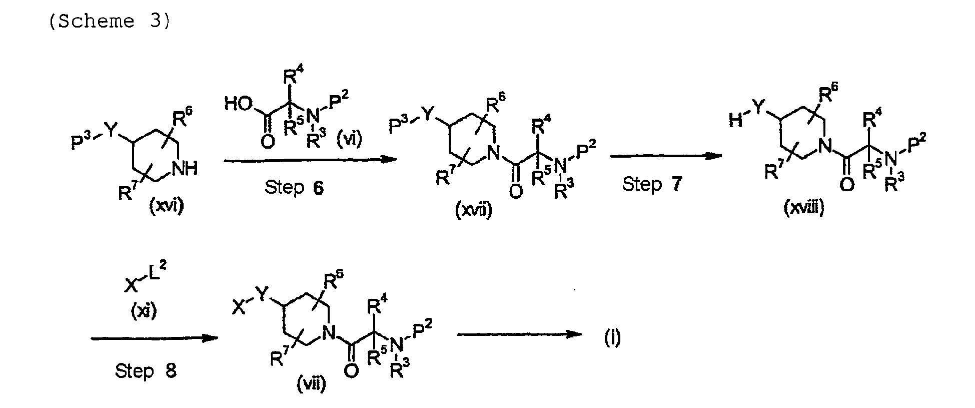

In the above formulae, R³, R⁴, R⁵, R⁶, R⁷, P², L², X and Y are the same as previously defined.

P³ represents an amino group or a protecting group of a hydroxy group. As examples of protecting groups, P² and P³ are both protecting groups of an amino group in the case where Y is represented by formula NRa. In other cases these protecting groups indicate different groups from each other, and groups for which P³ is stable under conditions for removing P² are respectively selected.

In the case where Y is an oxygen atom, examples of protecting groups include acyl-type protecting groups such as an acetyl group, an alkanoyl group or a benzoyl group, arylmethyl groups such as a benzyl group, silyl ether groups such as a t-butyldiphenylsilyl group or a t-butyldimethylsilyl group, a methoxymethyl group and a tetrahydropyranyl group, and preferably a t-butyldiphenylsilyl group is used.

### Step 6

This step is a step for obtaining a compound represented by the aforementioned formula (xvii) by a reaction of a piperidine derivative represented by the aforementioned formula (xvi) and an amino acid represented by the aforementioned formula (vi).

This reaction can be achieved by a method that complies with the reaction conditions used in Step 3.

### Step 7

This step is a step for obtaining a compound represented by the aforementioned formula (xviii), or a salt thereof, by removing a protecting group (P³) of a compound represented by the aforementioned formula (xvii).

In the case where Y in formula (xvii) is a group represented by formula NRa, this step can be achieved by a method that complies with the reaction conditions used in Step 2.

In the case where Y in formula (xvii) is an oxygen atom, the method used to remove the protecting group (P³) varies depending on the chemical properties of the protecting group used, and for example, an acyl group such as an alkanoyl group or aroyl group can be removed by hydrolysis with a suitable base such as an alkali metal hydroxide such as lithium hydroxide, sodium hydroxide or potassium hydroxide. In the case of an arylmethyl group, the protecting group can be removed by hydrolysis using a palladium-carbon catalyst. A silyl group such as a t-butyldimethylsilyl group or a t-butyldiphenylsilyl group can be removed by a hydrofluoride such as tetrabutylammonium fluoride. In addition, a methoxymethyl group or a tetrahydropyranyl group can be removed by acetic acid or hydrochloric acid and the like.

Examples of solvents used in this reaction include solvents that are inert in the reaction, including halogen-based solvents such as methylene chloride, chloroform, dichloroethane or carbon tetrachloride, ether-based solvents such as tetrahydrofuran, 1,2-dimethoxyethane or dioxane, aromatic-based solvents such as benzene or toluene, alcohol-based solvents such as methanol, ethanol or isopropanol, and amide-based solvents such as N,N-dimethylformamide and N-methylpyrrolidone.

The reaction temperature is from cooling to reflux heating (the temperature at which the solvent is heated and refluxed varies depending on the solvent), and specifically is 0 to 200°C and preferably 0 to 150°C.

Although the reaction time varies depending on the reaction temperature, raw material compounds, reaction reagents, solvent used in the reaction and the like, it is normally 30 minutes to 48 hours and preferably 1 to 8 hours.

### Step 8

This step is a step for synthesizing a compound represented by the aforementioned formula (vii) by reacting a compound represented by the aforementioned formula (xviii), or a salt thereof, with a compound represented by the aforementioned formula (xi).

This reaction can be achieved by a method that complies with the reaction conditions used in Step 1.

The compound represented by formula (vii) can be converted to a compound of the present invention represented by formula (i) by a method that complies with the scheme used in Manufacturing Method 1.

### <Manufacturing Method 3>

This manufacturing method is a method for obtaining a compound of the present invention represented by formula (i) by synthesizing a carboxylic acid represented by general formula (xxi) from a carboxylic acid derivative represented by general formula (ix), and reacting with a piperidine derivative represented by general formula (v), or a salt thereof.

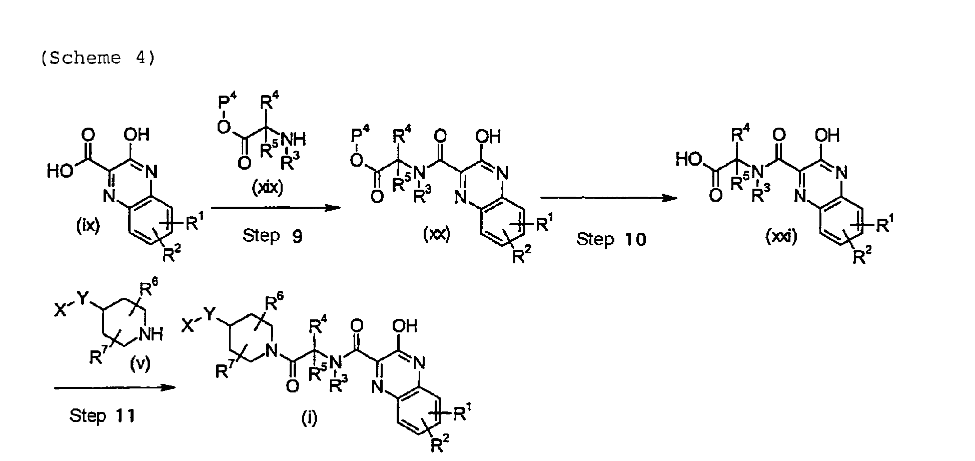

In the above formulae, R¹, R², R³, R⁴, R⁵, R⁶, R⁷, X and Y are the same as previously defined. P⁴ represents a protecting group of a carboxylic acid. Examples of protecting groups (P⁴) include a methyl group, an ethyl group, a t-butyl group and a benzyl group.

### Step 9

This step is a step for obtaining a compound represented by general formula (xx) by reacting a carboxylic acid represented by general formula (ix) with an amino acid represented by general formula (xix).

This reaction can be achieved by a method that complies with the reaction conditions used in Step 3.

### Step 10

This step is a step for obtaining a carboxylic acid derivative represented by general formula (xxi) by removing a protecting group (P⁴) of a compound represented by the aforementioned formula (xx) in a solvent.

The conditions of this reaction for removing the protecting group varies depending on the chemical properties of the protecting group used, and in the case of, for example, a methyl group or ethyl group, the protecting group can be removed by hydrolysis by using a suitable base such as an alkali metal hydroxide such as lithium hydroxide, sodium hydroxide or potassium hydroxide.

In the case of a t-butyl group, the protecting group can be removed by treating with trifluoroacetic acid or hydrochloric acid.

In addition, in the case of an arylmethyl group such as a benzyl group, the protecting group can be removed by hydrolysis using a palladium-carbon catalyst.

Examples of solvents used in this reaction include solvents that are inert in the reaction, including halogen-based solvents such as methylene chloride, chloroform, dichloroethane or carbon tetrachloride, ether-based solvents such as tetrahydrofuran, 1,2-dimethoxyethane or dioxane, aromatic-based solvents such as benzene or toluene, alcohol-based solvents such as methanol, ethanol or isopropanol, and amide-based solvents such as N,N-dimethylformamide or N-methylpyrrolidone.

The reaction temperature is from cooling to reflux heating (the temperature at which the solvent is heated and refluxed varies depending on the solvent), and specifically is 0 to 200°C and preferably 0 to 150°C.

Although the reaction time varies depending on the reaction temperature, raw material compounds, reaction reagents, solvent used in the reaction and the like, it is normally 30 minutes to 48 hours and preferably 1 to 8 hours.

### Step 11

This step is a step for obtaining a compound represented by general formula (i) by a condensation reaction between a carboxylic acid derivative represented by the aforementioned formula (xxi) and a piperidine derivative represented by the aforementioned formula (v), or a salt thereof.

This reaction can be achieved by a method that complies with the reaction conditions used in Step 3.

### <Manufacturing Method 4>

This manufacturing method is a method for obtaining a compound of the present invention represented by general formula (i) from a compound represented by general formula (xxi) by going through an intermediate represented by general formula (xxiv).

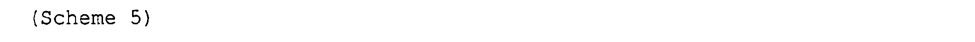

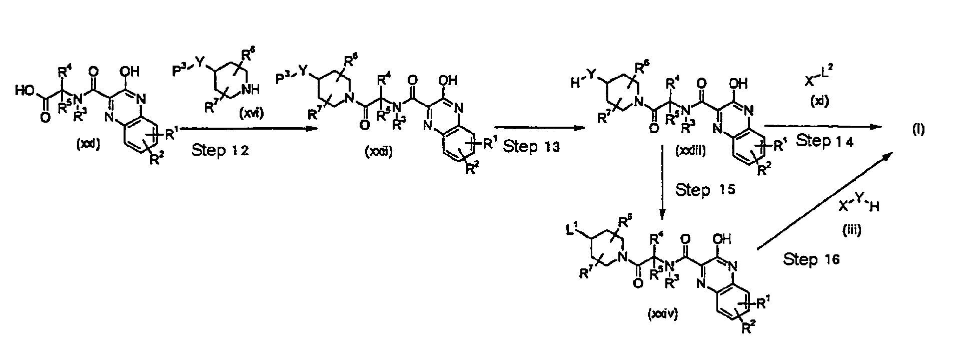

In the above formulae, R¹, R², R³, R⁴, R⁵, R⁶, R⁷, L¹, L², P³, X and Y are the same as previously defined.

### Step 12

This step is a step for obtaining a compound represented by general formula (xxii) from a carboxylic acid derivative represented by the aforementioned formula (xxi) and a piperidine derivative represented by the aforementioned formula (xvi).

This reaction can be achieved by a method that complies with the reaction conditions used in Step 3.

### Step 13

This step is a step for obtaining a compound represented by general formula (xxiii), or a salt thereof, by removing a protecting group (P³) of a compound represented by the aforementioned general formula (xxii).

This reaction can be achieved by a method that complies with the reaction conditions used in Step 7.

### Step 14

This step is a step for obtaining a compound represented by general formula (i) by reacting a compound represented by the aforementioned formula (xi) with a compound represented by the aforementioned formula (xxiii), or a salt thereof.

This reaction can be achieved by a method that complies with the reaction conditions used in Step 1.

### Step 15

This step is a step for obtaining a compound represented by the aforementioned formula (xxiv) by converting a hydroxy group of a compound represented by the aforementioned formula (xxiii) in which Y represents an oxygen atom to a leaving group (L¹ group).

Examples of leaving groups (L¹) used include a halogen atom such as a chloro group or a bromo group, and a methanesulfonyloxy group, a p-toluenesulfonyloxy group and a trifluoromethanesulfonyloxy group, and preferably a methanesulfonyloxy group is used.

In the case where a methanesulfonyloxy group is selected for the leaving group, for example, this reaction can be carried out by using methanesulfonyl chloride as a reagent and reacting the reaction reagent with a compound represented by the aforementioned formula (xxiii) in a solvent and in the presence of a base.

Although examples of bases include organic bases such as pyridine, triethylamine, N,N-diisopropylethylamine, 4-(dimethylamino)pyridine, N-methylmorpholine, 2,6-lutidine or collidine, and carbonates, alkoxides and hydroxides of alkali metals or alkaline earth metals such as sodium carbonate, potassium carbonate, sodium hydrogen carbonate, sodium ethoxide, potassium butoxide, sodium hydroxide or potassium hydride, preferably triethylamine and N,N-diisopropylethylamine are used.

Examples of solvents used include solvents that are inert in the reaction, including halogen-based solvents such as methylene chloride, chloroform, dichloroethane or carbon tetrachloride, ether-based solvents such as tetrahydrofuran, 1,2-dimethoxyethane or dioxane, aromatic-based solvents such as benzene or toluene, and amide-based solvents such as N,N-dimethylformamide or N-methylpyrrolidone.

The reaction temperature is from cooling to reflux heating (the temperature at which the solvent is heated and refluxed varies depending on the solvent), and specifically is 0 to 200°C and preferably 0 to 150°C.

Although the reaction time varies depending on the reaction temperature, raw material compounds, reaction reagents, solvent used in the reaction and the like, it is normally 30 minutes to 48 hours and preferably 1 to 8 hours.

### Step 16

This step is a step for obtaining a compound of the present invention represented by general formula (i) from a compound represented by the aforementioned formula (xxiv) and a compound represented by the aforementioned formula (iii).

This reaction can be achieved by a method that complies with the reaction conditions used in Step 1.

### <Manufacturing Method 5>

This manufacturing method is a method for obtaining a compound of the present invention represented by general formula (i) by synthesizing a compound represented by general formula (xxii) from a compound represented by the aforementioned formula (xvii).

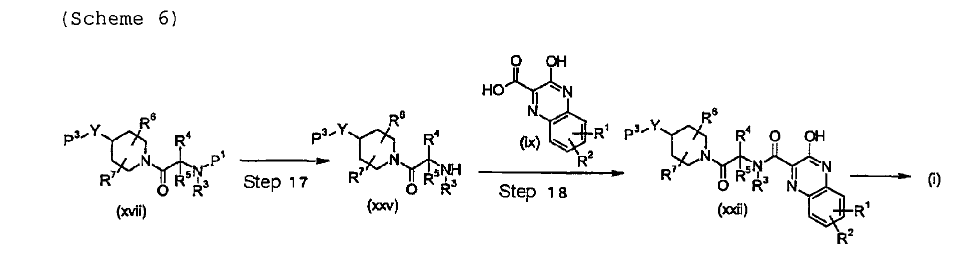

In the above formulae, R¹, R², R³, R⁴, R⁵, R⁶, R⁷, P², P³ and Y are the same as previously defined.

Although P² and P³ are both amino group protecting groups in the case where Y represents a group represented by formula NRa, these protecting groups indicate respective and separate groups, and groups for which P³ is stable under conditions for removing P² are respectively selected.

### Step 17

This step is a step for obtaining a compound represented by the aforementioned formula (xxv), or a salt thereof, by removing a protecting group (P²) of a compound represented by the aforementioned formula (xvii).

This reaction can be achieved by a method that complies with the reaction conditions used in Step 2.

### Step 18

This step is a step for obtaining a compound represented by the aforementioned formula (xxii) from an amine derivative represented by the aforementioned formula (xxv), or a salt thereof, and a carboxylic acid derivative represented by the aforementioned formula (ix).

This reaction can be achieved by a method that complies with the reaction conditions used in Step 5.

The compound represented by the aforementioned formula (xxii) can be converted to a compound of the present invention represented by general formula (i) by a method that complies with the scheme used in Manufacturing Method 4.

Following completion of each of the above reactions, the desired compound can be recovered from the reaction mixture in accordance with ordinary methods.

For example, after suitably neutralizing the reaction mixture or after removing insoluble matter by filtering in the case where insoluble matter is present, mutually immiscible solvents such as water and ethyl acetate are added followed by fractionating the organic layer, washing with water and the like, and separating the organic layer containing the desired compound. The desired compound is then obtained by drying with anhydrous magnesium sulfate and the like and distilling off the solvent.

The resulting desired compound can be separated and purified if necessary by suitably combining ordinary methods, such as recrystallization, reprecipitation, or methods commonly used to separate and purify organic compounds, examples of which include adsorption column chromatography using a carrier such as silica gel, alumina or magnesium-silica gel-based Florisil; a method using a synthetic adsorbent such as partition column chromatography using a carrier such as Sephadex LH-20 (Pharmacia), Amberlite XAD-11 (Rohm and Haas Chemical) or Diaion HP-20 (Mitsubishi Chemical), a method using ion exchange chromatography or forward phase-reverse phase column chromatography using a silica gel or alkylated silica gel (and preferably, high-performance liquid chromatography), followed by eluting with a suitable eluent.

Furthermore, each of the compounds (ii), (iii), (vi), (ix), (x), (xi), (xii), (xiii), (xiv), (xv), (xvi) and (xix) used as starting raw materials or auxiliary materials in the manufacturing methods of manufacturing methods 1 to 5 are either themselves known compounds or are compounds that are easily obtained from known compounds by treating in compliance with known methods.

Since the hydroxyquinoxalinecarboxamide derivatives of the present invention have superior anticoagulant action, they are useful as pharmaceuticals and particularly as preventive and/or therapeutic agents for blood coagulation disorders. The specific examples include preventive and/or therapeutic agents for blood coagulation disorders such as thromboembolism, congenital anticoagulant factor deficiency or plasminogen abnormality, and preventive agents for thrombogenesis following artificial valve replacement surgery, or thrombogenesis caused by nonvalvular atrial fibrillation or atrial fibrillation accompanying valvular heart disease.

Examples of administration forms of a compound of the present invention represented by general formula (i), or a pharmacologically acceptable salt thereof, include oral administration forms such as tablets, capsules, granules, powders or syrups, and parenteral administration forms such as injection preparations or suppositories. These preparations are produced by commonly known methods using additives such as vehicles, lubricants, binders, disintegrating agents, stabilizers, correctives or diluents.

In the case of administering a pharmaceutical composition having as the primary agent thereof a compound of the present invention represented by general formula (i), or a pharmacologically acceptable salt thereof, administration can be carried out systemically or locally and orally or parenterally. Examples of drug forms used for oral administration include tablets, pills, powders, granules, capsules, solutions, suspensions, emulsions, syrups and elixirs. Examples of drug forms used for parenteral administration include injection preparations, ointments, gels, creams, poultices, medicated patches, aerosols, inhalants, sprays, eye drops, nose drops, suppositories and inhalants. These drug forms can be prepared in accordance with ordinary methods using additives suitably selected as necessary from pharmaceutically acceptable additives such as diluents, vehicles, binders, stabilizers, antiseptics, colorants, solubilizers, suspending agents, buffers or wetting agents.

Although the amount used varies depending on the symptoms, body weight, age and administration method of the subject receiving administration (warm-blooded animal such as a human), in the case of oral administration, for example, the amount used has a lower limit of 0.001 mg/kg of body weight (and preferably 0.01 mg/kg of body weight) per administration and an upper limit of 500 mg/kg of body weight (and preferably 50 mg/kg of body weight) per administration, while in the case of intravenous administration, the amount used has a lower limit of 0.005 mg/kg of body weight (and preferably 0.05 mg/kg of body weight) per administration, and an upper limit of 50 mg/kg of body weight (and preferably 5 mg/kg of body weight) per administration, and administration is preferably carried out one to several times per day corresponding to symptoms.

### [Examples]

The following provides a more detailed explanation of the present invention through examples and test examples thereof, but the scope of the present invention is not limited thereto.

### Example 1

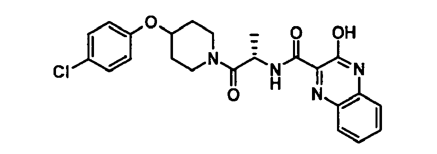

### N-{(1S)-2-[4-(4-Chlorophenoxy)piperidin-1-yl]-1-methyl-2-oxoethyl}-3-hydroxyquinoxaline-2-carboxamide

### Example 1-1

### t-Butyl [(1S)-2-(4-hydroxypiperidin-1-yl)-1-methyl-2-oxoethyl]carbamate

1-Ethyl-3-(3-dimethylaminopropyl)carbodiimide hydrochloride (2.30 g, 12.0 mmol) and 1-hydroxybenzotriazole monohydrate (1.84 g, 12.0 mmol)were added to a methylene chloride solution (50 ml) of 4-hydroxypiperidine (1.00 g, 10.0 mmol) and N-(t-butoxycarbonyl)-L-alanine (1.89 g, 10.0 mmol), at 0°C, under nitrogen stream, followed by further addition of triethylamine (1.21 ml, 12.0 mmol), and stirring was carried out at room temperature overnight. The reaction solution was diluted with methylene chloride, followed by sequential washing with a saturated aqueous sodium hydrogencarbonate solution, a saturated aqueous ammonium chloride solution, water and saline, and then the resulting organic layer was dried over anhydrous sodium sulfate. The organic layer was concentrated and the resulting residue was purified by a medium-pressure preparative liquid chromatograph (manufactured by Biotage, Inc., NH, 40+M) to afford t-butyl [(1S)-2-(4-hydroxypiperidin-l-yl)-1-methyl-2-oxoethyl]carbamate (2.02 g, yield 74%) as a colorless amorphous substance.
¹H-NMR (CDCl₃, 400 MHz) δ: 5.57 (1H, d, J=7.5 Hz), 4.64 (1H, m), 4.15-3.66 (3H, m), 3.40-3.14 (2H, m), 1.99-1.84 (2H, m), 1.71-1.49 (2H, m), 1.44 (9H, s), 1.31 and 1.30 (3H, d, J=7.0 Hz).
MS (FAB, m/z): 273 (M+H)⁺.

### Example 1-2

### t-Butyl {(1S)-2-[4-(4-chlorophenoxy)piperidin-1-yl]-1-methyl-2-oxoethyl}carbamate

A 40% toluene solution of diisopropyl azodicarboxylate (2.80 ml, 5.21 mmol) was added to a toluene solution (18 ml) of t-butyl [(1S)-2-(4-hydroxypiperidin-1-yl)-1-methyl-2-oxoethyl]carbamate (1.29 g, 4.74 mmol), 4-chlorophenol (670 mg, 5.21 mmol) and triphenylphosphine (1.37 g, 5.21 mmol), at room temperature, under nitrogen stream, and stirring was carried out at 90°C overnight. The reaction solution was concentrated and the resulting residue was purified by a medium-pressure preparative liquid chromatograph (manufactured by Biotage, Inc., 40+M) to afford t-butyl {(1S)-2-[4-(4-chlorophenoxy)piperidin-1-yl]-1-methyl-2-oxoethyl}carbamate (700 mg, yield 34%) as a pale yellow amorphous substance.
¹H-NMR (CDCl₃, 400 MHz) δ: 7.25 (2H, d, J=9.0 Hz), 6.85 (2H, d, J=9.0 Hz), 5.55 (1H, d, J=7.8 Hz), 4.65 (1H, m), 4.52 (1H, m), 3.88-3.36 (4H, m), 2.01-1.76 (4H, m), 1.44 (9H, s), 1.31 (3H, d, J=7.0 Hz).
MS (ESI, m/z): 383 (M+H)⁺.

### Example 1-3

### N-{(1S)-2-[4-(4-Chlorophenoxy)piperidin-1-yl]-1-methyl-2-oxoethyl}-3-hydroxyquinoxaline-2-carboxamide

A 4N-hydrochloric acid 1,4-dioxane solution (5 ml) was added to a 1,4-dioxane solution (5 ml) of t-butyl {(1S)-2-[4-(4-chlorophenoxy)piperidin-1-yl]-1-methyl-2-oxoethyl}carbamate (701 mg, 1.83 mmol), under nitrogen stream, and the mixture was allowed to react at room temperature overnight. The reaction solution was concentrated to afford (2S)-1-[4-(4-chlorophenoxy)piperidin-1-yl]-1-oxopropane-2-amine hydrochloride (558 mg) as a white solid.

1-Ethyl-3-(3-dimethylaminopropyl)carbodiimide hydrochloride (115 mg, 0.600 mmol) and 1-hydroxybenzotriazole monohydrate (91.0 mg, 0.600 mmol) were added to a methylene chloride solution (5.0 ml) of the resulting compound (160 mg, 0.500 mmol) and 3-hydroxyquinoxaline-2-carboxylic acid (98.0 mg, 0.500 mmol), at room temperature, under nitrogen stream, followed by further addition of N-methylmorpholine (0.270 ml, 2.50 mmol), and stirring was carried out at room temperature overnight. The reaction solution was diluted with methylene chloride, followed by sequential washing with a saturated aqueous sodium hydrogencarbonate solution, a saturated aqueous ammonium chloride solution, water and saline, and then the resulting organic layer was dried over anhydrous sodium sulfate. After the organic layer was concentrated and the resulting residue was purified by a medium-pressure preparative liquid chromatograph (manufactured by Biotage, Inc., 25+M), the residue resulting from concentration was suspended in a mixed solvent of methylene chloride-ethyl acetate, and the solid substance was collected by filtration to afford the desired title compound (130 mg, yield 57%) as a pale yellow solid.
¹H-NMR (DMSO-d₆, 400 MHz) δ: 12.86 (1H, brs), 9.71 (1H, brs), 7.88 (1H, dd, J=7.4 Hz, 6.8Hz), 7.65 (1H, dd, J=7.8 Hz, 7.4 Hz), 7.40 (1H, d, J=6.8 Hz), 7.38 (1H, d, J=7.8 Hz), 7.34 (2H, d, J=7.4 Hz), 7.04 (2H, m), 5.03 (1H, m), 4.66 (1H, m), 4.06-3.16 (4H, m), 2.08-1.87 (2H, m), 1.73-1.45 (2H, m), 1.31 (3H, d, J=6.6 Hz).
IR (KBr) cm⁻¹: 2945, 1690, 1640, 1500, 1240.
MS (ESI, m/z): 455 (M+H)⁺.
HRMS (ESI, m/z): 455.1478 (Calcd for C₂₃H₂₄ClN₄O₄: 455.1486).

### Example 2

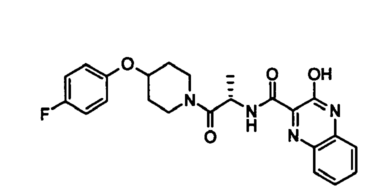

### N-{(1S)-2-[4-(4-Fluorophenoxy)piperidin-1-yl]-1-methyl-2-oxoethyl}-3-hydroxyquinoxaline-2-carboxamide

### Example 2-1

### t-Butyl {(1S)-2-[4-(4-fluorophenoxy)piperidin-1-yl]-1-methyl-2-oxoethyl}carbamate

1-Ethyl-3-(3-dimethylaminopropyl)carbodiimide hydrochloride (460 mg, 2.40 mmol) and 1-hydroxybenzotriazole monohydrate (367 mg, 2.40 mmol) were added to a methylene chloride solution (20 ml) of 4-(4-fluorophenoxy)piperidine hydrochloride (463 mg, 2.00 mmol) and N-(t-butoxycarbonyl)-L-alanine (416 mg, 2.20 mmol), at room temperature, under nitrogen stream, followed by further addition of N-methylmorpholine (1.10 ml, 10.0 mmol), and stirring was carried out at room temperature overnight. The reaction solution was poured into a 2N aqueous hydrochloric acid solution, followed by extraction with methylene chloride and sequential washing with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford t-butyl {(1S)-2-[4-(4-fluorophenoxy)piperidin-1-yl]-1-methyl-2-oxoethyl}carbamate (683 mg, yield 93%) as a colorless oil.
¹H-NMR (CDCl₃, 400 MHz) δ: 6.99 (2H, dd, J=8.6 Hz, 8.6 Hz), 6.87 (2H, m), 5.56(1H, d, J=6.6 Hz), 4.65 (1H, m), 4.48 (1H, m), 4.09-3.36 (4H, m), 2.00-1.74 (4H, m), 1.44 (9H, s), 1.31 (3H, d, J=7.0 Hz).
MS (FAB, m/z): 367 (M+H)⁺.

### Example 2-2

### N-{(1S)-2-[4-(4-Fluorophenoxy)piperidin-1-yl]-1-methyl-2-oxoethyl}-3-hydroxyquinoxaline-2-carboxamide

Concentrated hydrochloric acid (0.5 ml) was added to an ethanol solution (2.0 ml) of t-butyl {(1S)-2-[4-(4-fluorophenoxy)piperidin-1-yl]-1-methyl-2-oxoethyl}carbamate (111 mg, 0.300 mmol), under nitrogen stream, and stirring was carried out at room temperature overnight. The reaction solution was concentrated to afford (2S)-1-[4-(4-fluorophenoxy)piperidin-1-yl]-1-oxopropane-2-amine hydrochloride (100 mg) as a white solid.

1-Ethyl-3-(3-dimethylaminopropyl)carbodiimide hydrochloride (69.6 mg, 0.360 mmol) and 1-hydroxybenzotriazole monohydrate (55.6 mg, 0.360 mmol) were added to a methylene chloride solution (3.0 ml) of the resulting compound (100 mg, 0.300 mmol) and 3-hydroxyquinoxaline-2-carboxylic acid (57.5 mg, 0.300 mmol), at room temperature, under nitrogen stream, followed by further addition of N-methylmorpholine (0.170 ml, 1.50 mmol), and stirring was carried out at room temperature overnight. The reaction solution was poured into a 2N aqueous hydrochloric acid solution, followed by extraction with ethyl acetate and sequential washing with water, a saturated aqueous sodium hydrogencarbonate solution, water and saline, and then the resulting organic layer was dried over anhydrous sodium sulfate. After the organic layer was concentrated and the resulting residue was purified by a medium-pressure preparative liquid chromatograph (manufactured by Biotage, Inc., 25+M), the residue resulting from concentration was suspended in a mixed solvent of methylene chloride-diethyl ether, and the solid substance was collected by filtration to afford the desired title compound (59.1 mg, yield 44%) as a pale yellow solid.
¹H-NMR (DMSO-d₆, 400 MHz) δ: 12.9 (1H, brs), 9.70 (1H, brs), 7.88 (1H, dd, J=7.8 Hz, 4.6 Hz), 7.65 (1H, dt, J=7.8 Hz, 1.0 Hz), 7.40 (1H, d, J=7.8 Hz), 7.38(1H, d, J=7.8 Hz), 7.13 (2H, m), 7.03 (2H, m), 5.02 (1H, m), 4.60 (1H, m), 4.02-3.19 (4H, m), 2.06-1.85 (2H, m), 1.72-1.44 (2H, m), 1.31 (3H, d, J=6.6 Hz).
IR (KBr) cm⁻¹: 2935, 1685, 1640, 1505, 1205.
MS (ESI, m/z): 439 (M+H)⁺.
HRMS (ESI, m/z): 439.1791 (Calcd for C₂₃H₂₄FN₄O₄: 439.1782).

### Example 3

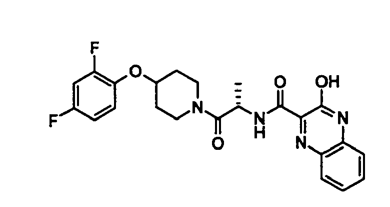

### N-{(1S)-2-[4-(2,4-Difluorophenoxy)piperidin-1-yl]-1-methyl-2-oxoethyl}-3-hydroxyquinoxaline-2-carboxamide

### Example 3-1

### t-Butyl {(1S)-2-[4-(2,4-difluorophenoxy)piperidin-1-yl]-1-methyl-2-oxoethyl}carbamate

Analogously to Example 1-2, t-butyl {(1S)-2-[4-(2,4-difluorophenoxy)piperidin-1-yl]-1-methyl-2-oxoethyl}carbamate (700 mg, yield 41%) was obtained from t-butyl [(1S)-2-(4-hydroxypiperidin-1-yl)-1-methyl-2-oxoethyl]carbamate (1.21 g, 4.44 mmol) and 2,4-difluorophenol (640 mg, 4.89 mmol as a colorless oil.
¹H-NMR (CDCl₃, 400 MHz) δ: 7.02-6.94 (1H, m), 6.92-6.84 (1H, m), 6.84-6.76 (1H, m), 5.55 (1H, d, J=7.4 Hz), 4.65 (1H, m), 4.42 (1H, m), 3.90-3.35 (4H, m), 2.00-1.77 (4H, m), 1.44 (9H, s), 1.32 and 1.31 (3H, d, J=7.0 Hz).
MS (ESI, m/z): 385 (M+H)⁺.

### Example 3-2

### N-{(1S)-2-[4-(2,4-Difluorophenoxy)piperidin-1-yl]-1-methyl-2-oxoethyl}-3-hydroxyquinoxaline-2-carboxamide

Analogously to Example 1-3, (2S)-1-[4-(2,4-difluorophenoxy)piperidin-1-yl]-1-oxopropane-2-amine hydrochloride (476 mg, yield 82%) was obtained from t-butyl {(1S)-2-[4-(2,4-difluorophenoxy)piperidin-1-yl]-1-methyl-2-oxoethyl}carbamate (700 mg, 1.82 mmol). The resulting compound (160 mg, 0.500 mmol) was condensed with 3-hydroxyquinoxaline-2-carboxylic acid (98.0 mg, 0.500 mmol) to afford the desired title compound (164 mg, yield 67%) as a pale yellow solid.
¹H-NMR (DMSO-d₆, 400 MHz) δ: 12.86 (1H, brs), 9.77 (1H, brs), 7.87 (1H, d, J=7.4 Hz), 7.64 (1H, dd, J=7.8 Hz, 7.8 Hz), 7.37 (1H, dd, J=7.8 Hz, 7.4 Hz), 7.38 (1H, d, J=7.8 Hz), 7.35-7.27 (2H, m), 7.07-6.99 (1H, m), 5.02 (1H, m), 4.59 (1H, m), 4.11-3.65 (2H, m), 3.57-3.11 (2H, m), 2.10-1.85 (2H, m), 1.80-1.46 (2H, m), 1.32 and 1.31 (3H, d, J=6.6 Hz). IR (KBr) cm⁻¹: 2950, 1685, 1640, 1630, 1500.
MS (ESI, m/z): 457 (M+H)⁺.
HRMS (ESI, m/z): 457.1709 (Calcd for C₂₃H₂₃F₂N₄O₄: 457.1687).

### Example 4

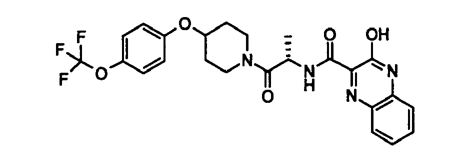

### 3-Hydroxy-N-[(1S)-1-methyl-2-oxo-2-{4-[4-(trifluoromethoxy)phenoxy]piperidin-1-yl}ethyl]quinoxaline-2-carboxamide

### Example 4-1

### t-Butyl [(1S)-1-methyl-2-oxo-2-{4-[4-(trifluoromethoxy)phenoxy]piperidin-1-yl}ethyl]carbamate

Analogously to Example 1-2, t-butyl [(1S)-1-methyl-2-oxo-2-{4-[4-(trifluoromethoxy)phenoxy]piperidin-1-yl}ethyl]carbamate (824 mg, yield 39%) was obtained from t-butyl {(1S)-2-[(4-hydroxypiperidin-1-yl)carbonyl]-1-methyl-2-oxoethyl}carbamate (1.32 g, 4.85 mmol) and 4-(trifluoromethoxy)phenol (950 mg, 5.33 mmol) as a pale yellow oil. ¹H-NMR (CDCl₃, 400 MHz) δ: 7.16 (2H, d, J=9.0 Hz), 6.90 (2H, d, J=9.0 Hz), 5.55 (1H, d, J=7.6 Hz), 4.65 (1H, m), 4.55 (1H, m), 3.88-3.38 (4H, m), 2.28-1.44 (4H, m), 1.44 (9H, s), 1.31 and 1.27 (3H, d, J=6.6 Hz).
MS (ESI, m/z): 433 (M+H)⁺.

### Example 4-2

### 3-Hydroxy-N-[(1S)-1-methyl-2-oxo-2-{4-[4-(trifluoromethoxy)phenoxylpiperidin-1-yl}ethyl]quinoxaline-2-carboxamide

Analogously to Example 1-3, (2S)-1-oxo-1-{4-[4-(trifluoromethoxy)phenoxy]piperidin-1-yl}propane-2-amine hydrochloride was obtained from t-butyl [(1S)-1-methyl-2-oxo-2-{4-[4-(trifluoromethoxy)phenoxy]piperidin-1-yl}ethyl]carbamate. The resulting compound (184 mg, 0.500 mmol) was condensed with 3-hydroxyquinoxaline-2-carboxylic acid (98.0 mg, 0.500 mmol) to afford the desired title compound (115 mg, 46%) as a pale yellow solid. ¹H-NMR (DMSO-d₆, 400 MHz) δ: 12.85 (1H, brs), 9.71 (1H, brs), 7.88 (1H, dd, J=7.8 Hz, 7.4 Hz), 7.65 (1H, dd, J=7.8 Hz, 7.8 Hz), 7.40 (1H, d, J=7.4 Hz), 7.38 (1H, d, J=7.8 Hz), 7.30 (2H, d, J=9.0 Hz), 7.11 (2H, dd, J=9.0 Hz, 3.8 Hz), 5.03 (1H, m), 4.69 (1H, m), 4.04-3.72 (2H, m), 3.56-3.19 (2H, m), 2.08-1.86 (2H, m), 1.75-1.48 (2H, m), 1.32 (3H, d, J=6.6 Hz).
IR (KBr) cm⁻¹: 2945, 1685, 1640, 1620, 1505, 1240.
MS (ESI, m/z): 505 (M+H)⁺.
HRMS (ESI, m/z): 505.1686 (Calcd for C₂₄H₂₄F₃N₄O₅: 505.1699).

### Example 5

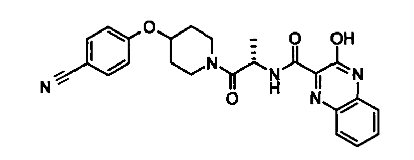

### N-{(1S)-2-[4-(4-Cyanophenoxy)piperidin-1-yl]-1-methyl-2-oxoethyl}-3-hydroxyquinoxaline-2-carboxamide

### Example 5-1

### t-Butyl {(1S)-2-[4-(4-cyanophenoxy)piperidin-1-yl]-1-methyl-2-oxoethyl}carbamate

Analogously to Example 2-1, t-butyl {(1S)-2-[4-(4-cyanophenoxy)piperidin-1-yl]-1-methyl-2-oxoethyl}carbamate (190 mg, yield 78%) was obtained from 4-[(piperidin-4-yl)oxy]benzonitrile hydrochloride (155 mg, 0.650 mmol) and N-(t-butoxycarbonyl)-L-alanine (123 mg, 0.650mmol) as a colorless oil.
¹H-NMR (CDCl₃, 400 MHz) δ: 7.60 (2H, d, J=9.0 Hz), 6.96 (2H, d, J=9.0 Hz), 5.52 (1H, d, J=7.8 Hz), 4.70-4.59 (2H, m), 4.90-4.30 (4H, m), 2.05-1.78 (4H, m), 1.44 (9H, s), 1.31 (3H, d, J=7.0 Hz).
MS (FAB, m/z): 374 (M+H)⁺.

### Example 5-2

### N-{(1S)-2-[4-(4-Cyanophenoxy)piperidin-1-yl]-1-methyl-2-oxoethyl}-3-hydroxyquinoxaline-2-carboxamide

Analogously to Example 1-3, 4-[(1-L-alanylpiperidin-4-yl)oxy]benzonitrile hydrochloride (133 mg, yield 84%) was obtained from t-butyl {(1S)-2-[4-(4-cyanophenoxy)piperidin-1-yl]-1-methyl-2-oxoethyl}carbamate (190 mg, 0.510 mmol). The resulting compound (108 mg, 0.350 mmol) was condensed with 3-hydroxyquinoxaline-2-carboxylic acid (68.6 mg, 0.350 mmol) to afford the desired title compound (107 mg, yield 68%) as a pale yellow solid.
¹H-NMR (DMSO-d₆, 400 MHz) δ: 12.87 (1H, brs), 9.70 (1H, brs), 7.88 (1H, dd, J=7.8 Hz, 7.4 Hz), 7.78 (2H, d, J=9.0 Hz), 7.65 (1H, dd, J=7.8 Hz, 7.8 Hz), 7.40 (1H, d, J=7.8 Hz), 7.38 (1H, d, J=7.4 Hz), 7.19 (2H, dd, J=9.0 Hz, 2.6 Hz), 5.03 (1H, m), 4.83 (1H, m), 4.07-3.73 (2H, m),
3.57-3.17 (2H, m), 2.12-1.89 (2H, m), 1.78-1.47 (2H, m), 1.31 (3H, d, J=7.0 Hz).
IR (KBr) cm⁻¹: 2945, 2220, 1685, 1640, 1605, 1505, 1255.
MS (ESI, m/z): 446 (M+H)⁺.
HRMS (ESI, m/z): 446.1851 (Calcd for C₂₄H₂₄N₅O₄: 446.1828).

### Example 6

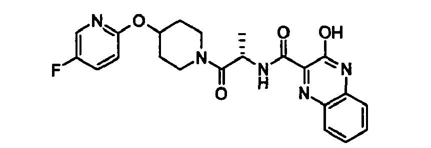

### N-[(1S)-2-{4-[(5-Fluoropyridin-2-yl)oxy]piperidin-1-yl}-1-methyl-2-oxoethyl]-3-hydroxyquinoxaline-2-carboxamide

### Example 6-1

### t-Butyl [(1S)-2-{4-[(5-fluoropyridin-2-yl)oxy]piperidin-1-yl}-1-methyl-2-oxoethyl]carbamate

Analogously to Example 2-1, t-butyl [(1S)-2-{4-[(5-fluoropyridin-2-yl)oxy]piperidin-1-yl}-1-methyl-2-oxoethyl]carbamate (316 mg, yield 86%) was obtained from 5-fluoro-2-[(piperidin-4-yl)oxy]pyridine dihydrochloride (269 mg, 1.00 mmol) and N-(t-butoxycarbonyl)-L-alanine (189 mg, 1.00 mmol) as a colorless amorphous substance.
¹H-NMR (CDCl₃, 400 MHz) δ: 7.98-7.94 (1H, m), 7.38-7.31 (1H, m), 6.70 (1H, dd, J=9.0 Hz, 3.4 Hz), 5.58 (1H, d, J=8.2 Hz), 5.23 (1H, m), 4.66 (1H, m), 4.01-3.34 (4H, m), 2.11-1.92 (2H, m), 1.89-1.72 (2H, m), 1.45 (9H, s), 1.32 (3H, d, J=7.0 Hz).
MS (FAB, m/z): 368 (M+H)⁺.

### Example 6-2

### N-[(1S)-2-{4-[(5-Fluoropyridin-2-yl)oxy]piperidin-1-yl}-1-methyl-2-oxoethyl]-3-hydroxyquinoxaline-2-carboxamide

Analogously to Example 1-3, (2S)-1-{4-[(5-fluoropyridin-2-yl)oxy]piperidin-1-yl}-1-oxopropane-2-amine dihydrochloride (283 mg, yield 100%) was obtained from t-butyl [(1S)-2-{4-[(5-fluoropyridin-2-yl)oxy]piperidin-1-yl}-1-methyl-2-oxoethyl]carbamate (306 mg, 0.831 mmol). From the resulting compound (119 mg, 0.350 mmol), through condensation with 3-hydroxyquinoxaline-2-carboxylic acid (68.6 mg, 0.350 mmol), the desired title compound (87.8 mg, yield 57%) was afforded as a yellow solid.
¹H-NMR (DMSO-d₆, 400 MHz) δ: 12.88 (1H, brs), 9.75 (1H, brs), 8.16 (1H, d, J=3.0 Hz), 7.88 (1H, dd, J=7.6 Hz, 4.2 Hz), 7.74-7.62 (2H, m), 7.40 (1H, d, J=7.6 Hz), 7.38 (1H, d, J=7.6 Hz), 6.92-6.83 (1H, m), 5.18 (1H, m), 5.03 (1H, m), 4.10-3.73 (2H, m), 3.56-3.16 (2H, m), 2.14-1.91 (2H, m), 1.79-1.46 (2H, m), 1.32 and 1.31(3H, d, J=6.6 Hz).
IR (KBr) cm⁻¹: 2950, 1690, 1630, 1480, 1220.
MS (ESI, m/z): 440 (M+H)⁺.
HRMS (ESI, m/z): 440.1736 (Calcd for C₂₃H₂₉FN₅O₄: 440.1734).

### Example 7

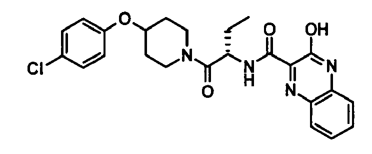

### N-[(1S)-1-{[4-(4-Chlorophenoxy)piperidin-1-yl]carbonyl}propyl]-3-hydroxyquinoxaline-2-carboxamide

### Example 7-1

### t-Butyl {(1S)-1-[(4-hydroxypiperidin-1-yl)carbonyl]propyl}carbamate

Analogously to Example 1-1, t-butyl {(1S)-1-[(4-hydroxypiperidin-1-yl)carbonyl]propyl}carbamate (5.95 g, yield 42%) was obtained from 4-hydroxypiperidine (5.06 g, 50.0 mmol) and (S)-2-(t-butoxycarbonylamino)butyric acid (10.1 g, 50.0 mmol) as a pale yellow amorphous subsutance.
¹H-NMR (CDCl₃, 500 MHz) δ: 5.48 (1H, d, J=7.4Hz), 4.58 (1H, m), 4.32-3.70 (3H, m), 3.39-3.18 (2H, m), 2.00-1.82 (2H, m), 1.82-1.63 (2H, m), 1.63-1.48 (3H, m), 1.43 (9H, s), 1.01-0.88 (3H, m).
MS (FAB/z): 287 (M+H)⁺.

### Example 7-2

### t-Butyl [(1S)-1-{[4-(4-chlorophenoxy)piperidin-1-yl]carbonyl}propyl]carbamate

Analogously to Example 1-2, a mixture (1.01 g) containing t-butyl [(1S)-1-{[4-(4-chlorophenoxy)piperidin-1-yl]carbonyl}propyl]carbamate was obtained from t-butyl {(1S)-1-[(4-hydroxypiperidin-1-yl)carbonyl]propyl}carbamate (1.00 g, 3.50 mmol) and 4-chlorophenol (490 mg, 3.85 mmol). The mixture was used as it is in the next reaction without further purificaiton.
MS (FAB, m/z): 397 (M+H)⁺.

### Example 7-3

### N-[(1S)-1-{[4-(4-Chlorophenoxy)piperidin-1-yl]carbonyl}propyl]-3-hydroxyquinoxaline-2-carboxamide

Analogously to Example 1-3, (2S)-1-[4-(4-chlorophenoxy)piperidin-1-yl]-1-oxobutan-2-amine hydrochloride (131 mg) was obtained from t-butyl [(1S)-1-{[4-(4-chlorophenoxy)piperidin-1-yl]carbonyl}propyl]carbamate (1.39 g, 3.50 mmol). From the resulting compound (117 mg, 0.350 mmol), through condensation with 3-hydroxyquinoxaline-2-carboxylic acid (68.6 mg, 0.350 mmol), the desired title compound (55.0 mg, yield 34%) was afforded as a pale yellow solid.
¹H-NMR (DMSO-d₆, 400 MHz) δ: 12.82 (1H, brs), 9.63 (1H, brs), 7.85 (1H, dd, J=7.4 Hz, 7.4 Hz), 7.63 (1H, dd, J=7.8 Hz, 7.4 Hz), 7.37 (1H, d, J=7.4 Hz), 7.36 (1H, d, J=7.8 Hz), 7.33 (2H, d, J=9.0 Hz), 7.04 and 7.03 (2H, d, J=9.0 Hz), 4.98 (1H, m), 4.66 (1H, m), 4.02-3.78 (2H, m), 3.56-3.19 (2H, m), 2.07-1.45 (6H, m), 0.91 (3H, t, J=7.4 Hz).
IR (KBr) cm⁻¹: 2935, 1690, 1640, 1490, 1240.
MS (ESI, m/z): 469 (M+H)⁺.
HRMS (ESI, m/z): 469.1650 (Calcd for C₂₄H₂₆ClN₄O₄: 469.1643).

### Example 8

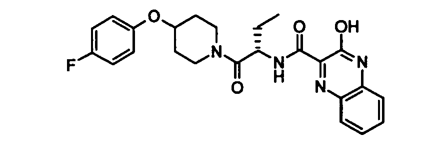

### N-[(1S)-1-{[4-(4-Fluorophenoxy)piperidin-1-yl]carbonyl}propyl]-3-hydroxyquinoxaline-2-carboxamide

### Example 8-1

### t-Butyl N-[(1S)-1-{[4-(4-fluorophenoxy)piperidin-1-yl]carbonyl}propyl]carbamate

Analogously to Example 2-1, t-butyl N-[(1S)-1-{[4-(4-fluorophenoxy)piperidin-1-yl]carbonyl}propyl]carbamate (703 mg, yield 92%) was obtained from 4-(4-fluorophenoxy)piperidine hydrochloride (463 mg, 2.00 mmol) and (2S)-2-(t-butoxycarbonylamino)butyric acid (407 mg, 2.00 mmol) as a colorless oil.
¹H-NMR (CDCl₃, 400 MHz) δ: 6.99 (2H, d, J=8.6 Hz), 6.87 (2H, m), 5.44 (1H, d, J=8.6 Hz), 4.59 (1H, m), 4.47 (1H, m), 3.83-3.38 (4H, m), 1.70-1.63 (4H, m), 1.63-1.50 (2H, m), 1.44 (9H, s), 0.94 (3H, t, J=7.4 Hz).
MS (ESI, m/z): 381 (M+H)⁺.

### Example 8-2

### N-[(1S)-1-{[4-(4-Fluorophenoxy)piperidin-1-yl]carbonyl}propyl]-3-hydroxyquinoxaline-2-carboxamide

Analogously to Example 2-2, (2S)-1-[4-(4-fluorophenoxy)piperidin-1-yl]-1-oxobutan-2-amine hydrochloride (535 mg) was obtained from t-butyl [(1S)-1-{[4-(4-fluorophenoxy)piperidin-1-yl]carbonyl}propyl]carbamate (703 mg, 1.80 mmol) as a white solid. The resulting compound (317 mg, 1.00 mmol) was condensed with 3-hydroxyquinoxaline-2-carboxylic acid (190 mg, 1.00 mmol) to afford the desired title compound (358 mg, yield 79%) as a pale yellow solid. ¹H-NMR (DMSO-d₆, 400 MHz) δ: 12.86 (1H, brs), 9.56 (1H, m), 7.88 (1H, dd, J=7.8 Hz, 7.8 Hz), 7.65 (1H, dd, J=7.8 Hz, 7.8 Hz), 7.40 (1H, d, J=7.8 Hz), 7.38 (1H, d, J=7.8 Hz), 7.14 (2H, m), 7.03 (2H, m), 5.00 (1H, m), 4.60 (1H, m), 4.03-3.79 (2H, m), 3.57-3.20 (2H, m), 2.07-1.44 (6H, m), 0.92 (3H, t, J=7.4 Hz).
IR (KBr) cm⁻¹: 2965, 1690, 1630, 1505, 1205.
MS (ESI, m/z): 453 (M+H)⁺.
HRMS (ESI, m/z): 453.1950 (Calcd for C₂₄H₂₆FN₄O₄: 453.1938)
Anal. Calcd for C₂₄H₂₅FN₄O₄: C, 63.71; H, 5.57; N, 12.38; F, 4.20. Found: C, 63.67; H, 5.46; N, 12.42; F, 4.09.

### Example 9

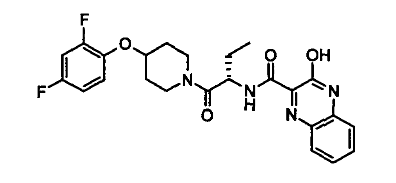

### N-[(1S)-1-{[4-(2,4-Difluorophenoxy)piperidin-1-yl]carbonyl}propyl]-3-hydroxyquinoxaline-2-carboxamide

Analogously to Example 1-2, a mixture (1.88 g) containing t-butyl [(1S)-1-{[4-(2,4-difluorophenoxy)piperidin-1-yl]carbonyl}propyl]carbamate was obtained from t-butyl {(1S)-2-[(4-hydroxypiperidin-1-yl)carbonyl]propyl}carbamate (1.18 g, 4.13 mmol) and 2,4-difluorophenol (590 mg, 4.54 mmol). The mixture was used as it is in the next reaction without further purification.

Analogously to Example 1-3, (2S)-1-[4-(2,4-difluorophenoxy)piperidin-1-yl]-1-oxobutan-2-amine hydrochloride (629 mg) was obtained from the resulting mixture (1.65 g, 4.13 mmol). The resulting compound (228 mg, 0.682 mmol) was condensed with 3-hydroxyquinoxaline-2-carboxylic acid (133 mg, 0.682 mmol) to afford the desired title compound (174 mg, yield 52%) as a pale yellow solid. ¹H-NMR (DMSO-d₆, 400 MHz) δ: 12.84 (1H, brs), 9.54 (1H, brs), 7.90-7.83 (1H, m), 7.65 (1H, dd, J=8.6 Hz, 8.2 Hz), 7.40 (1H, d, J=7.4 Hz), 7.38 (1H, d, J=8.6 Hz), 7.35-7.27 (2H, m), 7.27-7.07 (1H, m), 4.99 (1H, m), 4.59 (1H, m), 4.02-3.78 (2H, m), 3.57-3.20 (2H, m), 2.09-1.49 (6H, m), 0.92 (3H, t, J=7.0 Hz).
IR (KBr) cm⁻¹: 2940, 1685, 1640, 1505, 1210.
MS (ESI, m/z): 471 (M+H)⁺.
HRMS (ESI, m/z): 471.1848 (Calcd for C₂₄H₂₅F₂N₄O₄: 471.1844).

### Example 10

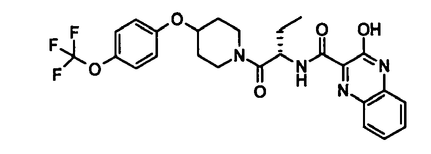

### 3-Hydroxy-N-[(1S)-1-({4-[4-(trifluoromethoxy)phenoxy]piperidin-1-yl}carbonyl)propyl]quinoxaline-2-carboxamide

### Example 10-1

### t-Butyl [(1S)-1-({4-[4-(trifluoromethoxy)phenoxy]piperidin-1-yl}carbonyl)propyl]carbamate

Analogously to Example 1-2, a mixture (1.58 g) containing t-butyl [(1S)-1-({4-[4-(trifluoromethoxy)phenoxy]piperidin-1-yl}carbonyl)propyl]carbamate was obtained from t-butyl {(1S)-1-[(4-hydroxypiperidin-1-yl)carbonyl}propyl}carbamate (1.13 g, 4.13 mmol) and 4-(trifluoromethoxy)phenol (590 mg, 4.54 mmol). The mixture was used as it is in the next reaction without further purification.
MS (FAB, m/z): 447 (M+H)⁺.

### Example 10-2

### 3-Hydroxy-N-[(1S)-1-({4-[4-(trifluoromethoxy)phenoxy]piperidin-1-yl}carbonyl)propyl]quinoxaline-2-carboxamide

Analogously to Example 1-3, (2S)-1-oxo-1-{4-[4-(trifluoromethoxy)phenoxylpiperidin-1-yl}butan-2-amine hydrochloride (310 mg) was obtained from t-butyl [(1S)-1-({4-[4-(trifluoromethoxy)phenoxy]piperidin-1-yl}carbonyl)propyl]carbamate (1.84 g, 4.12 mmol). The resulting compound (184 mg, 0.530 mmol) was condensed with 3-hydroxyquinoxaline-2-carboxylic acid (104 mg, 0.530 mmol) to afford the desired title compound (134 mg, yield 49%) as a pale yellow solid.
¹H-NMR (DMSO-d₆, 400 MHz) δ: 12.84 (1H, brs), 9.56 (1H, m), 7.87 (1H, dd, J=7.8 Hz, 7.8 Hz), 7.65 (1H, dd, J=7.8 Hz, 7.8 Hz), 7.40 (1H, d, J=7.8 Hz), 7.38 (1H, d, J=7.8 Hz), 7.30 (2H, d, J=8.6 Hz), 7.11 (2H, dd, J=8.6 Hz, 5.0 Hz), 5.00 (1H, m), 4.69 (1H, m), 4.08-3.79 (2H, m), 3.58-3.20 (2H, m), 2.10-1.46 (6H, m), 0.92 (3H, t, J=7.4 Hz).
IR (KBr) cm⁻¹: 2945, 1685, 1640, 1505, 1240.
MS (ESI, m/z): 519 (M+H)⁺.
HRMS (ESI, m/z): 519.1850 (Calcd for C₂₅H₂₆F₃N₄O₅: 519.1855).

### Example 11

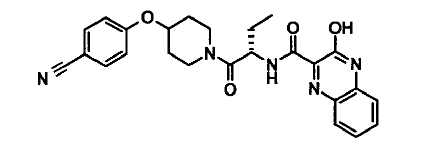

### N-[(1S)-1-{[4-(4-Cyanophenoxy)piperidin-1-yl]carbonyl}propyl]-3-hydroxyquinoxaline-2-carboxamide

### Example 11-1

### t-Butyl [(1S)-1-{[4-(4-cyanophenoxy)piperidin-1-yl]carbonyl}propyl]carbamate

Analogously to Example 2-1, t-butyl [(1S)-1-{[4-(4-cyanophenoxy)piperidin-1-yl]carbonyl}propyl]carbamate (226 mg, yield 90%) was obtained from 4-[(piperidin-4-yl)oxy]benzonitrile hydrochloride (155 mg, 0.650 mmol) and (2S)-2-(t-butoxycarbonylamino)butyric acid (132 mg, 0.650 mmol) as a colorless oil.
¹H-NMR (CDCl₃, 400 MHz) δ: 7.60 (2H, d, J=9.0 Hz), 6.96 (2H, d, J=9.0 Hz), 5.41 (1H, d, J=8.6 Hz), 4.70-4.55 (2H, m), 3.90-3.41 (4H, m), 2.07-1.70 (4H, m), 1.44 (9H, s), 1.57 (2H, q, J=7.4 Hz), 0.94 (3H, t, J=7.4 Hz).
MS (FAB, m/z): 388 (M+H)⁺.

### Example 11-2

### N-[(1S)-1-{[4-(4-Cyanophenoxy)piperidin-1-yl]carbonyl}propyl]-3-hydroxyquinoxaline-2-carboxamide

Analogously to Example 1-3, 4-({1-[(2S)-2-aminobutanoyl]piperidin-4-yl}oxy)benzonitrile hydrochloride (133 mg, yield 84%) was obtained from t-butyl [(1S)-1-{[4-(4-cyanophenoxy)piperidin-1-yl]carbonyl}propyl]carbamate (226 mg, 0.583 mmol). The resulting compound (113 mg, 0.350 mmol) was condensed with 3-hydroxyquinoxaline-2-carboxylic acid (68.6 mg, 0.350 mmol) to afford the desired title compound (107 mg, yield 66%) as a yellow solid. ¹H-NMR (DMSO-d₆, 400 MHz) δ: 12.85 (1H, brs), 9.56 (1H, m), 7.87 (1H, dd, J=8.2 Hz, 7.8 Hz), 7.78 (2H, d, J=9.0 Hz), 7.65 (1H, dd, J=7.8 Hz, 7.4 Hz), 7.40 (1H, d, J=7.4 Hz), 7.38 (1H, d, J=8.2 Hz), 7.19 (2H, dd, J=9.0 Hz, 4.2 Hz), 5.00 (1H, m), 4.83 (1H, m), 4.07-3.81 (2H, m), 3.61-3.16 (2H, m), 2.14-1.46 (6H, m), 0.92 (3H, t, J=7.4 Hz).
IR (KBr) cm⁻¹: 2965, 2220, 1685, 1630, 1505, 1250.
MS (ESI, m/z): 460 (M+H)⁺.
HRMS (ESI, m/z): 460.1973 (Calcd for C₂₅H₂₆N₅O₄: 460.1984).

### Example 12

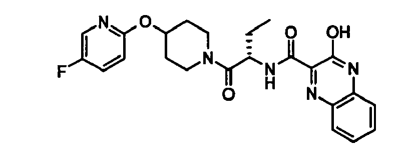

### N-[(1S)-1-({4-[(5-Fluoropyridin-2-yl)oxy]piperidin-1-yl}carbonyl)propyl]-3-hydroxyquinoxaline-2-carboxamide

### Example 12-1

### t-Butyl [(1S)-1-({4-[(5-fluoropyridin-2-yl)oxy]piperidin-1-yl}carbonyl)propyl]carbamate

Analogously to Example 2-1, t-butyl [(1S)-1-({4-[(5-fluoropyridin-2-yl)oxylpiperidin-1-yl}carbonyl)propyl]carbamate (317 mg, yield 83%) was obtained from 5-fluoro-2-[(piperidin-4-yl)oxy]pyridine dihydrochloride (269 mg, 1.00 mmol) and (2S)-2-(t-butoxycarbonylamino)butyric acid (203 mg, 1.00mmol) as a colorless oil.
¹H-NMR (CDCl₃, 400 MHz) δ: 7.96 (1H, dd, J=3.0 Hz, 3.0 Hz), 7.39-7.32 (1H, m), 6.70 (1H, dd, J=9.0 Hz, 3.4 Hz), 5.47 (1H, d, J=8.6 Hz), 5.23 (1H, m), 4.60 (1H, m), 3.99-3.38 (4H, m), 2.10-1.91 (2H, m), 1.88-1.70 (2H, m), 1.64-1.50 (2H, m), 1.44 (9H, s), 0.94 (3H, t, J=7.4 Hz).
MS (FAB, m/z): 382 (M+H)⁺.

### Example 12-2

### N-[(1S)-1-({4-[(5-Fluoropyridin-2-yl)oxy]piperidin-1-yl}carbonyl)propyl]-3-hydroxyquinoxaline-2-carboxamide

Analogously to Example 1-3, (2S)-1-{4-[(5-fluoropyridin-2-yl)oxy]piperidin-1-yl}-1-oxobutan-2-amine dihydrochloride (285 mg, yield 100%) was obtained from t-butyl [(1S)-1-({4-[(5-fluoropyridin-2-yl)oxylpiperidin-1-yl}carbonyl)propyl]carbamate (307 mg, 0.805 mmol). The resulting compound (124 mg, 0.350 mmol) was condensed with 3-hydroxyquinoxaline-2-carboxylic acid (68.6 mg, 0.350 mmol) to afford the desired title compound (74.2 mg, yield 47%) as a yellow solid. ¹H-NMR (DMSO-d₆, 400 MHz) δ: 12.85 (1H, brs), 9.58 (1H, d, J=7.0 Hz), 8.16 (1H, d, J=2.6 Hz), 7.88 (1H, dd, J=7.4 Hz, 5.4 Hz), 7.74-7.62 (2H, m), 7.40 (1H, d, J=7.4 Hz), 7.38 (1H, d, J=8.2 Hz), 6.93-6.84 (1H, m), 5.19 (1H, m), 5.00 (1H, m), 4.08-3.81 (2H, m), 3.58-3.16 (2H, m), 2.15-1.92 (2H, m), 1.88-1.48 (4H, m), 0.92 (3H, t, J=7.4 Hz).
IR (KBr) cm⁻¹: 2950, 1690, 1630, 1480, 1215.
MS (ESI, m/z): 454 (M+H)⁺.
HRMS (ESI, m/z): 454.1890 (Calcd for C₂₃H₂₅FN₅O₄: 454.1891).

### Example 13

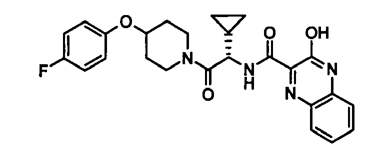

### N-{(1S)-1-Cyclopropyl-2-[4-(4-fluorophenoxy)piperidin-1-yl]-2-oxoethyl}-3-hydroxyquinoxaline-2-carboxamide

### Example 13-1

### t-Butyl {(1S)-1-cyclopropyl-2-[4-(4-fluorophenoxy)piperidin-1-yl)-2-oxoethyl}carbamate

Analogously to Example 2-1, t-butyl {(1S)-1-cyclopropyl-2-[4-(4-fluorophenoxy)piperidin-1-yl]-2-oxoethyl}carbamate (3.06 g, yield 90%) was obtained from 4-(4-fluorophenoxy)piperidine hydrochloride (2.00 g, 8.63 mmol) and (2S)-2-(t-butoxycarbonylamino)cyclopropylacetic acid (1.86 g, 8.63 mmol) as a colorless oil.
¹H-NMR (CDCl₃, 400 MHz) δ: 6.99 (2H, dd, J=8.6 Hz, 8.6 Hz), 6.87 (2H, dd, J=8.6Hz, 4.6 Hz), 5.46 (1H, d, J=7.8 Hz), 4.54-4.42 (2H, m), 3.84-3.44 (4H, m), 2.08-1.74 (4H, m), 1.44 (9H, s), 1.16-1.03 (1H, m), 0.58-0.31 (4H, m).
MS (ESI, m/z): 393 (M+H)⁺.

### Example 13-2

### N-{(1S)-1-Cyclopropyl-2-[4-(4-fluorophenoxy)piperidin-1-yl]-2-oxoethyl}-3-hydroxyquinoxaline-2-carboxamide

Analogously to Example 2-2, (1S)-1-cyclopropyl-2-[4-(4-fluorophenoxy)piperidin-1-yl]-2-oxoethanamine hydrochloride (2.00 g, yield 88%) was obtained from t-butyl {(1S)-1-cyclopropyl-2-[4-(4-fluorophenoxy)piperidin-1-yl]-2-oxoethyl}carbamate (3.06 g, 7.80 mmol) as a colorless oil. The resulting compound (2.00 g, 6.84 mmol) was condensed with 3-hydroxyquinoxaline-2-carboxylic acid (1.48 g, 7.80 mmol) under nitrogen stream to afford the desired title compound (2.71 g, yield 85%) as a pale yellow solid.
¹H-NMR (DMSO-d₆, 400 MHz) δ: 12.88 (1H, s), 9.62 (1H, d, J=6.0 Hz), 7.87 (1H, dd, J=7.6 Hz, 2.0 Hz), 7.65 (1H, dt, J=7.6 Hz, 2.0 Hz), 7.40 (1H, dt, J=7.6 Hz, 2.0 Hz), 7.37 (1H, d, J=7.6 Hz), 7.12 (2H, m), 7.02 (2H, m), 4.76 (1H, q, J=8.4 Hz), 4.61 (1H, m), 4.12-3.80 (2H, m), 3.60-3.20 (2H, m), 2.08-1.88 (2H, m), 1.75-1.45 (2H, m), 1.23 (1H, dd, J=6.4 Hz), 0.53-0.38 (4H, m).
MS (ESI, m/z): 465 (M+H)⁺.
Anal. Calcd for C₂₅H₂₅FN₄O₄: C, 64.64; H, 5.42; N, 12.06. Found: C, 64.27; H, 5.30; N, 12.01.

### Example 14

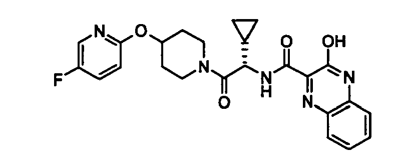

### N-[(1S)-1-Cyclopropyl-2-{4-[(5-fluoropyridin-2-yl)oxy]piperidin-1-yl}-2-oxoethyl]-3-hydroxyquinoxaline-2-carboxamide

### Example 14-1

### t-Butyl [(1S)-1-cyclopropyl-2-{4-[(5-fluoropyridin-2-yl)oxy]piperidin-1-yl}-2-oxoethyl]carbamate

Analogously to Example 2-1, t-butyl [(1S)-1-cyclopropyl-2-{4-[(5-fluoropyridin-2-yl)oxy]piperidin-1-yl}-2-oxoethyl]carbamate (276 mg, yield 70%) was obtained from 5-fluoro-2-[(piperidin-4-yl)oxy]pyridine dihydrochloride (269 mg, 1.00 mmol) and (2S)-2-(t-butoxycarbonylamino)-2-cyclopropylacetic acid (215 mg, 1.00 mmol) as a colorless oil.
¹H-NMR (CDCl₃, 400 MHz) δ: 7.97 (1H, d, J=2.6 Hz), 7.40-7.32 (1H, m), 6.71 (1H, dd, J=9.0 Hz, 3.4 Hz), 5.47 (1H, d, J=7.8 Hz), 5.24 (1H, m), 4.51 (1H, dd, J=7.4 Hz, 7.4 Hz), 3.99-3.73 (2H, m), 3.66-3.42 (2H, m), 2.12-1.94 (2H, m), 1.91-1.73 (2H, m), 1.44 (9H, s), 1.17-1.06 (1H, m), 0.58-0.34 (4H, m).
MS (FAB, m/z): 394 (M+H)⁺.

### Example 14-2

### N-[(1S)-1-Cyclopropyl-2-{4-[(5-fluoropyridin-2-yl)oxy]piperidin-1-yl}-2-oxoethyl]-3-hydroxyquinoxaline-2-carboxamide

Analogously to Example 1-3, (1S)-1-cyclopropyl-2-{4-[(5-fluoropyridin-2-yl)oxy]piperidin-1-yl}-2-oxoethanamine dihydrochloride (248 mg, yield 100%) was obtained from t-butyl [(1S)-1-cyclopropyl-2-{4-[(5-fluoropyridin-2-yl)oxy]piperidin-1-yl}-2-oxoethyl]carbamate (266 mg, 0.676 mmol). The resulting compound (160 mg, 0.439 mmol) was condensed with 3-hydroxyquinoxaline-2-carboxylic acid (85.6 mg, 0.439 mmol) to afford the desired title compound (78.3 mg, yield 38%) as a yellow solid.
¹H-NMR (DMSO-d₆, 400 MHz) δ: 12.87 (1H, brs), 9.67 (1H, brs), 8.16 (1H, d, J=2.6 Hz), 7.88 (1H, m), 7.76-7.62 (2H, m), 7.40 (1H, d, J=7.0 Hz), 7.39 (1H, d, J=8.6 Hz), 6.95-6.83 (1H, m), 5.19 (1H, m), 4.79 (1H, m), 4.10-3.82 (2H, m), 3.59-3.14 (2H, m), 2.17-1.45 (4H, m), 1.28-1.17 (1H, m), 0.53-0.36 (4H, m).
IR (KBr) cm⁻¹: 2960, 1690, 1630, 1530, 1480.
MS (ESI, m/z): 466(M+H)⁺.
HRMS (ESI, m/z): 466.1882 (Calcd for C2₄H₂₅FN₅O₄: 466.1891).

### Example 15

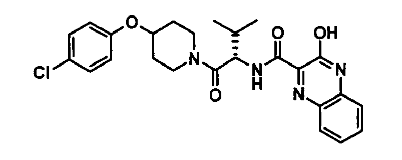

### N-[(1S)-1-{[4-(4-Chlorophenoxy)piperidin-1-yl]carbonyl}-2-methylpropyl]-3-hydroxyquinoxaline-2-carboxamide

### Example 15-1

### t-Butyl N-[(1S)-1-{[4-(4-chlorophenoxy)piperidin-1-yl]carbonyl}-2-methylpropyl] carbamate

1-Ethyl-3-(3-dimethylaminopropyl)carbodiimide hydrochloride (844 mg, 4.40 mmol) and 1-hydroxybenzotriazole monohydrate (595 mg, 4.40 mmol) were added to a methylene chloride solution (30 ml) of 4-(4-chlorophenoxy)piperidine hydrochloride (910 mg, 3.67 mmol) and N-(t-butoxycarbonyl)-L-valine (876 mg, 4.03 mmol), at room temperature, under nitrogen stream, followed by further addition of N-methylmorpholine (0.888 ml, 8.07 mmol), and stirring was carried out at room temperature overnight. The reaction solution was poured into a 2N aqueous hydrochloric acid solution, followed by extraction with methylene chloride and sequential washing with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford t-butyl [(1S)-1-{[4-(4-chlorophenoxy)piperidin-1-yl]carbonyl}-2-methylpropyl]carbamate (1.38 g, yield 92%) as a colorless oil.
¹H-NMR (CDCl₃, 400 MHz) δ: 7.41-7.23 (2H, m), 7.07-6.83 (2H, m), 5.34 (1H, d, J=8.3 Hz), 4.53-4.48 (2H, m), 3.80-3.46 (4H, m), 2.00-1.78 (5H, m), 1.48-1.40 (9H, m), 0.97 (3H, d, J=6.8 Hz), 0.89 (3H, d, J=6.8 Hz).
MS (FAB, m/z): 411 (M+H)⁺.

### Example 15-2

### N-[(1S)-1-{[4-(4-Chlorophenoxy)piperidin-1-yl]carbonyl}-2-methylpropyl]-3-hydroxyquinoxaline-2-carboxamide

Concentrated hydrochloric acid (6.0 ml) was added to an ethanol solution (12 ml) of t-butyl [(1S)-1-{[4-(4-chlorophenoxy)piperidin-1-yl]carbonyl}-2-methylpropyl]carbamate (1.38 g, 3.36 mmol), under nitrogen stream, and the mixture was allowed to react at room temperature overnight. The reaction solution was concentrated to afford (2S)-1-[4-(4-chlorophenoxy)piperidin-1-yl]-3-methyl-1-oxobutan-2-amine hydrochloride (1.17 g) as a white solid.

1-Ethyl-3-(3-dimethylaminopropyl)carbodiimide hydrochloride (149 mg, 0.777 mmol) and 1-hydroxybenzotriazole monohydrate (105 mg, 0.777 mmol), were added to a methylene chloride solution (5.0 ml) of the resulting compound (180 mg, 0.518 mmol) and 3-hydroxyquinoxaline-2-carboxylic acid (148 mg, 0.777 mmol), at room temperature, under nitrogen stream, followed by further addition of triethylamine (0.289 ml, 2.07 mmol), and stirring was carried out at room temperature overnight. The reaction solution was poured into a 2N aqueous hydrochloric acid solution, followed by extraction with ethyl acetate and sequential washing with water, a saturated aqueous sodium hydrogencarbonate solution, water and saline, and then the resulting organic layer was dried over anhydrous sodium sulfate. After the organic layer was concentrated and the resulting residue was purified by a medium-pressure preparative liquid chromatograph (manufactured by Biotage, Inc., 25+M), the residue resulting from concentration was suspended in a mixed solvent of methylene chloride-diethyl ether, and the solid substance was collected by filtration to afford the desired title compound (113 mg, yield 45%) as a pale yellow solid.
¹H-NMR (DMSO-d₆, 400 MHz) δ: 12.86 (1H, s), 10.19 (1H, s), 7.97 (1H, t, J=7.0 Hz), 7.61 (1H, s), 7.53 (1H, s), 7.36 (1H, s), 7.26 (2H, t, J=7.0 Hz), 6.86 (2H, t, J=7.0 Hz), 5.09-5.07 (1H, m), 4.57-4.55 (1H, m), 4.01-3.68 (4H, m), 2.21-2.02 (5H, m), 1.14-1.11 (6H, m).
IR (KBr) cm⁻¹: 2960, 1690, 1640, 1490, 1240, 1150.
MS (FAB, m/z): 483 (M+H)⁺.
HRMS (ESI, m/z): 483.1797 (Calcd for C₂₅H₂₈ClN₄O₄: 483.1799).

### Example 16

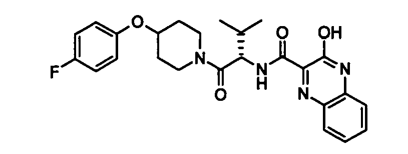

### N-[(1S)-1-{[4-(4-Fluorophenoxy)piperidin-1-yl]carbonyl}-2-methylpropyl]-3-hydroxyquinoxaline-2-carboxamide

### Example 16-1

### t-Butyl [(1S)-1-{[4-(4-fluorophenoxy)piperidin-1-yl]carbonyl}-2-methylpropyl] carbamate

Analogously to Example 2-1, t-butyl [(1S)-1-{[4-(4-fluorophenoxy)piperidin-1-yllcarbonyl}-2-methylpropyl]carbamate (745 mg, yield 86%) was obtained from 4-(4-fluorophenoxy)piperidine hydrochloride (510 mg, 2.20 mmol) and N-(t-butoxycarbonyl)-L-valine (478 mg, 2.20 mmol) as a colorless amorphous substance.
¹H-NMR (CDCl₃, 400 MHz) δ: 7.01-6.95 (2H, m), 6.89-6.84 (2H, m), 5.34 (1H, d, J=8.8 Hz), 4.52-4.45 (2H, m), 3.82-3.45 (4H, m), 2.05-1.76 (5H, m), 1.44 (9H, s), 0.97 (3H, d, J=6.8 Hz), 0.89 (3H, d, J=6.8 Hz). MS (ESI, m/z): 395 (M+H)⁺.

### Example 16-2

### N-[(1S)-1-{[4-(4-Fluorophenoxy)piperidin-1-yl]carbonyl}-2-methylpropyl]-3-hydroxyquinoxaline-2-carboxamide

Analogously to Example 2-2, (2S)-1-[4-(4-fluorophenoxy)piperidin-1-yl]-3-methyl-1-oxobutan-2-amine hydrochloride (550 mg, 70%) was obtained from t-butyl [(1S)-1-{[4-(4-fluorophenoxy)piperidin-1-yl]carbonyl}-2-methylpropyl]carbamate (745 mg, 1.89 mmol) as a colorless oil. The resulting compound (150 mg, 0.450 mmol) was condensed with 3-hydroxyquinoxaline-2-carboxylic acid (95.0 mg, 0.500 mmol) under nitrogen stream to afford the desired title compound (101 mg, yield 48%) as a pale yellow solid.
¹H-NMR (DMSO-d₆, 400 MHz) δ: 12.51 (1H, brs), 10.14 (1H, brs), 8.05-7.96 (1H, m), 7.70-7.32 (3H, m), 7.04-6.84 (4H, m), 5.08 (1H, dd, J=7.6 Hz, 7.1 Hz), 4.53-4.46 (1H, m), 4.00-3.68 (4H, m), 2.38-2.09 (1H, m), 2.02-1.85 (4H, m), 1.17-1.07 (6H, m).
IR (ATR) cm⁻¹, 1685, 1630, 1500, 1450, 1275, 1245.
MS (ESI, m/z): 467 (M+H)⁺.
Anal. Calcd for C₂₅H₂₇FN₄O₄: C, 64.37; H, 5.83; F, 4.07; N, 12.01. Found: C, 64.01; H, 5.81; F, 3.85; N, 11.69.

### Example 17

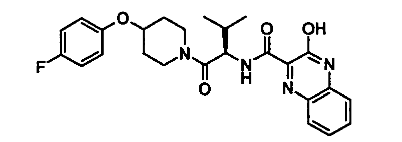

### N-[(1R)-1-{[4-(4-Fluorophenoxy)piperidin-1-yl]carbonyl}-2-methylpropyl]-3-hydroxyquinoxaline-2-carboxamide

### Example 17-1

### t-Butyl [(1R)-1-{[4-(4-fluorophenoxy)piperidin-1-yl]carbonyl}-2-methylpropyl] carbamate

Analogously to Example 2-1, t-butyl [(1R)-1-{[4-(4-fluorophenoxy)piperidin-1-yl]carbonyl}-2-methylpropyl]carbamate (326 mg, yield 83%) was obtained from 4-(4-fluorophenoxy)piperidine hydrochloride (232 mg, 1.00 mmol) and N-(t-butoxycarbonyl)-D-valine (217 mg, 1.00 mmol) as a colorless oil.
¹H-NMR (CDCl₃, 500 MHz) δ: 8.72 (2H, dd, J=8.8 Hz, 8.0 Hz), 8.57 (2H, dd, J=8.8 Hz, 4.4 Hz), 5.34 (1H, d, J=8.8 Hz), 4.54-4.43 (2H, m), 3.82-3.40 (4H, m), 2.00-1.80 (5H, m), 1.44 (9H, s), 0.97 and 0.89 (6H, d, J=6.8 Hz).
MS (ESI, m/z): 395 (M+H)⁺.

### Example 17-2

### N-[(1R)-1-{[4-(4-Fluorophenoxy)piperidin-1-yl]carbonyl}-2-methylpropyl]-3-hydroxyquinoxaline-2-carboxamide

Analogously to Example 2-2, (2R)-1-[4-(4-fluorophenoxy)piperidin-1-yl]-3-methyl-1-oxobutan-2-amine hydrochloride (113 mg, yield 46%) was obtained from t-butyl [(1R)-1-{[4-(4-fluorophenoxy)piperidin-1-yl]carbonyl}-2-methylpropyl]carbamate (326 mg, 0.828 mmol). The resulting compound (113 mg, 0.382 mmol) was condensed with 3-hydroxyquinoxaline-2-carboxylic acid (72.7 mg, 0.382 mmol) to afford the desired title compound (92.8 mg, yield 51%) as a yellow solid.
¹H-NMR (DMSO-d₆, 400 MHz) δ: 12.67 (1H, brs), 10.17 (1H, brs), 8.05-7.96 (1H, m), 7.70-7.32 (3H, m), 7.04-6.84 (4H, m), 5.08 (1H, dd, J=7.6 Hz, 7.1 Hz), 4.53-4.46 (1H, m), 4.00-3.68 (4H, m), 2.38-2.09 (1H, m), 2.02-1.85 (4H, m), 1.17-1.07 (6H, m).
IR (KBr)cm⁻¹: 2960, 1690, 1630, 1505, 1210.
MS (ESI, m/z): 467 (M+H)⁺.
HRMS (ESI, m/z): 467.2170 (Calcd for C₂₅H₂₈FN₄O₄: 467.2095).

### Example 18

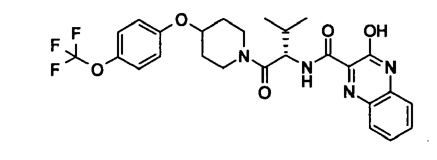

### 3-Hydroxy-N-[(1S)-2-methyl-1-({4-[4-(trifluoromethoxy)phenoxy]piperidin-1-yl}carbonyl)propyl]quinoxaline-2-carboxamide

### Example 18-1

### t-Butyl [(1S)-2-methyl-1-({4-[4-(trifluoromethoxy)phenoxy]piperidin-1-yl}carbonyl)propyl]carbamate

Analogously to Example 1-2, t-butyl N-[(1S)-2-methyl-1-{4-[4-(trifluoromethoxy)phenoxy]piperidin-1-yl}carbonyl}propyl]carbamate (2.09 g, yield 76%) was obtained from t-butyl {(1S)-1-[(4-hydroxypiperidin-1-yl)carbonyl]-2-methylpropyl}carbamate (1.80 g, 6.00 mmol) and 4-(trifluoromethoxy)phenol (1.28 g, 7.20 mmol) as a colorless oil.
¹H-NMR (CDCl₃, 400 MHz) δ: 7.15 (2H, d, J=9.6 Hz), 6.90 (2H, dd, J=9.6 Hz, 2.4 Hz), 5.34 (1H, d, J=9.6 Hz), 4.51 (2H, m), 3.80-3.45 (4H, m), 2.00-1.80 (5H, m), 1.44 (9H, s), 0.97 (3H, d, J=6.4 Hz), 0.89 (3H, d, J=6.4 Hz).
MS (ESI, m/z): 461 (M+H)⁺.

### Example 18-2

### 3-Hydroxy-N-[(1S)-2-methyl-1-({4-[4-(trifluoromethoxy)phenoxy]piperidin-1-yl}carbonyl)propyl]quinoxaline-2-carboxamide

Analogously to Example 1-3, (2S)-3-methyl-1-oxo-1-{4-[4-(trifluoromethoxy)phenoxylpiperidin-1-yl}butan-2-amine hydrochloride was obtained from t-butyl [(1S)-2-methyl-1-({4-[4-(trifluoromethoxy)phenoxy]piperidin-1-yl}carbonyl)propyl]carbamate (2.09g, 4.54 mmol) as a white solid. The resulting compound (940 mg, 2.37 mmol) was condensed with 3-hydroxyquinoxaline-2-carboxylic acid (540 mg, 2.84 mmol) to afford the desired title compound (680 mg, yield 54%) as a pale yellow solid.
¹H-NMR (DMSO-d₆, 400 MHz) δ: 12.83 (1H, brs), 9.55 (1H, brs), 7.86 (1H, m), 7.61 (1H, m), 7.38 (2H, m), 7.28 (1H, m), 7.11 (3H, m), 4.90 (1H, dd, J=7.6 Hz, 7.1 Hz), 4.68 (1H, m), 3.99-3.80 (2H, m), 3.57-3.20 (2H, m), 2.05 (4H, m), 1.68 (1H, m), 0.94 (6H, d, J=6.4 Hz).
IR (KBr)cm⁻¹: 2960, 1690, 1640, 1505, 1240, 1160.
MS (ESI, m/z): 533 (M+H)⁺.
HRMS (ESI, m/z): 533.1998 (Calcd for C₂₆H₂₅F₃N₄O₅: 533.2012).
Anal. Calcd for C₂₆H₂₇F₃N₄O₅: C, 58.64; H, 5.11; N, 10.52. Found: C, 58.28; H, 5.12; N, 10.51.

### Example 19

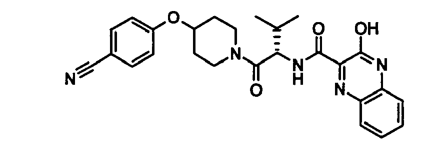

### N-[(1S)-1-{[4-(4-Cyanophenoxy)piperidin-1-yl]carbonyl}-2-methylpropyl]-3-hydroxyquinoxaline-2-carboxamide

### Example 19-1

### t-Butyl 4-(4-cyanophenoxy)piperidine-1-carboxylate

Analogously to Example 1-2, t-butyl 4-(4-cyanophenoxy)piperidine-1-carboxylate (2.30 g, yield 100%) was obtained from t-butyl 4-hydroxypiperidine-1-carboxylate (1.50 g, 7.45 mmol) and 4-cyanophenol (980 mg, 8.20 mmol) as a colorless oil. ¹H-NMR (CDCl₃, 400 MHz) δ: 7.60 (2H, d, J=9.6 Hz), 6.95 (2H, d, J=9.6 Hz), 4.55 (1H, m), 3.70-3.60 (2H, m), 3.40-3.32 (2H, m), 2.00-1.90 (2H, m), 1.80-1.70 (2H, m), 1.44 (9H, s).
MS (ESI, m/z): 303 (M+H)⁺.

### Example 19-2

### t-Butyl [(1S)-1-{[4-(4-cyanophenoxy)piperidin-1-yl]carbonyl}-2-methylpropyl]carbamate

Analogously to Example 2-2, 4-[(piperidin-4-yl)oxy]benzonitrile hydrochloride (1.43 g, yield 80%) was obtained from t-butyl 4-(4-cyanophenoxy)piperidine-1-carboxylate (2.25 g, 7.45 mmol) as a colorless oil. The resulting compound (1.43 g, 5.97 mmol) was condensed with N-(t-butoxycarbonyl)-L-valine (1.43 g, 6.56 mmol) to afford t-butyl [(1S)-1-{[4(4-cyanophenoxy)piperidin-1-yl]carbonyl}-2-methylpropyl]carbamate (2.28 g, yield 95%) as a colorless amorphous substance.
¹H-NMR (CDCl₃, 400 MHz) δ: 7.60 (2H, d, J=9.6 Hz), 6.95 (2H, d, J=9.6 Hz), 5.35 (1H, d, J=9.6 Hz), 4.65 (1H, m), 4.45 (1H, m), 3.90-3.50 (4H, m), 2.00-1.80 (5H, m), 1.44 (9H, s), 0.97 (3H, d, J=6.4 Hz), 0.89 (3H, d, J=6.4 Hz).
MS (ESI, m/z): 402 (M+H)⁺.

### Example 19-3

### N-[(1S)-1-{[4-(4-Cyanophenoxy)piperidin-1-yl]carbonyl}-2-methylpropyl]-3-hydroxyquinoxaline-2-carboxamide

Analogously to Example 1-3, 4-[(1-valylpiperidin-4-yl)oxy]benzonitrile hydrochloride (1.92 g, yield 100%) was obtained from t-butyl [(1S)-1-{[4-(4-cyanophenoxy)piperidin-1-yl]carbonyl}-2-methylpropyl]carbamate (2.28 g, 5.68 mmol) as a white solid. The resulting compound (301 mg, 1.00 mmol) was condensed with 3-hydroxyquinoxaline-2-carboxylic acid (228 mg, 1.20 mmol) to afford the desired title compound (255 mg, yield 54%) as a pale yellow solid. ¹H-NMR (DMSO-d₆, 400 MHz) δ: 12.82 (1H, brs), 9.48 (1H, brs), 7.86 (1H, t, J=7.2 Hz), 7.78 (2H, d, J=7.2 Hz), 7.64 (1H, t, J=5.6 Hz), 7.38 (2H, m), 7.18 (2H, m), 4.90 (1H, dd, J=12.4 Hz, 6.0 Hz), 4.84 (1H, m), 4.02-3.90 (2H, m), 3.57-3.20 (2H, m), 2.05 (4H, m), 1.55 (1H, m), 0.95 (6H, d, J=4.8 Hz).
LCMS (ESI, m/z): 474 (M+H)⁺.

### Example 20

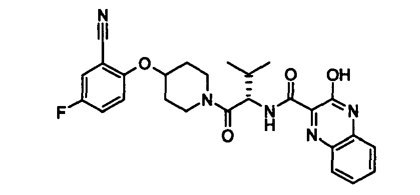

### N-[(1S)-1-{[4-(2-Cyano-4-fluorophenoxy)piperidin-1-yl]carbonyl}-2-methylpropyl]-3-hydroxyquinoxaline-2-carboxamide

### Example 20-1

### 2-Cyano-4-fluorophenol

Pyridine (3.00 ml, 37.5 mmol) was added to an ethanol solution (20 ml) of 5-fluorosalicylaldehyde (1.36 g, 9.71 mmol) and hydroxylamine hydrochloride (2.00 g, 29.1 mmol), at 0°C, under nitrogen stream, and stirring was carried out for 2.5 hours at room temperature. The reaction solution was poured into a 2N aqueous hydrochloric acid solution, followed by extraction with ethyl acetate and sequential washing with water and saline, and then the resulting organic layer was dried over anhydrous sodium sulfate. The organic layer was concentrated to afford 5-fluoro-2-hydroxybenzaldehyde oxime (1.47 g, yield 97%).

Anhydrous trifluoroacetic acid (3.93 ml, 28.4 mmol) was added to a methylene chloride solution (30 ml) of the resulting compound (1.47 g, 9.48 mmol) and triethylamine (3.96 ml, 28.4 mmol), at 0°C, under nitrogen stream, and stirring was carried out for 3 hours at room temperature. Further, potassium carbonate (1.96 g, 14.2 mmol) and methanol (10 ml) were added, and stirring was carried out for 10 minutes at room temperature. The reaction solution was concentrated, followed by pouring into water, extraction with ethyl acetate, and sequential washing with water and saline, and then the resulting organic layer was dried over anhydrous sodium sulfate. The organic layer was concentrated and the resulting residue was purified by silica gel column chromatography to afford 2-cyano-4-fluorophenol (637 mg, yield 49%).
¹H-NMR (CDCl₃, 400 MHz) δ: 7.24-7.18 (2H, m), 6.97 (1H, m), 5.15 (1H, brs).
MS (ESI, m/z): 138 (M+H)⁺.

### Example 20-2

### t-Butyl [(1S)-1-{[4-(2-cyano-4-fluorophenoxy)piperidin-1-yl]carbonyl}-2-methylpropyl]carbamate

Analogously to Example 1-2, t-butyl [(1S)-1-{[4-(2-cyano-4-fluorophenoxy)piperidin-1-yl]carbonyl}-2-methylpropyl]carbamate (1.33 g, yield 68%) was obtained from t-butyl {(1S)-1-[(4-hydroxypiperidin-1-yl)carbonyl]-2-methylpropyl}carbamate (1.40 g, 4.65 mmol) and 2-cyano-4-fluorophenol (637 mg, 4.65 mmol) as a colorless oil.
¹H-NMR (CDCl₃, 400 MHz) δ: 7.31-7.24 (2H, m), 6.96 (1H, dd, J=7.2 Hz, 3.2 Hz), 5.33 (1H, m), 4.68 (1H, m), 4.50 (1H, m), 4.02-3.50 (4H, m), 2.00-1.85 (5H, m), 1.44 (9H, s), 0.97 (3H, d, J=6.4 Hz), 0.89 (3H, d, J=6.4 Hz).
MS (ESI, m/z): 420 (M+H)⁺.

### Example 20-3

### N-[(1S)-1-{[4-(2-Cyano-4-fluorophenoxy)piperidin-1-yl]carbonyl}-2-methylpropyl]-3-hydroxyquinoxaline-2-carboxamide

Analogously to Example 1-3, 5-fluoro-2-[(1-L-valylpiperidin-4-yl)oxy]benzonitrile hydrochloride was obtained from t-butyl [(1S)-1-{[4-(2-cyano-4-fluorophenoxy)piperidin-1-yl]carbonyl}-2-methylpropyl]carbamate (910 mg, 2.17 mmol) as a white solid.

The resulting compound was condensed with 3-hydroxyquinoxaline-2-carboxylic acid (495 mg, 2.60 mmol) under nitrogen stream to afford the desired title compound (987 mg, yield 93%) as a pale yellow solid. ¹H-NMR (DMSO-d₆, 400 MHz) δ: 12.82 (1H, brs), 9.52 (1H, d, J=8.8 Hz), 7.86 (1H, d, J=8.0 Hz), 7.77 (1H, dt, J=5.2 Hz, 2.8 Hz), 7.75-7.55 (2H, m), 7.44-7.40 (3H, m), 4.90 (2H, m), 4.02-3.40 (4H, m), 2.08-1.59 (5H, m), 0.95 (6H, d, J=6.4 Hz).
IR (KBr)cm⁻¹: 2965, 2230, 1690, 1640, 1495, 1275, 1210.
MS (ESI, m/z): 492 (M+H)⁺.
HRMS (ESI, m/z): 514.1860 (Calcd for C₂₆H₂₆FN₅NaO₄: 514.1867). Anal. Calcd for C₂₆H₂₆FN₅O₄: C, 63.53; H, 5.33; N, 14.25. Found:: C, 63.53; H, 5.59; N, 14.29.

### Example 21

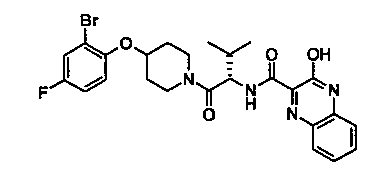

### N-[(1S)-1-{[4-(2-Bromo-4-fluorophenoxy)piperidin-1-yl]carbonyl}-2-methylpropyl]-3-hydroxyquinoxaline-2-carboxamide

### Example 21-1

### t-Butyl [(1S)-1-{[4-(2-bromo-4-fluorophenoxy)piperidin-1-yl]carbonyl}-2-methylpropyl]carbamate

Analogously to Example 2-1, t-butyl [(1S)-1-{[4-(2-bromo-4-fluorophenoxy)piperidin-1-yl]carbonyl}-2-methylpropyl]carbamate (997 mg, yield 83%) was obtained from 4-(2-bromo-4-fluorophenoxy)piperidine hydrochloride (786 mg, 2.53 mmol) and N-(t-butoxycarbonyl)-L-valine (550 mg, 2.53 mmol) as a colorless amorphous substance.
¹H-NMR (CDCl₃, 400 MHz) δ: 7.33-7.28 (1H, m), 7.01-6.95 (1H, m), 6.90-6.86 (1H, m), 5.35 (1H, d, J=9.0 Hz), 4.60-4.47 (2H, m), 4.01-3.50 (4H, m), 2.01-1.78 (5H, m), 1.44 (9H, s), 0.98 and 0.89 (6H, d, J=6.6 Hz).
MS (FAB, m/z): 473 (M+H)⁺.

### Example 21-2

### N-[(1S)-1-{[4-(2-Bromo-4-fluorophenoxy)piperidin-1-yl]carbonyl}-2-methylpropyl]-3-hydroxyquinoxaline-2-carboxamide

Analogously to Example 2-2, (2S)-1-[4-(2-bromo-4-fluorophenoxy)piperidin-1-yl]-3-methyl-1-oxobutan-2-amine hydrochloride (737 mg) was obtained from t-butyl [(1S)-1-{[4-(2-bromo-4-fluorophenoxy)piperidin-1-yl]carbonyl}-2-methylpropyl]carbamate (997 mg, 2.10 mmol). The resulting compound (737 mg, 1.80 mmol) was condensed with 3-hydroxyquinoxaline-2-carboxylic acid (353 mg, 1.80 mmol) to afford the desired title compound (609 mg, yield 62%) as a pale yellow solid.
¹H-NMR (DMSO-d₆, 400 MHz) δ: 12.83 (1H, brs), 9.50 (1H, d, J=9.0 Hz), 7.90-7.84 (1H, m), 7.69-7.62 (1H, m), 7.60-7.53 (1H, m), 7.41-7.35 (2H, m), 7.33-7.20 (2H, m), 4.91 (1H, m), 4.74 (1H, m), 3.92-3.41 (4H, m), 3.92-3.41 (5H, m), 0.96 and 0.95 (6H, d, J=6.2 Hz).
IR (KBr) cm⁻¹: 2960, 1690, 1630, 1530, 1485, 1190.
MS (ESI, m/z): 545 (M+H)⁺;
HRMS (ESI, m/z): 545.1210 (Calcd for C₂₅H₂₇BrFN₄O₄: 545.1200).

### Example 22

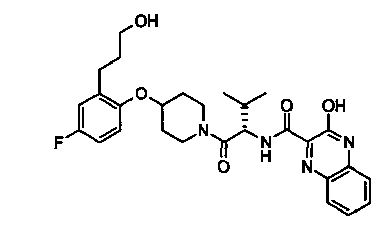

### N-[(1S)-1-({4-[4-Fluoro-2-(3-hydroxypropyl)phenoxy]piperidin-1-yl}carbonyl)-2-methylpropyl]-3-hydroxyquinoxaline-2-carboxamide

### Example 22-1

### t-Butyl [(1S)-1-({4-[4-fluoro-2-(3-hydroxy-1-propynyl)phenoxy]piperidin-1-yl}carbonyl)-2-methylpropyl]carbamate

Analogously to Example 1-2, t-butyl [(1S)-1-{[4-(2-bromo-4-fluorophenoxy)piperidin-1-yl]carbonyl}-2-methylpropyl]carbamate (1.58 g, yield 56%) was obtained from t-butyl {(1S)-1-[(4-hydroxypiperidin-1-yl)carbonyl]-2-methylpropyl}carbamate (1.80 g, 6.00 mmol) and 2-bromo-4-fluorophenol (1.38 g, 7.20 mmol) as a colorless oil.

The resulting compound (643 mg, 1.36 mmol) and 2-propyn-1-ol (107 mg, 1.90 mmol) were dissolved in a mixed solvent of N,N-dimethylformamide (3.0 ml) and triethylamine (3.0 ml), under nitrogen stream, and at room temperature bis(triphenylphosphine)palladium dichloride (98.0 mg, 0.140 mmol) and copper iodide (53.0 mg, 0.280 mmol) were added, followed by stirring at 70°C for 9 hours. The reaction solution was poured into water, followed by extraction with ethyl acetate and sequential washing with water and saline, and then the resulting organic layer was dried over anhydrous sodium sulfate. The organic layer was concentrated and the resulting residue was purified by silica gel column chromatography to afford t-butyl [(1S)-1-({4-[4-fluoro-2-(3-hydroxy-1-propynyl)phenoxy]piperidin-1-yl}carbonyl)-2-methylpropyl]carbamate (82.5 mg, yield 14%).
¹H-NMR (CDCl₃, 400 MHz) δ: 7.10 (1H, d, J=7.6 Hz), 6.99 (1H, m), 6.86 (1H, m), 5.33 (1H, m), 4.57 (1H, m), 4.50 and 4.35 (2H, d, J=4.8 Hz), 4.49 (1H, m), 3.90-3.50 (4H, m), 2.00-1.80 (5H, m), 1.44 (9H, s), 0.97 (3H, d, J=6.8 Hz), 0.89 (3H, d, J=6.8 Hz).
MS (ESI, m/z): 449 (M+H)⁺.

### Example 22-2

### t-Butyl [(1S)-1-({4-[4-fluoro-2-(3-hydroxypropyl)phenoxy]piperidin-1-yl}carbonyl)-2-methylpropyl]carbamate

The resulting compound (82.5 mg, 0.184 mmol) and 10% palladium carbon catalyst (14.2 mg) were suspended in methanol (4.0 ml), under hydrogen stream, and stirring was carried out at room temperature for 4 hours. The reaction solution was celite filtered, and the mother liquor was concentrated to afford t-butyl [(1S)-1-({4-[4-fluoro-2-(3-hydroxypropyl)phenoxy]piperidin-1-yl}carbonyl)-2-methylpropyl]carbamate (77.1 mg, yield 93%).
¹H-NMR (CDCl₃, 400 MHz) δ: 6.90 (1H, dd, J=7.2 Hz, 3.2 Hz), 6.84 (1H, m), 6.78 (1H, dd, J=8.4 Hz, 4.8 Hz), 5.34 (1H, d, J=9.2 Hz), 4.50 (2H, m), 3.90-3.50 (4H, m), 3.63 (2H, t, J=5.2 Hz), 2.71 (2H, q, J=8.0 Hz), 2.00-1.80 (5H, m), 1.85 (2H, q, J=8.0 Hz), 1.44 (9H, s), 0.97 (3H, d, J=6.8 Hz), 0.89 (3H, d, J=6.8 Hz).
MS (ESI, m/z): 453 (M+H)⁺.

### Example 22-3

### N-[(1S)-1-({4-[4-Fluoro-2-(3-hydroxypropyl)phenoxy]piperidin-1-yl}carbonyl)-2-methylpropyl]-3-hydroxyquinoxaline-2-carboxamide

Analogously to Example 1-3, 3-{5-fluoro-2-[(1-L-valylpiperidin-4-yl)oxy]phenyl}propan-1-ol hydrochloride was obtained from t-butyl [(1S)-1-({4-[4-fluoro-2-(3-hydroxypropyl)phenoxy]piperidin-1-yl}carbonyl)-2-methylpropyl]carbamate (75.6 mg, 0.167 mmol) as a white solid. The resulting compound was condensed with 3-hydroxyquinoxaline-2-carboxylic acid (38.0 mg, 0.200 mmol) to afford the desired title compound (44.6 mg, yield 51%) as a pale yellow solid.
¹H-NMR (DMSO-d₆, 400 MHz) δ: 12.82 (1H, brs), 9.56 (1H, brs), 7.85 (1H, t, J=8.0 Hz), 7.63 (1H, dt, J=8.0 Hz, 1.6 Hz), 7.37 (2H, m), 7.04-6.93 (3H, m), 4.90 (1H, t, J=7.6 Hz), 4.61 (1H, m), 3.90-3.40 (4H, m), 3.41 (2H, t, J=5.2 Hz), 2.67 (2H, q, J=8.0 Hz), 2.10 (1H, m), 2.00-1.55 (4H, m), 1.70 (2H, q, J=8.0 Hz), 0.97 (3H, d, J=6.4 Hz), 0.94 (3H, d, J=6.4 Hz).
LCMS (ESI, m/z): 525 (M+H)⁺.

### Example 23

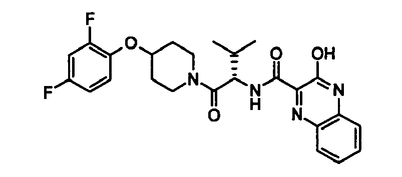

### N-[(1S)-1-{[4-(2,4-Difluorophenoxy)piperidin-1-yl]carbonyl}-2-methylpropyl]-3-hydroxyquinoxaline-2-carboxamide

### Example 23-1

### t-Butyl 4-(2,4-difluorophenoxy)piperidine-1-carboxylate

A 40% toluene solution of diisopropyl azodicarboxylate (4.71 ml, 8.94 mmol) was added to a toluene solution (30 ml) of t-butyl 4-hydroxypiperidine-1-carboxylate (1.50 g, 7.45 mmol), 2,4-difluorophenol (1.07 g, 8.20 mmol) and triphenylphosphine (2.34 g, 8.94 mmol), at room temperature, under nitrogen stream, and stirring was carried out at 90°C overnight. The reaction solution was concentrated and the resulting residue was purified by a medium-pressure preparative liquid chromatograph (manufactured by Biotage, Inc., 40+M) to afford t-butyl 4-(2,4-difluorophenoxy)piperidine-1-carboxylate (2.28 g, yield 98%) as a pale yellow oily substance. ¹H-NMR (CDCl₃, 400 MHz) δ: 6.99-6.75 (3H, m), 4.35-4.30 (1H, m), 3.77-3.71 (2H, m), 3.32-3.28 (2H, m), 1.92-1.88 (2H, m), 1.79-1.68 (2H, m), 1.53-1.38 (9H, m).
MS (FAB, m/z): 314 (M+H)⁺.

### Example 23-2

### t-Butyl [(1S)-1-{[4-(2,4-difluorophenoxy)piperidin-1-yl]carbonyl}-2-methylpropyl]carbamate

A 4N-hydrochloric acid 1,4-dioxane solution (30 ml) was added to a 1,4-dioxane solution (30 ml) of t-butyl 4-(2,4-difluorophenoxy)piperidine-1-carboxylate (2.28 g, 7.28 mmol), under nitrogen stream, and the mixture was allowed to react at room temperature overnight. The reaction solution was concentrated to afford 4-(2,4-difluorophenoxy)piperidine hydrochloride (1.63 g) as a white solid.

1-Ethyl-3-(3-dimethylaminopropyl)carbodiimide hydrochloride (1.50 g, 7.84 mmol) and 1-hydroxybenzotriazole monohydrate (1.06 g, 7.84 mmol) were added to a methylene chloride solution (30 ml) of the resulting compound (1.63 g, 6.53 mmol) and N-(t-butoxycarbonyl)-L-valine (1.56 g, 7.18 mmol), at room temperature, under nitrogen stream, followed by further addition of N-methylmorpholine (1.58 ml, 14.4 mmol), and stirring was carried out at room temperature overnight. The reaction solution was poured into a 2N aqueous hydrochloric acid solution, followed by extraction with methylene chloride and sequential washing with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford t-butyl [(1S)-1-{[4-(2,4-difluorophenoxy)piperidin-1-yl]carbonyl}-2-methylpropyl]carbamate (2.39 g, yield 89%) as a colorless amorphous substance.
¹H-NMR (CDCl₃, 500 MHz) δ: 7.00-6.99 (1H, m), 6.91-6.89 (1H, m), 6.83-6.81 (1H, m), 5.37-5.36 (1H, m), 4.52-4.45 (2H, m), 3.85-3.79 (2H, m), 3.64-3.45 (2H, m), 2.02-1.82 (5H, m), 1.51-1.48 (9H, m), 1.00 (3H, t, J=6.8 Hz), 0.92 (3H, t, J=6.8 Hz).
MS (FAB, m/z): 413 (M+H)⁺.

### Example 23-3

### N-[(1S)-1-{[4-(2,4-Difluorophenoxy)piperidin-1-yl]carbonyl}-2-methylpropyl]-3-hydroxyquinoxaline-2-carboxamide

A 4N-hydrochloric acid 1,4-dioxane solution (30 ml) was added to a 1,4-dioxane solution (30 ml) of t-butyl [(1S)-1-{[4-(2,4-difluorophenoxy)piperidin-1-yl]carbonyl}-2-methylpropyl]carbamate (2.39 g, 5.79 mmol), under nitrogen stream, and stirring was carried out at room temperature overnight. The reaction solution was concentrated to afford (2S)-1-[4-(2,4-difluorophenoxy)piperidin-1-yl]-3-methyl-1-oxobutan-2-amine hydrochloride (2.01 g) as a white solid.

1-Ethyl-3-(3-dimethylaminopropyl)carbodiimide hydrochloride (288 mg, 1.50 mmol) and 1-hydroxybenzotriazole monohydrate (203 mg, 1.50 mmol) were added to a methylene chloride solution (5.0 ml) of the resulting compound (350 mg, 1.00 mmol) and 3-hydroxyquinoxaline-2-carboxylic acid (285 mg, 1.50 mmol), at room temperature, under nitrogen stream, followed by further addition of triethylamine (0.558 ml, 4.00 mmol), and stirring was carried out at room temperature overnight. The reaction solution was diluted with methylene chloride, followed by sequential washing with a saturated aqueous sodium hydrogencarbonate solution, a saturated aqueous ammonium chloride solution, water and saline, and then the resulting organic layer was dried over anhydrous sodium sulfate. After the organic layer was concentrated and the resulting residue was purified by a medium-pressure preparative liquid chromatograph (manufactured by Biotage, Inc., 25+M), the residue resulting from concentration was suspended in a mixed solvent of methylene chloride-ethyl acetate, and the solid substance was collected by filtration to afford the desired title compound (283 mg, yield 58%) as a pale yellow solid.
¹H-NMR (DMSO-d₆, 400 MHz) δ: 12.83 (1H, s), 9.47 (1H, t, J=4.1 Hz), 7.86 (1H, d, J=8.6 Hz), 7.64 (1H, t, J=8.6 Hz), 7.40-7.27 (4H, m), 7.02 (1H, t, J=8.8 Hz), 4.90 (1H, q, J=8.1 Hz), 4.60 (1H, brs), 3.94-3.90 (2H, m), 3.57-3.22 (2H, m), 2.09-1.91 (3H, m), 1.73-1.52 (2H, m), 0.95 (6H, t, J=6.8 Hz).
IR (KBr) cm⁻¹: 2965, 1685, 1635, 1505, 1210.
MS (FAB, m/z): 485 (M+H)⁺.
HRMS (ESI, m/z): 485.2007 (Calcd for C₂₅H₂₇F₂N₄O₄: 485.2000).

### Example 24

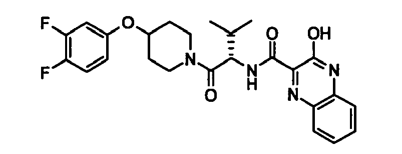

### N-[(1S)-1-{[4-(3,4-Difluorophenoxy)piperidin-1-yl]carbonyl}-2-methylpropyl]-3-hydroxyquinoxaline-2-carboxamide

### Example 24-1

### t-Butyl 4-(3,4-difluorophenoxy)piperidine-1-carboxylate

Analogously to Example 1-2, t-butyl 4-(3,4-difluorophenoxy)piperidine-1-carboxylate (1.69 g, yield 89%) was obtained from t-butyl 4-hydroxypiperidine-1-carboxylate (1.22 g, 6.07 mmol) and 3,4-difluorophenol (790 mg, 6.07 mmol) as a pale yellow oily substance.
¹H-NMR (CDCl₃, 500 MHz) δ: 7.05 (1H, q, J=9.3 Hz), 6.77-6.67 (1H, m), 6.62-6.59 (1H, m), 4.38-4.34 (1H, m), 3.71-3.66 (2H, m), 3.36-3.31 (2H, m), 1.92-1.88 (2H, m), 1.74-1.71 (2H, m), 1.49-1.44 (9H, m).
MS (FAB, m/z): 314 (M+H)⁺.

### Example 24-2

### t-Butyl [(1S)-1-{[4-(3,4-difluorophenoxy)piperidin-1-yl]carbonyl}-2-methylpropyl] carbamate

Analogously to Example 23-2, 4-(3,4-difluorophenoxy)piperidine hydrochloride (1.23 g) was obtained from t-butyl 4-(3,4-difluorophenoxy)piperidine-1-carboxylate (1.69 g, 5.39 mmol) as a white solid.

The resulting compound (1.23 g, 4.93 mmol) was condensed with N-(t-butoxycarbonyl)-L-valine (1.18 g, 5.42 mmol) under nitrogen stream to afford t-butyl [(1S)-1-{[4-(3,4-difluorophenoxy)piperidin-1-yl]carbonyl}-2-methylpropyl]carbamate (2.03 g, yield 100%) as a colorless amorphous substance.
¹H-NMR (CDCl₃, 400 MHz) δ: 7.07 (1H, dd, J=18.0 Hz, 9.4 Hz), 6.75-6.72 (1H, m), 6.64-6.59 (1H, m), 5.34 (1H, d, J=9.0 Hz), 4.51-4.43 (2H, m), 3.80-3.57 (4H, m), 1.97-1.79 (5H, m), 1.43-1.41 (9H, m), 0.97 (3H, d, J=7.0 Hz), 0.88 (3H, d, J=7.0 Hz).
MS (FAB, m/z): 413 (M+H)⁺.

### Example 24-3

### N-[(1S)-1-{[4-(3,4-Difluorophenoxy)piperidin-1-yl]carbonyl}-2-methylpropyl]-3-hydroxyquinoxaline-2-carboxamide

Analogously to Example 1-3, (2S)-1-[4-(3,4-difluorophenoxy)-piperidin-1-yl]-3-methyl-1-oxobutan-2-amine hydrochloride (1.73 g) was obtained from t-butyl [(1S)-1-{[4-(3,4-difluorophenoxy)piperidin-1-yl]carbonyl}-2-methylpropyl]carbamate (2.03 g, 4.92 mmol) as a white solid. The resulting compound (350 mg, 1.00 mmol) was condensed with 3-hydroxyquinoxaline-2-carboxylic acid (285 mg, 1.50 mmol) to afford the desired title compound (270 mg, yield 56%) as a pale yellow solid. ¹H-NMR (DMSO-d₆, 400 MHz) δ: 12.83 (1H, s), 9.48 (1H, dd, J=8.8 Hz, 4.9 Hz), 7.87-7.85 (1H, m), 7.66-7.62 (1H, m), 7.38-7.33 (3H, m), 7.21-7.14 (1H, m), 6.84 (1H, s), 4.90 (1H, dd, J=13.9 Hz, 6.8 Hz), 4.65-4.64 (1H, m), 3.95-3.90 (2H, m), 3.56-3.22 (2H, m), 2.02-1.96 (3H, m), 1.69-1.49 (2H, m), 0.95-0.94 (6H, m).
IR (KBr) cm⁻¹: 2965, 1685, 1630, 1515, 1215, 1160.
MS (FAB, m/z): 485 (M+H)⁺.
HRMS (ESI, m/z): 485.2022 (Calcd for C₂₅H₂₇F₂N₄O₄: 485.2000).

### Example 25

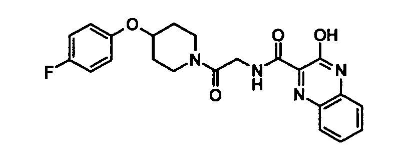

### N-{2-[4-(4-Fluorophenoxy)piperidin-1-yl]-2-oxoethyl}-3-hydroxyquinoxaline-2-carboxamide

### Example 25-1

### t-Butyl {2-[4-(4-fluorophenoxy)piperidin-1-yl]-2-oxoethyl}carbamate

Analogously to Example 2-1, t-butyl {2-[4-(4-fluorophenoxy)piperidin-1-yl]-2-oxoethyl}carbamate (273 mg, yield 77%) was obtained from 4-(4-fluorophenoxy)piperidine hydrochloride (231 mg, 1.00 mmol) and N-(t-butoxycarbonyl)glycine (175 mg, 1.00 mmol) as a colorless oil.
¹H-NMR (CDCl₃, 400 MHz) δ: 6.99 (2H, dd, J=9.0 Hz, 8.2 Hz), 6.87 (2H, dd, J=9.0 Hz, 4.2 Hz), 5.54 (1H, brs), 4.48 (1H, m), 3.99 (1H, d, J=4.6 Hz), 3.98 (1H, d, J=4.6 Hz), 3.73 (2H, t, J=5.8 Hz), 3.65-3.57 (1H, m), 3.38-3.30 (1H, m), 1.96-1.75 (4H, m), 1.96 (9H, s).
MS (ESI, m/z): 353 (M+H)⁺.

### Example 25-2

### N-[2-{[4-(4-Fluorophenoxy)piperidin-1-yl]carbonyl}-2-oxoethyl]-3-hydroxyquinoxaline-2-carboxamide

Analogously to Example 2-2, 2-[4-(4-fluorophenoxy)piperidin-1-yl]-2-oxoethanamine hydrochloride (190 mg, yield 85%) was obtained from t-butyl {2-[4-(4-fluorophenoxy)piperidin-1-yl]-2-oxoethyl}carbamate (273 mg, 0.773 mmol). From the resulting compound (144 mg, 0.500 mmol), through condensation with 3-hydroxyquinoxaline-2-carboxylic acid (95.1 mg, 0.500 mmol), a mixture containing the desired title compound was afforded. Repurification by preparative TLC afforded the desired title compound (28.5 mg, yield 13%) as a yellow solid.
¹H-NMR (DMSO-d₆, 400 MHz) δ: 12.85 (1H, brs), 9.79 (1H, brs), 7.88 (1H, d, J=8.2 Hz), 7.68-7.55 (1H, m), 7.41-7.30 (2H, m), 7.17-7.08 (2H, m), 7.07-6.98 (2H, m), 4.59 (1H, m), 4.28 (2H, d, J=4.6 Hz), 4.00-3.20 (4H, m), 2.04-1.85 (2H, m), 1.71-1.48 (2H, m).
IR (KBr) cm⁻¹: 2950, 1695, 1650, 1620, 1505, 1210.
MS (ESI, m/z): 425 (M+H)⁺.
HRMS (ESI, m/z): 425.1626 (Calcd for C₂₂H₂₂FN₄O₄: 425.1625).

### Example 26

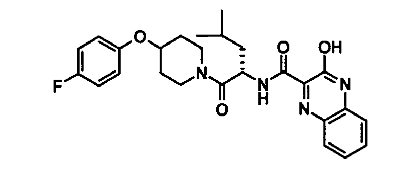

### N-[(1S)-1-{[4-(4-Fluorophenoxy)piperidin-1-yl]carbonyl}-3-methylbutyl]-3-hydroxyquinoxaline-2-carboxamide

### Example 26-1

### Benzyl [(1S)-1-{[4-(4-fluorophenoxy)piperidin-1-yl]carbonyl}-3-methylbutyl]carbamate

Analogously to Example 2-1, benzyl [(1S)-1-{[4-(4-fluorophenoxy)piperidin-1-yl]carbonyl}-3-methylbutyl]carbamate (755 mg, yield 85%) was obtained from 4-(4-fluorophenoxy)piperidine hydrochloride (463 mg, 2.00 mmol) and N-(benzyloxycarbonyl)-L-leucine (531 mg, 2.00 mmol) as a colorless oil.
¹H-NMR (CDCl₃, 400 MHz) δ: 7.38-7.30 (5H, m), 6.99 (2H, dd, J=9.0 Hz, 8.6 Hz), 6.87 (2H, dd, J=9.0 Hz, 4.2 Hz), 5.57 (1H, d, J=8.2 Hz), 5.10 (2H, s), 4.75 (1H, m), 4.47 (1H, m), 3.83-3.39 (4H, m), 2.00-1.64 (5H, m), 1.61-1.47 (1H, m), 1.44-1.34 (1H, m), 1.00 and 0.93 (6H, d, J=6.6 Hz).

### Example 26-2

### N-[(1S)-1-{[4-(4-Fluorophenoxy)piperidin-1-yl]carbonyl}-3-methylbutyl]-3-hydroxyquinoxaline-2-carboxamide

5% Palladium carbon catalyst (75.0 mg) was added to an ethanol solution (22.5 ml) of benzyl [(1S)-1-{[4-(4-fluorophenoxy)piperidin-1-yl]carbonyl}-3-methylbutyl]carbamate (755 mg, 1.70 mmol), under hydrogen stream, and stirring was carried out at room temperature for 2 hours. After the catalyst was celite filtered and washed with methylene chloride, the mother liquor was concentrated to afford (2S)-1-[4-(4-fluorophenoxy)piperidin-1-yl]-4-methyl-1-oxopentan-2-amine (521 mg) as a colorless oil.

1-Ethyl-3-(3-dimethylaminopropyl)carbodiimide hydrochloride (230 mg, 1.20 mmol) and 1-hydroxybenzotriazole monohydrate (184 mg, 1.20 mmol) were added to a methylene chloride solution (10 ml) of the resulting compound (308 mg, 1.00 mmol) and 3-hydroxyquinoxaline-2-carboxylic acid (190 mg, 1.00 mmol), at room temperature, under nitrogen stream, followed by further addition of N-methylmorpholine (0.550 ml, 5.00 mmol), and stirring was carried out at room temperature overnight. The reaction solution was diluted with methylene chloride, followed by sequential washing with a saturated aqueous sodium hydrogencarbonate solution, a saturated aqueous ammonium chloride solution, water and saline, and then the resulting organic layer was dried over anhydrous sodium sulfate. After the organic layer was concentrated and the resulting residue was purified by a medium-pressure preparative liquid chromatograph (manufactured by Biotage, Inc., 25+M), the residue resulting from concentration was suspended in a mixed solvent of methylene chloride-ethyl acetate, the solid substance was collected by filtration to afford the desired title compound (126 mg, yield 26%) as a pale yellow solid.
¹H-NMR (DMSO-d₆, 400 MHz) δ: 12.83 (1H, brs), 9.52 (1H, d, J=7.8 Hz), 7.86 (1H, dd, J=7.4 Hz, 7.0 Hz), 7.65 (1H, dd, J=7.8 Hz, 7.4 Hz), 7.40 (1H, d, J=7.0 Hz), 7.38 (1H, d, J=7.4 Hz), 7.17-7.09 (2H, m), 7.07-6.99 (2H, m), 5.08 (1H, m), 4.60 (1H, m), 4.02-3.18 (4H, m), 2.11-1.84 (2H, m), 1.76-1.44 (5H, m), 0.96 and 0.92 (6H, d, J=6.6 Hz).
IR (KBr) cm⁻¹: 2955, 1685, 1640, 1505, 1205.
MS (ESI, m/z): 481 (M+H)⁺.
HRMS (ESI, m/z): 481.2268 (Calcd for C₂₆H₃₀FN₄O₄: 481.2251).

### Example 27

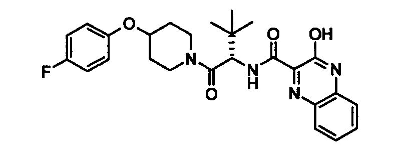

### N-[(1S)-1-{[4-(4-Fluorophenoxy)piperidin-1-yl]carbonyl}-2,2-dimethylpropyl]-3-hydroxyquinoxaline-2-carboxamide

### Example 27-1

### t-Butyl [(1S)-1-{[4-(4-fluorophenoxy)piperidin-1-yl]carbonyl}-2,2-dimethylpropyl]carbamate

Analogously to Example 2-1, t-butyl [(1S)-1-{[4-(4-fluorophenoxy)piperidin-1-yl]carbonyl}-2,2-dimethylpropyl]carbamate (619 mg, yield 76%) was obtained from 4-(4-fluorophenoxy)piperidine hydrochloride (463 mg, 2.00 mmol) and (2S)-N-(t-butoxycarbonyl)-2-amino-3,3-dimethylbutyric acid (462 mg, 2.00 mmol) as a colorless oil. ¹H-NMR (CDCl₃, 400 MHz) δ: 7.02-6.95 (2H, m), 6.95-6.89 (2H, m), 5.36 (1H, d, J=9.8 Hz), 4.56 (1H, d, J=9.8 Hz), 4.51-4.40 (1H, m), 3.93-3.47 (4H, m), 2.00-1.74 (4H, m), 1.43 (9H, s), 0.99 (9H, s). MS (ESI, m/z): 409 (M+H)⁺.

### Example 27-2

### N-[(1S)-1-{[4-(4-Fluorophenoxy)piperidin-1-yl]carbonyl}-2,2-dimethylpropyl]-3-hydroxyquinoxaline-2-carboxamide

Analogously to Example 2-2, (2S)-1-[4-(4-fluorophenoxy)piperidin-1-yl]-3,3-dimethyl-1-oxobutan-2-amine hydrochloride (522 mg, yield 100%) was obtained from t-butyl [(1S)-1-{[4-(4-fluorophenoxy)piperidin-1-yl]carbonyl}-2,2-dimethylpropyl]carbamate (619 mg, 1.52 mmol). From the resulting compound, through condensation with 3-hydroxyquinoxaline-2-carboxylic acid (190 mg, 1.00 mmol), the desired title compound (160 mg, yield 33%) was afforded as a pale yellow solid.
¹H-NMR (DMSO-d₆, 400 MHz) δ: 12.86 (1H, brs), 9.71 and 9.68 (1H, d, J=9.0 Hz), 7.89 (1H, d, J=8.2 Hz), 7.66 (1H, dd, J=8.2 Hz, 7.8 Hz), 7.44-7.37 (2H, m), 7.16-7.09 (2H, m), 7.07-6.99 (2H, m), 5.07 and 5.05 (1H, d, J=9.0 Hz), 4.61 (1H, m), 4.11-3.14 (4H, m), 2.07-1.84 (2H, m), 1.74-1.41 (2H, m), 1.03 (9H, s).
IR (KBr) cm⁻¹: 2950, 1690, 1630, 1505, 1205.
MS (ESI, m/z): 481 (M+H)⁺.
HRMS (ESI, m/z) : 481.2280 (Calcd for C₂₆H₃₀FN₄O₄: 481.2251).

### Example 28

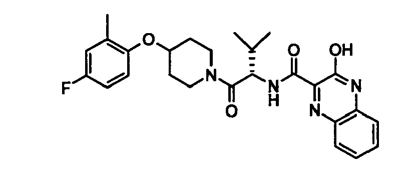

### N-[(1S)-1-{[4-(4-Fluoro-2-methylphenoxy)piperidin-1-yl]carbonyl}-2-methylpropyl]-3-hydroxyquinoxaline-2-carboxamide

### Example 28-1

### t-Butyl [(1S)-1-{[4-(4-fluoro-2-methylphenoxy)piperidin-1-yl]carbonyl}-2-methylpropyl]carbamate

Analogously to Example 1-2, t-butyl [(1S)-1-{[4-(4-fluoro-2-methylphenoxy)piperidin-1-yl]carbonyl}-2-methylpropyl]carbamate (480 mg, yield 60%) was obtained from t-butyl {(1S)-1-[(4-hydroxypiperidin-1-yl)carbonyl]-2-methylpropyl}carbamate (601 mg, 2.00 mmol) and 4-fluorocresol (277 mg, 2.20 mmol) as a colorless oil.
¹H-NMR (DMSO-d₆, 400 MHz) δ: 6.91-6.86 (1H, m), 6.85-6.78 (1H, m), 6.78-6.73 (1H, m), 5.36 (1H, d, J=9.4 Hz), 4.53-4.44 (2H, m), 3.88-3.45 (4H, m), 2.24 and 2.22 (3H, s), 2.02-1.74 (5H, m), 1.44 (9H, s), 0.98 and 0.90 (6H, d, J=7.0 Hz).
MS (FAB, m/z): 409 (M+H)⁺.

### Example 28-2

### N-[(1S)-1-{[4-(4-Fluoro-2-methylphenoxy)piperidin-1-yl]carbonyl}-2-methylpropyl]-3-hydroxyquinoxaline-2-carboxamide

Analogously to Example 1-3, (2S)-1-[4-(4-fluoro-2-methylphenoxy)piperidin-1-yl]-3-methyl-1-oxobutan-2-amine hydrochloride (388 mg, yield 96%) was obtained from t-butyl [(1S)-1-{[4-(4-fluoro-2-methylphenoxy)piperidin-1-yl]carbonyl}-2-methylpropyl]carbamate (480 mg, 1.18 mmol). The resulting compound (194mg, 0.564 mmol) was condensed with 3-hydroxyquinoxaline-2-carboxylic acid (110 mg, 0.564 mmol) to afford the desired title compound (89.5 mg, yield 33%) as a pale yellow solid.
¹H-NMR (DMSO-d₆, 400 MHz) δ: 12.82 (1H, brs), 9.52 (1H, brs), 7.90-7.82 (1H, m), 7.69-7.61 (1H, m), 7.43-7.36 (2H, m), 7.08-7.01 (2H, m), 7.00-6.92 (1H, m), 4.96 (1H, m), 4.60 (1H, m), 3.94-3.35 (4H, m), 2.19 and 2.16 (3H, s), 2.10-1.84 (3H, m), 1.76-1.64 (1H, m), 1.62-1.52 (1H, m), 0.98-0.92 (6H, m).
IR (KBr) cm⁻¹: 2965, 1690, 1630, 1495, 1205.
MS (ESI, m/z): 481 (M+H)⁺.
HRMS (ESI, m/z): 481.2255 (Calcd for C₂₆H₃₀FN₄O₄: 481.2251).

### Example 29

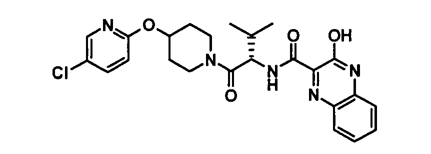

### N-[(1S)-1-({4-[(5-Chloropyridin-2-yl)oxy]piperidin-1-yl}carbonyl)-2-methylpropyl]-3-hydroxyquinoxaline-2-carboxamide

### Example 29-1

### t-Butyl 4-[(5-chloropyridin-2-yl)oxy]piperidine-1-carboxylate

t-Butyl 4-(methanesulfonyloxy)piperidine-1-carboxylate (3.02 g, 10.8 mmol) and potassium carbonate (2.35 g, 17.0 mmol) were added to a N,N-dimethylformamide solution (77 ml) of 5-chloro-2-hydroxypyridine (1.00 g, 7.72 mmol), at room temperature, and stirring was carried out at 75°C overnight. After the reaction solution was cooled to room temperature, water was added to the residue resulting from concentration, followed by extraction with methylene chloride. The resulting organic layer was dried over anhydrous sodium sulfate, and the residue resulting from concentration was purified by silica gel column chromatography to afford t-butyl 4-[(5-chloropyridin-2-yl)oxy]piperidine-1-carboxylate (1.57 g, yield 65%) as a colorless oil.
¹H-NMR (CDCl₃, 400 MHz) δ: 8.06 (1H, d, J=2.7 Hz), 7.51 (1H, dd, J=8.8 Hz, 2.7 Hz), 6.67 (1H, d, J=8.8 Hz), 5.18-5.13 (1H, m), 3.79-3.73 (2H, m), 3.31-3.24 (2H, m), 1.99-1.93 (2H, m), 1.74-1.66 (2H, m), 1.47 (9H, s) .

### Example 29-2

### t-Butyl [(1S)-1-({4-[(5-chloropyridin-2-yl)oxy]piperidin-1-yl}carbonyl)-2-methylpropyl]carbamate

Concentrated hydrochloric acid (5.0 ml) was added to an ethanol solution (10 ml) of t-butyl 4-[(5-chloropyridin-2-yl)oxy]piperidine-1-carboxylate (1.57 g, 5.02 mmol), at 0°C, and stirring was carried out at room temperature for 2 days. The reaction solution was concentrated to afford 5-chloro-2-[(piperidin-4-yl)oxy]pyridine dihydrochloride (1.61 g).
1-Ethyl-3-(3-dimethylaminopropyl)carbodiimide hydrochloride (1.44 g, 7.53 mmol) was added to a methylene chloride solution (50 ml) of the resulting compound (1.61 g), N-(t-butoxycarbonyl)-L-valine (1.09 g, 5.02 mmol), 1-hydroxybenzotriazole monohydrate (814 mg, 6.02 mmol) and N-methylmorpholine (3.31 ml, 30.1 mmol), at room temperature, and stirring was carried out at room temperature overnight. Water was added to the reaction solution, followed by extraction with methylene chloride and subsequent sequential washing with a saturated aqueous sodium hydrogencarbonate solution, water and saline, and then the resulting organic layer was dried over anhydrous sodium sulfate. The organic layer was concentrated and the resulting residue was purified by silica gel column chromatography to afford t-butyl [(1S)-1-({4-[(5-chloropyridin-2-yl)oxy]piperidin-1-yl}carbonyl)-2-methylpropyl]carbamate (1.94 g, yield 94%) as a colorless amorphous substance.
¹H-NMR (CDCl₃, 400 MHz) δ: 8.08-8.06 (1H, m), 7.53 (1H, dd, J=8.8 Hz, 2.8 Hz), 6.68 (1H, dd, J=8.8 Hz, 1.2 Hz), 5.35 (1H, d, J=8.8 Hz), 5.28-5.21 (1H, m), 4.50 (1H, dd, J=8.8 Hz, 5.7 Hz), 3.98-3.43 (4H, m), 2.09-1.73 (5H, m), 1.44 (9H, s), 0.97 (3H, d, J=6.6 Hz), 0.89 (3H, d, J=6.6 Hz).

### Example 29-3

### (2S)-1-{4-[(5-Chloropyridin-2-yl)oxy]piperidin-1-yl}-3-methyl-1-oxobutan-2-amine dihydrochloride

Concentrated hydrochloric acid (5.0 ml) was added to an ethanol solution (10 ml) of t-butyl [(1S)-1-({4-[(5-chloropyridin-2-yl)oxy]piperidin-1-yl}carbonyl)-2-methylpropyl]carbamate (1.94 g, 4.71 mmol), at 0°C, and stirring was carried out at room temperature overnight. The reaction solution was concentrated to afford (2S)-1-{4-[(5-chloropyridin-2-yl)oxy]piperidin-1-yl}-3-methyl-1-oxobutan-2-amine dihydrochloride (1.93 g, yield 100%) as a colorless amorphous substance.
¹H-NMR (CDCl₃, 400 MHz) δ: 8.43 (2H, brs), 8.13-8.12 (1H, m), 7.69 (1H, brs), 6.92 (1H, brs), 5.36 (1H, brs), 4.51 (1H, brs), 3.88-3.46 (4H, m), 2.28-1.86 (5H, m), 1.20-1.11 (6H, m).
MS (ESI, m/z): 312 (M+H)⁺.

### Example 29-4

### N-[(1S)-1-({4-[(5-Chloropyridin-2-yl)oxy]piperidin-1-yl}carbonyl)-2-methylpropyl]-3-hydroxyquinoxaline-2-carboxamide

1-Ethyl-3-(3-dimethylaminopropyl)carbodiimide hydrochloride (150 mg, 0.780 mmol), was added to a methylene chloride solution (5.2 ml) of (2S)-1-{4-[(5-chloropyridin-2-yl)oxy]piperidin-1-yl}-3-methyl-1-oxobutan-2-amine dihydrochloride (200 mg, 0.520 mmol), 3-hydroxyquinoxaline-2-carboxylic acid (102 mg, 0.520 mmol), 1-hydroxybenzotriazole monohydrate (84.3 mg, 0.620 mmol) and N-methylmorpholine (0.343 ml, 3.12 mmol), at room temperature, and stirring was carried out at room temperature for 3 days. Water was added to the reaction solution, followed by extraction with methylene chloride and subsequent sequential washing with a saturated aqueous sodium hydrogencarbonate solution, water and saline, and then the resulting organic layer was dried over anhydrous sodium sulfate. After the organic layer was concentrated and the resulting residue was purified by silica gel column chromatography, the residue resulting from concentration was suspended in a mixed solvent of ethanol-diethyl ether, and the solid substance was collected by filtration to afford the desired title compound (190 mg, yield 76%) as a yellow solid. ¹H-NMR (CDCl₃, 400 MHz) δ: 12.44 (1H, brs), 10.15 (1H, brs), 8.08 (1H, dd, J=6.3 Hz, 2.7 Hz), 8.02-8.00 (1H, m), 7.61-7.37 (4H, m), 6.70 (1H, t, J=9.2 Hz), 5.32-5.27 (1H, m), 5.09 (1H, t, J=7.4 Hz), 4.06-3.59 (4H, m), 2.34-1.82 (5H, m), 1.14-1.10 (6H, m).
IR (KBr) cm⁻¹: 2965, 1690, 1630, 1525, 1465.
MS (ESI, m/z): 484 (M+H)⁺.
HRMS (ESI, m/z): 484.1765 (Calcd for C₂₄H₂₇ClN₅O₄: 484.1752).
Anal. Calcd for C₂₄H₂₆ClN₅O₄: C, 59.56; H, 5.42; N, 14.47; Cl, 7.33. Found: C, 59.48; H, 5.50; N, 14.49; Cl, 7.16.

### Example 30

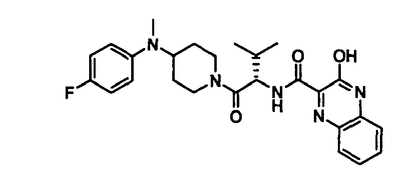

### N-[(1S)-1-({4-[(4-Fluorophenyl)(methyl)amino]piperidin-1-yl}carbonyl)-2-methylpropyl]-3-hydroxyquinoxaline-2-carboxamide

### Example 30-1

### t-Butyl 4-[(4-fluorophenyl)amino]piperidine-1-carboxylate

Sodium cyanoborohydride (661 mg, 10.0 mmol) was added to a methylene chloride solution (30 ml) of t-butyl 4-oxopiperidine-1-carboxylate (2.66 g, 13.1 mmol), 4-fluoroaniline (1.46 g, 13.1 mmol) and acetic acid (0.750 ml, 13.1 mmol), at 0°C, and stirring was carried out at room temperature for 15 hours. A saturated aqueous sodium hydrogencarbonate solution was added to the reaction solution, followed by extraction with ethyl acetate, and the extract was washed sequentially with water and saline, and dried over anhydrous sodium sulfate. The organic layer was concentrated and the resulting residue was washed with diisopropyl ether, followed by drying to afford t-butyl 4-[(4-fluorophenyl)amino]piperidine-1-carboxylate (3.09 g, yield 80%) as a white solid.
¹H-NMR (CDCl₃, 400 MHz) δ: 6.91-6.84 (2H, m), 6.56-6.51 (2H, m), 4.12-3.97 (2H, m), 3.36-3.32 (2H, m),2.95-2.87 (1H, m), 2.06-1.98 (2H, m), 1.47 (9H, s), 1.36-1.24 (2H, m).

### Example 30-2

### N-(4-Fluorophenyl)-N-methylpiperidin-4-amine dihydrochloride

Sodium cyanoborohydride (641 mg, 10.2 mmol) was added to an acetic acid solution (14 ml) of t-butyl 4-[(4-fluorophenyl)amino]piperidine-1-carboxylate (1.50 g, 5.10 mmol) and formaldehyde (2.07 ml, 25.5 mmol), at 0°C, and stirring was carried out at room temperature for 31 hours. The reaction solution was concentrated under reduced pressure, and a saturated aqueous sodium hydrogencarbonate solution was added to the residue, followed by extraction with ethyl acetate. The extract was washed sequentially with water and saline, and dried over anhydrous sodium sulfate. After the organic layer was concentrated and the resulting residue was purified by a medium-pressure preparative liquid chromatograph (manufactured by Yamazen Corporation, W-Prep 2XY), the residue resulting from concentration was dissolved in ethanol (24 ml), and then concentrated hydrochloric acid (10 ml) was added under ice cooling, followed by stirring at room temperature for 12 hours. The reaction solution was concentrated under reduced pressure to afford N-(4-fluorophenyl)-N-methylpiperidin-4-amine dihydrochloride (1.28 g, yield 89%) as an amorphous substance.
¹H-NMR (CDCl₃, 400 MHz) δ: 7.84-7.79 (2H, m), 7.43-7.37 (2H, m), 4.14-4.05 (1H, m), 3.61-3.49 (2H, m), 3.33 (3H, s), 3.17-3.04 (2H, m), 2.38-1.94 (4H, m).

### Example 30-3

### 1-[(2S)-2-Amino-3-methylbutanoyl]-N-(4-fluorophenyl)-N-methylpiperidin-4-amine dihydrochloride

1-Ethyl-3-(3-dimethylaminopropyl)carbodiimide hydrochloride (364 mg, 1.90 mmol) was added to a methylene chloride solution (30 ml) of N-(4-fluorophenyl)-N-methylpiperidin-4-amine dihydrochloride (445 mg, 1.58 mmol), N-(t-butoxycarbonyl)-L-valine (343 mg, 1.48 mmol), 1-hydroxybenzotriazole monohydrate (257 mg, 1.90 mmol) and N-methylmorpholine (0.556 ml, 5.06 mmol), at room temperature, and stirring was carried out under room temperature for 4 hours. The reaction solution was concentrated under reduced pressure, and water was added to the residue, followed by extraction with ethyl acetate. The extract was washed sequentially with water, a saturated aqueous sodium hydrogencarbonate solution and saturated saline, and then the resulting organic layer was dried over anhydrous sodium sulfate. The organic layer was concentrated and the resulting residue was purified by a medium-pressure preparative liquid chromatograph (manufactured by Yamazen Corporation, W-Prep 2XY), and the resulting residue was dissolved in ethanol (10 ml). To this was added concentrated hydrochloric acid (5.0 ml) under ice cooling, and stirring was carried out at room temperature for 12 hours. The reaction solution was concentrated under reduced pressure to afford the desired title compound (528 mg, yield 88%) as an amorphous substance.
¹H-NMR (CD₃OD, 400 MHz) δ: 7.84-7.72 (2H, m), 7.42-7.30 (2H, m), 4.74-4.63 (1H, m), 4.42-4.29 (1H, m), 4.16-3.08 (2H, m), 3.28 (3H, s), 3.27-3.18 (1H, m), 2.80-2.69 (1H, m), 2.26-1.60 (5H, m), 1.12-0.98 (6H, m).

### Example 30-4

### N-[(1S)-1-({4-[(4-Fluorophenyl)(methyl)amino]piperidin-1-yl}carbonyl)-2-methylpropyl]-3-hydroxyquinoxaline-2-carboxamide

1-Ethyl-3-(3-dimethylaminopropyl)carbodiimide hydrochloride (113 mg, 0.590 mmol) was added to a methylene chloride solution (5.0 ml) of 1-[(2S)-2-amino-3-methylbutanoyl]-N-(4-fluorophenyl)-N-methylpiperidin-4-amine dihydrochloride (150 mg, 0.390 mmol), 3-hydroxyquinoxaline-2-carboxylic acid (77.3 mg, 0.390 mmol), 1-hydroxybenzotriazole monohydrate (64.0 mg, 0.470 mmol) and N-methylmorpholine (0.217 ml, 1.97 mmol), at room temperature, and stirring was carried out at room temperature overnight. Water was added to the reaction solution, followed by extraction with methylene chloride and subsequent sequential washing with a saturated aqueous sodium hydrogencarbonate solution, water and saline, and the resulting organic layer was dried over anhydrous sodium sulfate. After the organic layer was concentrated and the resulting residue was purified by silica gel column chromatography, the residue resulting from concentration was suspended in a mixed solvent of ethanol-diethyl ether, and the solid substance was collected by filtration to afford the desired title compound (135 mg, yield 71%) as a yellow solid. ¹H-NMR (CDCl₃, 400 MHz) δ: 12.20 (1H, brs), 10.16 (1H, brs), 8.06-8.02 (1H, m), 7.64-7.40 (3H, m), 6.99-6.93 (2H, m), 6.80 (2H, dq, J=16.0 Hz, 3.6 Hz), 5.14-5.06 (1H, m), 4.84 (1H, s), 4.35 (1H, s), 3.67 (1H, s), 3.29-3.18 (1H, m), 2.75-2.69 (4H, m), 2.27 (1H, m), 1.96-1.62 (4H, m), 1.10 (6H, dd, J=13.2 Hz, 5.2 Hz).
IR (KBr) cm⁻¹: 2960, 1685, 1640, 1510.
MS (ESI, m/z): 480 (M+H)⁺.
HRMS (ESI, m/z): 480.2414 (Calcd for C₂₆H₃₁FN₅O₃: 480.2411).
Anal. Calcd for C₂₆H₃₀FN₅O₃: C, 65.12; H, 6.31; N, 14.60; F, 3.96. Found: C, 64.97; H, 6.32; N, 14.34; F, 3.94.

### Example 31

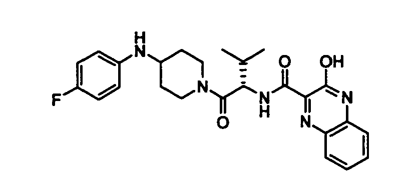

### N-[(1S)-1-({4-[(4-Fluorophenyl)amino]piperidin-1-yl}carbonyl]-2-methylpropyl]-3-hydroxyquinoxaline-2-carboxamide

Concentrated hydrochloric acid (5.0 ml) was added to an ethanol solution (10 ml) of t-butyl 4-[(4-fluorophenyl)amino]piperidine-1-carboxylate (1.00 g, 3.40 mmol), under ice cooling, and stirring was carried out at room temperature for 12 hours. The reaction solution was concentrated under reduced pressure, and the resulting residue was washed with diethyl ether and n-hexane to afford 1-[(2S)-2-amino-3-methylbutanoyl]-N-(4-fluorophenyl)piperidin-4-amine dihydrochloride (768 mg, yield 84%) as an orange oil.

1-Ethyl-3-(3-dimethylaminopropyl)carbodiimide hydrochloride (118 mg, 0.610 mmol) was added to a methylene chloride solution (5.0 ml) of 1-[(2S)-2-amino-3-methylbutanoyl]-N-(4-fluorophenyl)piperidin-4-amine dihydrochloride (150 mg, 0.410 mmol), 3-hydroxyquinoxaline-2-carboxylic acid (80.3 mg, 0.410 mmol), 1-hydroxybenzotriazole monohydrate (66.4 mg, 0.490 mmol) and N-methylmorpholine (0.225 ml, 2.05 mmol), at room temperature, and stirring was carried out at room temperature overnight. Water was added to the reaction solution, followed by extraction with methylene chloride and subsequent sequential washing with a saturated aqueous sodium hydrogencarbonate solution, water and saline, and the resulting organic layer was dried over anhydrous sodium sulfate. After the organic layer was concentrated and the resulting residue was purified by silica gel column chromatography, the residue resulting from concentration was suspended in a mixed solvent of ethanol-diethyl ether, and the solid substance was collected by filtration to afford the desired title compound (121 mg, yield 64%) as a yellow solid.
¹H-NMR (CDCl₃, 400 MHz) δ: 12.19 (1H, brs), 10.11 (1H, brs), 8.00 (1H, m), 7.62 (1H, m), 7.44-7.26 (2H, m), 6.90 (2H, q, J=7.2 Hz), 6.59-6.54 (2H, m), 5.08-5.05 (1H, m), 4.67-4.51 (1H, m), 4.24 (1H, brs), 3.51-3.30 (3H, m), 3.07-2.90 (1H, m), 2.32-2.12 (3H, m), 1.48-1.35 (2H, m), 1.13-1.09 (6H, m).
IR (KBr) cm⁻¹: 2960, 1685, 1630, 1510, 1215.
MS (FAB, m/z): 466 (M+H)⁺.
HRMS (FAB, m/z): 466.2242 (Calcd for C₂₅H₂₉FN₅O₃: 466.2255).
Anal. Calcd for C₂₅H₂₈FN₅O₃: C, 64.50; H, 6.06; N, 15.04; F, 4.08. Found: C, 64.18; H, 5.77; N, 14.93; F, 4.02.

### Example 32

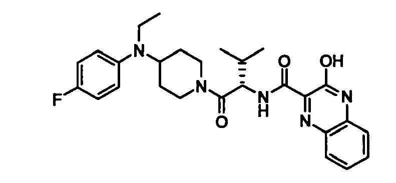

### N-[(1S)-1-({4-[(Ethyl)(4-fluorophenyl)amino]piperidin-1-yl}carbonyl)-2-methylpropyl]-3-hydroxyquinoxaline-2-carboxamide

### Example 32-1

### t-Butyl 4-[(ethyl)(4-fluorophenyl)amino]piperidine-1-carboxylate

Sodium cyanoborohydride (661 mg, 10.0 mmol) was added to an acetonitrile solution (5.0 ml) of t-butyl 4-[(4-fluorophenyl)amino]piperidine-1-carboxylate (1.47 g, 5.00 mmol), acetaldehyde (0.559 ml, 5.00 mmol) and acetic acid (0.0570 ml, 1.00 mmol), at 0°C, and stirring was carried out at room temperature for 3 hours. Further acetic acid (2.0 ml) was added, and stirring was carried out for 30 minutes. A saturated aqueous sodium hydrogencarbonate solution was added to the reaction solution, followed by extraction with ethyl acetate and subsequent sequential washing with water and saline, and then the resulting organic layer was dried over anhydrous sodium sulfate. The organic layer was concentrated and the resulting residue was purified by silica gel column chromatography to afford t-butyl 4-[(ethyl)(4-fluorophenyl)amino]piperidine-1-carboxylate (1.27 g, yield 79%) as a colorless oil.
¹H-NMR (CDCl₃, 400 MHz) δ: 6.94 (2H, t, J=8.8 Hz), 6.74 (2H, dd, J=8.8 Hz, 4.5 Hz), 4.20 (2H, brs), 3.48 (1H, tt, J=11.6 Hz, 3.6 Hz), 3.18 (2H, q, J=7.0 Hz), 2.78-2.72 (2H, m), 1.78 (2H, d, J=12.1 Hz), 1.62-1.46 (2H, m), 1.46 (9H, s), 1.08 (3H, t, J=7.0 Hz).
MS (ESI, m/z): 323 (M+H)⁺.

### Example 32-2

### t-Butyl [(1S)-1-({4-[(ethyl)(4-fluorophenyl)amino]piperidin-1-yl}carbonyl)-2-methylpropyl]carbamate

Analogously to Example 29-2, t-butyl [(1S)-1-({4-[(ethyl)(4-fluorophenyl)amino]piperidin-1-yl}carbonyl)-2-methylpropyl]carbamate (1.36 g, yield 82%) was obtained from t-butyl 4-[(ethyl)(4-fluorophenyl)amino]piperidine-1-carboxylate (1.27 g, 3.94 mmol) as a colorless amorphous substance.
¹H-NMR (CDCl₃, 400 MHz) δ: 6.97-6.92 (2H, m), 6.80-6.73 (2H, m), 5.34 (1H, d, J=6.6 Hz), 4.68 (1H, t, J=13.2 Hz), 4.50-4.47 (1H, m), 4.08-4.00 (1H, m), 3.59-3.51 (1H, m), 3.17-3.07 (3H, m), 2.68-2.61 (1H, m), 1.93-1.84 (3H, m), 1.68-1.43 (11H, m), 1.08-1.05 (3H, m), 0.97 (3H, dd, J=12.4 Hz, 5.2 Hz), 0.89 (3H, dd, J=8.4 Hz, 5.2 Hz).

### Example 32-3

### 1-[(2S)-2-Amino-3-methylbutanoyl]-N-ethyl-N-(4-fluorophenyl)piperidin-4-amine dihydrochloride

Analogously to Example 29-3, 1-[(2S)-2-amino-3-methylbutanoyl]-N-ethyl-N-(4-fluorophenyl)piperidin-4-amine dihydrochloride (1.37 g, yield 100%) was obtained from t-butyl [(1S)-1-({4-[ethyl(4-fluorophenyl)amino]piperidin-1-yl}carbonyl)-2-methylpropyl]carbamate (1.36 g, 3.23 mmol) as a colorless amorphous substance.
¹H-NMR (CD₃OD, 400 MHz) δ: 7.78-7.72 (2H, m), 7.44-7.39 (2H, m), 4.69-3.13 (8H, m), 2.74-1.73 (5H, m), 1.12-1.06 (6H, m), 0.99 (3H, t, J=7.6 Hz).
MS (ESI, m/z): 322 (M+H)⁺.

### Example 32-4

### N-[(1S)-1-({4-[Ethyl(4-fluorophenyl)amino]piperidin-1-yl}carbonyl)-2-methylpropyl]-3-hydroxyquinoxaline-2-carboxamide

Analogously to Example 29-4, the desired title compound (179 mg, yield 72%) was obtained from 1-[(2S)-2-amino-3-methylbutanoyl]-N-ethyl-N-(4-fluorophenyl)piperidin-4-amine dihydrochloride (200 mg, 0.510 mmol) as a yellow solid.
¹H-NMR (CDCl₃, 400 MHz) δ: 12.33 (1H, brs), 10.14 (1H, brs), 8.04 (1H, t, J=9.2 Hz), 7.65-7.26 (3H, m), 6.99-6.91 (2H, m), 6.84-6.74 (2H, m), 5.14-5.06 (1H, m), 4.82-4.74 (1H, m), 4.32 (1H, brs), 3.64-3.55 (1H, m), 3.24-3.12 (3H, m), 2.78-2.71 (1H, m), 2.28 (1H, brs), 2.03-1.55 (4H, m), 1.11-1.03 (9H, m).
IR (KBr) cm⁻¹: 2965, 1690, 1635, 1510, 1450.
MS (ESI, m/z): 494 (M+H)⁺, 516 (M+Na)⁺.
HRMS (ESI, m/z): 516.2399 (Calcd for C₂₇H₃₂FN₅NaO₃₁ 516.2387).
Anal. Calcd for C₂₇H₃₂FN₅O₃: C, 65.70; H, 6.53; N, 14.19; F, 3.85. Found: C, 65.34; H, 6.48; N, 14.02; F, 3.79.

### Example 33

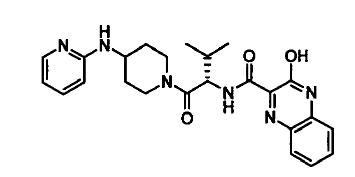

### N-[(1S)-2-Methyl-1-{[4-(pyridin-2-ylamino)piperidin-1-yl]carbonyl}propyl]-3-hydroxyquinoxaline-2-carboxamide

### Example 33-1

### t-Butyl [(1S)-2-methyl-1-({4-[(pyridin-2-ylamino)piperidin-1-yl]carbonyl}propyl]carbamate

Sodium triacetoxy borohydride (1.41 g, 6.31 mmol) was added to a methylene chloride solution (53 ml) of 2-aminopyridine (500 mg, 5.26 mmol), t-butyl 4-oxopiperidine-1-carboxylate (1.06 g, 5.26 mmol) and acetic acid (0.301 ml, 5.26 mmol), at room temperature, and stirring was carried out at room temperature overnight. A saturated aqueous sodium hydrogencarbonate solution was added to the reaction solution, followed by extraction with methylene chloride. The extract was concentrated and the resulting residue was purified by silica gel column chromatography to afford t-butyl 4-[(pyridin-2-ylamino)piperidine-1-carboxylate (335 mg).

Concentrated hydrochloric acid (3.5 ml) was added to an ethanol solution (7.0 ml) of the resulting compound (335 mg), at 0°C, and stirring was carried out at room temperature for 7 hours. The reaction solution was concentrated to afford N-(piperidin-4-yl)-2-aminopyridine trihydrochloride.

1-Ethyl-3-(3-dimethylaminopropyl)carbodiimide hydrochloride (347 mg, 1.81 mmol) was added to a methylene chloride solution (12 ml) of the resulting compound, N-(t-butoxycarbonyl)-L-valine (262 mg, 1.21 mmol), 1-hydroxybenzotriazole monohydrate (196 mg, 1.45 mmol) and N-methylmorpholine (0.797 ml, 7.25 mmol), at room temperature, and stirring was carried out at room temperature overnight. Water was added to the reaction solution, followed by extraction with methylene chloride and subsequent sequential washing with a saturated aqueous sodium hydrogencarbonate solution, water and saline, and then the resulting organic layer was dried over anhydrous sodium sulfate. The organic layer was concentrated and the resulting residue was purified by silica gel column chromatography to afford t-butyl [(1S)-2-methyl-1-{[4-(pyridin-2-ylamino)piperidin-1-yl]carbonyl)propyl]carbamate (316 mg, yield 16%) as a colorless amorphous substance.
¹H-NMR (CDCl₃, 400 MHz) δ: 8.09 (1H, d, J=5.1 Hz), 7.43-7.38 (1H, m), 6.60-6.56 (1H, m), 6.37 (1H, d, J=7.8 Hz), 5.36-5.34 (1H, m), 4.54-4.45 (2H, m), 4.32-4.18 (1H, m), 3.99-3.91 (2H, m), 3.32-3.21 (1H, m), 2.98-2.86 (1H, m), 2.23-2.08 (2H, m), 1.96-1.89 (1H, m), 1.44 (9H, s), 1.42-1.24 (2H, m), 0.99-0.87 (6H, m).
MS (ESI, m/z): 377 (M+H)⁺.

### Example 33-2

### N-[(1S)-2-Methyl-1-{[4-(pyridin-2-ylamino)piperidin-1-yl]carbonyl}propyl]-3-hydroxyquinoxaline-2-carboxamide

Concentrated hydrochloric acid (3.0 ml) was added to an ethanol solution (5.0 ml) of t-butyl [(1S)-2-methyl-1-({4-[(pyridin-2-yl)amino]piperidin-1-yl}carbonyl)propyl]carbamate (316 mg, 0.840 mmol), at 0°C, and stirring was carried out at room temperature overnight. The organic layer was concentrated to afford N-{1-[(2S)-2-amino-3-methylbutanoyl]piperidin-4-yl}pyridin-2-amine trihydrochloride as a colorless amorphous substance.

1-Ethyl-3-(3-dimethylaminopropyl)carbodiimide hydrochloride (241 mg, 1.26 mmol)was added to a methylene chloride solution (10 ml) of the resulting compound, 3-hydroxyquinoxaline-2-carboxylic acid (165 mg, 0.840 mmol), 1-hydroxybenzotriazole monohydrate (136 mg, 1.01 mmol) and N-methylmorpholine (0.646 ml, 5.88 mmol), at room temperature, and stirring was carried out at room temperature overnight. Water was added to the reaction solution, followed by extraction with methylene chloride and subsequent sequential washing with a saturated aqueous sodium hydrogencarbonate solution, water and saline, and the resulting organic layer was dried over anhydrous sodium sulfate. After the organic layer was concentrated and the resulting residue was purified by silica gel column chromatography, the residue resulting from concentration was suspended in a mixed solvent of ethanol-diethyl ether, and the solid substance was collected by filtration to afford the desired title compound (248 mg, yield 66%) as a yellow solid.
¹H-NMR (CDCl₃, 400 MHz) δ: 10.11-9.94 (2H, m), 8.12-8.10 (1H, m), 8.01 (1H, d, J=8.0 Hz), 7.64-7.37 (4H, m), 6.60 (1H, q, J=6.2 Hz), 6.43-6.39 (1H, m), 5.12-4.52 (3H, m), 4.23-4.17 (1H, m), 4.00-3.94 (1H, m), 3.49-3.32 (1H, m), 3.12-3.00 (1H, m), 2.36-2.17 (3H, m), 1.68-1.44 (2H, m), 1.12-1.08 (6H, m).
IR (KBr) cm⁻¹: 2960, 1685, 1635, 1605, 1520.
MS (ESI, m/z): 449 (M+H)⁺.
HRMS (ESI, m/z): 449.2299 (Calcd for C₂₄H₂₉N₆O₃: 449.2301).
Anal. Calcd for C₂₄H₂₈N₆O₃-0.5H₂O: C, 63.00; H, 6.39; N, 18.37. Found: C, 63.17; H, 6.48; N, 18.44.

### Example 34

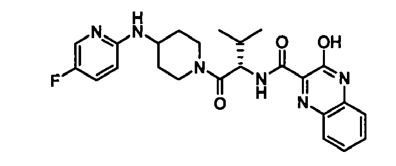

### N-[(1S)-1-({4-[(5-Fluoropyridin-2-yl)amino]piperidin-1-yl}carbonyl)-2-methylpropyl]-3-hydroxyquinoxaline-2-carboxamide

### Example 34-1

### t-Butyl 4-[(5-fluoropyridin-2-yl)amino]piperidine-1-carboxylate

Sodium triacetoxy borohydride (4.78 g, 21.4 mmol) was added to a methylene chloride solution (90 ml) of 2-amino-5-fluoropyridine (2.00 g, 17.8 mmol), t-butyl 4-oxopiperidine-1-carboxylate (3.59 g, 17.8 mmol) and acetic acid (1.02 ml, 17.8 mmol), at 0°C, and stirring was carried out at room temperature for 4 days. A saturated aqueous sodium hydrogencarbonate solution was added to the reaction solution, followed by extraction with methylene chloride. The organic layer was concentrated to afford t-butyl 4-[(5-fluoropyridin-2-yl)amino]piperidine-1-carboxylate (2.53 g, yield 48%) as a white solid.
¹H-NMR (CDCl₃, 400 MHz) δ: 7.94 (1H, d, J=2.4 Hz), 7.18 (1H, td, J=6.8 Hz, 2.4 Hz), 6.32 (1H, dd, J=6.8 Hz, 2.8 Hz), 4.22 (1H, d, J=6.3 Hz), 4.05 (2H, brs), 3.77-3.72 (1H, m), 2.96-2.92 (2H, m), 2.04-2.01 (2H, m), 1.46 (9H, s), 1.38-1.31 (2H, m).
MS (ESI, m/z): 196 (M-Boc+H)⁺.

### Example 34-2

### t-Butyl [(1S)-1-({4-[(5-fluoropyridin-2-yl)amino]piperidin-1-yl}carbonyl)-2-methylpropyl]carbamate

Analogously to Example 29-2, t-butyl [(1S)-1-({4-[(5-fluoropyridin-2-yl)amino]piperidin-1-yl}carbonyl)-2-methylpropyl]carbamate (1.29 g, yield 97%) was obtained from t-butyl 4-[(5-fluoropyridin-2-yl)amino]piperidine-1-carboxylate (1.00 g, 3.39 mmol) as a colorless amorphous substance.
¹H-NMR (CDCl₃, 400 MHz) δ: 7.95-7.94 (1H, m), 7.21-7.16 (1H, m), 6.34-6.32 (1H, m), 5.36-5.34 (1H, m), 4.54-4.46 (2H, m), 4.22 (1H, dd, J=12.8 Hz, 6.2 Hz), 3.99-3.87 (2H, m), 3.30-3.20 (1H, m), 2.96-2.84 (1H, m), 2.21-2.07 (2H, m), 1.96-1.90 (1H, m), 1.44 (9H, s), 1.43-1.31 (2H, m), 0.98-0.87 (6H, m).
MS (ESI, m/z): 295 (M-Boc+H)⁺.

### Example 34-3

### N-{1-[(2S)-2-Amino-3-methylbutanoyl]piperidin-4-yl}-5-fluoropyridin-2-amine trihydrochloride

Analogously to Example 29-3, N-{1-[(2S)-2-amino-3-methylbutanoyl]piperidin-4-yl}-5-fluoropyridin-2-amine trihydrochloride (1.25 g, yield 95%) was obtained from t-butyl [(1S)-1-({4-[(5-fluoropyridin-2-yl)amino]piperidin-1-yl}carbonyl)-2-methylpropyl]carbamate (1.29 g, 3.27 mmol) as a colorless amorphous substance.
¹H-NMR (CD₃OD, 400 MHz) δ: 7.96-7.93 (2H, m), 7.17-7.13 (1H, m), 4.60-4.35 (2H, m), 4.08-3.90 (2H, m), 3.44-3.25 (1H, m), 3.12-2.94 (1H, m), 2.24-2.09 (3H, m), 1.71-1.48 (2H, m), 1.12 (3H, dd, J=10.2 Hz, 6.8 Hz), 1.03 (3H, dd, J=12.0 Hz, 6.8 Hz).
MS (ESI, m/z): 295 (M+H)⁺.

### Example 34-4

### N-[(1S)-1-({4-[(5-Fluoropyridin-2-yl)amino]piperidin-1-yl}carbonyl)-2-methylpropyl]-3-hydroxyquinoxaline-2-carboxamide

Analogously to Example 29-4, the desired title compound (173 mg, yield 75%) was obtained from N-{1-[(2S)-2-amino-3-methylbutanoyl]piperidin-4-yl}-5-fluoropyridin-2-amine trihydrochloride (200 mg, 0.510 mmol) as a yellow solid.
¹H-NMR (CDCl₃, 400 MHz) δ: 12.59 (1H, brs), 10.10 (1H, brs), 8.01-7.95 (2H, m), 7.63-7.39 (3H, m), 7.24-7.17 (1H, m), 6.36 (1H, td, J=7.6 Hz, 2.8 Hz), 5.08 (1H, t, J=5.6 Hz), 4.68-4.35 (2H, m), 4.23 (1H, d, J=10.6 Hz), 4.01-3.90 (1H, m), 3.47-3.32 (1H, m), 3.08-2.93 (1H, m), 2.36-2.14 (3H, m), 1.52-1.39 (2H, m), 1.12-1.09 (6H, m).
IR (KBr) cm⁻¹: 2965, 1685, 1635, 1525, 1495.
MS (ESI, m/z): 467 (M+H)⁺, 489 (M+Na)⁺.
HRMS (ESI, m/z): 489.2013 (Calcd for C₂₄H₂₇FN₆NaO₃: 489.2026).
Anal. Calcd for C₂₄H₂₇FN₆O₃·0.25H₂O: C, 61.20; H, 5.88; N, 17.84; F, 4.03. Found: C, 61.24; H, 5.68; N, 17.86; F, 4.04.

### Example 35

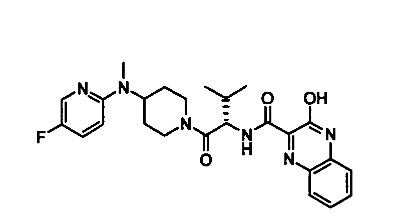

### N-[(1S)-1-({4-[(5-Fluoropyridin-2-yl)(methyl)amino]piperidin-1-yl}carbonyl)-2-methylpropyl]-3-hydroxyquinoxaline-2-carboxamide

### Example 35-1

### t-Butyl 4-[N-(5-fluoropyridin-2-yl)(methyl)amino]piperidine-1-carboxylate

Sodium cyanoborohydride (685 mg, 10.4 mmol) was added to an acetonitrile solution (52 ml) of t-butyl 4-[(5-fluoropyridin-2-yl)amino]piperidine-1-carboxylate (1.53 g, 5.18 mmol), formaldehyde (1.93 ml, 25.9 mmol) and acetic acid (0.297 ml, 5.18 mmol), at 0°C, and stirring was carried out at room temperature for 4 hours. Further acetic acid (1.0 ml) was added, and stirring was carried out overnight. Ethanol (26 ml) was added to the reaction solution, and stirring was carried out overnight. A saturated aqueous sodium hydrogencarbonate solution was added to the reaction solution, and the organic layer was concentrated, followed by extraction with methylene chloride. The resulting organic layer was dried over anhydrous sodium sulfate. The organic layer was concentrated and the resulting residue was purified by silica gel column chromatography to afford t-butyl 4-[N-(5-fluoropyridin-2-yl)(methyl)amino]piperidine-1-carboxylate (332 mg, yield 22%) as a white solid.
¹H-NMR (CDCl₃, 400 MHz) δ: 8.01 (1H, d, J=2.4 Hz), 7.25-7.21 (1H, m), 6.43 (1H, dd, J=7.2 Hz, 2.4 Hz), 4.65-4.59 (1H, m), 4.24 (2H, brs), 2.86-2.81 (2H, m), 2.81 (3H, s), 1.66-1.60 (4H, m), 1.48 (9H, s).
MS (ESI, m/z): 210 (M-Boc+H)⁺.

### Example 35-2

### t-Butyl [(1S)-1-({4-[(5-fluoropyridin-2-yl)(methyl)amino]piperidin-1-yl}carbonyl)-2-methylpropyl]carbamate

Analogously to Example 29-2, t-butyl [(1S)-1-({4-[(5-fluoropyridin-2-yl)(methyl)amino]piperidin-1-yl}carbonyl)-2-methylpropyl]carbamate (368 mg, yield 84%) was obtained from t-butyl 4-[(5-fluoropyridin-2-yl)(methyl)amino]piperidine-1-carboxylate (332 mg, 1.07 mmol) as a colorless amorphous substance.
¹H-NMR (CDCl₃, 400 MHz) δ: 8.01 (1H, dd, J=4.4 Hz, 2.4 Hz), 7.26-7.22 (1H, m), 6.45-6.41 (1H, m), 5.37 (1H, d, J=7.4 Hz), 4.85-4.74 (2H, m), 4.53-4.50 (1H, m), 4.08-4.05 (1H, m), 3.25-3.17 (1H, m), 2.79 (3H, d, J=9.0 Hz), 2.70 (1H, t, J=10.4 Hz), 1.98-1.90 (1H, m), 1.84-1.58 (4H, m), 1.45-1.44 (9H, m), 1.02-0.88 (6H, m).
MS (ESI, m/z): 309 (M-Boc+H)⁺.

### Example 35-3

### N-{1-[(2S)-2-Amino-3-methylbutanoyl]piperidin-4-yl}-5-fluoro-N-methylpyridin-2-amine trihydrochloride

Analogously to Example 29-3, N-{1-[(2S)-2-amino-3-methylbutanoyl]piperidin-4-yl}-5-fluoro-N-methylpyridin-2-amine trihydrochloride (370 mg, yield 98%) was obtained from t-butyl [(1S)-1-({4-[(5-fluoropyridin-2-yl)(methyl)amino]piperidin-1-yl}carbonyl)-2-methylpropyl]carbamate (368 mg, 0.900 mmol) as a colorless amorphous substance.
¹H-NMR (CD₃OD, 400 MHz) δ: 8.08-8.03 (2H, m), 7.56-7.53 (1H, m), 4.71 (1H, d, J=14.0 Hz), 4.42-4.35 (2H, m), 4.14-4.08 (1H, m), 3.43-3.34 (1H, m), 3.11 (3H, s), 2.95-2.87 (1H, m), 2.27-2.15 (1H, m), 2.06-1.79 (4H, m), 1.17-1.02 (6H, m).
MS (ESI, m/z): 309 (M+H)⁺.

### Example 35-4

### N-[(1S)-1-({4-[(5-Fluoropyridin-2-yl)(methyl)amino]piperidin-1-yl}carbonyl)-2-methylpropyl]-3-hydroxyquinoxaline-2-carboxamide

Analogously to Example 29-4, the desired title compound (171 mg, yield 82%) was obtained from N-{1-[(2S)-2-amino-3-methylbutanoyl]piperidin-4-yl}-5-fluoro-N-methylpyridin-2-amine trihydrochloride (181 mg, 0.430 mmol), as a yellow solid.
¹H-NMR (CDCl₃, 400 MHz) δ: 12.59 (1H, brs), 10.21 (1H, brs), 8.05-8.01 (2H, m), 7.61-7.45 (3H, m), 7.28-7.23 (1H, m), 6.48-6.42 (1H, m), 5.17-5.09 (1H, m), 4.89-4.80 (2H, m), 4.44-4.33 (1H, m), 3.37-3.28 (1H, m), 2.86-2.78 (4H, m), 2.39-2.29 (1H, m), 1.92-1.63 (4H, m), 1.12 (6H, dd, J=16.2 Hz, 5.3 Hz).
IR (KBr) cm⁻¹: 3460, 2960, 1685, 1640, 1490.
MS (ESI, m/z): 481 (M+H)⁺.
HRMS (ESI, m/z): 503.2200 (Calcd for C₂₅H₂₉FN₆NaO₃: 503.2183).
Anal. Calcd for C₂₅H₂₉FN₆O₃: C, 62.49; H, 6.08; N, 17.49; F, 3.95. Found: C, 62.10; H, 6.25; N, 17.19; F, 4.00.

### Example 36

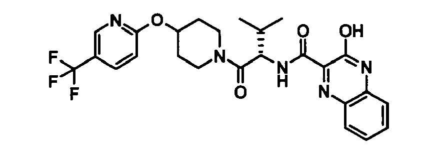

### 3-Hydroxy-N-{(1S)-2-methyl-1-[(4-{[5-(trifluoromethyl)pyridin-2-yl]oxy}piperidin-1-yl)carbonyl]propyl}quinoxaline-2-carboxamide

### Example 36-1

### t-Butyl 4-{[5-(trifluoromethyl)pyridin-2-yl]oxy}piperidine-1-carboxylate

Analogously to Example 29-1, t-butyl 4-{[5-(trifluoromethyl)pyridin-2-yl]oxy}piperidine-1-carboxylate (1.26 g, yield 61%) was obtained from 5-trifluoromethyl-2-pyridinol (1.00 g, 5.95 mmol) as a white solid.
¹H-NMR (CDCl₃, 400 MHz) δ: 8.41 (1H, m), 7.76 (1H, dd, J=8.6 Hz, 2.4 Hz), 6.79 (1H, d, J=8.6 Hz), 5.32-5.26 (1H, m), 3.81-3.75 (2H, m), 3.33-3.26 (2H, m), 2.02-1.96 (2H, m), 1.78-1.69 (2H, m), 1.48 (9H, s).

### Example 36-2

### t-Butyl {(1S)-2-methyl-1-[(4-{[5-(trifluoromethyl)pyridin-2-yl]oxy}piperidin-1-yl)carbonyl]propyl}carbamate

Analogously to Example 29-2, t-butyl {(1S)-2-methyl-1-[(4-{[5-(trifluoromethyl)pyridin-2-yl]oxy}piperidin-1-yl)carbonyl]propyl}carbamate (1.57 g, yield 97%) was obtained from t-butyl 4-{[5-(trifluoromethyl)pyridin-2-yl]oxy}piperidine-1-carboxylate (1.26 g, 3.64 mmol) as a colorless amorphous substance.
¹H-NMR (CDCl₃, 400 MHz) δ: 8.41 (1H, s), 7.78 (1H, dd, J=9.0 Hz, 2.4 Hz), 6.80 (1H, d, J=8.6 Hz), 5.39-5.30 (2H, m), 4.51 (1H, dd, J=9.0 Hz, 5.6 Hz), 4.01-3.44 (4H, m), 2.12-1.74 (5H, m), 1.44 (9H, s), 0.98 (3H, d, J=6.8 Hz), 0.90 (3H, d, J=6.8 Hz).
MS (ESI, m/z): 446 (M+H)⁺.

### Example 36-3

### (2S)-3-Methyl-1-oxo-1-(4-{[5-(trifluoromethyl)pyridin-2-yl]oxy}piperidin-1-yl)butan-2-amine dihydrochloride

Analogously to Example 29-3, (2S)-3-methyl-1-oxo-1-(4-{[5-(trifluoromethyl)pyridin-2-yl]oxy}piperidin-1-yl)butan-2-amine dihydrochloride (1.51 g, yield 100%) was obtained from t-butyl {(1S)-2-methyl-1-[(4-{[5-(trifluoromethyl)pyridin-2-yl]oxy}piperidin-1-yl)carbonyl]propyl}carbamate (1.57 g, 3.52 mmol) as a colorless amorphous substance.
¹H-NMR (CDCl₃, 400 MHz) δ: 8.44 (5H, brs), 7.90 (1H, brs), 7.00 (1H, brs), 5.46 (1H, brs), 4.54 (1H, brs), 4.13-3.48 (4H, m), 2.31-1.89 (5H, m), 1.28-1.14 (6H, m).
MS (ESI, m/z): 346 (M+H)⁺.

### Example 36-4

### 3-Hydroxy-N-{(1S)-2-methyl-1-[(4-{[5-(trifluoromethyl)pyridin-2-yl]oxy}piperidin-1-yl)carbonyl]propyl}quinoxaline-2-carboxamide

Analogously to Example 29-4, the desired title compound (214 mg, yield 87%) was obtained from (2S)-3-methyl-1-oxo-1-(4-{[5-(trifluoromethyl)pyridin-2-yl]oxy}piperidin-1-yl)butan-2-amine dihydrochloride (200 mg, 0.480 mmol) as a yellow solid.
¹H-NMR (CDCl₃, 400 MHz) δ: 12.12 (1H, brs), 10.12 (1H, brs), 8.44-8.42 (1H, m), 8.04-8.01 (1H, m), 7.81-7.37 (4H, m), 6.82 (1H, t, J=9.2 Hz), 5.45-5.39 (1H, m), 5.11-5.08 (1H, m), 4.08-3.57 (4H, m), 2.34-1.89 (5H, m), 1.11 (6H, d, J=7.0 Hz).
IR (KBr) cm⁻¹: 2965, 1690, 1630, 1615, 1525, 1325.
MS (ESI, m/z): 518 (M+H)⁺.
HRMS (ESI, m/z): 518.1993 (Calcd for C₂₅H₂₇F₃N₅O₄: 518.2015).
Anal. Calcd for C₂₅H₂₆F₃N₅O₄: C, 58.02; H, 5.06; N, 13.53; F, 11.01. Found: C, 57.80; H, 5.00; N, 13.51; F, 11.01.

### Example 37

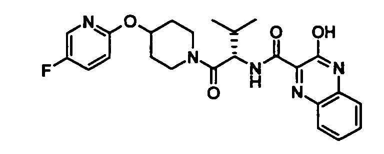

### N-[(1S)-1-({4-[(5-Fluoropyridin-2-yl)oxy]piperidin-1-yl}carbonyl)-2-methylpropyl]-3-hydroxyquinoxaline-2-carboxamide

### Example 37-1

### t-Butyl 4-{(5-fluoropyridin-2-yl)oxy}piperidine-1-carboxylate

Analogously to Example 29-1, t-butyl 4-[(5-fluoropyridin-2-yl)oxy]piperidine-1-carboxylate (1.46 g, yield 48%) was obtained from 5-fluoro-2-hydroxypyridine (1.00 g, 8.84 mmol) as a white solid. ¹H-NMR (CDCl₃, 400 MHz) δ: 7.95 (1H, d, J=3.2 Hz), 7.35-7.30 (1H, m), 6.68 (1H, dd, J=9.2 Hz, 3.6 Hz), 5.16-5.10 (1H, m), 3.80-3.74 (2H, m), 3.30-3.24 (2H, m), 1.99-1.94 (2H, m), 1.74-1.66 (2H, m), 1.47 (9H, s). MS (ESI, m/z): 241 (M-tBu+H)⁺.

### Example 37-2

### t-Butyl [(1S)-1-({4-[(5-fluoropyridin-2-yl)oxy]piperidin-1-yl}carbonyl)-2-methylpropyl]carbamate

Analogously to Example 29-2, t-butyl [(1S)-1-({4-[(5-fluoropyridin-2-yl)oxylpiperidin-1-yl}carbonyl)-2-methylpropyl]carbamate (1.92 g, yield 99%) was obtained from t-butyl 4-[(5-fluoropyridin-2-yl)oxy]piperidine-1-carboxylate (1.46 g, 4.93 mmol) as a colorless amorphous substance.
¹H-NMR (CDCl₃, 400 MHz) δ: 7.97-7.95 (1H, m), 7.34 (1H, ddd, J=9.0 Hz, 7.4 Hz, 3.1 Hz), 6.69 (1H, ddd, J=9.0 Hz, 3.1 Hz, 2.0 Hz), 5.36 (1H, d, J=9.0 Hz), 5.24-5.19 (1H, m), 4.51 (1H, dd, J=8.6 Hz, 5.6 Hz), 3.98-3.42 (4H, m), 2.09-1.71 (5H, m), 1.44 (9H, s), 0.98 (3H, d, J=6.6 Hz), 0.89 (3H, d, J=6.6 Hz).
MS (ESI, m/z): 396 (M+H)⁺.

### Example 37-3

### (2S)-1-{4-[(5-Fluoropyridin-2-yl)oxy]piperidin-1-yl}-3-methyl-1-oxobutan-2-amine dihydrochloride

Analogously to Example 29-3, (2S)-1-{4-[(5-fluoropyridin-2-yl)oxy]piperidin-1-yl}-3-methyl-1-oxobutan-2-amine dihydrochloride (1.93 g, yield 100%) was obtained from t-butyl [(1S)-1-({4-[(5-fluoropyridin-2-yl)oxy]piperidin-1-yl}carbonyl)-2-methylpropyl]carbamate (1.92 g, 4.85 mmol) as a colorless amorphous substance.
¹H-NMR (CDCl₃, 400 MHz) δ: 8.42 (4H, brs), 7.99 (1H, brs), 7.46 (1H, brs), 7.24 (1H, brs), 5.33 (1H, brs), 4.48 (1H, brs), 4.09-3.41 (4H, m), 2.26-1.83 (5H, m), 1.18-1.10 (6H, m).
MS (ESI, m/z): 296 (M+H)⁺.

### Example 37-4

### N-[(1S)-1-({4-[(5-Fluoropyridin-2-yl)oxy]piperidin-1-yl}carbonyl)-2-methylpropyl]-3-hydroxyquinoxaline-2-carboxamide

Analogously to Example 29-4, the desired title compound (200 mg, yield 79%) was obtained from (2S)-1-{4-[(5-fluoropyridin-2-yl)oxy]piperidin-1-yl}-3-methyl-1-oxobutan-2-amine dihydrochloride (200 mg, 0.540 mmol) as a yellow solid.
¹H-NMR (CDCl₃, 400 MHz) δ: 12.32 (1H, brs), 10.13 (1H, brs), 8.02-7,96 (2H, m), 7.62-7.32 (4H, m), 6.71 (1H, td, J=9.2 Hz, 3.6 Hz), 5.29-5.24 (1H, m), 5.11-5.08 (1H, m), 4.04-3.59 (4H, m), 2.33-1.85 (5H, m), 1.11 (6H, d, J=6.6 Hz).
IR (KBr) cm⁻¹: 2965, 1685, 1640, 1530, 1480.
MS (ESI, m/z): 468 (M+H)⁺.
HRMS (ESI, m/z): 468.2042 (Calcd for C₂₄H₂₇FN₅O₄: 468.2047).

### Example 38

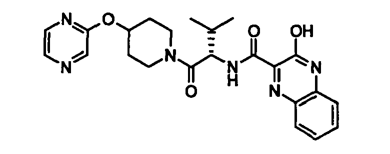

### 3-Hydroxy-N-[(1S)-2-methyl-1-({4-[(pyrazin-2-yl)oxy]piperidin-1-yl}carbonyl)propyl]quinoxaline-2-carboxamide

### Example 38-1

### t-Butyl 4-[pyrazin-2-yl)oxy]piperidine-1-carboxylate

t-Butyl 4-hydroxypiperidine-1-carboxylate (3.73 g, 18.0 mmol) was added to a N,N-dimethylformamide suspension (10 ml) of 60% sodium hydride (533 mg, 14.0 mmol), at 0°C, and stirring was carried out at room temperature for 30 minutes. Chloropyrazine (1.15 g, 10.0 mmol) was further added at room temperature, and stirring was carried out for 1.5 hours. Water was added to the reaction solution, followed by extraction with ethyl acetate and subsequent sequential washing with water and saline, and the resulting organic layer was dried over anhydrous sodium sulfate. The organic layer was concentrated and the resulting residue was purified by silica gel column chromatography to afford t-butyl 4-[(pyrazin-2-yl)oxy]piperidine-1-carboxylate (2.79 g, yield 100%) as a white solid.
¹H-NMR (CDCl₃, 400 MHz) δ: 8.20 (1H, s), 8.10 (1H, d, J=2.8 Hz), 8.05 (1H, dd, J=2.8 Hz, 1.2 Hz), 5.24-5.18 (1H, m), 3.82-3.76 (2H, m), 3.33-3.27 (2H, m), 2.02-1.97 (2H, m), 1.79-1.70 (2H, m), 1.48 (9H, s). MS (ESI, m/z): 224 (M-tBu+H)⁺.

### Example 38-2

### 2-[(Piperidin-4-yl)oxy]pyrazine dihydrochloride

Concentrated hydrochloric acid (10 ml) was added to an ethanol solution (20 ml) of t-butyl 4-[(pyrazin-2-yl)oxy]piperidine-1-carboxylate (2.79 g, 10.0 mmol), at 0°C, and stirring was carried out under room temperature overnight. The organic layer was concentrated to afford 2-[(piperidin-4-yl)oxy]pyrazine dihydrochloride (2.50 g, yield 99%) as a white solid.
¹H-NMR (CD₃OD, 400 MHz) δ: 8.27-8.17 (3H, m), 5.39 (1H, brs), 3.48-3.23 (4H, m), 2.24 (2H, s), 2.12 (2H, s).
MS (ESI, m/z): 180 (M+H)⁺.

### Example 38-3

### t-Butyl [(1S)-2-methyl-1-({4-[(pyrazin-2-yl)oxy]piperidin-1-yl}carbonyl)propyl]carbamate

1-Ethyl-3-(3-dimethylaminopropyl)carbodiimide hydrochloride (1.44 g, 7.50 mmol) was added to a methylene chloride solution (50 ml) of 2-[(piperidin-4-yl)oxy]pyrazine dihydrochloride (1.26 g, 5.00 mmol), N-(t-butoxycarbonyl)-L-valine (1.09 g, 5.00 mmol), 1-hydroxybenzotriazole monohydrate (811 mg, 6.00 mmol) and N-methylmorpholine (3.30 ml, 30.0 mmol), at room temperature, and stirring was carried out at room temperature overnight. Water was added to the reaction solution, followed by extraction with methylene chloride and subsequent sequential washing with a saturated aqueous sodium hydrogencarbonate solution, water and saline, and the resulting organic layer was dried over anhydrous sodium sulfate. The organic layer was concentrated and the resulting residue was purified by silica gel column chromatography to afford t-butyl [(1S)-2-methyl-1-({4-[(pyrazin-2-yl)oxy]piperidin-1-yl}carbonyl)propyl]carbamate (1.82 g, yield 96%) as a colorless amorphous substance.
¹H-NMR (CDCl₃, 400 MHz) δ: 8.22 (1H, d, J=1.2 Hz), 8.13 (1H, d, J=2.8 Hz), 8.07-8.05 (1H, m), 5.36-5.26 (2H, m), 4.53-4.49 (1H, m), 3.98-3.49 (4H, m), 2.11-1.77 (5H, m), 1.44 (9H, s), 0.98 (3H, d, J=7.0 Hz), 0.90 (3H, d, J=6.6 Hz).
MS (ESI, m/z): 379 (M+H)⁺.

### Example 38-4

### (2S)-3-Methyl-1-oxo-1-{4-[(pyrazin-2-yl)oxy]piperidin-1-yl}butan-2-amine dihydrochloride

Analogously to Example 29-3, 2S)-3-methyl-1-oxo-1-14-[(pyrazin-2-yl)oxylpiperidin-1-yl}butan-2-amine dihydrochloride (1.85 g, yield 100%) was obtained from t-butyl [(1S)-2-methyl-1-({4-[(pyrazin-2-yl)oxy]piperidin-1-yl}carbonyl)propyl]carbamate (1.82 g, 4.81 mmol) (as a colorless amorphous substance.
¹H-NMR (DMSO-d₆, 400 MHz) δ: 8.30 (1H, d, J=3.9 Hz), 8.22-8.17 (6H, m), 5.32-5.22 (1H, m), 4.31-4.28 (1H, m), 4.11-3.75 (2H, m), 3.56-3.20 (2H, m), 2.11-1.97 (3H, m), 1.78-1.57 (2H, m), 1.00-0.92 (6H, m).
MS (ESI, m/z): 279 (M+H)⁺.

### Example 38-5

### 3-Hydroxy-N-[(1S)-2-methyl-1-({4-[(pyrazin-2-yl)oxy]piperidin-1-yl}carbonyl)propyl]quinoxaline-2-carboxamide

Analogously to Example 29-4, the desired title compound (232 mg, yield 91%) was obtained from (2S)-3-methyl-1-oxo-1-{4-[(pyrazin-2-yl)oxy]piperidin-1-yl}butan-2-amine dihydrochloride (200 mg, 0.570 mmol) as a yellow solid.
¹H-NMR (CDCl₃, 400 MHz) δ: 12.37 (1H, brs), 10.16 (1H, brs), 8.25-8.21 (1H, m), 8.15-8.13 (1H, m), 8.09-8.00 (2H, m), 7.63-7.26 (3H, m), 5.38-5.30 (1H, m), 5.12-5.07 (1H, m), 4.09-3.58 (4H, m), 2.36-1.85 (5H, m), 1.11 (6H, d, J=6.6 Hz).
IR (KBr) cm⁻¹: 2960, 1685, 1640, 1525, 1410.
MS (ESI, m/z): 451 (M+H)⁺, 473 (M+Na)⁺.
HRMS (ESI, m/z): 473.1929 (Calcd for C₂₃H₂₆5N₆NaO₄: 473.1913).
Anal. Calcd for C₂₃H₂₆N₆O₄: C, 61.32; H, 5.82; N, 18.66. Found: C, 61.23; H, 5.83; N, 18.60.

### Example 39

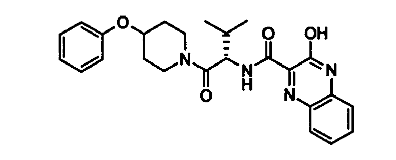

### 3-Hydroxy-N-{(1S)-2-methyl-1-[(4-phenoxypiperidin-1-yl)carbonyl]propyl}quinoxaline-2-carboxamide

### Example 39-1

### (2S)-3-Methyl-1-oxo-1-(4-phenoxypiperidin-1-yl)butan-2-amine hydrochloride

A 40% toluene solution of diisopropyl azodicarboxylate (2.10 ml, 3.99 mmol) was added to a toluene solution (15 ml) of t-butyl {(1S)-1-[(4-hydroxypiperidin-1-yl)carbonyl]-2-methylpropyl}carbamate (1.00 g, 3.33 mmol), phenol (344 mg, 3.33 mmol) and triphenylphosphine (1.05 g, 3.99 mmol), at room temperature, and stirring was carried out at room temperature overnight. The reaction solution was concentrated and the resulting residue was diluted with ethyl acetate, followed by sequential washing with a 1N aqueous sodium hydroxide solution, water and saline, and then the resulting organic layer was dried over anhydrous sodium sulfate. The organic layer was concentrated and the resulting residue was purified by silica gel column chromatography to afford t-butyl N-{(1S)-2-methyl-1-[(4-phenoxypiperidin-1-yl)carbonyl]propyl}carbamate (1.04 g) as a colorless oil. Concentrated hydrochloric acid (2.8 ml) was added to an ethanol solution (5.6 ml) of the resulting compound (1.04 g), at 0°C, and stirring was carried out under room temperature overnight. The reaction solution was concentrated to afford (2S)-3-methyl-1-oxo-1-(4-phenoxypiperidin-1-yl)butan-2-amine hydrochloride (880 mg, yield 84%) as a colorless amorphous substance.
¹H-NMR (CD₃OD, 400 MHz) δ: 7.30-7.25 (2H, m), 6.98-6.92 (3H, m), 4.71-4.62 (1H, m), 4.34 (1H, d, J=5.1 Hz), 4.02-3.46 (4H, m), 2.24-1.70 (5H, m), 1.11 (3H, d, J=7.0 Hz), 1.02 (3H, d, J=7.0 Hz).
MS (ESI, m/z): 277 (M+H)⁺.

### Example 39-2

### 3-Hydroxy-N-{(1S)-2-methyl-1-[(4-phenoxypiperidin-1-yl)carbonyl]propyl}quinoxaline-2-carboxamide

Analogously to Example 29-4, the desired title compound (174 mg, yield 61%) was obtained from (2S)-3-methyl-1-oxo-1-(4-phenoxypiperidin-1-yl)butan-2-amine hydrochloride (200 mg, 0.640 mmol) as a yellow solid.
¹H-NMR (CDCl₃, 400 MHz) δ: 12.69 (1H, brs), 10.16 (1H, brs), 8.00-7.97 (1H, m), 7.61-7.26 (5H, m), 7.00-6.92 (3H, m), 5.11-5.06 (1H, m), 4.65-4.59 (1H, m), 4.00-3.71 (4H, m), 2.35-1.88 (5H, m), 1.14-1.10 (6H, m).
IR (KBr) cm⁻¹: 2960, 1685, 1640, 1530, 1455.
MS (ESI, m/z): 448 (M+H)⁺.
HRMS (ESI, m/z): 471.2004 (Calcd for C₂₅H₂₈N₄NaO₄: 471.2008).
Anal. Calcd for C₂₅H₂₈N₄O₄·0.25H₂O: C, 66.28; H, 6.34; N, 12.37. Found: C, 66.34; H, 6.32; N, 12.32.

### Example 40

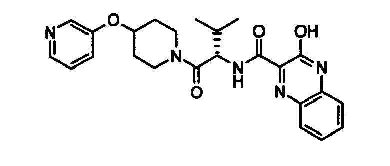

### 3-Hydroxy-N-[(1S)-2-methyl-1-({4-[(pyridin-3-yl)oxy]piperidin-1-yl}carbonyl)propyl]quinoxaline-2-carboxamide

### Example 40-1

### t-Butyl [(1S)-2-methyl-1-({4-[(pyridin-3-yl)oxy]piperidin-1-yl}carbonyl)propyl]carbamate

A 40% toluene solution of diisopropyl azodicarboxylate (2.10 ml, 3.99 mmol) was added to a toluene solution (15 ml) of t-butyl 4-hydroxypiperidine-1-carboxylate (1.00 g, 3.33 mmol), 3-hydroxypyridine (348 mg, 3.66 mmol) and triphenylphosphine (1.05 g, 3.99 mmol), at room temperature, and stirring was carried out at room temperature for 4 days. The reaction solution was concentrated and the resulting residue was diluted with ethyl acetate, followed by sequential washing with a 1N aqueous sodium hydroxide solution, water and saline. The resulting organic layer was extracted with a 1N hydrochloric acid, and a 1N aqueous sodium hydroxide solution was added to the aqueous layer to make it alkaline. The aqueous layer was extracted with ethyl acetate, and the resulting organic layer was dried over anhydrous sodium sulfate. The organic layer was concentrated to afford t-butyl [(1S)-2-methyl-1-({4-[(pyridin-3-yl)oxy]piperidin-1-yl}carbonyl)propyl]carbamate (767 mg, yield 61%) as a colorless amorphous substance.
¹H-NMR (CDCl₃, 400 MHz) : 8.33 (1H, brs), 8.24 (1H, brs), 7.26-7.22 (2H, m), 5.34-5.30 (1H, m), 4.61 (1H, brs), 4.49 (1H, brs), 3.83-3.47 (4H, m), 2.05-1.82 (5H, m), 1.44 (9H, s), 0.97 (3H, d, J=6.6 Hz), 0.90 (3H, d, J=6.3 Hz).
MS (ESI, m/z): 378 (M+H)⁺.

### Example 40-2

### (2S)-3-Methyl-1-oxo-1-{4-[(pyridin-3-yl)oxy]piperidin-1-yl}butan-2-amine dihydrochloride

Concentrated hydrochloric acid (2.0 ml) was added to an ethanol solution (4.0 ml) of t-butyl [(1S)-2-methyl-1-({4-[(pyridin-3-yl)oxy]piperidin-1-yl}carbonyl)propyl]carbamate (767 mg, 2.03 mmol), at 0°C, and stirring was carried out at room temperature overnight. The organic layer was concentrated to afford (2S)-3-methyl-1-oxo-1-{4-[(pyridin-3-yl)oxy]piperidin-1-yl}butan-2-amine dihydrochloride (786 mg, yield 100%) as a colorless amorphous substance.
¹H-NMR (CD₃OD, 400 MHz) δ: 8.71-8.70 (1H, m), 8.48 (1H, d, J=5.6 Hz), 8.35-8.33 (1H, m), 8.05 (1H, dd, J=8.8 Hz, 5.6 Hz), 5.06-4.95 (1H, m), 4.39 (1H, d, J=5.2 Hz), 4.14-3.45 (4H, m), 2.25-1.77 (5H, m), 1.12 (3H, d, J=6.6 Hz), 1.03 (3H, d, J=6.6 Hz).
MS (ESI, m/z): 278 (M+H)⁺.

### Example 40-3

### 3-Hydroxy-N-[(1S)-2-methyl-1-({4-[(pyridin-3-yl)oxy]piperidin-1-yl}carbonyl)propyl]quinoxaline-2-carboxamide

Analogously to Example 29-4, the desired title compound (148 mg, yield 58%) was obtained from (2S)-3-methyl-1-oxo-1-{4-[(pyridin-3-yl)oxy]piperidin-1-yl}butan-2-amine dihydrochloride (200 mg, 0.570 mmol) as a yellow solid.
¹H-NMR (CDCl₃, 400 MHz) δ: 8.37-8.34 (1H, m), 8.26-8.25 (1H, m), 8.04-8.01 (1H, m), 7.65-7.24 (5H, m), 5.10-5.06 (1H, m), 4.67 (1H, brs), 4.03-3.68 (4H, m), 2.33-1.88 (5H, m), 1.13-1.10 (6H, m).
IR (KBr) cm⁻¹: 2965, 1685, 1630, 1525, 1475.
MS (ESI, m/z): 472 (M+Na)⁺.
HRMS (ESI, m/z) : 472.1971 (Calcd for C₂₄H₂₇N₅NaO₄: 472.1961).
Anal. Calcd for C₂₄H₂₇N₅O₄·0.25H₂O: C, 63.49; H, 6.11; N, 15.43. Found: C, 63.41; H, 5.90; N, 15.39.

### Example 41

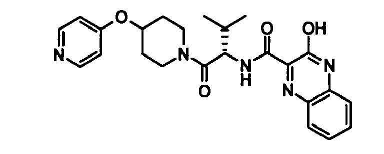

### 3-Hydroxy-N-[(1S)-2-methyl-1-({4-[(pyridin-4-yl)oxy]piperidin-1-yl}carbonyl)propyl]quinoxaline-2-carboxamide

### Example 41-1

### t-Butyl [(1S)-2-methyl-1-({4-[(pyridin-4-yl)oxy]piperidin-1-yl}carbonyl)propyl]carbamate

Analogously to Example 2-1, t-butyl [(1S)-2-methyl-1-({4-[(pyridin-4-yl)oxy]piperidin-1-yl}carbonyl)propyl]carbamate (940 mg, yield 100%) was obtained from 4-[(piperidin-4-yl)oxy]pyridine dihydrochloride (580 mg, 2.30 mmol) and N-(t-butoxycarbonyl)-L-valine (500 mg, 2.30 mmol) as a colorless oil.
¹H-NMR (CDCl₃ 400 MHz) δ: 8.44 (2H, dd, J=6.3 Hz, 1.5 Hz), 6.81 (2H, dd, J=6.3 Hz, 1.5 Hz), 5.33 (1H, d, J=9.5 Hz), 4.72-4.40 (1H, m), 4.49 (1H, dd, J=8.3 Hz, 6.1 Hz), 3.88-3.50 (4H, m), 2.15-1.80 (5H, m), 1.44 (9H, s), 0.97 (3H, d, J=6.8 Hz), 0.90 (3H, d, J=6.8 Hz).
IR (ATR) cm⁻¹: 2965, 1700, 1635, 1590, 1500, 1440, 1365, 1280.
MS (ESI, m/z): 378 (M+H)⁺.

### Example 41-2

### 3-Hydroxy-N-[(1S)-2-methyl-1-({4-[(pyridin-4-yl)oxy]piperidin-1-yl}carbonyl)propyl]quinoxaline-2-carboxamide

Analogously to Example 2-2, (2S)-3-methyl-1-oxo-1-{4-[(pyridin-4-yl)oxy]piperidin-1-yl}butan-2-amine dihydrochloride was obtained from N-(t-butoxycarbonyl)-L-valine (500 mg, 2.30 mmol) and from t-butyl [(1S)-2-methyl-1-({4-[(pyridin-4-yl)oxy]piperidin-1-yl}carbonyl)propyl]carbamate (940 mg, 2.49 mmol) as an amorphous substance. The resulting compound (150 mg, 0.430 mmol) was condensed with 3-hydroxyquinoxaline-2-carboxylic acid (81.0 mg, 0.430 mmol) to afford the desired title compound (104 mg, yield 63%) as a white solid.
¹H-NMR (CDCl₃ 400 MHz) δ: 10.16 (1H, s), 8.47 (2H, dt, J=4.6 Hz, 1.2 Hz), 7.98 (1H, dd, J=7.3 Hz, 6.4 Hz), 7.59 (1H, dd, J=7.5 Hz, 7.1 Hz), 7.53 (1H, d, J=8.3 Hz), 7.36 (1H, t, J=7.5 Hz), 6.84 (2H, t, J=6.4 Hz), 5.08 (1H, t, J=7.3 Hz), 4.78-4.68 (1H, m), 4.08-3.99 (1H, m), 3.97-3.63 (3H, m), 2.40-1.86 (5H, m), 1.13 (3H, d, J=6.8 Hz), 1.10 (3H, d, J=6.8 Hz).
IR (ATR) cm⁻¹: 1685, 1630, 1590, 1525, 1495, 1470, 1450, 1275.
MS (ESI, m/z): 450 (M+H)⁺.
Anal. Calcd for C₂₄H₂₇N₅O₃·0.25H₂O: C, 63.49; H, 6.11; N, 15.43. Found: C, 63.10; H, 6.13; N, 15.34.

### Example 42

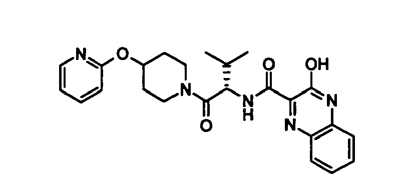

### 3-Hydroxy-N-[(1S)-2-methyl-1-({4-[(pyridin-2-yl)oxy]piperidin-1-yl}carbonyl)propyl]quinoxaline-2-carboxamide

### Example 42-1

### t-Butyl [(1S)-2-methyl-1-({4-[(pyridin-2-yl)oxy]piperidin-1-yl}carbonyl)propyl]carbamate

Analogously to Example 2-1, t-butyl [(1S)-2-methyl-1-({4-[(pyridin-2-yl)oxy]piperidin-1-yl]carbonyl}propyl)carbamate (948 mg, yield 100%) was obtained from 2-[(piperidin-4-yl)oxy]pyridine dihydrochloride (580 mg, 2.30 mmol) and N-(t-butoxycarbonyl)-L-valine (500 mg, 2.30 mmol) as a colorless oil.
¹H-NMR (CDCl₃ 400 MHz) δ: 8.14-8.11 (1H, m), 7.57 (1H, dt, J=7.1 Hz, 2.0 Hz), 6.86 (1H, dd, J=6.8 Hz, 6.1 Hz), 6.72 (1H, d, J=8.3 Hz), 5.37 (1H, d, J=9.1 Hz), 5.34-5.28 (1H, m), 4.51 (1H, dd, J=8.5 Hz, 5.9 Hz), 3.98-3.73 (2H, m), 3.62-3.42 (2H, m), 2.10-1.73 (5H, m), 1.44 (9H, s), 0.98 (3H, d, J=6.6 Hz), 0.89 (3H, d, J=6.6 Hz).
IR (ATR) cm⁻¹: 2965, 1700, 1635, 1595, 1470, 1430, 1365, 1305.
MS (ESI, m/z): 378 (M+H)⁺.

### Example 42-2

### 3-Hydroxy-N-[(1S)-2-methyl-1-({4-[(pyridin-2-yl)oxy]piperidin-1-yl}carbonyl)propyl]quinoxaline-2-carboxamide

Analogously to Example 2-2, (2S)-3-methyl-1-oxo-1-{4-[(pyridin-4-yl)oxylpiperidin-1-yl}butan-2-amine dihydrochloride (734 mg, yield 91%) was obtained from t-butyl [(1S)-2-methyl-1-({4-[(pyridin-2-yl)oxylpiperidin-1-yl}carbonyl)propyl]carbamate (948 mg, 2.21 mmol) as an amorphous substance. The resulting compound (150 mg, 0.430 mmol) was condensed with 3-hydroxyquinoxaline-2-carboxylic acid (81.0 mg, 0.430 mmol) to afford the desired title compound (71.0 mg, yield 43%) as a white solid.
¹H-NMR (CDCl₃ 400 MHz) δ: 12.77 and 10.14 (1H, brs), 8.14 (1H, t, J=4.9 Hz), 7.98 (1H, d, J=8.1 Hz), 7.66-7.50 (3H, m), 7.37 (1H, brs), 6.86 (1H, dd, J=11.7 Hz, 4.9 Hz), 6.73 (1H, t, J=8.8 Hz), 5.40-5.32 (1H, m), 5.09 (1H, t, J=7.1 Hz), 4.10-3.91 (2H, m), 3.82-3.62 (2H, m), 2.39-2.17 (1H, m), 2.12-1.98 (2H, m), 1.95-1.83 (2H, m), 1.11 (6H, d, J=6.6 Hz).
IR (ATR) cm⁻¹: 1685, 1630, 1525, 1470, 1430, 1270, 1250, 1215.
MS (ESI, m/z): 450 (M+H)⁺.
Anal. Calcd for C₂₄H₂₇N₅O₃·0.75H₂O: C, 62.26; H, 6.20, N, 15.13. Found: C, 61.95; H, 5.86; N, 15.15.

### Example 43

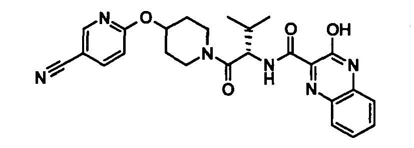

### N-[(1S)-1-({4-[(5-Cyanopyridin-2-yl)oxy]piperidin-1-yl}carbonyl)-2-methylpropyl]-3-hydroxyquinoxaline-2-carboxamide

### Example 43-1

### t-Butyl 4-[(5-cyanopyridin-2-yl)oxy]piperidine-1-carboxylate

t-Butyl 4-hydroxypiperidine-1-carboxylate (2.49 g, 12.0 mmol) was added to a N,N-dimethylformamide suspension (10 ml) of 60% sodium hydride (419 mg, 11.0 mmol), at 0°C, and stirring was carried out at room temperature for 30 minutes. Further, at 0°C, 6-chloropyridine-3-carbonitrile (1.43 g, 10.0 mmol) was added, and stirring was carried out for 1.25 hours. Water was added to the reaction solution, followed by extraction with ethyl acetate and subsequent sequential washing with water and saline, and then the resulting organic layer was dried over anhydrous sodium sulfate. The organic layer was concentrated and the resulting residue was purified by silica gel column chromatography to afford t-butyl 4-[(5-cyanopyridin-2-yl)oxy]piperidine-1-carboxylate (2.71 g, yield 89%) as a white solid.
¹H-NMR (CDCl₃, 400 MHz) δ: 8.45 (1H, dd, J=2.4 Hz, 0.8 Hz), 7.78 (1H, dd, J=8.6 Hz, 2.4 Hz), 6.79 (1H, dd, J=8.6 Hz, 0.8 Hz), 5.32-5.26 (1H, m), 3.81-3.75 (2H, m), 3.32-3.25 (2H, m), 2.01-1.96 (2H, m), 1.77-1.69 (2H, m), 1.47 (9H, s).
MS (ESI, m/z): 304 (M+H)⁺, 204 (M-Boc+H)⁺.

### Example 43-2

### t-Butyl [(1S)-1-({4-[(5-cyanopyridin-2-yl)oxy]piperidin-1-yl}carbonyl)-2-methylpropyl]carbamate

Concentrated hydrochloric acid (8.9 ml) was added to an ethanol solution (17.8 ml) of t-butyl 4-[(5-cyanopyridin-2-yl)oxy]piperidine-1-carboxylate (2.71 g, 8.93 mmol), at 0°C, and stirring was carried out at room temperature overnight. The organic layer was concentrated to afford 6-(piperidin-4-yl)oxypyridine-3-carbonitrile dihydrochloride (2.34 g) as a white solid.

1-Ethyl-3-(3-dimethylaminopropyl)carbodiimide hydrochloride (2.57 g, 13.4 mmol) was added to a methylene chloride solution (90 ml) of the resulting compound (2.34 g), N-(t-butoxycarbonyl)-L-valine (1.94 g, 8.93 mmol), 1-hydroxybenzotriazole monohydrate (1.45 g, 10.7 mmol) and N-methylmorpholine (4.91 ml, 44.7 mmol), at room temperature, and stirring was carried out at room temperature for 3 days. Water was added to the reaction solution, followed by extraction with ethyl acetate and subsequent sequential washing with a saturated aqueous sodium hydrogencarbonate solution, water and saline, and the resulting organic layer was dried over anhydrous sodium sulfate. The organic layer was concentrated and the resulting residue was purified by silica gel column chromatography to afford t-butyl [(1S)-1-({4-[(5-cyanopyridin-2-yl)oxy]piperidin-1-yl}carbonyl)-2-methylpropyl]carbamate (3.50 g, yield 97%) as a colorless amorphous substance.
¹H-NMR (CDCl₃, 400 MHz) δ: 8.46-8.45 (1H, m), 7.79 (1H, dd, J=8.6 Hz. 2.3 Hz), 6.80 (1H, d, J=8.6 Hz), 5.42-5.30 (2H, m), 4.52-4.48 (1H, m), 4.03-3.43 (4H, m), 2.11-1.76 (5H, m), 1.44 (9H, s), 0.97 (3H, d, J=6.6 Hz), 0.90 (3H, d, J=7.0 Hz).
MS (ESI, m/z): 302 (M-Boc+H)⁺.

### Example 43-3

### 6-[(1-L-Valylpiperidin-4-yl)oxy]nicotinonitrile dihydrochloride

Analogously to Example 29-3, 6-[(1-L-valylpiperidin-4-yl)oxy]nicotinonitrile dihydrochloride (3.28 g, yield 100%) was obtained from t-butyl [(1S)-1-({4-(5-cyanopyridin-2-yl)oxy}piperidin-1-yl)carbonyl]-2-methylpropyl]carbamate (3.50 g, 8.70 mmol) as a colorless amorphous substance.
¹H-NMR (CD₃OD, 400 MHz) δ: 8.54 (1H, d, J=2.4 Hz), 8.00-7.97 (1H, m), 6.94 (1H, dd, J=12.0 Hz, 8.4 Hz), 5.48-5.39 (1H, m), 4.37 (1H, d,
J=3.9 Hz), 4.12-3.41 (4H, m), 2.24-1.74 (5H, m), 1.11 (3H, d, J=6.6 Hz), 1.03 (3H, d, J=6.6 Hz).
MS (ESI, m/z): 303 (M+H)⁺.

### Example 43-4

### N-[(1S)-1-({4-[(5-Cyanopyridin-2-yl)oxy]piperidin-1-yl}carbonyl)-2-methylpropyl]-3-hydroxyquinoxaline-2-carboxamide

Analogously to Example 29-4, the desired title compound (182 mg, yield 72%) was obtained from 6-[(1-L-valylpiperidin-4-yl)oxy]nicotinonitrile dihydrochloride (200 mg, 0.530 mmol) as a yellow solid.
¹H-NMR (CDCl₃, 400 MHz) δ: 12.00 (1H, brs), 10.11 (1H, brs), 8.47 (1H, dd, J=6.0 Hz, 2.4 Hz), 8.04-8.02 (1H, m), 7.82-7.78 (1H, m), 7.63 (1H, brs), 7.44-7.22 (2H, m), 6.82 (1H, t, J=9.6 Hz), 5.45-5.39 (1H, m), 5.08 (1H, t, J=7.6 Hz), 4.11-3.53 (4H, m), 2.35-1.85 (5H, m), 1.10 (5H, d, J=6.6 Hz).
IR (KBr) cm⁻¹: 2965, 2225, 1685, 1630, 1480.
MS (ESI, m/z): 475 (M+H)⁺, 497 (M+Na)⁺.
HRMS (ESI, m/z): 497.1920 (Calcd for C₂₅H₂₆N₆NaO₄: 497.1913).
Anal. Calcd for C₂₅H₂₆N₆O₄·1/4H₂O: C, 62.68; H, 5.58; N, 17.54. Found: C, 62.76; H, 5.47; N, 17.48.

### Example 44

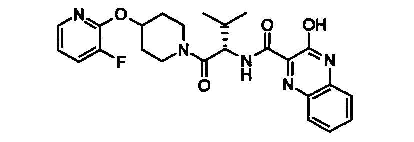

### N-[(1S)-1-({4-[(3-Fluoropyridin-2-yl)oxy]piperidin-1-yl}carbonyl)-2-methylpropyl]-3-hydroxyquinoxaline-2-carboxamide

### Example 44-1

### t-Butyl 4-[(3-fluoropyridin-2-yl)oxy]piperidine-1-carboxylate

t-Butyl 4-(methanesulfonyloxy)piperidine-1-carboxylate (2.96 g, 10.6 mmol) and potassium carbonate (2.69 g, 19.5 mmol) were added to a N,N-dimethylformamide solution (45 ml) of 3-fluoro-2-hydroxypyridine (1.00 g, 8.84 mmol), at room temperature, and stirring was carried out at 75°C overnight. The reaction solution was cooled to room temperature, and water was added to the residue resulting from concentration, followed by extraction with methylene chloride. After the resulting organic layer was dried over anhydrous sodium sulfate, the residue resulting from concentration was purified by silica gel column chromatography to afford t-butyl 4-[(3-fluoropyridin-2-yl)oxy]piperidine-1-carboxylate (1.63 g, yield 62%) as a colorless oil.
¹H-NMR (CDCl₃, 400 MHz) δ: 7.88 (1H, d, J=3.9 Hz), 7.33-7.29 (1H, m), 6.84-6.81 (1H, m), 5.32-5.27 (1H, m), 3.81-3.77 (2H, m), 3.33-3.28 (2H, m), 2.02-1.98 (2H, m), 1.82-1.75 (2H, m), 1.47 (9H, s).
MS (ESI, m/z): 241 (M-tBu+H)⁺.

### Example 44-2

### t-Butyl [(1S)-1-({4-[(3-fluoropyridin-2-yl)oxy]piperidin-1-yl}carbonyl)-2-methylpropyl]carbamate

Concentrated hydrochloric acid (2.7 ml) was added to an ethanol solution (5.4 ml) of t-butyl 4-[(3-fluoropyridin-2-yl)oxy]piperidine-1-carboxylate (800 mg, 2.70 mmol), at 0°C, and stirring was carried out under room temperature overnight. The reaction solution was concentrated to afford 3-fluoro-2-[(piperidin-4-yl)oxy]pyridine dihydrochloride (633 mg) as a white solid.

1-Ethyl-3-(3-dimethylaminopropyl)carbodiimide hydrochloride (776 mg, 4.05 mmol) was added to a methylene chloride solution (27 ml) of the resulting compound (633 mg), N-(t-butoxycarbonyl)-L-valine (586 mg, 2.70 mmol), 1-hydroxybenzotriazole monohydrate (438 mg, 3.24 mmol) and N-methylmorpholine (1.48 ml, 13.5 mmol), at room temperature, and stirring was carried out under room temperature overnight. Water was added to the reaction solution, followed by extraction with ethyl acetate and subsequent sequential washing with a saturated aqueous sodium hydrogencarbonate solution, water and saline, and the resulting organic layer was dried over anhydrous sodium sulfate. The organic layer was concentrated and the resulting residue was purified by silica gel column chromatography to afford t-butyl [(1S)-1-({4-[(3-fluoropyridin-2-yl)oxy]piperidin-1-yl}carbonyl)-2-methylpropyl]carbamate (1.00 g, yield 94%) as a colorless amorphous substance.
¹H-NMR (CDCl₃, 400 MHz) δ: 7.90-7.88 (1H, m), 7.35-7.31 (1H, m), 6.87-6.84 (1H, m), 5.42-5.34 (2H, m), 4.53-4.50 (1H, m), 3.98-3.46 (4H, m), 2.11-1.80 (5H, m), 1.44 (9H, s), 0.98 (3H, d, J=5.5 Hz), 0.90 (3H, dd, J=5.5 Hz, 2.0 Hz).
MS (ESI, m/z): 295 (M-Boc+H)⁺.

### Example 44-3

### (2S)-1-{4-[(3-Fluoropyridin-2-yl)oxy]piperidin-1-yl}-3-methyl-1-oxobutan-2-amine dihydrochloride

Analogously to Example 29-3, (2S)-1-{4-[(3-fluoropyridin-2-yl)oxy]piperidin-1-yl}-3-methyl-1-oxobutan-2-amine dihydrochloride (975 mg, yield 100%) was obtained from t-butyl [(1S)-1-({4-[(3-fluoropyridin-2-yl)oxy]piperidin-1-yl}carbonyl)-2-methylpropyl]carbamate (1.00 g, 2.53 mmol) as a colorless amorphous substance.
¹H-NMR (CD₃OD, 400 MHz) δ: 7.92 (1H, d, J=4.8 Hz), 7.53-7.48 (1H, m), 6.99-6.95 (1H, m), 5.45-5.39 (1H, m), 4.37 (1H, d, J=5.2 Hz), 4.08-3.45 (4H, m), 2.24-2.01 (3H, m), 1.95-1.77 (2H, m), 1.12 (3H, d, J=6.6 Hz), 1.03 (3H, d, J=7.0 Hz).
MS (ESI, m/z): 296 (M+H)⁺.

### Example 44-4

### N-[(1S)-1-({4-[(3-Fluoropyridin-2-yl)oxy]piperidin-1-yl}carbonyl)-2-methylpropyl]-3-hydroxyquinoxaline-2-carboxamide

Analogously to Example 29-4, the desired title compound (189 mg, yield 74%) was obtained from (2S)-1-{4-[(3-fluoropyridin-2-yl)oxy]piperidin-1-yl}-3-methyl-1-oxobutan-2-amine dihydrochloride (200 mg, 0.540 mmol) as a yellow solid.
¹H-NMR (CDCl₃, 400 MHz) δ: 12.20 (1H, brs), 10.13 (1H, brs), 8.02 (1H, brs), 7.92-7.89 (1H, m), 7.62-7.26 (4H, m), 6.89-6.84 (1H, m), 5.44 (1H, brs), 5.13-5.09 (1H, m), 4.07-3.65 (4H, m), 2.37-1.90 (5H, m), 1.11 (6H, d, J=6.3 Hz).
IR (KBr) cm⁻¹: 2965, 1685, 1635, 1530, 1460.
MS (ESI, m/z): 490 (M+Na)⁺.
HRMS (ESI, m/z): 490.1852 (Calcd for C₂₅H₂₆FN₅NaO₄: 490.1867).
Anal. Calcd for C₂₅H₂₆FN₅O₄: C, 61.66; H, 5.61; N, 14.98; F, 4.06. Found: C, 61.54; H, 5.51; N, 14.95; F, 4.03.

### Example 45

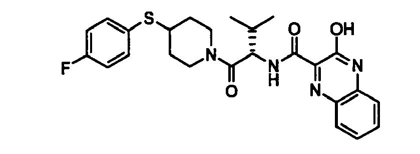

### N-[(1S)-1-({4-[(4-Fluorophenyl)thio]piperidin-1-yl}carbonyl)-2-methylpropyl]-3-hydroxyquinoxaline-2-carboxamide

### Example 45-1

### t-Butyl [(1S)-1-({4-[(4-fluorophenyl)thio]piperidin-1-yl}carbonyl)-2-methylpropyl] carbamate

1-Ethyl-3-(3-dimethylaminopropyl)carbodiimide hydrochloride (947 mg, 4.94 mmol) was added to a methylene chloride solution (41 ml) of 4-[(4-fluorophenyl)sulfanyl]piperidine hydrochloride (1.02 g, 4.12 mmol), N-(t-butoxycarbonyl)-L-valine (894 mg, 4.12 mmol), 1-hydroxybenzotriazole monohydrate (612 mg, 4.53 mmol) and N-methylmorpholine (1.36 ml, 12.4 mmol), at room temperature, and stirring was carried out at room temperature overnight. Water was added to the reaction solution, followed by extraction with ethyl acetate and subsequent sequential washing with a saturated aqueous sodium hydrogencarbonate solution, water and saline, and the resulting organic layer was dried over anhydrous sodium sulfate. The organic layer was concentrated and the resulting residue was purified by silica gel column chromatography to afford t-butyl [(1S)-1-({4-[(4-fluorophenyl)thio]piperidin-1-yl}carbonyl)-2-methylpropyl]carbamate (1.64 g, yield 97%) as a colorless oil.
¹H-NMR (CDCl₃, 400 MHz) δ: 7.43 (2H, dd, J=8.6 Hz, 5.6 Hz), 7.02 (2H, td, J=8.6 Hz, 2.4 Hz), 5.31 (1H, t, J=7.8 Hz), 4.46-4.27 (2H, m), 3.96-3.85 (1H, m), 3.25-3.12 (2H, m), 3.03-2.87 (1H, m), 2.03-1.84 (3H, m), 1.57-1.46 (2H, m), 1.43 (9H, d, J=6.0 Hz), 0.94 (3H, dd, J=8.4 Hz, 6.8 Hz), 0.86 (3H, dd, J=6.8 Hz, 4.4 Hz).
MS (ESI, m/z): 312 (M-Boc+H)⁺.

### Example 45-2

### N-[(1S)-1-({4-[(4-Fluorophenyl)thio]piperidin-1-yl}carbonyl)-2-methylpropyl]-3-hydroxyquinoxaline-2-carboxamide

Analogously to Example 2-2, the desired title compound (213 mg, yield 73%) was obtained from t-butyl [(1S)-1-({4-[(4-fluorophenyl)thio]piperidin-1-yl}carbonyl)-2-methylpropyl]carbamate (248 mg, 0.600 mmol) as a yellow solid.
¹H-NMR (CDCl₃, 400 MHz) δ: 12.78-12.45 (1H, m), 10.13 (1H, brs), 8.04-7.98 (1H, m), 7.62-6.98 (7H, m), 5.08-5.01 (1H, m), 4.48-4.16 (2H, m), 3.43-3.00 (3H, m), 2.29-1.55 (5H, m), 1.12-1.05 (6H, m).
IR (KBr) cm⁻¹: 2965, 1685, 1635, 1525, 1490.
MS (ESI, m/z): 505 (M+Na)⁺.
HRMS (ESI, m/z): 505.1675 (Calcd for C₂₅H₂₇FN₄NaO₃S: 505.1686).
Anal. Calcd for C₂₅H₂₇FN₄O₃S: C, 62.22; H, 5.64; N, 11.61; F, 3.94; S, 6.64. Found: C, 62.03; H, 5.36; N, 11.59; F, 4.24; S, 6.82.

### Example 46

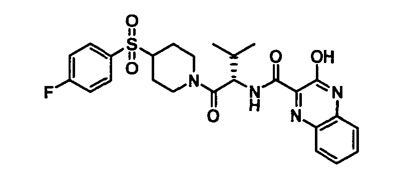

### N-[(1S)-1-({4-[(4-Fluorophenyl)sulfonyl]piperidin-1-yl}carbonyl)-2-methylpropyl]-3-hydroxyquinoxaline-2-carboxamide

### Example 46-1

### t-Butyl [(1S)-1-({4-[(4-fluorophenyl)sulfonyl]piperidin-1-yl}carbonyl)-2-methylpropyl]carbamate

m-Chloroperbenzoic acid (504 mg, 1.90 mmol) was added to a methylene chloride solution (6.3 ml) of t-butyl [(1S)-1-({4-[(4-fluorophenyl)thio]piperidin-1-yl}carbonyl)-2-methylpropyl]carbamate (260 mg, 0.630 mmol), at room temperature, and stirring was carried out at room temperature for 30 minutes. Further m-chloroperbenzoic acid (504 mg, 1.90 mmol) was added at room temperature, and stirring was carried out at room temperature for 30 minutes. The reaction solution was diluted with methylene chloride, followed by sequential washing with a 1N aqueous sodium hydroxide solution and saline, and then the resulting organic layer was dried over anhydrous sodium sulfate. The organic layer was concentrated and the resulting residue was purified by silica gel column chromatography to afford t-butyl [(1S)-1-({4-[(4-fluorophenyl)sulfonyl]piperidin-1-yl}carbonyl)-2-methylpropyl]carbamate (282 mg, yield 100%) as a colorless amorphous substance.
¹H-NMR (CDCl₃, 400 MHz) δ: 7.92-7.86 (2H, m), 7.30-7.24 (2H, m), 5.26-5.19 (1H, m), 4.79-4.66 (1H, m), 4.43-4.35 (1H, m), 4.17-4.08 (1H, m), 3.16-2.98 (2H, m), 2.65-2.51 (1H, m), 2.10-1.57 (5H, m), 1.41 (9H, s), 0.94-0.85 (6H, m).
MS (ESI, m/z): 343 (M-Boc+H)⁺.

### Example 46-2

### N-[(1S)-1-({4-[(4-Fluorophenyl)sulfonyl]piperidin-1-yl}carbonyl)-2-methylpropyl]-3-hydroxyquinoxaline-2-carboxamide

Analogously to Example 2-2, the desired title compound (221 mg, yield 68%) was obtained from t-butyl [(1S)-1-({4-[(4-fluorophenyl)sulfonyl]piperidin-1-yl}carbonyl)-2-methylpropyl]carbamate (280 mg, 0.630 mmol) as a yellow solid.
¹H-NMR (CDCl₃, 400 MHz) δ: 12.36 (1H, brs), 10.12 (1H, brs), 8.08-7.21 (8H, m), 5.07-4.74 (2H, m), 4.42 (1H, brs), 3.29-3.10 (2H, m), 2.77-2.63 (1H, m), 2.31-2.05 (3H, m), 1.86-1.57 (2H, m), 1.10-1.03 (6H, m). IR (KBr) cm⁻¹: 3470, 2965, 1690, 1645, 1530.
MS (ESI, m/z): 537 (M+Na)⁺.
HRMS (ESI, m/z): 537.1587 (Calcd for C₂₅H₂₇FN₄NaO₅S: 537.1584).
Anal. Calcd for C₂₅H₂₇FN₄O₅S: C, 58.35; H, 5.29; N, 10.89; F, 3.69; S, 6.23. Found:: C, 58.09; H, 5.26; N, 10.82; F, 3.72; S, 6.28.

### Example 47

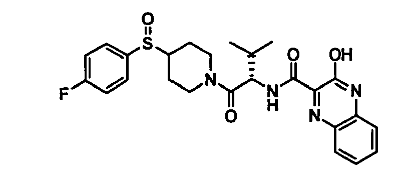

### N-[(1S)-1-({4-[(4-Fluorophenyl)sulfinyl]piperidin-1-yl}carbonyl)-2-methylpropyl]-3-hydroxyquinoxaline-2-carboxamide

### Example 47-1

### t-Butyl [(1S)-1-({4-[(4-fluorophenyl)sulfinyl]piperidin-1-yl}carbonyl)-2-methylpropyl]carbamate

m-Chloroperbenzoic acid (268 mg, 1.01 mmol) was added to a methylene chloride solution (10 ml) of t-butyl [(1S)-1-({4-[(4-fluorophenyl)thio]piperidin-1-yl}carbonyl)-2-methylpropyl]carbamate (415 mg, 1.01 mmol), at 0°C, and stirring was carried out at 0°C for 1 hour. A 1N aqueous sodium hydroxide solution was added to the reaction solution, followed by extraction with methylene chloride. After the extract was washed with saline, the resulting organic layer was dried over anhydrous sodium sulfate. The organic layer was concentrated and the resulting residue was purified by silica gel column chromatography to afford t-butyl [(1S)-1-({4-[(4-fluorophenyl)sulfinyl]piperidin-1-yl}carbonyl)-2-methylpropyl]carbamate (379 mg, yield 88%) as a colorless amorphous substance.
¹H-NMR (CDCl₃, 400 MHz) δ: 7.62-7.58 (2H, m), 7.26-7.22 (2H, m), 5.30-5.24 (1H, m), 4.75-4.62 (1H, m), 4.44-4.38 (1H, m), 4.15-4.04 (1H, m), 3.15-3.00 (1H, m), 2.82-2.56 (2H, m), 1.93-1.54 (5H, m), 1.44-1.41 (9H, m), 0.96-0.84 (6H, m).
MS (ESI, m/z): 328 (M-Boc+H)⁺.

### Example 47-2

### N-[(1S)-1-({4-[(4-Fluorophenyl)sulfinyl]piperidin-1-yl}carbonyl)-2-methylpropyl]-3-hydroxyquinoxaline-2-carboxamide

Analogously to Example 2-2, the desired title compound (203 mg, yield 46%) was obtained from t-butyl [(1S)-1-({4-[(4-fluorophenyl)sulfinyl]piperidin-1-yl}carbonyl)-2-methylpropyl]carbamate (377 mg, 0.880 mmol) as a yellow solid.
¹H-NMR (CDCl₃, 400 MHz) δ: 9.95 (1H, brs), 8.05-8.00 (1H, m), 7.64-7.20 (7H, m), 5.06-4.98 (1H, m), 4.85-4.69 (1H, m), 4.40-4.35 (1H, m), 3.72 (1H, t, J=3.6 Hz), 3.32-3.12 (1H, m), 2.81-2.67 (2H, m), 2.42-1.55 (5H, m), 1.10-1.02 (6H, m).
IR (KBr) cm⁻¹: 2960, 1685, 1640, 1530, 1450.
MS (ESI, m/z): 521 (M+Na)⁺.
HRMS (ESI, m/z) : 521.1623 (Calcd for C₂₅H₂₇FN₄NaO₄S: 521.1635).

### Example 48

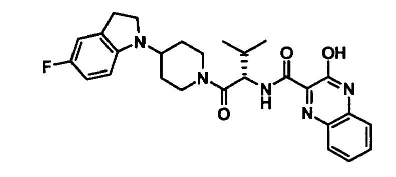

### N-[(1S)-1-{[4-(5-Fluoro-2,3-dihydro-1H-indol-1-yl)piperidin-1-yl]carbonyl}-2-methylpropyl]-3-hydroxyquinoxaline-2-carboxamide

### Example 48-1

### t-Butyl 4-(5-fluoro-2,3-dihydro-1H-indol-1-yl)piperidine-1-carboxylate

Sodium triacetoxy borohydride (1.79 g, 8.04 mmol) was added to a methylene chloride solution (33.5 ml) of 5-fluoro-2,3-dihydro-1H-indol (919 mg, 6.70 mmol), t-butyl 4-oxopiperidine-1-carboxylate (1.35 g, 6.70 mmol) and acetic acid (0.384 ml, 6.70 mmol), at 0°C, and stirring was carried out at room temperature for 3 hours. A saturated aqueous sodium hydrogencarbonate solution was added to the reaction solution, followed by extraction with methylene chloride. The extract was concentrated and the resulting residue was purified by silica gel column chromatography to afford t-butyl 4-(5-fluoro-2,3-dihydro-1H-indol-1-yl)piperidine-1-carboxylate (1.68 g, yield 78%).
¹H-NMR (CDCl₃, 400 MHz) δ: 6.80 (1H, dd, J=8.4 Hz, 2.8 Hz), 6.76-6.71 (1H, m), 6.29 (1H, dd, J=8.6 Hz, 4.3 Hz), 4.25 (2H, brs), 3.47-3.39 (1H, m), 3.33 (2H, t, J=8.2 Hz), 2.92 (2H, t, J=8.2 Hz), 2.80-2.73 (2H, m), 1.77 (2H, d, J=12.5 Hz), 1.55-1.48 (2H, m), 1.47 (9H, s).
MS (ESI, m/z): 220 (M-tBu+H)⁺.

### Example 48-2

### t-Butyl N-[(1S)-1-{[4-(5-fluoro-2,3-dihydro-1H-indol-1-yl)piperidin-1-yl]carbonyl}-2-methylpropyl]carbamate

Analogously to Example 29-2, t-butyl N-[(1S)-1-{[4-(5-fluoro-2,3-dihydro-1H-indol-1-yl)piperidin-1-yl]carbonyl}-2-methylpropyl]carbamate (2.05 g, yield 93%) was obtained from t-butyl 4-(5-fluoro-2,3-dihydro-1H-indol-1-yl)piperidine-1-carboxylate (1.68 g, 5.24 mmol) as a colorless amorphous substance.
¹H-NMR (CDCl₃, 400 MHz) δ: 6.81 (1H, dd, J=8.4 Hz, 2.4 Hz), 6.78-6.71 (1H, m), 6.33-6.28 (1H, m), 5.34 (1H, d, J=8.4 Hz), 4.80-4.75 (1H, m), 4.52-4.48 (1H, m), 4.13-4.07 (1H, m), 3.58-3.50 (1H, m), 3.31 (2H, dd, J=16.8 Hz, 8.4 Hz), 3.19-3.09 (1H, m), 2.94-2.90 (2H, m), 2.68-2.60 (1H, m), 1.96-1.82 (3H, m), 1.69-1.49 (2H, m), 1.44 (9H, s), 0.97 (3H, dd, J=9.2 Hz, 6.8 Hz), 0.89 (3H, dd, J=9.6 Hz, 6.8 Hz).
MS (ESI, m/z): 420 (M+H)⁺.

### Example 48-3

### (2S)-1-[4-(5-Fluoro-2,3-dihydro-1H-indol-1-yl)piperidin-1-yl]-3-methyl-1-oxobutan-2-amine dihydrochloride

Analogously to Example 29-3, (2S)-1-[4-(5-fluoro-2,3-dihydro-1H-indol-1-yl)piperidin-1-yl]-3-methyl-1-oxobutan-2-amine dihydrochloride (1.96 g, yield 100%) was obtained from t-butyl [(1S)-1-{[4-(5-fluoro-2,3-dihydro-1H-indol-1-yl)piperidin-1-yl]carbonyl}-2-methylpropyl]carbamate (2.05 g, 4.89 mmol) as a pale red amorphous substance.
¹H-NMR (CD₃OD, 400 MHz) δ: 7.71-7.67 (1H, m), 7.31 (1H, d, J=8.2 Hz), 7.28-7.23 (1H, m), 4.70 (1H, d, J=13.6 Hz), 4.40-4.05 (5H, m), 3.36 (2H, t, J=7.2 Hz), 3.31-3.24 (1H, m), 2.81 (1H, t, J=12.8 Hz), 2.25-1.65 (5H, m), 1.14-1.00 (6H, m).
MS (ESI, m/z): 320 (M+H)⁺.

### Example 48-4

### N-[(1S)-1-{[4-(5-Fluoro-2,3-dihydro-1H-indol-1-yl)piperidin-1-yl]carbonyl}-2-methylpropyl]-3-hydroxyquinoxaline-2-carboxamide

Analogously to Example 29-4, the desired title compound (171 mg, yield 68%) was obtained from (2S)-1-[4-(5-fluoro-2,3-dihydro-1H-indol-1-yl)piperidin-1-yl]-3-methyl-1-oxobutan-2-amine dihydrochloride (200 mg, 0.510 mmol) as a yellow solid.
¹H-NMR (CDCl₃, 400 MHz) δ: 12.45 (1H, brs), 10.18 (1H, brs), 8.04-8.01 (1H, m), 7.64-7.39 (3H, m), 6.83-6.72 (2H, m), 6.35-6.30 (1H, m), 5.17-5.07 (1H, m), 4.90-4.86 (1H, m), 4.38 (1H, brs), 3.62-3.56 (1H, m), 3.38-3.21 (3H, m), 2.95 (1H, t, J=6.6 Hz), 2.90 (1H, t, J=6.6 Hz), 2.74 (1H, t, J=10.0 Hz), 2.36-2.28 (1H, m), 2.03-1.60 (4H, m), 1.16-1.09 (6H, m).
IR (KBr) cm⁻¹: 2960, 1685, 1640, 1490, 1475.
MS (ESI, m/z): 492 (M+H)⁺, 514 (M+Na)⁺.
HRMS (ESI, m/z): 492.2422 (Calcd for C₂₇H₃₁FN₅O₃: 492.2411).
Anal. Calcd for C₂₇H₃₀FN₅O₃: C, 65.97; H, 6.15; N, 14.25; F, 3.86. Found:: C, 65.74; H, 5.92; N, 14.07; F, 3.77.

### Example 49

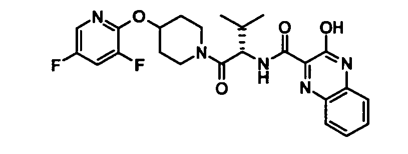

### N-[(1S)-1-({4-[(3,5-Difluoropyridin-2-yl)oxy]piperidin-1-yl}carbonyl)-2-methylpropyl}-3-hydroxyquinoxaline-2-carboxamide

### Example 49-1

### t-Butyl 4-[(3,5-difluoropyridin-2-yl)oxy]piperidine-1-carboxylate and t-butyl 4-[(2,3-difluoropyridin-5-yl)oxy]piperidine-1-carboxylate

t-Butyl 4-hydroxypiperidine-1-carboxylate (1.38 g, 6.63 mmol) was added to a N,N-dimethylformamide suspension (5.5 ml) of 60% sodium hydride (232 mg, 6.08 mmol), at 0°C, and stirring was carried out at room temperature for 30 minutes. Further, at 0°C, 2,3,5-trifluoropyridine (750 mg, 5.52 mmol) was added, and stirring was carried out at room temperature for 2 hours. Water was added to the reaction solution, followed by extraction with ethyl acetate and subsequent sequential washing with water and saline, and then the resulting organic layer was dried over anhydrous sodium sulfate. The organic layer was concentrated and the resulting residue was purified by silica gel column chromatography to afford t-butyl 4-[(3,5-difluoropyridin-2-yl)oxy]piperidine-1-carboxylate (654 mg, yield 38%) as a colorless oil, and t-butyl 4-[(2,3-difluoropyridin-5-yl)oxy]piperidine-1-carboxylate (1.00 g, yield 58%) as a colorless oil.
t-butyl 4-[(3,5-difluoropyridin-2-yl)oxy]piperidine-1-carboxylate ¹H-NMR (CDCl₃, 400 MHz) δ: 7.80 (1H, d, J=2.8 Hz), 7.22-7.17 (1H, m), 5.26-5.20 (1H, m), 3.81-3.75 (2H, m), 3.33-3.27 (2H, m), 2.02-1.96 (2H, m), 1.81-1.73 (2H, m), 1.47 (9H, s).
MS (ESI, m/z): 215 (M-Boc+H)⁺.
t-butyl 4-[(2,3-difluoropyridin-5-yl)oxy]piperidine-1-carboxylate ¹H-NMR (CDCl₃, 400 MHz) δ: 7.63 (1H, t, J=2.4 Hz), 7.12-7.07 (1H, m), 4.52-4.46 (1H, m), 3.73-3.67 (2H, m), 3.41-3.34 (2H, m), 1.97-1.90 (2H, m), 1.84-1.76 (2H, m), 1.47 (9H, s).
MS (ESI, m/z): 215 (M-Boc+H)⁺.

### Example 49-2

### t-Butyl [(1S)-1-({4-[(3,5-difluoropyridin-2-yl)oxy]piperidin-1-yl}carbonyl)-2-methylpropyl]carbamate

Analogously to Example 29-2, t-butyl [(1S)-1-({4-[(3,5-difluoropyridin-2-yl)oxy]piperidin-1-yl}carbonyl)-2-methylpropyl]carbamate (803 mg, yield 86%) was obtained from t-butyl 4-[(3,5-difluoropyridin-2-yl)oxy]piperidine-1-carboxylate (654 mg, 2.08 mmol) as a colorless amorphous substance.
¹H-NMR (CDCl₃, 400 MHz) δ: 7.80 (1H, t, J=3.2 Hz), 7.24-7.19 (1H, m), 5.37-5.28 (2H, m), 4.53-4.48 (1H, m), 3.97-3.45 (4H, m), 2.10-1.78 (5H, m), 1.44 (9H, s), 0.98-0.88 (6H, m).
MS (ESI, m/z): 414 (M+H)⁺.

### Example 49-3

### (2S)-1-{4-[(3,5-Difluoropyridin-2-yl)oxy]piperidin-1-yl}-3-methyl-1-oxobutan-2-amine dihydrochloride

Analogously to Example 29-3, (2S)-1-{4-[(3,5-difluoropyridin-2-yl)oxy]piperidin-1-yl}-3-methyl-1-oxobutan-2-amine dihydrochloride (673 mg, yield 90%) was obtained from t-butyl [(1S)-1-({4-[(3,5-difluoropyridin-2-yl)oxylpiperidin-1-yl}carbonyl]-2-methylpropyl}carbamate (803 mg, 1.94 mmol) as a colorless amorphous substance.
¹H-NMR (CD₃OD, 400 MHz) δ: 7.88 (1H, d, J=2.4 Hz), 7.57-7.51 (1H, m), 5.42-5.34 (1H, m), 4.35 (1H, d, J=5.2 Hz), 4.08-3.45 (4H, m), 2.25-1.75 (5H, m), 1.11 (3H, d, J=7.0 Hz), 1.03 (3H, d, J=7.0 Hz).
MS (ESI, m/z): 314 (M+H)⁺.

### Example 49-4

### N-[(1S)-1-({4-[(3,5-Difluoropyridin-2-yl)oxy]piperidin-1-yl}carbonyl)-2-methylpropyl]-3-hydroxyquinoxaline-2-carboxamide

Analogously to Example 29-4, the desired title compound (148 mg, yield 59%) was obtained from (2S)-1-{4-[(3,5-difluoropyridin-2-yl)oxy]piperidin-1-yl}-3-methyl-1-oxobutan-2-amine dihydrochloride (200 mg, 0.520 mmol) as a yellow solid.
¹H-NMR (CDCl₃, 400 MHz) δ: 12.58 (1H, brs), 10.16 (1H, brs), 8.00 (1H, brs), 7.81 (1H, dd, J=7.0 Hz, 2.0 Hz), 7.62-7.19 (4H, m), 5.40-5.33 (1H, m), 5.11-5.08 (1H, m), 4.04-3.66 (4H, m), 2.35-1.88 (5H, m), 1.14-1.10 (6H, m).
IR (KBr) cm⁻¹: 2965, 1690, 1640, 1470, 1440.
MS (ESI, m/z): 486 (M+H)⁺, 508 (M+Na)⁺.
HRMS (ESI, m/z): 486.1979 (Calcd for C₂₉H₂₆F₂N₅O₄: 486.1953).
Anal. Calcd for C₂₉H₂₅F₂N₅O₄: C, 59.38; H, 5.19; N, 14.43; F, 7.83. Found: C, 59.56; H, 5.15; N, 14.41; F, 8.09.

### Example 50

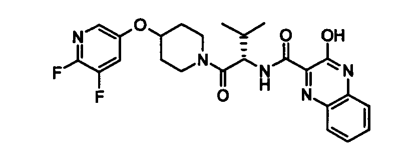

### N-[(1S)-1-({4-[(2,3-Difluoropyridin-5-yl)oxy]piperidin-1-yl}carbonyl)-2-methylpropyl]-3-hydroxyquinoxaline-2-carboxamide

### Example 50-1

### t-Butyl [(1S)-1-({4-[(2,3-difluoropyridin-5-yl)oxy]piperidin-1-yl}carbonyl)-2-methylpropyl]carbamate

Analogously to Example 29-2, t-butyl [(1S)-1-({4-[(2,3-difluoropyridin-5-yl)oxy]piperidin-1-yl}carbonyl)-2-methylpropyl]carbamate (1.13 g, yield 89%) was obtained from t-butyl 4-[(2,3-difluoropyridin-5-yl)oxy]piperidine-1-carboxylate (893 mg, 2.84 mmol) as a colorless amorphous substance.
¹H-NMR (CDCl₃, 400 MHz) δ: 7.66-7.64 (1H, m), 7.10 (1H, td, J=8.0 Hz, 2.8 Hz), 5.33-5.29 (1H, m), 4.63-4.46 (2H, m), 3.90-3.48 (4H, m), 2.06-1.82 (5H, m), 1.44 (9H, s), 0.97 (3H, d, J=6.6 Hz), 0.90 (3H, d, J=6.6 Hz).
MS (ESI, m/z): 314 (M-Boc+H)⁺.

### Example 50-2

### (2S)-1-{4-[(2,3-Difluoropyridin-5-yl)oxy]piperidin-1-yl}-3-methyl-1-oxobutan-2-amine dihydrochloride

Analogously to Example 29-3, (2S)-1-{4-[(2,3-difluoropyridin-5-yl)oxy]piperidin-1-yl}-3-methyl-1-oxobutan-2-amine dihydrochloride (968 mg, yield 92%) was obtained from t-butyl [(1S)-1-({4-[(2,3-difluoropyridin-5-yl)oxy]piperidin-1-yl}carbonyl)-2-methylpropyl}carbamate (1.13 g, 2.73 mmol) as a colorless amorphous substance.
¹H-NMR (CD₃OD, 400 MHz) δ: 7.65-7.59 (2H, m), 4.82-4.74 (1H, m), 4.35 (1H, d, J=4.0 Hz), 4.04-3.59 (3H, m), 3.53-3.47 (1H, m), 2.24-2.16 (1H, m), 2.12-1.99 (2H, m), 1.90-1.75 (2H, m), 1.11 (3H, d, J=5.6 Hz), 1.02 (3H, d, J=5.6 Hz).
MS (ESI, m/z): 314 (M+H)⁺.

### Example 50-3

### N-[(1S)-1-({4-[(2,3-Difluoropyridin-5-yl)oxy]piperidin-1-yl}carbonyl)-2-methylpropyl]-3-hydroxyquinoxaline-2-carboxamide

Analogously to Example 29-4, the desired title compound (196 mg, yield 78%) was obtained from (2S)-1-{4-[(2,3-difluoropyridin-5-yl)oxy]piperidin-1-yl}-3-methyl-1-oxobutan-2-amine dihydrochloride (200 mg, 0.520 mmol) as a yellow solid.
¹H-NMR (CDCl₃, 400 MHz) δ: 12.55 (1H, brs), 10.17 (1H, brs), 8.00 (1H, dd, J=12.6 Hz, 6.6 Hz), 7.66-7.39 (4H, m), 7.16-7.11 (1H, m), 5.07 (1H, q, J=5.7 Hz), 4.67-4.60 (1H, m), 4.12-3.63 (4H, m), 2.36-1.93 (5H, m), 1.15-1.09 (6H, m).
IR (KBr) cm⁻¹: 2965, 1690, 1630, 1525, 1460.
MS (ESI, m/z): 486 (M+H)⁺, 508 (M+Na)⁺.
HRMS (ESI, m/z): 486.1944 (Calcd for C₂₄H₂₆F₂N₅O₄: 486.1953).
Anal. Calcd for C₂₄H₂₅F₂N₅O₄: C, 59.38; H, 5.19; N, 14.43; F, 7.83. Found: C, 59.29; H, 5.11; N, 14.42; F, 7.58.

### Example 51

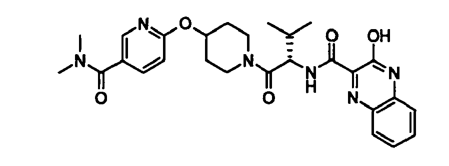

### N-{(1S)-1-[(4-{[5-(Dimethylcarbamoyl)pyridin-2-yl]oxy}piperidin-1-yl)carbonyl]-2-methylpropyl}-3-hydroxyquinoxaline-2-carboxamide

### Example 51-1

### 6-Chloro-N,N-dimethylnicotinamide

A tetrahydrofuran solution (14.2 ml, 28.4 mmol) of 2M-N,N-dimethylamine was added to a methylene chloride solution (17 ml) of 6-chloronicotinyl chloride (1.00 g, 5.68 mmol), at 0°C, and stirring was carried out at room temperature overnight. The reaction solution was concentrated and the resulting residue was diluted with methylene chloride, followed by sequential washing with water and saline, and the resulting organic layer was dried over anhydrous sodium sulfate. The organic layer was concentrated and the resulting residue was purified by silica gel column chromatography to afford 6-chloro-N,N-dimethylnicotinamide (1.02 g, yield 97%) as a pale red oil.
¹H-NMR (CDCl₃, 400 MHz) δ: 8.47 (1H, d, J=2.4 Hz), 7.75 (1H, dd, J=8.2 Hz, 2.4 Hz), 7.40 (1H, d, J=8.2 Hz), 3.13 (3H, s), 3.03 (3H, s).
MS (ESI, m/z): 185 (M+H)⁺.

### Example 51-2

### t-Butyl 4-{[5-(dimethylcarbamoyl)pyridin-2-yl]oxy}piperidine-1-carboxylate

t-Butyl 4-hydroxypiperidine-1-carboxylate (1.38 g, 6.63 mmol) was added to a N,N-dimethylformamide suspension (3.0 ml) of 60% sodium hydride (232 mg, 6.08 mmol), at 0°C, and stirring was carried out at room temperature for 30 minutes. Further, at 0°C, a N,N-dimethylformamide solution (2.5 ml) of 6-chloro-N,N-dimethylnicotinamide (1.02 g, 5.52 mmol) was added, and stirring was carried out at room temperature for 3 hours. Water was added to the reaction solution, followed by extraction with ethyl acetate, the extract was washed sequentially with water and saline, and the resulting organic layer was dried over anhydrous sodium sulfate. The organic layer was concentrated and the resulting residue was purified by silica gel column chromatography to afford t-butyl 4-{[5-(dimethylcarbamoyl)pyridin-2-yl]oxy}piperidine-1-carboxylate (1.63 g, yield 84%) as a colorless oil.
¹H-NMR (CDCl₃, 400 MHz) δ: 8.26-8.25 (1H, m), 7.70-7.67 (1H, m), 6.73 (1H, d, J=8.6 Hz), 5.29-5.22 (1H, m), 3.80-3.76 (2H, m), 3.32-3.26 (2H, m), 3.13-3.03 (6H, m), 2.01-1.96 (2H, m), 1.74-1.60 (2H, m), 1.47 (9H, s).
MS (ESI, m/z): 350 (M+H)⁺.

### Example 51-3

### t-Butyl {(1S)-1-[(4-{[5-(dimethylcarbamoyl)pyridin-2-yl]oxy}piperidin-1-yl)carbonyl]-2-methylpropyl}carbamate

Analogously to Example 29-2, t-butyl {(1S)-1-[(4-{[5-(dimethylcarbamoyl)pyridin-2-yl]oxy}piperidin-1-yl)carbonyl]-2-methylpropyl}carbamate (1.57 g, yield 78%) was obtained from t-butyl 4-{[5-(dimethylcarbamoyl)pyridin-2-yl]oxy}piperidine-1-carboxylate (1.56 g, 4.46 mmol) as a colorless amorphous substance.
¹H-NMR (CDCl₃, 400 MHz) δ: 8.25-8.25 (1H, m), 7.71-7.68 (1H, m), 6.74 (1H, d, J=6.6 Hz), 5.38-5.32 (2H, m), 4.53-4.50 (1H, m), 3.99-3.44 (4H, m), 3.09 (6H, brs), 2.09-1.80 (5H, m), 1.44 (9H, s), 0.98 (3H, d, J=5.6 Hz), 0.89 (3H, d, J=5.2 Hz).
MS (ESI, m/z): 349 (M-Boc+H)⁺.

### Example 51-4

### N,N-Dimethyl-6-[(1-L-valylpiperidin-4-yl)oxy]nicotinamide dihydrochloride

Analogously to Example 29-3, N,N-dimethyl-6-[(1-L-valylpiperidin-4-yl)oxy]nicotinamide dihydrochloride (1.61 g, yield 100%) was obtained from t-butyl {(1S)-1-[(4-{[5-(dimethylcarbamoyl)pyridin-2-yl]oxy}piperidin-1-yl)carbonyl]-2-methylpropyl}carbamate (1.57 g, 3.50 mmol) as a colorless amorphous substance.
¹H-NMR (CD₃OD, 400 MHz) δ: 8.39 (1H, t, J=3.2 Hz), 8.08 (1H, dq, J=8.8 Hz, 2.4 Hz), 7.22 (1H, t, J=8.8 Hz), 5.42-5.32 (1H, m), 4.38 (1H, d, J=4.3 Hz), 4.13-3.44 (4H, m), 3.09 (6H, d, J=10.9 Hz), 2.25-1.81 (5H, m), 1.12 (3H, d, J=7.0 Hz), 1.03 (3H, d, J=6.6 Hz).
MS (ESI, m/z): 349 (M+H)⁺.

### Example 51-5

### N-{(1S)-1-[(4-{[5-(Dimethylcarbamoyl)pyridin-2-yl]oxy}piperidin-1-yl)carbonyl]-2-methylpropyl}-3-hydroxyquinoxaline-2-carboxamide

Analogously to Example 29-4, the desired title compound (157 mg, yield 64%) was obtained from N,N-dimethyl-6-[(1-L-valylpiperidin-4-yl)oxy]nicotinamide dihydrochloride (200 mg, 0.470 mmol) as a yellow solid.
¹H-NMR (CDCl₃, 400 MHz) δ: 12.73 (1H, brs), 10.19 (1H, brs), 8.27 (1H, s), 8.00 (1H, d, J=7.4 Hz), 7.74-7.27 (4H, m), 6.79-6.73 (1H, m), 5.39 (1H, brs), 5.10 (1H, t, J=7.0 Hz), 4.11-3.60 (4H, m), 3.10 (6H, s), 2.35-1.89 (5H, m), 1.14-1.10 (6H, m);
IR (KBr) cm⁻¹: 3470, 2960, 1690, 1635, 1600, 1475.
MS (ESI, m/z): 521 (M+H)⁺, 543 (M+Na)⁺.
HRMS (ESI, m/z): 521.2527 (Calcd for C₂₇H₃₃N₆O₅: 521.2512).

### Example 52

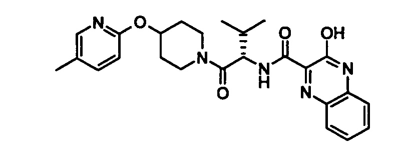

### 3-Hydroxy-N-[(1S)-1-({4-[(5-methylpyridin-2-yl)oxy]piperidin-1-yl}carbonyl)-2-methylpropyl]quinoxaline-2-carboxamide

A 40% toluene solution of diisopropyl azodicarboxylate (2.10 ml, 3.99 mmol)was added to a toluene solution (15 ml) of t-butyl {(1S)-1-[(4-hydroxypiperidin-1-yl)carbonyl]-2-methylpropyl}carbamate (1.00 g, 3.33 mmol), 2-hydroxy-5-methylpyridine (400 mg, 3.66 mmol) and triphenylphosphine (1.05 g, 3.99 mmol), at room temperature, and stirring was carried out at room temperature overnight. The reaction solution was concentrated and the resulting residue was purified by silica gel column chromatography to afford t-butyl [(1S)-1-({4-[(5-methylpyridin-2-yl)oxy]piperidin-1-yl}carbonyl)-2-methylpropyl]carbamate (2.06 g) as a mixture with impurities. This product was used as it is in the next reaction without further purification.

Concentrated hydrochloric acid (1.7 ml)was added to an ethanol solution (3.4 ml) of the resulting mixture (2.06 g), at 0°C, and stirring was carried out at room temperature overnight. The reaction solution was concentrated and the resulting residue was diluted with water, and washed with ethyl acetate. A saturated aqueous sodium hydrogencarbonate solution was added to the aqueous layer, followed by extraction with ethyl acetate. The resulting organic layer was dried over anhydrous sodium sulfate, and then concentrated to afford (2S)-3-methyl-1-{4-[(5-methylpyridin-2-yl)oxy]piperidin-1-yl}-1-oxobutan-2-amine (815 mg).

1-Ethyl-3-(3-dimethylaminopropyl)carbodiimide hydrochloride (157 mg, 0.820 mmol) was added to a methylene chloride solution (6.9 ml) of the resulting compound (200 mg), 3-hydroxyquinoxaline-2-carboxylic acid (135 mg, 0.690 mmol), 1-hydroxybenzotriazole monohydrate (102 mg, 0.760 mmol) and N-methylmorpholine (0.226 ml, 2.06 mmol), at room temperature, and stirring was carried out at room temperature overnight. Water was added to the reaction solution, followed by extraction with methylene chloride and subsequent sequential washing with a saturated aqueous sodium hydrogencarbonate solution, water and saline, and the resulting organic layer was dried over anhydrous sodium sulfate. After the organic layer was concentrated and the resulting residue was purified by silica gel column chromatography and molecular sieving, the residue resulting from concentration was suspended in a mixed solvent of ethanol-diethyl ether, and the solid substance was collected by filtration to afford the desired title compound (161 mg, 10%) as a yellow solid.
¹H-NMR (CDCl₃, 400 MHz) δ: 12.59 (1H, brs), 10.05 (1H, brs), 8.00 (1H, d, J=6.6 Hz), 7.95-7.93 (1H, m), 7.62-7.36 (4H, m), 6.64 (1H, dd, J=12.0 Hz, 8.4 Hz), 5.32-5.27 (1H, m), 5.13-5.09 (1H, m), 4.05-3.91 (2H, m), 3.77-3.63 (2H, m), 2.32-1.85 (8H, m), 1.10 (6H, d, J=6.6 Hz). IR (KBr) cm⁻¹: 3440, 2965, 1690, 1640, 1485.
MS (ESI, m/z): 464 (M+H)⁺, 486 (M+Na)⁺.
HRMS (ESI, m/z): 464.2319 (Calcd for C₂₅H₃₀N₅O₄: 464.2298).
Anal. Calcd for C₂₅H₂₉N₅O₄·0.5H₂O: C, 63.54; H, 6.40; N, 14.82. Found: C, 63.36; H, 6.20; N, 14.72.

### Example 53

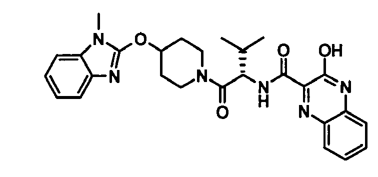

### 3-Hydroxy-N-[(1S)-2-methyl-1-({4-[(1-methyl-1H-benzimidazol-2-yl)oxy]piperidin-1-yl}carbonyl)propyl]quinoxaline-2-carboxamide

### Example 53-1

### 2-Chloro-1-methyl-1H-benzimidazole

Sodium hydride (purity 55%)(516 mg, 12.0 mmol) was added to a N,N-dimethylformamide solution (8.0 ml) of 2-chloro-1H-benzimidazole (1.52 g, 10.0 mmol), at 0°C, under nitrogen stream, and stirring was carried out for 20 minutes. Methyl iodide (0.750 ml, 12.0 mmol) was further added, and stirring was carried out under room temperature for 2 hours. Water was added to the reaction solution, and the resulting solid substance was collected by filtration to afford 2-chloro-1-methyl-1H-benzimidazole (1.51 g, yield 91%) as a white solid.

### Example 53-2

### t-Butyl 4-[(1-methyl-1H-benzimidazol-2-yl)oxy]piperidine-1-carboxylate

Sodium hydride (purity 55%)(129 mg, 3.00 mmol) was added to a N,N-dimethylformamide solution (4.0 ml) of 2-chloro-1-methyl-1H-benzimidazole (333 mg, 2.00 mmol) and t-butyl 4-hydroxypiperidine-1-carboxylate (402 mg, 2.00 mmol), at 0°C, under nitrogen stream, and stirring was carried out at room temperature overnight. The reaction solution was poured into water, followed by extraction with ethyl acetate and subsequent sequential washing with water and saline, and then the resulting organic layer was dried over anhydrous sodium sulfate. The organic layer was concentrated and the resulting residue was purified by silica gel column chromatography to afford t-butyl 4-[(1-methyl-1H-benzimidazol-2-yl)oxy]piperidine-1-carboxylate (548 mg, yield 83%) as a white solid.
¹H-NMR (CDCl₃, 400 MHz) δ: 7.53 (1H, m), 7.17 (3H, m), 5.32 (1H, m), 3.77 (2H, m), 3.56 (3H, s), 3.34 (2H, m), 2.11 (2H, m), 1.84 (2H, m), 1.48 (9H, s).
MS (ESI, m/z): 332 (M+H)⁺.

### Example 53-3

### t-Butyl [(1S)-2-methyl-1-({4-[(1-methyl-1H-benzimidazol-2-yl)oxy]piperidin-1-yl}carbonyl)propyl]carbamate

Analogously to Example 29-2, t-butyl [(1S)-2-methyl-1-({4-[(1-methyl-1H-benzimidazol-2-yl)oxy]piperidin-1-yl}carbonyl)propyl]carbamate (504 mg, yield 71%) was obtained from t-butyl 4-[(1-methyl-1H-benzimidazol-2-yl)oxy]piperidine-1-carboxylate (548 mg, 1.66 mmol) as a colorless oil.
¹H-NMR (CDCl₃, 400 MHz) δ: 7.53 (1H, m), 7.17 (3H, m), 5.38 (1H, m), 5.34 (1H, d, J=9.2 Hz), 4.19-3.60 (2H, m), 3.57 and 3.56 (3H, s), 3.47 (2H, m), 2.13 (2H, m), 1.92 (3H, m), 1.44 (9H, s), 0.99 (3H, d, J=6.4 Hz), 0.90 (3H, d, J=6.4 Hz).
MS (ESI, m/z): 431 (M+H)⁺.

### Example 53-4

### 3-Hydroxy-N-[(1S)-2-methyl-1-({4-[(1-methyl-1H-benzimidazol-2-yl)oxy]piperidin-1-yl}carbonyl)propyl]quinoxaline-2-carboxamide

Analogously to Example 1-3, (2S)-3-methyl-1-{4-[(1-methyl-1H-benzimidazol-2-yl)oxy]piperidin-1-yl}-1-oxobutan-2-amine hydrochloride was afforded from t-butyl [(1S)-2-methyl-1-({4-[(1-methyl-1H-benzimidazol-2-yl)oxy]piperidin-1-yl}carbonyl)propyl]carbamate (504 mg, 1.17 mmol) as a white solid. The resulting compound was condensed with 3-hydroxyquinoxaline-2-carboxylic acid (222 mg, 1.17 mmol) to afford the desired title compound (524 mg, yield 89%) as a pale yellow solid.
¹H-NMR (DMSO-d₆, 400 MHz) δ: 12.83 (1H, s), 9.50 (1H, d, J=8.4 Hz), 7.87 (1H, t, J=6.8 Hz), 7.64 (1H, dt, J=8.0 Hz, 1.6.Hz), 7.41-7.32 (4H, m), 7.12-7.07 (2H, m), 5.31 (1H, m), 4.94 (1H, q, J=7.6 Hz), 3.96-3.28 (4H, m), 3.57 and 3.56 (3H, s), 2.30-1.70 (5H, m), 0.95 (6H, m).
IR (KBr) cm⁻¹: 2965, 1690, 1630, 1530, 1450, 1020.
MS (ESI, m/z): 503 (M+H)⁺.
HRMS (ESI, m/z): 503.2425 (Calcd for C₂₇H₃₁N₆O₄: 503.2407).

### Example 54

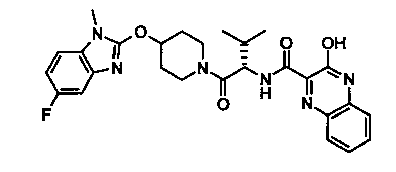

### N-[(1S)-1-({4-[(5-Fluoro-1-methyl-1H-benzimidazol-2-yl)oxy]piperidin-1-yl}carbonyl)-2-methylpropyl]-3-hydroxyquinoxaline-2-carboxamide

### Example 54-1

### N-Methyl-4-fluoro-2-nitroaniline

Potassium carbonate (2.61 g, 18.9 mmol) was added to a N,N-dimethylformamide solution (20 ml) of 2,5-difluoronitrobenzene (1.00 g, 6.29 mmol) and methylamine hydrochloride (509 mg, 7.54 mmol), under nitrogen stream, and stirring was carried out at 120°C for 1.5 hours. The reaction solution was filtered, water was added to the filtrate, followed by extraction with ethyl acetate and sequential washing with water and saline, and then the resulting organic layer was dried over anhydrous sodium sulfate. The organic layer was concentrated and the resulting residue was purified by a medium-pressure preparative liquid chromatograph (manufactured by Biotage, Inc., 40+M) to afford N-methyl-4-fluoro-2-nitroaniline (919 mg, yield 87%) as an orange solid. ¹H-NMR (CDCl₃, 400 MHz) δ: 7.94 (1H, brs), 7.90 (1H, dd, J=9.4 Hz, 2.9 Hz), 7.31-7.27 (1H, m), 6.83 (1H, dd, J=9.4 Hz, 4.7 Hz), 3.04 (3H, d, J=5.5 Hz).

### Example 54-2

### 5-Fluoro-1-methyl-1H-benzimidazol-2-ol

5% Palladium carbon catalyst (1.15 g) was added to a methanol solution (30 ml) of N-methyl-4-fluoro-2-nitroaniline (919 mg, 5.40 mmol), under hydrogen atmosphere, and stirring was carried out at room temperature for 4 hours. After the reaction solution was celite filtered, the filtrate was concentrated and the resulting residue was dissolved in a 1,4-dioxane solution (30 ml), and N,N'-carbonyldiimidazole (1.31 g, 8.10 mmol) was added, followed by heating to reflux overnight. The reaction solution was cooled to room temperature, and water was added, followed by extraction with ethyl acetate. The extract was washed sequentially with water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford 5-fluoro-1-methyl-1H-benzimidazol-2-ol (583 mg, yield 65%) as a colorless amorphous substance.
¹H-NMR (CDCl₃, 400 MHz) δ: 8.86 (1H, brs), 7.12-7.06 (1H, m), 6.87-6.82 (2H, m), 3.42 (3H, d, J=6.3 Hz).
MS (EI, m/z): 166 (M⁺).

### Example 54-3

### t-Butyl 4-[(5-fluoro-1-methyl-1H-benzimidazol-2-yl)oxy]piperidine-1-carboxylate

Phosphorus oxychloride (5.0 ml) was added to 5-fluoro-1-methyl-1H-benzimidazol-2-ol (225 mg, 1.35mmol), under nitrogen stream, followed by heating to reflux for 5 hours. A saturated aqueous sodium hydrogencarbonate solution was poured under ice cooling into the residue resulting from concentration of the reaction solution under reduced pressure, followed by extraction with ethyl acetate. The extract was washed sequentially with water and saline, and then the resulting organic layer was dried over anhydrous sodium sulfate. The organic layer was concentrated and the resulting compound was dissolved in a N,N-dimethylformamide solution (10 ml), and, at 0°C, 63% sodium hydride (93.0 mg, 2.44 mmol) and t-butyl 4-hydroxypiperidine-1-carboxylate (491 mg, 2.44 mmol) were added, followed by stirring under room temperature for 6 hours. Water was added to the reaction solution under ice cooling, followed by extraction with ethyl acetate. The extract was washed sequentially with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford t-butyl 4-[(5-fluoro-1-methyl-1H-benzimidazol-2-yl)oxy]piperidine-1-carboxylate (108 mg, yield 20%) as a white solid.
¹H-NMR (CDCl₃) δ: 7.22 (1H, dd, J=9.6 Hz, 2.0 Hz), 7.04 (1H, dd, J=8.0 Hz, 4.7 Hz), 6.89 (1H, ddd, J=10.5 Hz, 8.0 Hz, 2.0 Hz), 5.32-5.26 (1H, m), 3.80-3.78 (2H, m), 3.54 (3H, s), 3.36-3.32 (2H, m), 2.11-2.09 (2H, m), 1.85-1.82 (2H, m), 1.48-1.46 (9H, m).
MS (FAB, m/z): 350 (M+H)⁺.

### Example 54-4

### t-Butyl [(1S)-1-({4-[(5-fluoro-1-methyl-1H-benzimidazol-2-yl)oxy]piperidin-1-yl}carbonyl)-2-methylpropyl]carbamate

Analogously to Example 29-2, t-butyl [(1S)-1-({4-[(5-fluoro-1-methyl-1H-benzimidazol-2-yl)oxy]piperidin-1-yl}carbonyl)-2-methylpropyl]carbamate (128 mg, yield 93%) was obtained from t-butyl 4-[(5-fluoro-1-methyl-1H-benzimidazol-2-yl)oxy]piperidine-1-carboxylate (108 mg, 0.309 mmol) as a colorless amorphous substance. ¹H-NMR (CDCl₃, 500 MHz) δ: 7.22 (1H, dd, J=9.6 Hz, 2.0 Hz), 7.04 (1H, dd, J=8.0 Hz, 4.7 Hz), 6.89 (1H, ddd, J=10.5 Hz, 8.0 Hz, 2.0 Hz), 5.37-5.36 (2H, m), 4.52-4.50 (1H, m), 4.07-3.76 (2H, m), 3.68-3.46 (2H, m), 3.55 (3H, d, J=7.3 Hz), 2.24-2.10 (2H, m), 1.96-1.89 (3H, m), 1.44-1.40 (9H, m), 0.98 (3H, t, J=6.6 Hz), 0.91 (3H, t, J=6.6 Hz).
MS (FAB, m/z): 406 (M+H)⁺.

### Example 54-5

### N-[(1S)-1-({4-[(5-Fluoro-1-methyl-1H-benzimidazol-2-yl)oxy]piperidin-1-yl}carbonyl)-2-methylpropyl]-3-hydroxyquinoxaline-2-carboxamide

Analogously to Example 1-3, (2S)-1-{4-[(5-fluoro-1-methyl-1H-benzimidazol-2-yl)oxy]piperidin-1-yl}-3-methyl-1-oxobutan-2-amine hydrochloride (119 mg) was obtained from t-butyl [(1S)-1-({4-[(5-fluoro-1-methyl-1H-benzimidazol-2-yl)oxy]piperidin-1-yl}carbonyl)-2-methylpropyl]carbamate (128 mg, 0.285 mmol) as a white solid. The resulting compound (119 mg, 0.285 mmol) was condensed with 3-hydroxyquinoxaline-2-carboxylic acid (81.0 mg, 0.428 mmol) to afford the desired title compound (111 mg, yield 75%) as a pale yellow solid. ¹H-NMR (DMSO-d₆, 400 MHz) δ: 12.84 (1H, brs), 9.57 (1H, brs), 7.87 (1H, t, J=6.8 Hz), 7.64 (1H, d, J=16.4 Hz), 7.40-7.33 (3H, m), 7.24 (1H, dd, J=9.6 Hz, 2.0 Hz), 6.96 (1H, ddd, J=10.5 Hz, 8.0 Hz, 2.0 Hz), 5.31-5.29 (1H, m), 4.96-4.92 (1H, m), 3.94-3.63 (4H, m), 3.57 (3H, d, J=9.4 Hz), 2.00-1.89 (5H, m), 0.96 (6H, t, J=6.6 Hz).
IR (KBr) cm⁻¹: 2965, 1690, 1640, 1530, 1440, 1140.
MS (ESI, m/z): 521 (M+H)⁺.
HRMS (ESI, m/z): 521.2320 (Calcd for C₂₇H₃₀FN₆O₄: 521.2312).

### Example 55

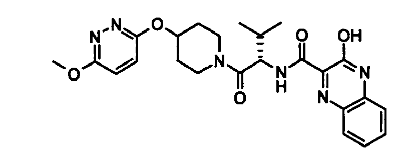

### 3-Hydroxy-N-[(1S)-1-({4-[(6-methoxypyridazin-3-yl)oxy]piperidin-1-yl}carbonyl)-2-methylpropyl]quinoxaline-2-carboxamide

### Example 55-1

### t-Butyl 4-[(6-methoxypyridazin-3-yl)oxy]piperidine-1-carboxylate

Analogously to Example 53-2, t-butyl 4-[(6-methoxypyridazin-3-yl)oxy]piperidine-1-carboxylate (964 mg, yield 78%) was obtained from 3-chloro-6-methoxypyridazine (578 mg, 4.00 mmol) and t-butyl 4-hydroxypiperidine-1-carboxylate (924 mg, 4.60 mmol) as a white solid. ¹H-NMR (CDCl₃, 400 MHz) δ: 6.94 (1H, d, J=8.8 Hz), 6.89 (1H, d, J=8.8 Hz), 5.32 (1H, m), 4.04 (3H, s), 3.80 (2H, m), 3.22 (2H, m), 2.10 (2H, m), 1.75 (2H, m), 1.47 (9H, s).
MS (ESI, m/z): 310 (M+H)⁺.

### Example 55-2

### t-Butyl [(1S)-1-({4-[(6-methoxypyridazin-3-yl)oxy]piperidin-1-yl}carbonyl)-2-methylpropyl]carbamate

Analogously to Example 29-2, t-butyl [(1S)-1-({4-[(6-methoxypyridazin-3-yl)oxy]piperidin-1-yl}carbonyl)-2-methylpropyl]carbamate (157 mg, yield 68%) was obtained from t-butyl 4-[(6-methoxypyridazin-3-yl)oxy]piperidine-1-carboxylate (175 mg, 0.566 mmol) as a colorless oil.
¹H-NMR (CDCl₃, 400 MHz) δ: 6.93 (1H, d, J=9.2 Hz), 6.90 (1H, dd, J=9.2 Hz, 2.4 Hz), 5.40 (1H, m), 5.37 (1H, m), 4.50 (1H, q, J=5.6 Hz), 4.05 (3H, s), 4.05-3.75 (2H, m), 3.55-3.35 (2H, m), 2.20-2.05 (2H, m), 1.95 (1H, m), 1.95-1.75 (2H, m), 1.44 (9H, s), 0.98 (3H, d, J=6.8 Hz), 0.89 (3H, d, J=6.8 Hz).
MS (ESI, m/z): 409 (M+H)⁺.

### Example 55-3

### 3-Hydroxy-N-[(1S)-1-({4-[(6-methoxypyridazin-3-yl)oxy]piperidin-1-yl}carbonyl)-2-methylpropyl]quinoxaline-2-carboxamide

Analogously to Example 1-3, (2S)-1-{4-[(6-methoxypyridazin-3-yl)oxy]piperidin-1-yl}-3-methyl-1-oxobutan-2-amine hydrochloride was obtained from t-butyl [(1S)-1-({4-[(6-methoxypyridazin-3-yl)oxy]piperidin-1-yl}carbonyl)-2-methylpropyl]carbamate (156 mg, 0.382 mmol) as a white solid. The resulting compound was condensed with 3-hydroxyquinoxaline-2-carboxylic acid (72.6 mg, 0.382 mmol) to afford the desired title compound (82.7 mg, yield 45%) as a pale yellow solid.
¹H-NMR (DMSO-d₆, 400 MHz) δ: 12.83 (1H, brs), 9.52 (1H, brs), 7.86 (1H, t, J=6.8 Hz), 7.64 (1H, dt, J=8.0 Hz, 1.6 Hz), 7.41-7.36 (2H, m), 7.22-7.16 (2H, m), 5.31 (1H, m), 4.92 (1H, q, J=7.6 Hz), 4.08-3.28 (4H, m), 3.94 (3H, s), 2.13-2.00 (2H, m), 2.05 (1H, m), 1.79-1.57 (2H, m), 0.95 (6H, t, J=6.4 Hz).
IR (KBr) cm⁻¹: 2960, 1685, 1640, 1670, 1420, 1270, 1015.
MS (ESI, m/z): 481 (M+H)⁺.
HRMS (ESI, m/z): 481.2206 (Calcd for C₂₄H₂₉N₆O₅: 481.2199).
Anal. Calcd for C₂₄H₂₈N₆O₅·0.5H₂O: C, 58.89; H, 5.97; N, 17.17. Found: C, 59.01; H, 5.82; N, 16.77.

### Example 56

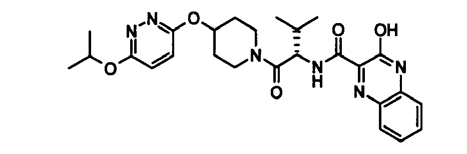

### 3-Hydroxy-N-[(1S)-1-({4-[(6-isopropoxypyridazin-3-yl)oxy]piperidin-1-yl}carbonyl)-2-methylpropyl]quinoxaline-2-carboxamide

### Example 56-1

### 3-Chloro-6-isopropoxypyridazine

2-Propanol (0.770 ml, 10.0 mmol) was added to a N,N-dimethylformamide solution (2.0 ml) of sodium hydride (purity 55%)(218 mg, 5.00 mmol), under nitrogen stream, and stirring was carried out for 15 minutes. Further, 3,6-dichloropyridazine (745 mg, 5.00 mmol) was added, and stirring was carried out at room temperature for 1 hour. The reaction solution was poured into a saturated aqueous ammonium chloride solution, followed by extraction with ethyl acetate and subsequent sequential washing with water and saline, and then the resulting organic layer was dried over anhydrous sodium sulfate. The organic layer was concentrated and the resulting residue was purified by silica gel column chromatography to afford 3-chloro-6-isopropoxypyridazine (486 mg, yield 56%) as a white solid.
¹H-NMR (CDCl₃, 400 MHz) δ: 7.33 (1H, d, J=7.2 Hz), 6.87 (1H, d, J=7.2 Hz), 5.18 (1H, m), 1.40 (6H, d, J=6.0 Hz).

### Example 56-2

### t-Butyl 4-[(6-isopropoxypyridazin-3-yl)oxy]piperidine-1-carboxylate

Analogously to Example 53-2, t-butyl 4-[(6-isopropoxypyridazin-3-yl)oxy]piperidine-1-carboxylate (631 mg, yield 66%) was obtained from 3-chloro-6-isopropoxypyridazine (486 mg, 2.86 mmol) and t-butyl 4-hydroxypiperidine-1-carboxylate (790 mg, 3.92 mmol) as a white solid.
¹H-NMR (CDCl₃, 400 MHz) δ: 6.87 (2H, s), 5.41 (1H, m), 5.32 (1H, m), 3.80 (2H, brs), 3.22 (2H, m), 2.05 (2H, m), 1.75 (2H, m), 1.46 (9H, s), 1.38 (6H, d, J=6.0 Hz).

### Example 56-3

### t-Butyl [(1S)-1-({4-[(6-isopropoxypyridazin-3-yl)oxy]piperidin-1-yl}carbonyl)-2-methylpropyl]carbamate

Analogously to Example 29-2, t-butyl [(1S)-1-({4-[(6-isopropoxypyridazin-3-yl)oxy]piperidin-1-yl}carbonyl)-2-methylpropyl]carbamate (586 mg, yield 72%) was obtained from t-butyl 4-[(6-isopropoxypyridazin-3-yl)oxy]piperidine-1-carboxylate (631 mg, 1.87 mmol) as a colorless amorphous substance.
¹H-NMR (CDCl₃, 400 MHz) δ: 6.88 (2H, s), 6.88-5.44 (3H, m), 6.88-5.44 (1H, m), 4.10-3.76 (2H, m), 3.53-3.37 (2H, m), 2.19-1.76 (5H, m), 1.44 (9H, s), 1.39 (6H, d, J=5.9 Hz), 0.99 (3H, d, J=6.8 Hz), 0.89 (3H, d, J=6.8 Hz).
MS (ESI, m/z): 437 (M+H)⁺.

### Example 56-4

### 3-Hydroxy-N-[(1S)-1-({4-[(6-isopropoxypyridazin-3-yl)oxy]piperidin-1-yl}carbonyl)-2-methylpropyl]quinoxaline-2-carboxamide

Analogously to Example 1-3, (2S)-1-{4-[(6-isopropoxypyridazin-3-yl)oxy]piperidin-1-yl}-3-methyl-1-oxybutan-2-amine hydrochloride was obtained from t-butyl [(1S)-1-({4-[(6-isopropoxypyridazin-3-yl)oxy]piperidin-1-yl}carbonyl)-2-methylpropyl]carbamate (574 mg, 1.31 mmol) as a white solid. The resulting compound was condensed with 3-hydroxyquinoxaline-2-carboxylic acid (249 mg, 1.31 mmol) to afford the desired title compound (581 mg, yield 87%) as a pale yellow solid. ¹H-NMR (DMSO-d₆, 400 MHz) δ: 12.83 (1H, brs), 9.51 (1H, m), 7.88 (1H, m), 7.66 (1H, t, J=3.2 Hz), 7.41-7.37 (2H, m), 7.19-7.11 (2H, m), 5.35-5.26 (2H, m), 4.96-4.91 (1H, m), 4.08-3.92 (2H, m), 3.60-3.21 (2H, m), 2.20-2.02 (3H, m), 1.80-1.53 (2H, m), 1.34 (6H, d, J=6.0 Hz), 0.97-0.94 (6H, m).
IR (KBr) cm⁻¹: 2970, 1690, 1645, 1630, 1440, 1270.
MS (ESI, m/z): 509 (M+H)⁺.
HRMS (ESI, m/z): 509.2523 (Calcd for C₂₆H₃₃N₆O₅: 509.2512).
Anal. Calcd for C₂₆H₃₂N₆O₅·1/2H₂O: C, 60.34; H, 6.43; N, 16.24. Found: C, 60.43; H, 6.27; N, 16.15.

### Example 57

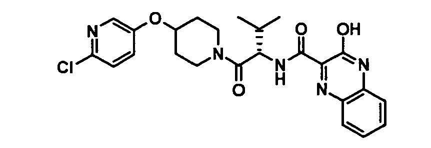

### N-[(1S)-1-({4-[(2-Chloropyridin-5-yl)oxy]piperidin-1-yl}carbonyl)-2-methylpropyl]-3-hydroxyquinoxaline-2-carboxamide

### Example 57-1

### t-Butyl 4-[(2-chloropyridin-5-yl)oxy]piperidine-1-carboxylate

Analogously to Example 53-2, t-butyl 4-[(2-chloropyridin-5-yl)oxy]piperidine-1-carboxylate (811 mg, yield 86%) was obtained from 2-chloro-5-fluoropyridine (393 mg, 3.00 mmol) and t-butyl 4-hydroxypiperidine-1-carboxylate (603 mg, 3.00 mmol) as a white solid. ¹H-NMR (CDCl₃, 400 MHz) δ: 8.07 (1H, dd, J=2.8 Hz, 0.8Hz), 7.25 (1H, d, J=8.2 Hz), 7.20 (1H, dd, J=8.8 Hz, 2.8 Hz), 4.49-4.45 (1H, m), 3.73-3.67 (2H, m), 3.38-3.32 (2H, m), 1.97-1.90 (2H, m), 1.79-1.71 (2H, m), 1.47 (9H, s).

### Example 57-2

### t-Butyl [(1S)-1-({4-[(2-chloropyridin-5-yl)oxy]piperidin-1-yl}carbonyl)-2-methylpropyl]carbamate

Analogously to Example 29-2, t-butyl [(1S)-1-({4-[(2-chloropyridin-5-yl)oxy]piperidin-1-yl}carbonyl)-2-methylpropyl]carbamate (321 mg, yield 52%) was obtained from t-butyl 4-[(2-chloropyridin-5-yl)oxy]piperidine-1-carboxylate (468 mg, 1.50 mmol) as a colorless amorphous substance.
¹H-NMR (CDCl₃, 400 MHz) δ: 8.08 (1H, d, J=2.8 Hz), 7.26 (1H, d, J=9.4 Hz), 7.21 (1H, dd, J=8.8 Hz, 3.0 Hz), 5.33 (1H, d, J=8.2 Hz), 4.62-4.53 (1H, m), 4.50 (1H, dd, J=9.2 Hz, 5.7 Hz), 3.86-3.47 (4H, m), 2.04-1.78 (5H, m), 1.44 (9H, s), 0.97 (3H, d, J=6.7 Hz), 0.90 (3H, d, J=7.1Hz).

### Example 57-3

### N-[(1S)-1-({4-[(2-Chloropyridin-5-yl)oxy]piperidin-1-yl}carbonyl)-2-methylpropyl]-3-hydroxyquinoxaline-2-carboxamide

Analogously to Example 1-3, (2S)-1-{4-[(2-chloropyridin-5-yl)oxy]piperidin-1-yl}-3-methyl-1-oxobutan-2-amine hydrochloride was obtained from t-butyl [(1S)-1-({4-[(2-chloropyridin-5-yl)oxy]piperidin-1-yl}carbonyl)-2-methylpropyl]carbamate (315 mg, 0.766 mmol) as a white solid. The resulting compound was condensed with 3-hydroxyquinoxaline-2-carboxylic acid (146 mg, 0.766 mmol) to afford the desired title compound (298 mg, yield 80%) as a pale yellow solid.
¹H-NMR (DMSO-d₆, 400 MHz) δ: 12.85 (1H, brs), 9.51 (1H, brs), 8.19 (1H, dd, J=5.4 Hz, 3.2 Hz), 7.87 (1H, dd, J=7.6 Hz, 4.9 Hz), 7.65 (1H, t, J=7.8 Hz), 7.62-7.57 (1H, m), 7.46 (1H, d, J=8.6 Hz), 7.41-7.37 (2H, m), 4.91 (1H, dd, J=13.8 Hz, 7.7 Hz), 4.81-4.71 (1H, m), 4.04-3.84 (2H, m), 3.58-3.20 (2H, m), 2.12-1.90 (3H, m), 1.76-1.47 (2H, m), 0.96 (3H, d, J=3.9 Hz), 0.94 (3H, d, J=3.9 Hz).
IR (KBr) cm⁻¹: 2960, 1685, 1640, 1450, 1270.
MS (ESI, m/z): 484 (M+H)⁺.
HRMS (ESI, m/z): 484.1769 (Calcd for C₂₄H₂₇ClN₅O₄: 484.1752).
Anal. Calcd for C₂₄H₂₆ClN₅O₄: C, 59.56; H, 5.42; N, 14.47. Found: C, 59.60; H, 5.42; N, 14.38.

### Example 58

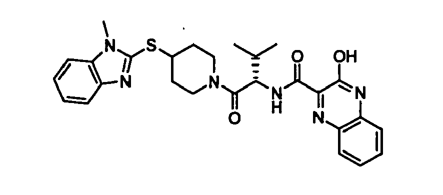

### 3-Hydroxy-N-[(1S)-2-methyl-1-({4-[(1-methyl-1H-benzimidazol-2-yl)thio]piperidin-1-yl}carbonyl)propyl]quinoxaline-2-carboxamide

### Example 58-1

### t-Butyl 4-[(1H-benzimidazol-2-yl)thio]piperidine-1-carboxylate

Potassium carbonate (414 mg, 3.00 mmol) was added to an ethanol solution (10 ml) of 4-methanesulfonyloxypiperidine (834 mg, 3.00 mmol) and 2-mercaptobenzimidazole (450 mg, 3.00 mmol), at room temperature, under nitrogen stream, and stirring was carried out for 16 hours under heating to reflux. The reaction solution was poured into a saturated aqueous ammonium chloride solution, followed by extraction with ethyl acetate. The extract was washed sequentially with water and saline, and then the resulting organic layer was dried over anhydrous sodium sulfate. The organic layer was concentrated and the resulting residue was suspended in a mixed solvent of ethyl acetate-methanol, and the resulting precipitate was collected by filtration to afford t-butyl 4-[(1H-benzimidazol-2-yl)thio]piperidine-1-carboxylate (473 mg, yield 47%) as a white solid.
¹H-NMR (CDCl₃, 400 MHz) δ: 9.19 (1H, brs), 7.68 (1H, brs), 7.35 (1H, brs), 7.22 (2H, m), 4.01 (1H, m), 3.39 (2H, m), 3.06 (1H, t, J=10.8 Hz), 2.13 (2H, m), 1.70 (2H, m), 1.46 (9H, s).

### Example 58-2

### t-Butyl 4-[(1-methyl-1H-benzimidazol-2-yl)thio]piperidine-1-carboxylate

Butyllithium (1.58M hexane solution)(1.08 ml, 1.70 mmol) was added to a tetrahydrofuran solution (10 ml) of t-butyl 4-[(1H-benzimidazol-2-yl)thio]piperidine-1-carboxylate (473 mg, 1.42 mmol), at 0°C, under nitrogen stream, and stirring was carried out for 15 minutes, followed by further addition of methyl iodide (0.180 ml, 2.84 mmol) and stirring at room temperature for 2 hours. The reaction solution was poured into a saturated aqueous ammonium chloride solution, followed by extraction with ethyl acetate. The extract was washed sequentially with water and saline, and then the resulting organic layer was dried over anhydrous sodium sulfate. The organic layer was concentrated and the resulting residue was purified by silica gel column chromatography to afford t-butyl 4-[(1-methyl-1H-benzimidazol-2-yl)thio]piperidine-1-carboxylate (493 mg, yield 100%) as a colorless oil.
¹H-NMR (CDCl₃, 400 MHz) δ: 7.66 (1H, m), 7.26-7.22 (3H, m), 4.11 (1H, m), 3.95 (2H, m), 3.70 (3H, s), 3.11 (1H, t, J=10.8 Hz), 2.16 (2H, m), 1.71 (2H, m), 1.46 (9H, s).

### Example 58-3

### t-Butyl [(1S)-1-({4-[(1-methyl-1H-benzimidazol-2-yl)thio]piperidin-1-yl}carbonyl)-2-methylpropyl]carbamate

Analogously to Example 29-2, t-butyl [(1S)-1-({4-[(1-methyl-1H-benzimidazol-2-yl)thio]piperidin-1-yl}carbonyl)-2-methylpropyl]carbamate (361 mg, yield 57%) was obtained from t-butyl 4-[(1-methyl-1H-benzimidazol-2-yl)thio]piperidine-1-carboxylate (493 mg, 1.42 mmol) as a colorless amorphous substance.
¹H-NMR (CDCl₃, 400 MHz) δ: 7.67 (1H, m), 7.24 (3H, m), 5.34 (1H, d, J=7.2 Hz), 4.48 (1H, dd, J=7.2 Hz, 3.2 Hz), 4.39-4.29 (1H, m), 4.20 (1H, m), 3.93 (1H, t, J=12.0 Hz), 3.70 and 3.69 (3H, s), 3.43-3.33 (1H, m), 3.19-3.08 (1H, m), 2.34-2.20 (2H, m), 1.92 (1H, m), 1.81-1.68 (2H, m), 1.44 (9H, s), 0.97 (3H, t, J=6.4 Hz), 0.89 (3H, d, J=6.4 Hz). MS (ESI, m/z): 447 (M+H)⁺.

### Example 58-4

### 3-Hydroxy-N-[(1S)-2-methyl-1-({4-[(1-methyl-1H-benzimidazol-2-yl)thio]piperidin-1-yl}carbonyl)propyl]quinoxaline-2-carboxamide

Analogously to Example 1-3, (2S)-3-methyl-1-{4-[(1-methyl-1H-benzimidazol-2-yl)thio]piperidin-1-yl}-1-oxobutan-2-amine hydrochloride was obtained from t-butyl [(1S)-1-({4-[(1-methyl-1H-benzimidazol-2-yl)thio]piperidin-1-yl}carbonyl)-2-methylpropyl]carbamate (361 mg, 0.809 mmol) as a white solid. The resulting compound was condensed with 3-hydroxyquinoxaline-2-carboxylic acid (154 mg, 0.809 mmol) to afford the desired title compound (331 mg, yield 79%) as a pale yellow solid.
¹H-NMR (DMSO-d₆, 400 MHz) δ: 12.85 (1H, brs), 9.50 (1H, t, J=9.6 Hz), 7.88 (1H, dd, J=8.4 Hz, 1.4 Hz), 7.63 (1H, t, J=17.0 Hz), 7.57 (1H, d, J=16.2 Hz), 7.50 (1H, m), 7.41-7.37 (2H, m), 7.24-7.15 (2H, m), 4.91 (1H, dd, J=13.3 Hz, 7.8 Hz), 4.33-4.06 (3H, m), 3.70 (3H, d, J=7.8 Hz), 3.49-3.38 (1H, m), 3.12-2.99 (1H, m), 2.29-2.15 (2H, m), 2.06 (1H, m), 1.85-1.53 (2H, m), 0.96-0.93 (6H, m).
IR (KBr) cm⁻¹: 2960, 1690, 1630, 1530, 1450, 1275.
MS (ESI, m/z): 519 (M+H)⁺.
HRMS (ESI, m/z): 519.2178 (Calcd for C₂₇H₃₁N₆O₃S: 519.2178).
Anal. Calcd for C₂₇H₃₀N₆O₃S: C, 62.53; H, 5.83; N, 16.20. Found: C, 62.39; H, 5.79; N, 16.09.

### Example 59

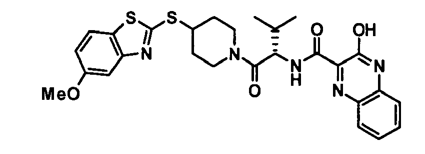

### 3-Hydroxy-N-[(1S)-1-({4-[(5-methoxybenzothiazol-2-yl)thio]piperidin-1-yl}carbonyl)-2-methylpropyl]quinoxaline-2-carboxamide

### Example 59-1

### t-Butyl 4-[(5-methoxybenzothiazol-2-yl)thio]piperidine-1-carboxylate

Analogously to Example 58-1, t-butyl 4-[(5-methoxybenzothiazol-2-yl)thio]piperidine-1-carboxylate (922 mg, yield 81%) was obtained from 4-methanesulfonyloxypiperidine (834 mg, 3.00 mmol) and 2-mercapto-5-methoxybenzothiazole (592 mg, 3.00 mmol) as a colorless oil.
¹H-NMR (CDCl₃, 400 MHz) δ: 7.61 (1H, d, J=8.8 Hz), 7.39 (1H, d, J=2.4 Hz), 6.95 (1H, dd, J=8.8 Hz, 2.4 Hz), 4.06 (1H, m), 3.95 (2H, m), 3.88 (3H, s), 3.12 (2H, t, J=10.8 Hz), 2.20-2.10 (2H, m), 1.76-1.70 (2H, m), 1.47 (9H, s).

### Example 59-2

### t-Butyl N-[(1S)-1-({4-[(5-methoxybenzothiazol-2-yl)thio]piperidin-1-yl}carbonyl)-2-methylpropyl]carbamate

Analogously to Example 29-2, t-butyl [(1S)-1-({4-[(5-methoxybenzothiazol-2-yl)thio]piperidin-1-yl}carbonyl)-2-methylpropyl]carbamate (873 mg, yield 98%) was obtained from t-butyl 4-[(5-methoxybenzothiazol-2-yl)thio]piperidine-1-carboxylate (921 mg, 2.42 mmol) as a colorless amorphous substance.
¹H-NMR (CDCl₃, 400 MHz) δ: 7.61 (1H, d, J=8.8 Hz), 7.39 (1H, d, J=2.4 Hz), 6.97 (1H, dd, J=8.8 Hz, 2.4 Hz), 5.38 (1H, t, J=7.2 Hz), 4.49
(1H, m), 4.39-4.29 (1H, m), 4.20 (1H, m), 3.88 (3H, s), 3.41-3.14 (3H, m), 2.40-2.20 (2H, m), 1.95 (1H, m), 1.88-1.68 (2H, m), 1.44 (9H, s), 0.97 (3H, t, J=6.4 Hz), 0.89 (3H, d, J=6.4 Hz).
MS (ESI, m/z): 448 (M+H)⁺.

### Example 59-3

### 3-Hydroxy-N-[(1S)-1-({4-[(5-methoxybenzthiazol-2-yl)thio]piperidin-1-yl}carbonyl)-2-methylpropyl]quinoxaline-2-carboxamide

Analogously to Example 1-3, (2S)-1-{4-[(5-methoxybenzothiazol-2-yl)thio]piperidin-1-yl}-3-methyl-1-oxobutan-2-amine hydrochloride was obtained from t-butyl [(1S)-1-({4-[(5-methoxybenzthiazol-2-yl)thio]piperidin-1-yl}carbonyl)-2-methylpropyl]carbamate (873 mg, 1.95 mmol) as a white solid. The resulting compound was condensed with 3-hydroxyquinoxaline-2-carboxylic acid (380 mg, 2.00 mmol) to afford the desired title compound (883 mg, yield 82%) as a pale yellow solid. ¹H-NMR (DMSO-d₆, 500 MHz) δ: 12.83 (1H, s), 9.50 (1H, brs), 7.88 (1H, dd, J=8.8 Hz, 5.3 Hz), 7.86 (1H, t, J=7.5 Hz), 7.64 (1H, t, J=7.5 Hz), 7.45 (1H, brs), 7.39-7.36 (2H, m), 7.04-6.98 (1H, m), 4.89 (1H, dd, J=18.6 Hz, 8.3 Hz), 4.31-4.07 (3H, m), 3.83 (3H, s), 3.49-3.39 (1H, m), 3.13-3.01 (1H, m), 2.31-2.18 (2H, m), 2.06 (1H, m), 1.85-1.56 (2H, m), 0.97-0.94 (6H, m).
IR (KBr) cm⁻¹: 2960, 1690, 1640, 1450, 1430, 1275.
MS (ESI, m/z): 552 (M+H)⁺.
HRMS (ESI, m/z): 574.1609 (Calcd for C₂₇H₂₉N₅NaO₄S₂: 574.1559).
Anal. Calcd for C₂₇H₂₉N₅O₄S₂·0.5H₂O, C: 57.84, H: 5.39, N: 12.49; Found: C, 57.69; H, 5.13; N, 12.46.

### Example 60

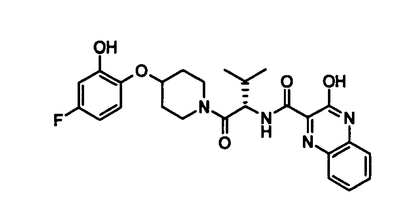

### N-[(1S)-1-{[4-(4-Fluoro-2-hydroxyphenoxy)piperidin-1-yl]carbonyl}-2-methylpropyl]-3-hydroxyquinoxaline-2-carboxamide

### Example 60-1

### 1-[2-(Benzyloxy)-4-fluorophenyl]ethanone

Benzyl bromide (1.03 g, 6.00 mmol) was added to a N,N-dimethylformamide solution (5 ml) of 1-(4-fluoro-2-hydroxyphenyl)ethanone (770 mg, 5.00 mmol) and potassium carbonate (860 mg, 5.00 mmol), at room temperature, under nitrogen stream, and stirring was carried out at 70°C for 1.5 hours. The reaction solution was poured into a 2N aqueous hydrochloric acid solution, followed by extraction with ethyl acetate. The extract was washed sequentially with water and saline, and then the resulting organic layer was dried over anhydrous sodium sulfate. The organic layer was concentrated and the resulting residue was purified by silica gel column chromatography to afford 1-[2-(benzyloxy)-4-fluorophenyl]ethanone (1.20 g, yield 98%) as a white solid.
¹H-NMR (CDCl₃, 400 MHz) δ: 7.83 (1H, dd, J=8.0 Hz, 7.2 Hz), 7.44-7.39 (5H, m), 6.76-6.70 (2H, m), 5.15 (2H, s), 2.58 (3H, s).

### Example 60-2

### 2-(benzyloxy)-4-fluorophenyl acetate

m-Chloroperbenzoic acid (2.54 g, 14.7 mmol) was added to a methylene chloride solution (20 ml) of 1-[2-(benzyloxy)-4-fluorophenyl]ethanone (1.20 g, 4.91 mmol), at room temperature, under nitrogen stream, and stirring was carried out for 10 hours under heating to reflux. The reaction solution was cooled to room temperature, the solid substance was filtered out, and then an aqueous sodium thiosulfate solution was added, followed by stirring for 15 minutes. The solution was separated, and the resulting organic layer was washed sequentially with a saturated aqueous sodium hydrogencarbonate solution, water and saline, followed by drying over anhydrous sodium sulfate. The organic layer was concentrated to afford 2-(benzyloxy)-4-fluorophenyl acetate (1.19 g, yield 93%) as a colorless oil.
¹H-NMR (CDCl₃, 400 MHz) δ: 7.40-7.34 (5H, m), 7.01 (1H, dd, J=8.8 Hz, 5.7 Hz), 6.74 (1H, dd, J=10.0 Hz, 3.0 Hz), 6.66 (1H, dt, J=12.1 Hz, 4.2 Hz), 5.07 (2H, s), 2.27 (3H, s).

### Example 60-3

### 2-(Benzyloxy)-4-fluorophenol

Sodium methoxide (494 mg, 9.14 mmol) was added to a methanol solution (20 ml) of 2-(benzyloxy)-4-fluorophenyl acetate (1.19 g, 4.57 mmol), at room temperature, under nitrogen stream, and stirring was carried out for 1.5 hours. The reaction solution was concentrated, followed by pouring into a 2N aqueous hydrochloric acid solution and extraction with ethyl acetate, and the extract was washed sequentially with water and saturated saline. The resulting organic layer was dried over anhydrous sodium sulfate, and then the residue resulting from concentration was purified by silica gel chromatography to afford 2-(benzyloxy)-4-fluorophenol (900 mg, yield 90%) as a colorless oil. ¹H-NMR (CDCl₃, 400 MHz) δ: 7.42-7.34 (5H, m), 6.87 (1H, dd, J=8.8 Hz, 5.7 Hz), 6.69 (1H, dd, J=10.0 Hz, 3.0 Hz), 6.60 (1H, dt, J=12.1 Hz, 4.2 Hz), 5.41 (1H, s), 5.08 (2H, s).

### Example 60-4

### t-Butyl [(1S)-1-{[4-(2-benzyloxy-4-fluorophenoxy)piperidin-1-yl]carbonyl}-2-methylpropyl]carbamate

Analogously to Example 1-2, t-butyl [(1S)-1-{[4-(2-benzyloxy-4-fluorophenoxy)piperidin-1-yl]carbonyl}-2-methylpropyl]carbamate (1.18 g) was obtained from t-butyl [(1S)-1-{(4-hydroxypiperidin-1-yl)carbonyl}-2-methylpropyl]carbamate (1.24 g, 4.12 mmol) and 2-(benzyloxy)-4-fluorophenol (900 mg, 4.12 mmol) as a mixture of colorless oil.
¹H-NMR (CDCl₃, 400 MHz) δ: 7.40-7.34 (5H, m), 6.90 (1H, m), 6.70 (1H, m), 6.60 (1H, m), 5.36 (1H, d, J=8.8 Hz), 5.41 (1H, s), 5.07 (2H, s), 4.48 (1H, m), 4.38 (1H, m), 4.10-3.40 (4H, m), 1.93-1.81 (5H, m), 1.44 (9H, s), 0.97 (3H, d, J=6.4 Hz), 0.89 (3H, d, J=6.4 Hz).
MS (ESI, m/z): 501 (M+H)⁺.

### Example 60-5

### t-Butyl [(1S)-1-{[4-(4-fluoro-2-hydroxyphenoxy)piperidin-1-yl]carbonyl}-2-methylpropyl]carbamate

10% Palladium carbon catalyst (120 mg) was added to t-butyl [(1S)-1-{[4-(2-benzyloxy-4-fluorophenoxy)piperidin-1-yl]carbonyl}-2-methylpropyl]carbamate (1.18 g, 2.36 mmol), and stirring was carried out at room temperature for 2 hours under hydrogen stream. The reaction solution was celite filtered, the filtrate was concentrated and the resulting residue was purified by silica gel chromatography to afford t-butyl [(1S)-1-{[4-(4-fluoro-2-hydroxyphenoxy)piperidin-1-yl]carbonyl}-2-methylpropyl]carbamate (478 mg, yield 49%) as a colorless amorphous substance.
¹H-NMR (CDCl₃, 400 MHz) δ: 6.81(1H, dd, J=9.0 Hz, 5.1 Hz), 6.71 (1H, dd, J=9.2 Hz, 2.9 Hz), 6.58-6.53 (1H, m), 5.75 (1H, d, J=4.3 Hz), 5.33 (1H, t, J=8.0 Hz), 4.52-4.42 (2H, m), 4.10-3.75 (2H, m), 3.58-3.31 (2H, m), 2.11-1.72 (5H, m), 1.44 (9H, s), 0.97 (3H, t, J=6.0 Hz), 0.90 (3H, d, J=6.7 Hz).
MS (ESI, m/z): 411 (M+H)⁺.

### Example 60-6

### N-[(1S)-1-{[4-(4-Fluoro-2-hydroxyphenoxy)piperidin-1-yl]carbonyl}-2-methylpropyl]-3-hydroxyquinoxaline-2-carboxamide

Analogously to Example 1-3, (2S)-1-[4-(4-fluoro-2-hydroxyphenoxy)piperidin-1-yl]-3-methyl-1-oxobutan-2-amine hydrochloride was obtained from t-butyl [(1S)-1-{[4-(4-fluoro-2-hydroxyphenoxy)piperidin-1-yl]carbonyl}-2-methylpropyl]carbamate (478 mg, 1.16 mmol) as a white solid. The resulting compound was condensed with 3-hydroxyquinoxaline-2-carboxylic acid (220 mg, 1.16 mmol) to afford the desired title compound (411 mg, yield 73%) as a pale yellow solid.
¹H-NMR (DMSO-d₆, 400 MHz) δ: 12.84 (1H, s), 9.50 (1H, t, J=8.0 Hz), 9.46 (1H, s), 7.88 (1H, dd, J=7.8 Hz, 4.3 Hz), 7.65 (1H, t, J=7.6 Hz), 7.39 (2H, t, J=6.5 Hz), 7.04-7.00 (1H, m), 6.82 (1H, dt, J=10.0 Hz, 3.2 Hz), 6.57-6.52 (1H, m), 4.92 (1H, t, J=7.0 Hz), 4.48-4.47 (1H, m), 4.00-3.86 (2H, m), 3.54-3.25 (2H, m), 2.09-1.52 (5H, m), 0.96 (3H, d, J=6.8 Hz), 0.93 (3H, d, J=6.8 Hz).
IR (KBr) cm⁻¹: 2965, 1690, 1630, 1525, 1505, 1230.
MS (ESI, m/z): 483 (M+H)⁺.
HRMS (ESI, m/z): 505.1882 (Calcd for C₂₅H₂₇FN₄NaO₅: 505.1863).
Anal. Calcd for C₂₅H₂₇FN₄O₅·H₂O: C, 59.99; H, 5.84; N, 11.19. Found: C, 60.23; H, 5.57; N, 11.00.

### Example 61

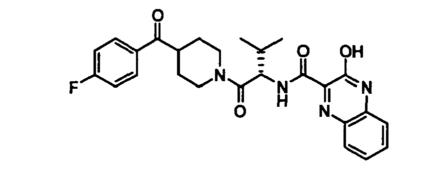

### N-[(1S)-1-{[4-(4-Fluorobenzoyl)piperidin-1-yl]carbonyl}-2-methylpropyl]-3-hydroxyquinoxaline-2-carboxamide

### Example 61-1

### t-Butyl [(1S)-1-{[4-(4-fluorobenzoyl)piperidin-1-yl]carbonyl}-2-methylpropyl] carbamate

Analogously to Example 2-1, t-butyl [(1S)-1-{[4-(4-fluorobenzoyl)piperidin-1-yl]carbonyl}-2-methylpropyl]carbamate (3.88 g, yield 96%) was obtained from 4-(4-fluorobenzoyl)piperidine hydrochloride (2.44 g, 10.0 mmol) and N-(t-butoxycarbonyl)-L-valine (2.17 g, 10.0 mmol) as a colorless amorphous substance.

### Example 61-2

### [(1S)-1-{[4-(4-Fluorobenzoyl)piperidin-1-yl]carbonyl}-2-methylpropyl]-3-hydroxyquinoxaline-2-carboxamide

Analogously to Example 2-2, (4-fluorophenyl)(1-L-valylpiperidin-4-yl)methanone hydrochloride (3.17 g, yield 97%) was obtained from t-butyl [(1S)-1-{[4-(4-fluorobenzoyl)piperidin-1-yl]carbonyl}-2-methylpropyl]carbamate (3.88 g, 9.55 mmol) as a colorless amorphous substance. The resulting compound (200 mg, 0.580 mmol) was condensed with 3-hydroxyquinoxaline-2-carboxylic acid (110 mg, 0.580 mmol) to afford the desired title compound (143 mg, yield 51%) as a pale yellow solid.
¹H-NMR (CDCl₃ 400 MHz) δ: 12.90 and 10.21 (1H, brs), 8.05-7.97 (2H, m), 7.61 (1H, d, J=6.8 Hz), 7.58 (1H, m), 7.37 (1H, brs), 7.21-7.13 (2H, m), 5.14-5.07 (1H, m), 4.68 and 4.59 (1H, m), 4.34 (1H, d, J=11.7 Hz), 3.60-3.35 (2H, m), 3.10-2.96 (1H, m), 3.10-2.96 (1H, m), 2.38-2.29 (1H, m), 2.11-0.96 (5H, m), 1.12-1.09 (6H, m).
IR (ATR) cm⁻¹: 1680, 1630, 1595, 1525, 1505, 1475.
MS (ESI, m/z): 479 (M+H)⁺.
Anal. Calcd for C₂₆H₂₇FN₄O₄·0.5H₂O: C, 64.05; H, 5.79; N, 11.49. Found: C, 64.20; H, 5.58; N, 11.34.

### Example 62

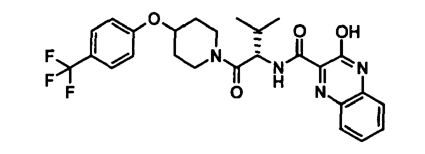

### 3-Hydroxy-N-[(1S)-2-methyl-1-({4-[4-(trifluoromethyl)phenoxy]piperidin-1-yl}carbonyl)propyl]quinoxaline-2-carboxamide

### Example 62-1

### t-Butyl [(1S)-2-methyl-1-({4-[4-(trifluoromethyl)phenoxy]piperidin-1-yl}carbonyl)propyl]carbamate

Analogously to Example 2-1, t-butyl [(1S)-2-methyl-1-({4-[4-(trifluoromethyl)phenoxy]piperidin-1-yl}carbonyl)propyl]carbamate (1.41 g, yield 98%) was obtained from 4-[4-(trifluoromethyl)phenoxy]piperidine hydrochloride (913 mg, 3.24 mmol) and N-(t-butoxycarbonyl)-L-valine (704 mg, 3.24 mmol) as a colorless oil.
¹H-NMR (CDCl₃ 400 MHz) δ: 7.55 (2H, d, J=8.5 Hz), 6.97 (2H, d, J=8.5 Hz), 5.33 (1H, d, J=8.8 Hz), 4.68-4.60 (1H, m), 4.49 (1H, dd, J=8.1 Hz, 6.4 Hz), 3.90-3.49 (4H, m), 2.04-1.80 (5H, m), 1.44 (9H, s), 0.98 (3H, d, J=6.8 Hz), 0.90 (3H, d, J=6.8 Hz).
IR (ATR) cm⁻¹: 2965, 1705, 1635, 1615, 1515, 1440, 1320, 1250.
MS (ESI, m/z): 445 (M+H)⁺.

### Example 62-2

### 3-Hydroxy-N-[(1S)-2-methyl-1-({4-[4-(trifluoromethyl)phenoxy]piperidin-1-yl}carbonyl)propyl]quinoxaline-2-carboxamide

Analogously to Example 2-2, (2S)-3-methyl-1-oxo-1-{4-[4-(trifluoromethyl)phenoxylpiperidin-1-yl}butan-2-amine hydrochloride (1.14 g, yield 95%) was obtained from t-butyl [(1S)-2-methyl-1-({4-[4-(trifluoromethyl)phenoxy]piperidin-1-yl}carbonyl)propyl]carbamate (1.41 g, 3.16 mmol) as an amorphous substance. The resulting compound (220 mg, 0.580 mmol) was condensed with 3-hydroxyquinoxaline-2-carboxylic acid (110 mg, 0.580 mmol) to afford the desired title compound (132 mg, yield 45%) as a white solid.
¹H-NMR (CDCl₃, 400 MHz) δ: 12.57 and 10.15 (1H, brs), 7.98 (1H, d, J=4.6 Hz), 7.65-7.53 (4H, m), 7.37 (1H, brs), 6.99 (2H, d, J=8.6 Hz), 5.07 (1H, t, J=7.3 Hz), 4.72-4.65 (1H, m), 4.03-3.62 (5H, m), 2.36-2.10 (2H, m), 2.02-1.80 (3H, m), 1.12 (3H, d, J=6.8 Hz), 1.11 (3H, d, J=6.8 Hz).
IR (ATR) cm⁻¹: 1685, 1630, 1610, 1515, 1445, 1320, 1250.
MS (ESI, m/z): 517 (M+H)⁺.
Anal. Calcd for C₂₆H₂₇F₃N₄O₄: C, 60.46; H, 5.27; N, 10.85. Found: C, 60.23; H, 5.23; N, 10.82.

### Example 63

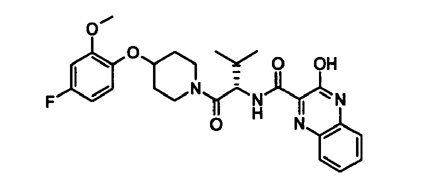

### N-[(1S)-1-{[4-(4-Fluoro-2-methoxyphenoxy)piperidin-1-yl]carbonyl}-2-methylpropyl]-3-hydroxyquinoxaline-2-carboxamide

### Example 63-1

### t-Butyl [(1S)-1-{[4-(4-fluoro-2-methoxyphenoxy)piperidin-1-yl]carbonyl}-2-methylpropyl]carbamate

A 40% toluene solution of diisopropyl azodicarboxylate (0.930 ml, 1.83 mmol) was added to a toluene solution (15 ml) of t-butyl {(1S)-1-[(4-hydroxypiperidin-1-yl)carbonyl]-2-methylpropyl}carbamate (500 mg, 1.66 mmol), 4-fluoro-2-methoxyphenol (260 mg, 1.83 mmol) and triphenylphosphine (480 mg, 1.83 mmol), at room temperature, under nitrogen stream, and stirring was carried out at room temperature overnight. The reaction solution was concentrated and the resulting residue was purified by a medium-pressure preparative liquid chromatograph (manufactured by Yamazen Corporation, W-Prep 2XY) to afford t-butyl [(1S)-1-{[4-(4-fluoro-2-methoxyphenoxy)piperidin-1-yl]carbonyl}-2-methylpropyl]carbamate (520 mg, yield 74%) as a colorless amorphous substance.
¹H-NMR (CDCl₃, 400 MHz) δ: 6.89 and 6.87 (1H, d, J=8.8 Hz), 6.65 (1H, m), 6.66 and 6.65 (1H, d, J=2.4 Hz), 6.58 and 6.57 (1H, dd, J=8.8 Hz, 2.4 Hz), 5.36 (1H, d, J=9.2 Hz), 4.50 (1H, dd, J=9.2 Hz, 5.4 Hz), 3.92-3.78 (5H, m), 3.63-3.39 (2H, m), 1.98-1.75 (5H, m), 1.44 and 1.43 (9H, s), 0.97 (3H, t, J=7.3 Hz), 0.89 (3H, dd, J=6.4 Hz).
MS (ESI, m/z): 425 (M+H)⁺.

### Example 63-2

### N-[(1S)-1-{[4-(4-Fluoro-2-methoxyphenoxy)piperidin-1-yl]carbonyl}-2-methylpropyl]-3-hydroxyquinoxaline-2-carboxamide

Concentrated hydrochloric acid (5.0 ml) was added to an ethanol solution (10 ml) of t-butyl [(1S)-1-{[4-(4-fluoro-2-methoxyphenoxy)piperidin-1-yl]carbonyl}-2-methylpropyl]carbamate (400 mg, 0.940 mmol), under nitrogen stream, and the mixture was allowed to react at room temperature overnight. The reaction solution was concentrated to afford (2S)-1-[4-(4-fluoro-2-methoxyphenoxy)piperidin-1-yl]-3-methyl-1-oxobutan-2-amine hydrochloride (340 mg) as a colorless amorphous substance.

1-Ethyl-3-(3-dimethylaminopropyl)carbodiimide hydrochloride (270 mg, 1.41 mmol), 1-hydroxybenzotriazole monohydrate (150 mg, 1.13 mmol) and N-methylmorpholine (0.410 ml, 3.77 mmol) were added to a methylene chloride solution (10 ml) of the resulting compound (340 mg, 0.940 mmol) and 3-hydroxyquinoxaline-2-carboxylic acid (180 mg, 0.940 mmol), at room temperature, under nitrogen stream, and stirring was carried out at room temperature overnight. The reaction solution was washed sequentially with water, a saturated aqueous sodium hydrogencarbonate solution, water and saline, and then the resulting organic layer was dried over anhydrous sodium sulfate. The organic layer was concentrated, the resulting residue was purified by a medium-pressure preparative liquid chromatograph (manufactured by Yamazen Corporation, W-Prep 2XY), the residue resulting from concentration was suspended in diethyl ether, and the solid substance was collected by filtration to afford the desired title compound (459 mg, yield 98%) as a pale yellow solid.
¹H-NMR (DMSO-d₆, 400 MHz) δ: 12.86 (1H, brs), 9.55 (1H, brs), 7.89 (1H, d, J=8.3 Hz), 7.66 (1H, t, J=7.2 Hz), 7.43-7.37 (2H, m), 7.11-7.06 (1H, m), 6.98-6.93 (1H, m), 6.78-6.73 (1H, m), 4.93 (1H, q, J=7.8 Hz), 4.51-4.43 (1H, m), 3.99-3.87 (2H, m), 3.81 (3H, d, J=6.8 Hz), 3.58-3.45 (2H, m), 2.11-1.84 (3H, m), 1.75-1.49 (2H, m), 0.98 (3H, d, J=6.8 Hz), 0.97 (3H, d, J=6.8 Hz).
IR (KBr) cm⁻¹: 2960, 1685, 1640, 1500, 1450, 1215, 1190, 1150, 1030.
MS (ESI, m/z): 497 (M+H)⁺.
Anal. Calcd for C₂₆H₂₉FN₄O₅·1/4H₂O: C, 62.33; H, 5.93; N, 11.18; F, 3.79. Found: C, 62.07; H, 6.02; N, 11.04; F, 3.68.

### Example 64

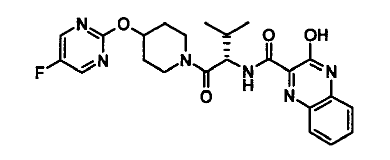

### N-[(1S)-1-({4-[(5-Fluoropyrimidin-2-yl)oxy]piperidin-1-yl}carbonyl)-2-methylpropyl]-3-hydroxyquinoxaline-2-carboxamide

### Example 64-1

### t-Butyl [(1S)-1-({4-[(5-fluoropyrimidin-2-yl)oxy]piperidin-1-yl}carbonyl)-2-methylpropyl]carbamate

63% Sodium hydride (278 mg, 7.32 mmol) was added to a N,N-dimethylformamide solution (20 ml) of 2-chloro-5-fluoropyrimidine (452 mg, 3.66 mmol) and t-butyl N-{(1S)-1-[(4-hydroxypiperidin-1-yl)carbonyl]-2-methylpropyl}carbamate (1.00 g, 3.32 mmol), at 0°C, under nitrogen stream, and stirring was carried out at room temperature overnight. Water was added to the reaction solution under ice cooling, followed by extraction with ethyl acetate. The extract was washed sequentially with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford t-butyl [(1S)-1-({4-[(5-fluoropyrimidin-2-yl)oxy]piperidin-1-yl}carbonyl)-2-methylpropyl]carbamate (568 mg, yield 43%) as a colorless oil.
¹H-NMR (CDCl₃, 400 MHz) δ: 8.36 (2H, s), 5.41 (1H, d, J=9.0 Hz), 5.26-5.16 (1H, m), 4.53-4.47 (1H, m), 4.06-3.78 (2H, m), 3.74-3.45 (2H, m), 2.02-1.92 (5H, m), 1.50-1.39 (9H, m), 0.98 (3H, t, J=6.8 Hz), 0.86 (3H, t, J=6.8 Hz).
MS (FAB, m/z): 397 (M+H)⁺.

### Example 64-2

### N-[(1S)-1-({4-[(5-Fluoropyrimidin-2-yl)oxy]piperidin-1-yl}carbonyl)-2-methylpropyl]-3-hydroxyquinoxaline-2-carboxamide

Analogously to Example 1-3, (2S)-1-{4-[(5-fluoropyrimidin-2-yl)oxy]piperidin-1-yl}-3-methyl-1-oxobutan-2-amine hydrochloride (163 mg) was obtained from t-butyl [(1S)-1-({4-[(5-fluoropyrimidin-2-yl)oxy]piperidin-1-yl}carbonyl)-2-methylpropyl]carbamate (194 mg, 0.489 mmol) as a white solid. The resulting compound (163 mg, 0.489 mmol) was condensed with 3-hydroxyquinoxaline-2-carboxylic acid (139 mg, 0.734 mmol) to afford the desired title compound (90.0 mg, yield 39%) as a pale yellow solid.
¹H-NMR (DMSO-d₆, 400 MHz) δ: 12.80 (1H, brs), 9.46 (1H, brs), 8.68 (1H, d, J=3.1 Hz), 8.60 (1H, d, J=3.1 Hz), 7.84 (1H, d, J=8.2 Hz), 7.62 (1H, t, J=7.6 Hz), 7.37-7.35 (2H, m), 4.92-4.85 (2H, m), 3.95-3.90 (2H, m), 3.55-3.19 (2H, m), 2.07-1.55 (5H, m), 0.94-0.92 (6H, m).
IR (KBr) cm⁻¹ : 2960, 1685, 1640, 1530, 1430, 1275.
MS (FAB, m/z): 469 (M+H)⁺.
HRMS (ESI, m/z): 469.2001 (Calcd for C₂₃H₂₆FN₆O₄: 469.1999).

### Example 65

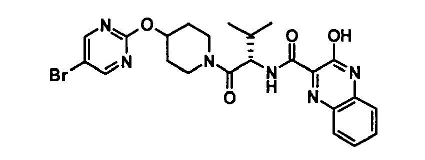

### N-[(1S)-1-({4-[(5-Bromopyrimidin-2-yl)oxy]piperidin-1-yl}carbonyl)-2-methylpropyl]-3-hydroxyquinoxaline-2-carboxamide

### Example 65-1

### t-Butyl [(1S)-1-({4-[(5-bromopyrimidin-2-yl)oxy]piperidin-1-yl}carbonyl)-2-methylpropyl]carbamate

Analogously to Example 64-1, t-butyl N-[(1S)-1-({4-(5-bromopyrimidin-2-yl)oxy}piperidin-1-yl)carbonyl]-2-methylpropyl]carbamate (1.74 g, yield 57%) was obtained from 5-bromo-2-chloropyrimidine (1.42 g, 7.32 mmol) and t-butyl {(1S)-1-[(4-hydroxypiperidin-1-yl)carbonyl]-2-methylpropyl}carbamate (2.00 g, 6.66 mmol) as a white solid.
¹H-NMR (CDC1₃, 400 MHz) δ: 8.53 (2H, s), 5.36-5.35 (1H, m), 5.27-5.18 (1H, m), 4.51-4.49 (1H, m), 4.02-3.68 (3H, m), 3.59-3.44 (1H, m), 2.10-1.82 (5H, m), 1.44 (9H, brs), 0.97 (3H, d, J=6.6 Hz), 0.89 (3H, d, J=6.6 Hz).
MS (FAB, m/z): 457 (M+H)⁺.

### Example 65-2

### N-[(1S)-1-({4-[(5-Bromopyrimidin-2-yl)oxy]piperidin-1-yl}carbonyl)-2-methylpropyl]-3-hydroxyquinoxaline-2-carboxamide

Analogously to Example 1-3, (2S)-1-{4-[(5-bromopyrimidin-2-yl)oxy]piperidin-1-yl}-3-methyl-1-oxobutan-2-amine hydrochloride (1.50 g) was obtained from t-butyl [(1S)-1-({4-[(5-bromopyrimidin-2-yl)oxy]piperidin-1-yl}carbonyl)-2-methylpropyl]carbamate (1.74 g, 3.80 mmol) as a white solid. The resulting compound (200 mg, 0.508 mmol) was condensed with 3-hydroxyquinoxaline-2-carboxylic acid (145 mg, 0.762 mmol) to afford the desired title compound (136 mg, yield 51%) as a pale yellow solid.
¹H-NMR (DMSO-d₆, 500 MHz) δ: 12.83 (1H, brs), 9.55 (1H, brs), 8.77 (2H, d, J=3.9 Hz), 7.86 (1H, d, J=7.8 Hz), 7.63 (1H, t, J=7.8 Hz), 7.38-7.36 (2H, m), 5.19 (1H, brs), 4.91 (1H, q, J=7.8 Hz), 4.04-3.91 (2H, m), 3.59-3.22 (2H, m), 2.06-2.01 (3H, m), 1.78-1.57 (2H, m), 0.96-0.95 (6H, m).
IR (KBr) cm⁻¹: 2965, 1690, 1630, 1540, 1425, 1320.
MS (ESI, m/z): 529 (M+H)⁺.
HRMS (ESI, m/z): 529.1197 (Calcd for C₂₃H₂₆BrN₆O₄: 529.1198).

### Example 66

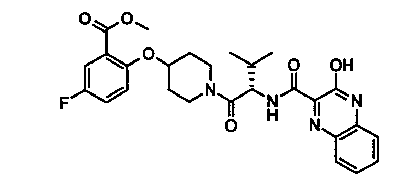

### Methyl 5-fluoro-2-[(1-{N-[(3-hydroxyquinoxalin-2y1)carbonyl]-L-valyl}piperidin-4-yl)oxy]benzoate

### Example 66-1

### t-Butyl 4-[4-fluoro-2-(methoxycarbonyl)phenoxy]piperidine-1-carboxylate

Analogously to Example 23-1, t-butyl 4-[4-fluoro-2-(methoxycarbonyl)phenoxy]piperidine-1-carboxylate (2.39 g, yield 91%) was obtained from t-butyl 4-hydroxypiperidine-1-carboxylate (1.50 g, 7.45 mmol) and methyl 5-fluoro-2-hydroxybenzoate (1.40 g, 8.20 mmol) as a colorless oil.
¹H-NMR (CDCl₃, 400 MHz) δ: 7.52-7.49 (1H, m), 7.17-7.12 (1H, m), 6.95 (1H, dd, J=9.4 Hz, 4.3 Hz), 4.52-4.48 (1H, m), 3.90 (3H, s), 3.66-3.60 (2H, m), 3.48-3.42 (2H, m), 1.91-1.78 (4H, m), 1.49-1.43 (9H, m).
MS (FAB, m/z): 354 (M+H)⁺.

### Example 66-2

### Methyl 2-({1-[N-(t-Butoxycarbonyl)-L-valyl]piperidin-4-yl}oxy)-5-fluorobenzoate

Analogously to Example 23-2, methyl 5-fluoro-2-[(piperidin-4-yl)oxy]benzoate hydrochloride (1.87 g, yield 95%) was obtained from t-butyl 4-[4-fluoro-2-(methoxycarbonyl)phenoxy]piperidine-1-carboxylate (2.39 g, 6.76 mmol) as a white solid. The resulting compound (1.87 g, 6.45 mmol) was condensed with N-(t-butoxycarbonyl)-L-valine (1.54 g, 7.10 mmol) to afford methyl 2-({1-[N-(t-butoxycarbonyl)-L-valyl]piperidin-4-yl}oxy)-5-fluorobenzoate (2.62 g, yield 90%) as a colorless amorphous substance.
¹H-NMR (CDCl₃, 400 MHz) δ: 7.53 (1H, dd, J=8.6 Hz, 4.3 Hz), 7.18-7.16 (1H, m), 6.94 (1H, dd, J=9.0 Hz, 4.3 Hz), 5.36-5.34 (1H, m), 4.62-4.60 (1H, m), 4.50 (1H, dd, J=9.0 Hz, 5.5 Hz), 3.97 (3H, s), 3.89-3.54 (4H, m), 1.97-1.83 (5H, m), 1.44 (9H, s), 0.98 (3H, d, J=6.8 Hz), 0.89 (3H, d, J=6.8 Hz).
MS (FAB, m/z): 453 (M+H)⁺.

### Example 66-3

### Methyl 5-fluoro-2-[(1-{N-[(3-hydroxyquinoxalin-2-yl)carbonyl]-L-valyl}piperidin-4-yl)oxy]benzoate

Analogously to Example 23-3, methyl 5-fluoro-2-[(1-L-valylpiperidin-4-yl)oxy]benzoate hydrochloride (1.72 g) was obtained from methyl 2-({1-[N-(t-butoxycarbonyl)-L-valyl]piperidin-4-yl}oxy)-5-fluorobenzoate (1.86 g, 5.79 mmol) as a white solid. The resulting compound (1.00 g, 2.57 mmol) was condensed with 3-hydroxyquinoxaline-2-carboxylic acid (734 mg, 3.86 mmol) to afford the desired title compound (1.09 g, yield 74%) as a pale yellow solid.
¹H-NMR (DMSO-d₆, 400 MHz) δ: 12.83 (1H, s), 9.49 (1H, d, J=8.2 Hz), 7.87 (1H, d, J=8.2 Hz), 7.65 (1H, t, J=8.2 Hz), 7.50-7.29 (5H, m), 4.92 (1H, q, J=7.4 Hz), 4.78 (1H, brs), 3.81 (3H, s), 3.71-3.69 (4H, m), 2.09-1.67 (5H, m), 0.97-0.95 (6H, m),
IR (KBr) cm⁻¹: 2960, 1685, 1640, 1495, 1270, 1185.
MS (FAB, m/z): 525 (M+H)⁺.
HRMS (ESI, m/z): 525.2127 (Calcd for C₂₇H₃₀FN₄O₆: 525.2149).

### Example 67

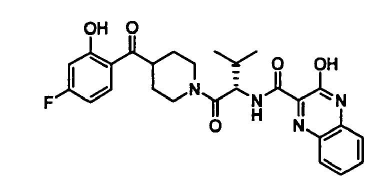

### N-[(1S)-1-{[4-(4-Fluoro-2-hydroxybenzoyl)piperidin-1-yl]carbonyl}-2-methylpropyl]-3-hydroxyquinoxaline-2-carboxamide

### Example 67-1

### t-Butyl [(1S)-1-{[4-(6-fluoro-1,2-benzisoxazol-3-yl)piperidin-1-yl]carbonyl}-2-methylpropyl}carbamate

Analogously to Example 2-1, 6-fluoro-3-(piperidin-4-yl)-1,2-benzisoxazole (2.00 g, 9.08 mmol) was condensed with N-(t-butoxycarbonyl)-L-valine (2.17 g, 9.99 mmol) to afford t-butyl [(1S)-1-{[4-(6-fluoro-1,2-benzisoxazol-3-yl)piperidin-1-yl]carbonyl}-2-methylpropyl}carbamate (3.70 g, yield 97%) as a colorless amorphous substance.
¹H-NMR (CDCl₃, 400 MHz) δ: 7.71-7.61 (1H, m), 7.29-7.25 (1H, m), 7.09-7.04 (1H, m), 5.35 (1H, dd, J=17.8 Hz, 9.2 Hz), 4.67-4.55 (2H, m), 4.19-4.09 (1H, m), 3.42-3.27 (2H, m), 3.02-2.92 (1H, m), 2.24-1.82 (5H, m), 1.48-1.46 (9H, m), 1.00 (3H, t, J=6.6 Hz), 0.90 (3H, t, J=6.6 Hz).
MS (FAB, m/z): 420 (M+H)⁺.

### Example 67-2

### N-[(1S)-1-{[4-(6-Fluoro-1,2-benzisoxazol-3-yl)piperidin-1-yl]carbonyl}-2-methylpropyl}-3-hydroxyquinoxaline-2-carboxamide

Analogously to Example 1-3, (2S)-1-[4-(6-fluoro-1,2-benzisoxazol-3-yl)piperidin-1-yl]-3-methyl-1-oxobutan-2-amine hydrochloride (3.20 g) was obtained from t-butyl [(1S)-1-{[4-(6-fluoro-1,2-benzisoxazol-3-yl)piperidin-1-yl]carbonyl}-2-methylpropyl}carbamate (3.70 g, 8.78 mmol) as a white solid. The resulting compound (400 mg, 1.12 mmol) was condensed with 3-hydroxyquinoxaline-2-carboxylic acid (319 mg, 1.68 mmol) to afford N-[(1S)-1-{[4-(6-fluoro-1,2-benzisoxazol-3-yl)piperidin-1-yl]carbonyl}-2-methylpropyl}-3-hydroxyquinoxaline-2-carboxamide (220 mg, yield 40%) as a pale yellow solid.
¹H-NMR (DMSO-d₆, 400 MHz) δ: 9.50 (1H, t, J=7.6 Hz), 8.07-8.03 (1H, m), 7.88-7.61 (3H, m), 7.42-7.21 (3H, m), 4.95-4.91 (1H, m), 4.51 (1H, t, J=12.1 Hz), 4.27 (1H, d, J=12.1 Hz), 3.53-3.51 (1H, m), 3.41-3.25 (1H, m), 3.00-2.93 (2H, m), 2.11-1.75 (5H, m), 0.97 (6H, t, J=6.8 Hz).
IR (KBr) cm⁻¹: 2960, 1685, 1635, 1530, 1450, 1275, 1120.
MS (FAB, m/z): 492 (M+H)⁺.
HRMS (ESI, m/z): 492.2035 (Calcd for C₂₆H₂₇FN₅O₄: 492.2047).

### Example 67-3

### N-[(1S)-1-{[4-(4-Fluoro-2-hydroxybenzoyl)piperidin-1-yl]carbonyl}-2-methylpropyl]-3-hydroxyquinoxaline-2-carboxamide

5% Palladium carbon catalyst (50.0 mg) was added to a methanol solution (3.0 ml) of N-[(1S)-1-{[4-(6-fluoro-1,2-benzisoxazol-3-yl)piperidin-1-yl]carbonyl}-2-methylpropyl]-3-hydroxyquinoxaline-2-carboxamide (150 mg, 0.305 mmol), and stirring was carried out at room temperature for 3 hours under hydrogen atmosphere. The reaction solution was celite filtered, and the filtrate was concentrated. The resulting residue was dissolved in a tetrahydrofuran solution (3.0 ml), and a 2N aqueous sodium hydroxide solution (3.0 ml) was added, followed by reaction at room temperature overnight. Under ice cooling, the reaction solution was poured into a 2N aqueous hydrochloric acid solution, followed by extraction with ethyl acetate and sequential washing with water and saline, and then the resulting organic layer was dried over anhydrous sodium sulfate. The organic layer was concentrated to afford the desired title compound (122 mg, yield 81%) as a pale yellow solid.
¹H-NMR (DMSO-d₆, 400 MHz) δ: 12.83 (1H, brs), 12.25 (1H, d, J=6.3 Hz), 9.48 (1H, dd, J=16.2 Hz, 8.8 Hz), 8.11-8.07 (1H, m), 7.87 (1H, dd, J=8.8 Hz, 4.0 Hz), 7.65 (1H, t, J=7.8 Hz), 7.40-7.38 (2H, m), 6.86-6.84 (2H, m), 4.93-4.92 (1H, m), 4.49-4.46 (1H, m), 4.22-4.20 (1H, m), 3.81-3.79 (1H, m), 3.33-3.30 (1H, m), 2.83 (1H, m), 2.05-1.88 (3H, m), 1.67-1.40 (2H, m), 0.96 (6H, t, J=6.8 Hz).
IR (KBr) cm⁻¹: 2965, 1685, 1635, 1530, 1200.
MS (ESI, m/z): 517 (M+Na)⁺.
HRMS (ESI, m/z) : 517.1827 (Calcd for C₂₆H₂₇FN₄NaO₅: 517.1863).

### Example 68

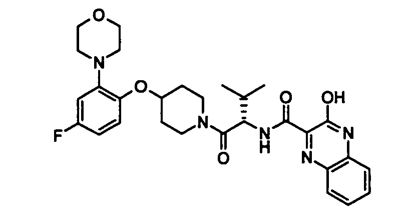

### N-[(1S)-1-({4-[4-Fluoro-2-(morpholin-4-yl)phenoxy]piperidin-1-yl}carbonyl)-2-methylpropyl]-3-hydroxyquinoxaline-2-carboxamide

### Example 68-1

### t-Butyl 4-(2-bromo-4-fluorophenoxy)piperidine-1-carboxylate

Analogously to Example 1-2, t-butyl 4-(2-bromo-4-fluorophenoxy)piperidine-1-carboxylate (2.45 g, yield 88%) was obtained from t-butyl 4-hydroxypiperidine-1-carboxylate (1.50 g, 7.45 mmol) and t-butyl 2-bromo-4-fluorophenol (1.57 g, 8.20 mmol) as a colorless oil.
¹H-NMR (CDCl₃, 400 MHz) δ: 7.31-7.27 (1H, m), 6.99-6.93 (1H, m), 6.91-6.86 (1H, m), 4.47-4.43 (1H, m), 3.69-3.62 (2H, m), 3.47-3.44 (2H, m), 1.91-1.78 (4H, m), 1.47-1.43 (9H, m).
MS (FAB, m/z): 374 (M+H)⁺.

### Example 68-2

### t-Butyl 4-[4-fluoro-2-(morpholin-4-yl)phenoxy]piperidine-1-carboxylate

Sodium t-butoxide (77.0 mg, 0.801 mmol), 2,2'-bis(diphenylphosphino)-1,1'-binaphthyl(200 mg, 0.320 mmol) and tris(dibenzylideneacetone)dipalladium (61.0 mg, 0.107 mmol) were added sequentially to a toluene solution (5 ml) of t-butyl 4-(2-bromo-4-fluorophenoxy)piperidine-1-carboxylate (910 mg, 3.67 mmol) and morpholine (0.0700 ml, 0.801 mmol), at room temperature, under nitrogen stream, followed by heating to reflux for 10 hours. The reaction solution was poured into ice water, followed by extraction with ethyl acetate, the extract was washed sequentially with a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford t-butyl 4-[4-fluoro-2-(morpholin-4-yl)phenoxy]piperidine-1-carboxylate (154 mg, yield 76%) as a yellow oily substance.
¹H-NMR (CDCl₃, 400 MHz) δ: 6.83-6.80 (1H, m), 6.63-6.61 (2H, m), 4.47-4.42 (1H, m), 3.84 (4H, t, J=4.7 Hz), 3.73-3.67 (2H, m), 3.35-3.31 (2H, m), 3.08 (4H, t, J=4.7 Hz), 1.91-1.84 (2H, m), 1.77-1.69 (2H, m), 1.51-1.43 (9H, m).
MS (FAB, m/z): 381 (M+H)⁺.

### Example 68-3

### t-Butyl [(1S)-1-{[4-(4-fluoro-2-morpholin-4-ylphenoxy)piperidin-1-yl]carbonyl}-2-methylpropyl]carbamate

Analogously to Example 23-2, 4-{5-fluoro-2-[(piperidin-4-yl)oxy]phenyl}morpholine hydrochloride (128 mg, yield 100%) was obtained from t-butyl 4-[4-fluoro-2-(morpholin-4-yl)phenoxy]piperidine-1-carboxylate (154 mg, 0.405 mmol) as a white solid. The resulting compound (128 mg, 0.405 mmol) was condensed with N-(t-butoxycarbonyl)-L-valine (132 mg, 0.607 mmol) to afford t-butyl [(1S)-1-({4-[4-fluoro-2-(morpholin-4-yl)phenoxy]piperidin-1-yl}carbonyl)-2-methylpropyl]carbamate (199 mg, yield 100%) as a colorless amorphous substance.
¹H-NMR (CDCl₃, 400 MHz) δ: 6.85-6.81 (1H, m), 6.66-6.62 (2H, m), 5.35 (1H, t, J=4.5 Hz), 4.53-4.51 (2H, m), 3.85-3.43 (4H, m), 3.84 (4H, t, J=4.7 Hz), 3.08 (4H, t, J=4.7 Hz), 1.99-1.76 (5H, m), 1.43-1.41 (9H, m), 1.00 (3H, t, J=6.8 Hz), 0.90 (3H, t, J=6.8 Hz).
MS (FAB, m/z): 480 (M+H)⁺.

### Example 68-4

### N-[(1S)-1-({4-[4-Fluoro-2-(morpholin-4-yl)phenoxy]piperidin-1-yl}carbonyl)-2-methylpropyl]-3-hydroxyquinoxaline-2-carboxamide

Analogously to Example 2-2, (2S)-1-{4-[4-fluoro-2-(morpholin-4-yl)phenoxy]piperidin-1-yl}-3-methyl-1-oxobutan-2-amine hydrochloride (223 mg) was obtained from t-butyl [(1S)-1-({4-[4-fluoro-2-(morpholin-4-yl)phenoxy]piperidin-1-yl}carbonyl)-2-methylpropyl]carbamate (195 mg, 0.405 mmol) as a white solid. The resulting compound (223 mg, 0.405 mmol) was condensed with 3-hydroxyquinoxaline-2-carboxylic acid (116 mg, 0.608 mmol) to afford the desired title compound (133 mg, yield 57%) as a pale yellow solid.
¹H-NMR (DMSO-d₆, 400 MHz) δ: 12.81 (1H, s), 9.51 (1H, d, J=7.4 Hz), 7.86 (1H, d, J=7.4 Hz), 7.64-7.63 (1H, m), 7.39-7.37 (2H, m), 7.05-7.02 (1H, m), 6.75-6.73 (2H, m), 4.91 (1H, dd, J=14.5 Hz, 4.5 Hz), 4.60 (1H, brs), 3.85-3.57 (8H, m), 3.01 (4H, t, J=4.7 Hz), 2.07-1.59 (5H, m), 0.97-0.93 (6H, m).
IR (KBr) cm⁻¹: 2965, 1685, 1630, 1495, 1215, 1120.
MS (FAB, m/z): 552 (M+H)⁺.
HRMS (ESI, m/z): 552.2600 (Calcd for C₂₉H₃₅FN₅O₅: 552.2622).

### Example 69

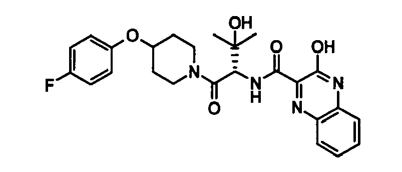

### N-[(1S)-1-{[4-(4-Fluorophenoxy)piperidin-1-yl]carbonyl}-2-hydroxy-2-methylpropyl]-3-hydroxyquinoxaline-2-carboxamide

### Example 69-1

### t-Butyl [(1S)-1-{[4-(4-fluorophenoxy)piperidin-1-yl]carbonyl}-2-hydroxy-2-methylpropyl]carbamate

1-Ethyl-3-(3-dimethylaminopropyl)carbodiimide hydrochloride (115 mg, 0.600 mmol), 1-hydroxybenzotriazole monohydrate (81.0 mg, 0.600 mmol) and N-methylmorpholine (0.440 ml, 2.00 mmol) were added to a methylene chloride solution (2.0 ml) of 4-(4-fluorophenoxy)piperidine hydrochloride (116 mg, 0.500 mmol) and N-(t-butoxycarbonyl)-(S)-2-amino-3-hydroxy-3-methylbutanoic acid (117 mg, 0.500 mmol), at 0°C, under nitrogen stream, and stirring was carried out at room temperature overnight. The reaction solution was diluted with methylene chloride, followed by sequential washing with a saturated aqueous sodium hydrogencarbonate solution, a saturated aqueous ammonium chloride solution, water and saline, and then the resulting organic layer was dried over anhydrous sodium sulfate. The organic layer was concentrated and the resulting residue was purified by silica gel chromatography to afford t-butyl [(1S)-1-{[4-(4-fluorophenoxy)piperidin-1-yl]carbonyl}-2-hydroxy-2-methylpropyl]carbamate (89.0 mg, yield 44%) as a colorless amorphous substance.
Major: ¹H-NMR (CDCl₃, 500 MHz) δ: 7.00-6.96 (2H, m), 6.86-6.85 (2H, m), 5.50 (1H, d, J=8.4 Hz), 4.72 (1H, s), 4.50-4.48 (1H, m), 4.45 (1H, s), 3.81-3.79 (2H, m), 3.66-3.64 (2H, m), 1.99-1.84 (4H, m), 1.44 (9H, s), 1.25 (6H, s).
Minor: ¹H-NMR (CDCl₃, 500 MHz) δ: 7.00-6.96 (2H, m), 6.86-6.85 (2H, m), 5.52 (1H, d, J=8.4 Hz), 4.79 (1H, s), 4.45-4.43 (1H, m), 4.43 (1H, s), 3.96-3.94 (2H, m), 3.82-3.79 (2H, m), 1.99-1.84 (4H, m), 1.44 (9H, s), 1.21 (6H, s).
MS (FAB, m/z): 410 (M+H)⁺.

### Example 69-2

### N-[(1S)-1-{[4-(4-Fluorophenoxy)piperidin-1-yl]carbonyl}-2-hydroxy-2-methylpropyl]-3-hydroxyquinoxaline-2-carboxamide

Water (0.5 ml) and concentrated hydrochloric acid (0.5 ml) were added to a 1,4-dioxane solution (2.0 ml) of t-butyl [(1S)-1-{[4-(4-fluorophenoxy)piperidin-1-yl]carbonyl}-2-hydroxy-2-methylpropyl]carbamate (86.0 mg, 0.210 mmol), and the mixture was allowed to react at room temperature overnight. The reaction solution was concentrated to afford (3S)-3-amino-4-[4-(4-fluorophenoxy)piperidin-1-yl]-2-methyl-4-oxobutan-2-ol hydrochloride as a colorless oil. This product was used as it is in the next reaction without purification.

1-Ethyl-3-(3-dimethylaminopropyl)carbodiimide hydrochloride (48.0 mg, 0.250 mmol), 1-hydroxybenzotriazole monohydrate (34.0 mg, 0.250 mmol) and N-methylmorpholine (0.0930 ml, 0.840 mmol), were added to a methylene chloride solution (4.0 ml) of the resulting compound and 3-hydroxyquinoxaline-2-carboxylic acid (40.0 mg, 0.210 mmol), at room temperature, under nitrogen stream, and stirring was carried out at room temperature overnight. The reaction solution was poured into a 2N aqueous hydrochloric acid solution, followed by extraction with ethyl acetate. The extract was washed sequentially with water and saline, and then dried over anhydrous sodium sulfate. The organic layer was concentrated, the resulting residue was purified by silica gel chromatography, the resulting residue was suspended in diethyl ether, and then the solid substance was collected by filtration to afford the desired title compound (62.0 mg, yield 62%) as a pale yellow solid.
¹H-NMR (DMSO-d₆, 400 MHz) δ: 12.86 (1H, s), 9.74 (1H, m), 7.88 (1H, dd, J=8.8 Hz, 1.6 Hz), 7.41-7.38 (2H, m), 7.15-7.10 (2H, m), 7.04-7.01
(2H, m), 5.11 (1H, m), 4.93 (1H, s), 4.62-4.58 (1H, m), 4.02-3.93 (2H, m), 3.57-3.53 (1H, m), 3.32-3.30 (1H, m), 2.01-1.98 (1H, m), 1.93-1.89 (1H, m), 1.70-1.68 (1H, m), 1.53-1.49 (1H, m), 1.24 (3H, s), 1.17 (3H, s).
IR (KBr) cm⁻¹: 2975, 1685, 1625, 1505, 1205.
HRMS (ESI, m/z): 483.2050 (Calcd for C₂₅H₂₈FN₄O₅: 483.2043).
Anal. Calcd for C₂₄H₂₇FN₄O_{5·}0.2H₂O: C, 61.77; H, 5.68; N, 11.53; F, 3.91. Found: C, 61.73; H, 5.51; N, 11.59; F, 4.14.

### Example 70

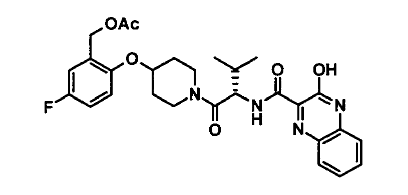

### 5-fluoro-2-[(1-{N-[(3-hydroxyquinoxalin-2-yl)carbonyl]-L-valyl}piperidin-4-yl)oxy]benzyl acetate

### Example 70-1

### 2-({1-[N-(t-butoxycarbonyl)-L-valyl]piperidin-4-yl}oxy)-5-fluorobenzyl acetate

A toluene solution of diisobutylaluminium hydride (0.990 M, 13.6 ml, 13.5 mmol) was slowly added dropwise to a tetrahydrofuran solution (50 ml) of t-butyl 4-(4-fluoro-2-methoxycarbonyphenoxy)piperidine-1-carboxylate (1.59 g, 4.50 mmol), at 0°C, under nitrogen stream, and stirring was carried out at 0°C for 1 hour. After completion of the reaction, an aqueous Rochelle salt solution and ethyl acetate were sequentially added, and stirring was carried out at room temperature overnight. The reaction solution was extracted with ethyl acetate, and then the extract was washed sequentially with a saturated aqueous sodium hydrogencarbonate solution, water and saline. The resulting organic layer was dried over anhydrous sodium sulfate, and then the residue resulting from concentration was purified by a medium-pressure preparative liquid chromatograph (manufactured by Biotage, Inc., 25+M) to afford t-butyl 4-(4-fluoro-2-hydroxymethylphenoxy)piperidine-1-carboxylate (1.13 g, yield 77%) as a colorless oil.
Acetic anhydride (0.304 ml, 3.24 mmol) was added to a pyridine solution (5 ml) of the resulting compound (350 mg, 1.08 mmol), under nitrogen stream, and stirring was carried out at room temperature overnight. The reaction solution was concentrated to afford t-butyl 4-(2-acetoxymethyl-4-fluorophenoxy)piperidine-1-carboxylate (396 mg) as a colorless oil.

Analogously to Example 23-2, 5-fluoro-2-[(piperidin-4-yl)oxy]benzyl acetate hydrochloride (328 mg) was obtained from the resulting compound (396 mg, 1.08 mmol) as a white solid. The resulting compound (328 mg, 1.08 mmol) was condensed with N-(t-butoxycarbonyl)-L-valine (352 mg, 1.62 mmol) to afford 2-({1-[N-(t-butoxycarbonyl)-L-valyl]piperidin-4-yl}oxy)-5-fluorobenzyl acetate (475 mg, yield 94%) as a colorless amorphous substance.
¹H-NMR (CDCl₃, 400 MHz) δ: 7.08 (1H, dd, J=9.0 Hz, 2.9 Hz), 6.99-6.96 (1H, m), 6.82 (1H, dd, J=9.0 Hz, 4.3 Hz), 5.35 (1H, d, J=9.0 Hz), 5.14 (2H, d, J=8.6 Hz), 4.58-4.48 (2H, m), 3.88-3.48 (4H, m), 2.11 (3H, s), 1.99-1.81 (5H, m), 1.47-1.44 (9H, m), 0.97 (3H, d, J=6.6 Hz), 0.89 (3H, d, J=6.6 Hz).
MS (FAB, m/z): 467 (M+H)⁺.

### Example 70-2

### 5-fluoro-2-[(1-{N-[(3-hydroxyquinoxalin-2-yl)carbonyl]-L-valyl}piperidin-4-yl)oxy]benzyl acetate

Analogously to Example 23-3, 5-fluoro-2-[(1-valylpiperidin-4-yl)oxy]benzyl acetate hydrochloride (410 mg) was obtained from 2-({1-[N-(t-butoxycarbonyl)-L-valyl]piperidin-4-yl}oxy)-5-fluorobenzyl acetate (475 mg, 1.02 mmol) as a white solid. The resulting compound (230 mg, 0.571 mmol) was condensed with 3-hydroxyquinoxaline-2-carboxylic acid (163 mg, 0.856 mmol) to afford the desired title compound (240 mg, yield 78%) as a pale yellow solid.
¹H-NMR (DMSO-d₆, 400 MHz) δ: 9.57 (1H, d, J=9.0 Hz), 7.86-7.85 (1H, m), 7.64-7.62 (1H, m), 7.38 (2H, d, J=7.8 Hz), 7.19-7.15 (3H, m), 5.07 (2H, d, J=8.6 Hz), 4.91 (1H, m), 4.71 (1H, brs), 4.10 (1H, brs), 3.70-3.54 (4H, m), 2.08-1.60 (8H, m), 0.95 (6H, t, J=5.9 Hz).
IR (KBr) cm⁻¹: 2960, 1685, 1640, 1495, 1225, 1030.
MS (ESI, m/z): 539 (M+H)⁺, 561 (M+Na)⁺.
HRMS (ESI, m/z): 561.2138 (Calcd for C₂₈H₃₁FN₄NaO₆: 561.2125).

### Example 71

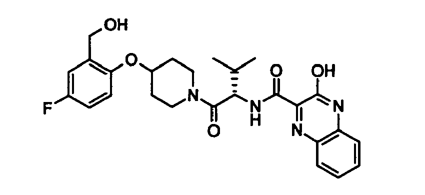

### N-[(1S)-1-({4-[4-Fluoro-2-(hydroxymethyl)phenoxy]piperidin-1-yl}carbonyl)-2-methylpropyl]-3-hydroxyquinoxaline-2-carboxamide

A 2N aqueous sodium hydroxide solution (3 ml) was added to a tetrahydrofuran solution (3.0 ml) of methyl 5-fluoro-2-[(1-{N-[(3-hydroxyquinoxalin-2-yl)carbonyl]-L-valyl}piperidin-4-yl)oxy]benzoate (120 mg, 0.223 mmol), and stirring was carried out at room temperature overnight. Under ice cooling, the reaction solution was poured into a 2N aqueous hydrochloric acid solution, followed by extraction with ethyl acetate, and the extract was washed sequentially with water and saline. The resulting organic layer was dried over anhydrous sodium sulfate, and then concentrated to afford the desired title compound (84.0 mg, yield 76%) as a pale yellow solid.
¹H-NMR (DMSO-d₆, 400 MHz) δ: 12.82 (1H, s), 9.52 (1H, d, J=8.8 Hz), 7.87 (1H, d, J=7.8 Hz), 7.64 (1H, m), 7.39-7.37 (2H, m), 7.18-7.16 (1H, m), 7.07-6.98 (2H, m), 5.19-5.18 (1H, m), 4.92 (1H, dd, J=14.7 Hz, 6.5 Hz), 4.64 (1H, brs), 4.53-4.51 (2H, m), 3.85-3.37 (4H, m), 2.10-1.58 (5H, m), 0.95 (6H, t, J=5.9 Hz).
IR (KBr) cm⁻¹: 2960, 1685, 1635, 1490, 1195, 1045.
MS (ESI, m/z): 497 (M+H)⁺.
HRMS (ESI, m/z): 519.2026 (Calcd for C₂₆H₂₉FN₄NaO₅: 519.2019).

### Example 72

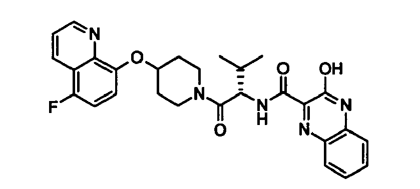

### N-[(1S)-1-({4-[(5-Fluoroquinolin-8-yl)oxy]piperidin-1-yl}carbonyl)-2-methylpropyl]-3-hydroxyquinoxaline-2-carboxamide

### Example 72-1

### t-Butyl 4-[(5-fluoroquinolin-8-yl)oxy]piperidine-1-carboxylate

Analogously to Example 23-1, t-butyl 4-[(5-fluoroquinolin-8-yl)oxy]piperidine-1-carboxylate (1.14 g, yield 90%) was obtained from t-butyl 4-hydroxypiperidine-1-carboxylate (740 mg, 3.68 mmol) and 5-fluoro-8-hydroxyquinolin (600 mg, 3.68 mmol) as a colorless oil. ¹H-NMR (CDCl₃, 500 MHz) δ: 8.99 (1H, dd, J=4.2 Hz, 1.5 Hz), 8.40 (1H, dd, J=8.3 Hz, 1.5 Hz), 7.49 (1H, dd, J=8.8 Hz, 4.2 Hz), 7.12 (1H, t, J=8.8 Hz), 7.06 (1H, dd, J=8.3 Hz, 4.9 Hz), 4.72-4.67 (1H, m), 3.95 (2H, brs), 3.22-3.17 (2H, m), 2.09-2.05 (2H, m), 1.94-1.91 (2H, m), 1.47-1.45 (9H, m).
MS (FAB, m/z): 347 (M+H)⁺.

### Example 72-2

### t-Butyl [(1S)-1-({4-[(5-fluoroquinolin-8-yl)oxy]piperidin-1-yl}carbonyl)-2-methylpropyl]carbamate

Analogously to Example 23-2, 5-fluoro-8-[(piperidin-4-yl)oxy]quinolinhydrochloride (1.02 g) was obtained from t-butyl 4-[(5-fluoro-quinolin-8-yl)oxy]piperidine-1-carboxylate (1.14 g, 3.29 mmol) as a white solid. The resulting compound (720 mg, 2.26 mmol) was condensed with N-(t-butoxycarbonyl)-L-valine (588 mg, 2.71 mmol) to afford t-butyl [(1S)-1-({4-[(5-fluoroquinolin-8-yl)oxy]piperidin-1-yl}carbonyl)-2-methylpropyl]carbamate (772 mg, yield 77%) as a colorless amorphous substance.
¹H-NMR (CDCl₃, 400 MHz) δ: 8.99 (1H, dd, J=4.2 Hz, 1.5 Hz), 8.42 (1H, dd, J=8.4 Hz, 1.5 Hz), 7.51 (1H, dd, J=8.4 Hz, 4.2 Hz), 7.17-7.06 (2H, m), 5.38-5.36 (1H, m), 4.82-4.80 (1H, m), 4.55-4.52 (1H, m), 4.15-4.09 (1H, m), 4.02-3.94 (1H, m), 3.56-3.42 (2H, m), 2.18-1.93 (5H, m), 1.45-1.45 (9H, m), 0.99 (3H, t, J=6.8 Hz), 0.90 (3H, t, J=6.8 Hz).
MS (FAB, m/z): 445 (M+H)⁺.

### Example 72-3

### N-[(1S)-1-({4-[(5-Fluoroquinolin-8-yl)oxy]piperidin-1-yl}carbonyl)-2-methylpropyl]-3-hydroxyquinoxaline-2-carboxamide

Analogously to Example 23-3, (2S)-1-{4-[(5-fluoroquinolin-8-yl)oxy]piperidin-1-yl}-3-methyl-1-oxobutan-2-amine dihydrochloride (725 mg) was obtained from t-butyl [(1S)-1-({4-[(5-fluoroquinolin-8-yl)oxy]piperidin-1-yl}carbonyl)-2-methylpropyl]carbamate (772 mg, 1.73 mmol) as a white solid. The resulting compound (200 mg, 0.478 mmol) was condensed with 3-hydroxyquinoxaline-2-carboxylic acid (136 mg, 0.717 mmol) to afford the desired title compound (160 mg, yield 65%) as a pale yellow solid.
¹H-NMR (DMSO-d₆, 400 MHz) δ: 12.81 (1H, brs), 9.66 (1H, brs), 8.98 (1H, dd, J=4.2 Hz, 1.5 Hz), 8.45 (1H, dd, J=8.4 Hz, 1.5 Hz), 7.85 (1H, dd, J=8.4 Hz, 4.2 Hz), 7.66-7.63 (2H, m), 7.38-7.36 (4H, m), 4.95-4.91 (2H, m), 4.03-4.01 (2H, m), 3.59-3.33 (2H, m), 2.09-1.68 (5H, m), 0.97 (6H, t, J=7.0 Hz).
IR (KBr) cm⁻¹: 2960, 1685, 1630, 1530, 1475, 1255, 1090.
MS (ESI, m/z): 518 (M+H)⁺.
HRMS (ESI, m/z): 518.2191 (Calcd for C₂₈H₂₈FN₅O₄: 518.2203).

### Example 73

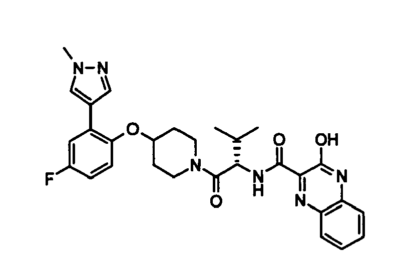

### N-[(1S)-1-({4-[4-Fluoro-2-(1-methyl-1H-pyrazol-4-yl)phenoxy]piperidin-1-yl}carbonyl)-2-methylpropyl]-3-hydroxyquinoxaline-2-carboxamide

### Example 73-1

### t-Butyl 4-[4-fluoro-2-(1-methyl-1H-pyrazol-4-yl)phenoxy]piperidine-1-carboxylate

Potassium carbonate (775 mg, 5.61 mmol) and tetrakis(triphenylphosphine)palladium (108 mg, 0.0935 mmol) were added sequentially to a 1,2-dimethoxyethane-water (5.0 ml, 4:4, v/v) mixed solution of t-butyl 4-(2-bromo-4-fluorophenoxy)piperidine-1-carboxylate (350 mg, 0.935 mmol) and 1-methyl-4-(4,4,5,5-tetramethyl-1,3,2-dioxaboran-2-yl)-1H-pyrazol (233 mg, 1.12 mmol), under nitrogen stream, and stirring was carried out overnight under heating to reflux. After completion of the reaction, the reaction solution was cooled to room temperature, and poured into ice water, followed by extraction with ethyl acetate. The extract was washed sequentially with water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford t-butyl 4-[4-fluoro-2-(1-methyl-1H-pyrazol-4-yl)phenoxy]piperidine-1-carboxylate (253 mg, yield 72%) as a colorless oil.
¹H-NMR (CDCl₃, 400 MHz) δ: 7.83 (1H, d, J=15.2 Hz), 7.75 (1H, d, J=11.3 Hz), 7.20-7.16 (1H, m), 6.91-6.83 (2H, m), 4.45-4.43 (1H, m), 3.95 (3H, s), 3.74-3.72 (2H, m), 3.28-3.21 (2H, m), 1.94-1.88 (2H, m), 1.74-1.69 (2H, m), 1.47-1.46 (9H, m).
MS (FAB, m/z): 376 (M+H)⁺.

### Example 73-2

### t-Butyl [(1S)-1-({4-[4-fluoro-2-(1-methyl-1H-pyrazol-4-yl)phenoxy]piperidin-1-yl}carbonyl)-2-methylpropyl]carbamate

Analogously to Example 23-2, 4-[4-fluoro-2-(1-methyl-1H-pyrazol-4-yl)phenoxy]piperidine hydrochloride (210 mg, yield 100%) was obtained from t-butyl 4-[4-fluoro-2-(1-methyl-1H-pyrazol-4-yl)phenoxy]piperidine-1-carboxylate as a white solid. The resulting compound (210 mg, 0.674 mmol) was condensed with N-(t-butoxycarbonyl)-L-valine (176 mg, 0.809 mmol) to afford t-butyl [(1S)-1-({4-[4-fluoro-2-(1-methyl-1H-pyrazol-4-yl)phenoxy]piperidin-1-yl}carbonyl)-2-methylpropyl]carbamate (158 mg, yield 49%) as a colorless oil.
¹H-NMR (CDCl₃, 400 MHz) δ: 7.83 (1H, d, J=15.2 Hz), 7.75 (1H, d, J=11.3 Hz), 7.20-7.15 (1H, m), 6.91-6.84 (2H, m), 5.33 (1H, t, J=8.6 Hz), 4.53-4.47 (2H, m), 3.95 (3H, s), 3.79-3.60 (2H, m), 3.47-3.40 (2H, m), 2.05-1.80 (5H, m), 1.45-1.42 (9H, m), 0.96 (3H, d, J=6.8 Hz), 0.89 (3H, d, J=6.8 Hz).
MS (FAB, m/z): 475 (M+H)⁺.

### Example 73-3

### N-[(1S)-1-({4-[4-Fluoro-2-(1-methyl-1H-pyrazol-4-yl)phenoxy]piperidin-1-yl}carbonyl)-2-methylpropyl]-3-hydroxyquinoxaline-2-carboxamide

Analogously to Example 23-3, (2S)-1-{4-[4-fluoro-2-(1-methyl-1H-pyrazol-4-yl)phenoxy]piperidin-1-yl}-3-methyl-1-oxobutan-2-amine hydrochloride (137 mg) was obtained from t-butyl [(1S)-1-({4-[4-fluoro-2-(1-methyl-1H-pyrazol-4-yl)phenoxy]piperidin-1-yl}carbonyl)-2-methylpropyl]carbamate (158 mg, 0.333 mmol) as a white solid. The resulting compound (70.0 mg, 0.170 mmol) was condensed with 3-hydroxyquinoxaline-2-carboxylic acid (49.0 mg, 0.256 mmol) to afford the desired title compound (92.0 mg, yield 99%) as a pale yellow solid.
¹H-NMR (DMSO-d₆, 400 MHz) δ: 12.80 (1H, brs), 9.54 (1H, d, J=8.2 Hz), 8.13 (1H, d, J=5.9 Hz), 7.93 (1H, d, J=5.5 Hz), 7.84 (1H, dd, J=16.0 Hz, 7.6 Hz), 7.63 (1H, t, J=7.6 Hz), 7.44 (1H, dd, J=10.0 Hz, 2.9 Hz), 7.39-7.37 (2H, m), 7.18-7.16 (1H, m), 7.01-6.97 (1H, m), 4.94-4.91 (1H, m), 4.71-4.68 (1H, m), 4.02-3.87 (2H, m), 3.86 (3H, d, J=12.9 Hz), 3.53-3.16 (2H, m), 2.18-1.54 (5H, m), 1.05-0.85 (6H, m).
IR (KBr) cm⁻¹: 2960, 1685, 1640, 1530, 1495, 1450, 1210.
MS (ESI, m/z): 569 (M+Na)⁺.
HRMS (ESI, m/z): 569.2304 (Calcd for C₂₉H₃₁FN₆NaO₄: 569.2288).

### Example 74

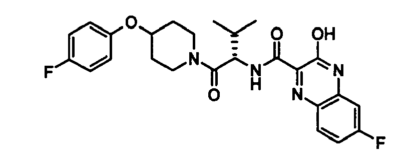

### N-[(1S)-1-{[4-(4-Fluorophenoxy)piperidin-1-yl]carbonyl}-2-methylpropyl]-6-fluoro-3-hydroxyquinoxaline-2-carboxamide

### Example 74-1

### Ethyl 6-fluoro-3-hydroxyquinoxaline-2-carboxylate

4-Fluoro-2-nitroaniline (672 mg, 4.31 mmol) and diethyl ketomalonate (500 mg, 2.87 mmol) were dissolved in ethanol (30 ml), under nitrogen stream, and stirring was carried out at room temperature for 1.5 hours. Subsequently, tin (II) chloride dihydrate (1.62 g, 7.18 mmol) was added, and further stirring was carried out overnight. The reaction solution was concentrated to 5.0 ml under reduced pressure and poured into a 2N aqueous hydrochloric acid solution, followed by extraction with methylene chloride, the extract was washed sequentially with water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford ethyl 6-fluoro-3-oxo-1,2,3,4-tetrahydroquinoxaline-2-carboxylate (105 mg, yield 16%) as a pale yellow solid.

2,3-Dichloro-5,6-dicyanobenzoquinone (66.0 mg, 0.290 mmol) was added to a methylene chloride solution (3.0 ml) of the resulting compound (59.0 mg, 0.248 mmol), at room temperature, under nitrogen stream, and stirring was carried out at room temperature for 3 hours. After completion of the reaction, the reaction solution was poured into ice water, followed by extraction with ethyl acetate, and the extract was washed sequentially with water and saline and then dried over anhydrous sodium sulfate. The organic layer was concentrated and the resulting residue was purified by a medium-pressure preparative liquid chromatograph (manufactured by Biotage, Inc., 25+M) to afford ethyl 6-fluoro-3-hydroxyquinoxaline-2-carboxylate (52.0 mg, yield 90%) as a white solid.
¹H-NMR (CDCl₃, 400 MHz) δ: 7.68 (1H, d, J=8.0 Hz), 7.45-7.43 (2H, m), 4.57 (2H, q, J=7.0 Hz), 1.49 (3H, t, J=7.0 Hz).
MS (FAB, m/z): 237 (M+H)⁺.

### Example 74-2

### N-[(1S)-1-{[4-(4-Fluorophenoxy)piperidin-1-yl]carbonyl}-2-methylpropyl]-6-fluoro-3-hydroxyquinoxaline-2-carboxamide

Lithium hydroxide monohydrate (32.0 mg, 0.773 mmol) was added to an aqueous solution (2.0 ml) of ethyl 6-fluoro-3-hydroxyquinoxaline-2-carboxylate (73.0 mg, 0.309 mmol), under nitrogen stream, and the mixture was allowed to react at room temperature for 2.5 hours. Under ice cooling, the reaction solution was poured into a 2N aqueous hydrochloric acid solution, and the precipitated solid was collected by filtration, followed by drying to afford 6-fluoro-3-hydroxy-2-quinoxaline carboxylic acid (52.0 mg, yield 81%) as a pale yellow solid.

1-Ethyl-3-(3-dimethylaminopropyl)carbodiimide hydrochloride (72.0 mg, 0.375 mmol) and 1-hydroxybenzotriazole monohydrate (51.0 mg, 0.375 mmol) were added to a methylene chloride solution (5.0 ml) of the resulting compound (52.0 mg, 0.250 mmol) and (2S)-1-[4-(4-fluorophenoxy)piperidin-1-yl]-3-methyl-1-oxobutan-2-amine (74.0 mg, 0.250 mmol), at room temperature, under nitrogen stream, and stirring was carried out at room temperature overnight. The reaction solution was poured into a 2N aqueous hydrochloric acid solution, followed by extraction with ethyl acetate, and the extract was washed sequentially with water, a saturated aqueous sodium hydrogencarbonate solution, water and saline, and then dried over anhydrous sodium sulfate. After the organic layer was concentrated and the resulting residue was purified by a medium-pressure preparative liquid chromatograph (manufactured by Biotage, Inc., 25+M), the residue resulting from concentration was suspended in a mixed solvent of methylene chloride-diethyl ether, and the solid substance was collected by filtration to afford the desired title compound (90.0 mg, yield 74%) as a pale yellow solid.
¹H-NMR (DMSO-d₆, 500 MHz) δ: 12.86 (1H, brs), 9.46 (1H, brs), 7.72 (1H, t, J=7.2 Hz), 7.57-7.55 (1H, m), 7.40 (1H, dd, J=9.3 Hz, 4.6 Hz), 7.13-7.11 (2H, m), 7.04-7.00 (2H, m), 4.90 (1H, dd, J=13.2 Hz, 7.3 Hz), 4.60-4.59 (1H, m), 3.93-3.90 (2H, m), 3.49-3.31 (2H, m), 2.09-1.91 (3H, m), 1.66-1.55 (2H, m), 0.94 (6H, t, J=6.8 Hz).
IR (KBr) cm⁻¹: 2965, 1690, 1640, 1505, 1245, 1205.
MS (ESI, m/z): 507 (M+Na)⁺.
HRMS (ESI, m/z): 507.1804 (Calcd for C₂₅H₂₆F₂N₄NaO₄: 507.1819).

### Example 75

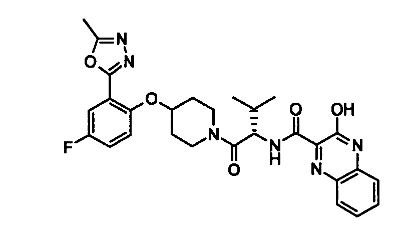

### N-[(1S)-1-({4-[4-Fluoro-2-(5-methyl-1,3,4-oxadiazol-2-yl)phenoxy]piperidin-1-yl}carbonyl)-2-methylpropyl]-3-hydroxyquinoxaline-2-carboxamide

### Example 75-1

### Methyl 2-[(1-{N-[(benzyloxycarbonyl)amino]-L-valyl}piperidin-4-yl)oxy]-5-fluorobenzoate

Analogously to Example 23-2, methyl 5-fluoro-2-[(piperidin-4-yl)oxy]benzoate hydrochloride (985 mg, yield 100%) was obtained from t-butyl 4-[4-fluoro-2-(methoxycarbonyl)phenoxy]piperidine-1-carboxylate (1.20 g, 3.40 mmol) as a white solid. The resulting compound (985 mg, 3.40 mmol) was condensed with N-(benzyloxycarbonyl)-L-valine (1.02 g, 4.07 mmol) to afford methyl 2-[(1-{N-[(benzyloxycarbonyl)amino]-L-valyl}piperidin-4-yl)oxy]-5-fluorobenzoate (1.49 g, yield 90%) as a colorless amorphous substance. ¹H-NMR (CDCl₃, 400 MHz) δ: 7.53 (1H, dd, J=8.6 Hz, 3.0 Hz), 7.35-7.32 (5H, m), 7.19-7.14 (1H, m), 6.93 (1H, dd, J=8.6 Hz, 4.3 Hz), 5.62 (1H, t, J=8.0 Hz), 5.10-5.09 (2H, m), 4.62-4.55 (2H, m), 4.01-3.98 (1H, m), 3.88 (3H, s), 3.88-3.53 (3H, m), 1.93-1.89 (5H, m), 0.99 (3H, d, J=6.6 Hz), 0.89 (3H, d, J=6.6 Hz).
MS (FAB, m/z): 487 (M+H)⁺.

### Example 75-2

### Benzyl {(1S)-1-[(4-{2-[(2-acetylhydrazino)carbonyl]-4-fluorophenoxy}piperidin-1-yl)carbonyl]-2-methylpropyl}carbamate

A 2N aqueous sodium hydroxide solution (30 ml) was added to a tetrahydrofuran solution (30 ml) of methyl 2-[(1-{N-[(benzyloxycarbonyl)amino]-L-valyl}piperidin-4-yl)oxy]-5-fluorobenzoate (1.23 g, 2.53 mmol), and the mixture was allowed to react at room temperature overnight. Under ice cooling, the reaction solution was poured into a 2N aqueous hydrochloric acid solution, followed by extraction with ethyl acetate. The extract was washed sequentially with water and saline and dried over anhydrous sodium sulfate, and then concentrated to afford 2-[(1-{N-[(benzyloxycarbonyl)amino]-L-valyl}piperidin-4-yl)oxy]-5-fluorobenzoic acid (840 mg).

1-Ethyl-3-(3-dimethylaminopropyl)carbodiimide hydrochloride (512 mg, 2.67 mmol) and 1-hydroxybenzotriazole monohydrate (361 mg, 2.67 mmol) were added to a tetrahydrofuran solution (25 ml) of the resulting compound (840 mg, 1.78 mmol) and acetohydrazide (198 mg, 2.67 mmol), at room temperature, and stirring was carried out at room temperature overnight. The reaction solution was poured into a 2N aqueous hydrochloric acid solution, followed by extraction with ethyl acetate, the extract was washed sequentially with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford benzyl {(1S)-1-[(4-{2-[(2-acetylhydrazino)carbonyl]-4-fluorophenoxy}piperidin-1-yl)carbonyl]-2-methylpropyl}carbamate (854 mg, yield 91%) as a colorless amorphous substance.
¹H-NMR (CDCl₃, 400 MHz) δ: 10.82-10.74 (1H, m), 9.33-9.23 (1H, m), 7.90 (1H, dd, J=8.6 Hz, 3.0 Hz), 7.35-7.33 (5H, m), 7.21-7.18 (1H, m), 6.99 (1H, dd, J=8.6 Hz, 4.3 Hz), 5.67 (1H, m), 5.11-5.10 (2H, m), 4.73-4.70 (1H, m), 4.58-4.56 (1H, m), 4.11-3.95 (2H, m), 3.55-3.46 (2H, m), 2.12 (3H, d, J=7.4 Hz), 1.97-1.79 (5H, m), 1.01 (3H, t, J=6.6 Hz), 0.91 (3H, t, J=6.6 Hz).

### Example 75-3

### Benzyl [(1S)-1-({4-[4-fluoro-2-(5-methyl-1,3,4-oxadiazol-2-yl)phenoxy]piperidin-1-yl}carbonyl)-2-methylpropyl]carbamate

p-Toluenesulfonyl chloride (420 mg, 0.874 mmol) and triethylamine (0.244 ml, 1.75 mmol) were added to a methylene chloride solution (15 ml) of benzyl {(1S)-1-[(4-{2-[(2-acetylhydrazino)carbonyl]-4-fluorophenoxy}piperidin-1-yl)carbonyl]-2-methylpropyl}carbamate (420 mg, 0.795 mmol), under nitrogen stream, and stirring was carried out at room temperature overnight. Under ice cooling, the reaction solution was poured into a 2N aqueous hydrochloric acid solution, followed by extraction with ethyl acetate, the extract was washed sequentially with water and saline, and then the resulting organic layer was dried over anhydrous sodium sulfate. The organic layer was concentrated and the resulting residue was purified by a medium-pressure preparative liquid chromatograph (manufactured by Biotage, Inc., 25+M) to afford benzyl [(1S)-1-({4-[4-fluoro-2-(5-methyl-1,3,4-oxadiazol-2-yl)phenoxy]piperidin-1-yl}carbonyl)-2-methylpropyl]carbamate (227 mg, yield 56%) as a colorless amorphous substance.
¹H-NMR (CDCl₃, 400 MHz) δ: 7.69-7.63 (1H, m), 7.36-7.31 (5H, m), 7.19-7.16 (1H, m), 7.00 (1H, dd, J=8.6 Hz, 4.3 Hz), 5.60 (1H, d, J=8.6 Hz), 5.09 (2H, s), 4.72-4.67 (1H, m), 4.56 (1H, dd, J=8.6 Hz, 5.7 Hz), 4.07-3.49 (4H, m), 2.60 (3H, s), 1.97-1.91 (5H, m), 0.99 (3H, t, J=6.6 Hz), 0.89 (3H, t, J=6.6 Hz).

### Example 75-4

### N-[(1S)-1-({4-[4-Fluoro-2-(5-methyl-1,3,4-oxadiazol-2-yl)phenoxy]piperidin-1-yl}carbonyl)-2-methylpropyl]-3-hydroxyquinoxaline-2-carboxamide

5% Palladium carbon catalyst (193 mg) was added to a methanol solution (10 ml) of benzyl [(1S)-1-({4-[4-fluoro-2-(5-methyl-1,3,4-oxadiazol-2-yl)phenoxy]piperidin-1-yl}carbonyl)-2-methylpropyl]carbamate (464 mg, 0.909 mmol), and stirring was carried out at room temperature for 3 hours under hydrogen atmosphere. The reaction solution was celite filtered, and the filtrate was concentrated to afford (2S)-1-{4-[4-fluoro-2-(5-methyl-1,3,4-oxadiazol-2-yl)phenoxy]piperidin-1-yl}-3-methyl-1-oxobutan-2-amine (334 mg, yield 98%) as white crystals.

1-Ethyl-3-(3-dimethylaminopropyl)carbodiimide hydrochloride (126 mg, 0.657 mmol) and 1-hydroxybenzotriazole monohydrate (89.0 mg, 0.657 mmol) were added to a methylene chloride solution (5.0 ml) of the resulting compound (165 mg, 0.438 mmol) and 3-hydroxyquinoxaline-2-carboxylic acid (125 mg, 0.657 mmol), at room temperature, and stirring was carried out at room temperature overnight. The reaction solution was poured into a 2N aqueous hydrochloric acid solution, followed by extraction with ethyl acetate, the extract was washed sequentially with water, a saturated aqueous sodium hydrogencarbonate solution, water and saline, and then the resulting organic layer was dried over anhydrous sodium sulfate. The organic layer was concentrated, the resulting residue was purified by a medium-pressure preparative liquid chromatograph (manufactured by Biotage, Inc., 25+M), the residue resulting from concentration was suspended in a mixed solvent of methylene chloride-dimethylether, and the solid substance was collected by filtration to afford the desired title compound (217 mg, yield 90%) as a pale yellow solid.
¹H-NMR (DMSO-d₆, 400 MHz) δ: 12.82 (1H, brs), 9.50 (1H, d, J=9.0 Hz), 7.86 (1H, t, J=7.0 Hz), 7.65-7.63 (2H, m), 7.49-7.36 (4H, m), 4.94-4.85 (2H, m), 3.71-3.61 (4H, m), 2.55 (3H, d, J=12.9 Hz), 1.96-1.84 (5H, m), 0.97-0.93 (6H, m).
IR (KBr) cm⁻¹: 2965, 1685, 1640, 1540, 1480, 1265.
MS (ESI, m/z): 571 (M+Na)⁺.
HRMS (ESI, m/z): 571.2075 (Calcd for C₂₈H₂₉FN₆NaO₅: 571.2081).

### Example 76

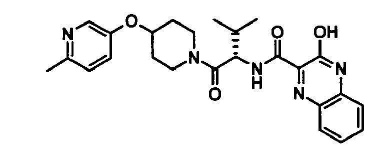

### 3-Hydroxy-N-[(1S)-2-methyl-1-({4-[(6-methylpyridin-3-yl)oxy]piperidin-1-yl}carbonyl)propyl]quinoxaline-2-carboxamide

### Example 76-1

### t-Butyl [(1S)-2-methyl-1-({4-[(6-methylpyridin-3-yl)oxy]piperidin-1-yl}carbonyl)propyl]carbamate

A 40% toluene solution of diisopropyl azodicarboxylate (2.10 ml, 3.99 mmol) was added to a toluene solution (15 ml) of t-butyl [(1S)-4-(4-hydroxypiperidine-1-carbonyl)-2-methylpropyl]carbamate (1.00 g, 3.33 mmol), 5-hydroxy-2-methylpyridine (404 mg, 3.66 mmol) and triphenylphosphine (1.05 g, 3.99 mmol), at room temperature, and stirring was carried out for 4 days. The reaction solution was concentrated under reduced pressure and the resulting residue was diluted with ethyl acetate, followed by sequential washing with a 1N aqueous sodium hydroxide solution, water and saline, and then the resulting organic layer was extracted with a 1N hydrochloric acid. A 5N aqueous sodium hydroxide solution was used to make the aqueous layer basic, followed by extraction with ethyl acetate. The resulting organic layer was dried over anhydrous sodium sulfate, and the organic layer was concentrated to afford t-butyl [(1S)-2-methyl-1-({4-[(6-methylpyridin-3-yl)oxy]piperidin-1-yl}carbonyl)propyl]carbamate (980 mg, yield 72%) as a colorless amorphous substance.
¹H-NMR (CDCl₃) δ: 8.20 (1H, s), 7.14-7.07 (2H, m), 5.34 (1H, d, J=7.8 Hz), 4.56-4.48 (2H, m), 3.82-3.46 (4H, m), 2.50 (3H, s), 1.99-1.80 (5H, m), 1.44 (9H, s), 0.97 (3H, d, J=6.6 Hz), 0.89 (3H, d, J=6.6 Hz). MS (ESI, m/z): 392 (M+H)⁺.

### Example 76-2

### (2S)-3-Methyl-1-{4-[(6-methylpyridin-3-yl)oxy]piperidin-1-yl}-1-oxobutan-2-amine dihydrochloride

Analogously to Example 29-3, (2S)-3-methyl-1-{4-[(6-methylpyridin-3-yl)oxy]piperidin-1-yl}-1-oxobutan-2-amine dihydrochloride (907 mg, yield 100%) was obtained from t-butyl [(1S)-2-methyl-1-({4-[(6-methylpyridin-3-yl)oxy]piperidin-1-yl}carbonyl)propyl]carbamate (980 mg, 2.50 mmol) as a colorless amorphous substance.
¹H-NMR (CD₃OD) δ: 8.52 (1H, s), 8.21-8.18 (1H, m), 7.85 (1H, d, J=9.0 Hz), 4.96 (1H, s), 4.38 (1H, d, J=4.3 Hz), 4.12-3.43 (4H, m), 2.71 (3H, s), 2.24-2.03 (3H, m), 1.93-1.74 (2H, m), 1.11 (3H, d, J=6.6 Hz), 1.02 (3H, d, J=6.6 Hz).
MS (ESI, m/z): 292 (M+H)⁺.

### Example 76-3

### 3-Hydroxy-N-[(1S)-2-methyl-1-({4-[(6-methylpyridin-3-yl)oxy]piperidin-1-yl}carbonyl)propyl]quinoxaline-2-carboxamide

Analogously to Example 29-4, the desired title compound (228 mg, yield 90%) was obtained from (2S)-3-methyl-1-{4-[(6-methylpyridin-3-yl)oxy]piperidin-1-yl}-1-oxobutan-2-amine dihydrochloride (200 mg, 0.549 mmol) as a yellow solid.
¹H-NMR (CDCl₃) δ: 12.73 (1H, br s), 10.04 (1H, br s), 8.23 (1H, dd, J=8.8 Hz, 2.9 Hz), 8.00 (1H, t, J=7.0 Hz), 7.63 (1H, t, J=7.4 Hz), 7.55 (1H, br s), 7.41-7.37 (1H, m), 7.17-7.13 (1H, m), 7.09 (1H, dd, J=8.6 Hz, 4.7 Hz), 5.10-5.05 (1H, m), 4.63-4.57 (1H, m), 4.03-3.68 (4H, m), 2.51 (3H, d, J=2.3 Hz), 2.40-1.87 (5H, m), 1.14-1.09 (6H, m). IR (KBr) cm⁻¹: 3440, 2960, 1685, 1635, 1525.
MS (ESI, m/z): 464 (M+H)⁺.
HRMS (ESI, m/z): 464.2316 (Calcd for C₂₅H₃₀N₅O₄: 464.2298).
Anal. Calcd for C₂₅H₂₉N₅O₄·1/2H₂O: C, 63.54; H, 6.40; N, 14.82. Found: C, 63.76; H, 6.13; N, 14.80.

### Example 77

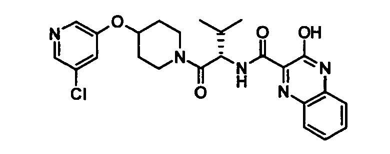

### N-[(1S)-1-({4-[(5-Chloropyridin-3-yl)oxy]piperidin-1-yl}carbonyl)-2-methylpropyl]-3-hydroxyquinoxaline-2-carboxamide

### Example 77-1

### t-Butyl [(1S)-1-({4-[(5-chloropyridin-3-yl)oxy]piperidin-1-yl}carbonyl)-2-methylpropyl]carbamate

Analogously to Example 1-2, t-butyl [(1S)-1-({4-[(5-chloropyridin-3-yl)oxy]piperidin-1-yl}carbonyl)-2-methylpropyl]carbamate (253 mg, yield 18%) was obtained from t-butyl [(1S)-1-(4-hydroxypiperidine-1-carbonyl)-2-methylpropyl]carbamate (1.00 g, 3.33 mmol) and 3-chloro-5-hydroxypyridine (474 mg, 3.66 mmol) as a colorless amorphous substance.
¹H-NMR (CDCl₃) δ: 8.21 (2H, s), 7.24-7.22 (1H, m), 5.34-5.32 (1H, m), 4.64-4.48 (2H, m), 3.87-3.53 (4H, m), 2.05-1.86 (5H, m), 1.44 (9H, s), 0.97 (3H, d, J=6.6 Hz), 0.90 (3H, d, J=6.6 Hz).
MS (ESI, m/z): 412 (M+H)⁺.

### Example 77-2

### (2S)-1-{4-[(5-Chloropyridin-3-yl)oxy]piperidin-1-yl}-3-methyl-1-oxobutan-2-amine dihydrochloride

Analogously to Example 29-3, (2S)-1-{4-[(5-chloropyridin-3-yl)oxy]piperidin-1-yl}-3-methyl-1-oxobutan-2-amine dihydrochloride (236 mg, yield 100%) was obtained from t-butyl [(1S)-1-({4-[(5-chloropyridin-3-yl)oxy]piperidin-1-yl}carbonyl)-2-methylpropyl]carbamate (253 mg, 0.614 mmol) as a colorless amorphous substance.
¹H-NMR (CD₃OD) δ: 8.56-8.51 (2H, m), 8.21-8.19 (1H, m), 4.97 (1H, s), 4.38 (1H, s), 4.10-3.45 (4H, m), 2.23-1.77 (5H, m), 1.12 (3H, d, J=6.6 Hz), 1.02 (3H, d, J=6.6 Hz).
MS (ESI, m/z): 312 (M+H)⁺.

### Example 77-3

### N-[(1S)-1-({4-[(5-Chloropyridin-3-yl)oxy]piperidin-1-yl}carbonyl)-2-methylpropyl]-3-hydroxyquinoxaline-2-carboxamide

Analogously to Example 29-4, the desired title compound (189 mg, yield 64%) was obtained from (2S)-1-{4-[(5-chloropyridin-3-yl)oxy]piperidin-1-yl}-3-methyl-1-oxobutan-2-amine dihydrochloride (236 mg, 0.613 mmol) as a yellow solid.
¹H-NMR (CDCl₃) δ: 12.35 (1H, br s), 10.18 (1H, br s), 8.25-8.22 (2H, m), 8.04-8.00 (1H, m), 7.64-7.22 (4H, m), 5.10-5.05 (1H, m), 4.68-4.62 (1H, m), 4.03-3.66 (4H, m), 2.35-1.88 (5H, m), 1.14-1.09 (6H, m).
IR (KBr) cm⁻¹: 2965, 1685, 1640, 1520, 1420.
MS (ESI, m/z): 484 (M+H)⁺.
HRMS (ESI, m/z): 506.1579 (Calcd for C₂₄H₂₆ClN₅NaO₄: 506.1571).
Anal. Calcd for C₂₄H₂₆ClN₅O₄: C, 59.56; H, 5.42; N, 14.47; Cl, 7.33. Found: C, 59.63; H, 5.32; N, 14.51; Cl, 7.64.

### Example 78

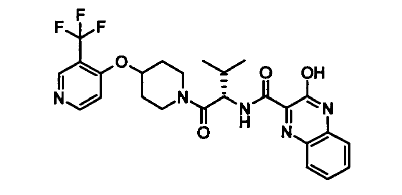

### 3-Hydroxy-N-{(1S)-2-methyl-1-[(4-{[3-(trifluoromethyl)pyridin-4-yl]oxy}piperidin-1-yl)carbonyl]propyl}quinoxaline-2-carboxamide

### Example 78-1

### t-Butyl 4-{[3-(trifluoromethyl)pyridin-4-yl]oxy}piperidine-1-carboxylate

t-Butyl 4-hydroxypiperidine-1-carboxylate (1.30 g, 5.78 mmol) was added to a N,N-dimethylformamide suspension (4.8 ml) of 60% sodium hydride (404 mg, 10.6 mmol), at 0°C, and stirring was carried out at room temperature for 30 minutes. Further, at 0°C, 4-chloro-3-(trifluoromethyl)pyridine hydrochloride (1.00 g, 4.82 mmol) was added, and stirring was carried out at room temperature for 5 hours. Water was added to the reaction solution, and a 1N hydrochloric acid was used to make it acidic, followed by washing with ethyl acetate. A 1N aqueous sodium hydroxide solution was used to make the aqueous layer basic, followed by extraction with ethyl acetate, and then the extract was washed with water and saturated saline. The resulting organic layer was dried over anhydrous sodium sulfate, and subsequently concentrated to afford t-butyl 4-{[3-(trifluoromethyl)pyridin-4-yl]oxy}piperidine-1-carboxylate (884 mg, yield 53%) as a colorless oil.
¹H-NMR (CDCl₃) δ: 8.70 (1H, s), 8.61 (1H, d, J=4.7 Hz), 6.90 (1H, d, J=4.7 Hz), 4.79-4.76 (1H, m), 3.65-3.60 (2H, m), 3.50-3.46 (2H, m), 1.92-1.87 (4H, m), 1.47 (9H, s).
MS (ESI, m/z): 347 (M+H)⁺.

### Example 78-2

### t-Butyl {(1S)-2-methyl-1-[(4-{[3-(trifluoromethyl)pyridin-4-yl]oxy}piperidin-1-yl)carbonyl]propyl}carbamate

Analogously to Example 29-2, t-butyl {(1S)-2-methyl-1-[(4-{[3-(trifluoromethyl)pyridin-4-yl]oxy}piperidin-1-yl)carbonyl]propyl}carbamate (610 mg, yield 54%) was obtained from t-butyl 4-{[3-(trifluoromethyl)pyridin-4-yl]oxy}piperidine-1-carboxylate (884 mg, 2.55 mmol) as a colorless amorphous substance.
¹H-NMR (CDCl₃, 400 MHz) δ: 8.72 (1H, s), 8.64 (1H, d, J=4.7 Hz), 6.90 (1H, d, J=4.7 Hz), 5.32 (1H, d, J=7.4 Hz), 4.89-4.84 (1H, m), 4.49 (1H, dd, J=7.2 Hz, 4.5 Hz), 4.22-3.98 (1H, m), 3.76-3.29 (3H, m), 2.02-1.91 (5H, m), 1.44 (9H, s), 0.99-0.88 (6H, m).
MS (ESI, m/z): 446 (M+H)⁺.

### Example 78-3

### (2S)-3-Methyl-1-oxo-1-(4-{[3-(trifluoromethyl)pyridin-4-yl]oxy}piperidin-1-yl)butan-2-amine dihydrochloride

Analogously to Example 29-3, (2S)-3-methyl-1-oxo-1-(4-{[3-(trifluoromethyl)pyridin-4-yl]oxy}piperidin-1-yl)butan-2-amine dihydrochloride (583 mg, yield 100%) was obtained from t-butyl {(1S)-2-methyl-1-[(4-{[3-(trifluoromethyl)pyridin-4-yl]oxy}piperidin-1-yl)carbonyl]propyl}carbamate (610 mg, 1.37 mmol) as a colorless amorphous substance.
¹H-NMR (CD₃OD) δ: 9.10 (1H, s), 8.95 (1H, d, J=7.0 Hz), 8.02 (1H, dd, J=10.2 Hz, 7.0 Hz), 5.46-5.37 (1H, m), 4.40 (1H, t, J=5.5 Hz), 4.07-3.52 (4H, m), 2.24-1.90 (5H, m), 1.14-1.02 (6H, m).
MS (ESI, m/z): 346 (M+H)⁺.

### Example 78-4

### 3-Hydroxy-N-{(1S)-2-methyl-1-[(4-{[3-(trifluoromethyl)pyridin-4-yl]oxy}piperidin-1-yl)carbonyl]propyl}quinoxaline-2-carboxamide

Analogously to Example 29-4, the desired title compound (152 mg, yield 61%) was obtained from (2S)-3-methyl-1-oxo-1-(4-{[3-(trifluoromethyl)pyridin-4-yl]oxy}piperidin-1-yl)butan-2-amine dihydrochloride (200 mg, 0.478 mmol) as a colorless solid.
¹H-NMR (CDCl₃) δ: 12.21 (1H, brs), 10.16 (1H, brs), 8.74-8.64 (2H, m), 8.06-8.00 (1H, m), 7.63-7.22 (3H, m), 6.92 (1H, d, J=5.9 Hz), 5.10-5.03 (1H, m), 4.93-4.88 (1H, m), 4.41-3.33 (4H, m), 2.34-2.01 (5H, m), 1.15-1.10 (6H, m).
IR (KBr) cm⁻¹: 3430, 2965, 1690, 1645, 1600.
MS (ESI, m/z): 518 (M+H)⁺.
HRMS (ESI, m/z): 540.1825 (Calcd for C₂₅H₂₆F₃N₅NaO₄: 540.1835).
Anal. Calcd for C₂₅H₂₆F₃N₅O₄: C, 58.02; H, 5.06; N, 13.53; F, 11.01. Found: C, 57.77; H, 4.93; N, 13.48; F, 11.19.

### Example 79

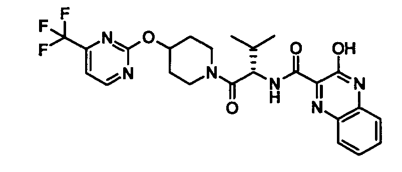

### 3-Hydroxy-N-{(1S)-2-methyl-1-[(4-{[4-(trifluoromethyl)pyrimidin-2-yl]oxy}piperidin-1-yl)carbonyl]propyl}quinoxaline-2-carboxamide

### Example 79-1

### t-Butyl {(1S)-2-methyl-1-[(4-{[4-(trifluoromethyl)pyrimidin-2-yl]oxy}piperidin-1-yl)carbonyl]propyl}carbamate

Analogously to Example 64-1, t-butyl {(1S)-2-methyl-1-[(4-{[4-(trifluoromethyl)pyrimidin-2-yl]oxy}piperidin-1-yl)carbonyl]propyl}carbamate (2.74 g, yield 92%) was obtained from 2-chloro-4-(trifluoromethyl)pyrimidine (0.883 ml, 7.32 mmol) and t-butyl {(1S)-1-[(4-hydroxypiperidin-1-yl)carbonyl]-2-methylpropyl}carbamate (2.00 g, 6.66 mmol) as a colorless oil.
¹H-NMR (CDCl₃, 500 MHz) δ: 8.80 (1H, d, J=4.8 Hz), 7.31 (1H, d, J=4.8 Hz), 5.41-5.33 (2H, m), 4.53 (1H, dd, J=8.8 Hz, 6.0 Hz), 4.04-3.49 (4H, m), 2.16-1.89 (5H, m), 1.47 (9H, s), 1.00 (3H, d, J=6.8 Hz), 0.92 (3H, d, J=6.8 Hz).
MS (FAB, m/z): 447 (M+H)⁺.

### Example 79-2

### 3-Hydroxy-N-{(1S)-2-methyl-1-[(4-{[4-(trifluoromethyl)pyrimidin-2-yl]oxy}piperidin-1-yl)carbonyl]propyl}quinoxaline-2-carboxamide

Analogously to Example 1-3, (2S)-3-methyl-1-oxo-1-(4-{[4-(trifluoromethyl)pyrimidin-2-yl]oxy}piperidin-1-yl)butan-2-amine hydrochloride (2.48 g) was obtained from t-butyl {(1S)-2-methyl-1-[(4-{[4-(trifluoromethyl)pyrimidin-2-yl]oxy}piperidin-1-yl)carbonyl]propyl}carbamate (2.74 g, 6.14 mmol) as a white solid. The resulting compound (200 mg, 0.522 mmol) was condensed with 3-hydroxyquinoxaline-2-carboxylic acid (136 mg, 0.784 mmol) to afford the desired title compound (240 mg, yield 92%) as a pale yellow solid. ¹H-NMR (DMSO-d₆, 400 MHz) δ: 12.86 (1H, brs), 9.56 (1H, brs), 9.03 (1H, t, J=4.0 Hz), 7.89 (1H, dd, J=6.4 Hz, 3.2 Hz), 7.67-7.66 (2H, m), 7.42-7.40 (2H, m), 5.33-5.32 (1H, m), 4.95 (1H, q, J=8.0 Hz), 4.05-3.90 (2H, m), 3.59-3.40 (2H, m), 2.06-1.74 (5H, m), 1.12-0.97 (6H, m). IR (KBr) cm⁻¹: 2965, 1690, 1645, 1425, 1155.
MS (ESI, m/z): 519 (M+H)⁺.
HRMS (ESI, m/z): 519.1956 (Calcd for C₂₄H₂₆F₃N₆O₄: 519.1967).

### Example 80

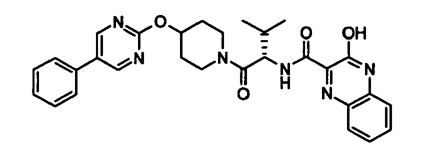

### 3-Hydroxy-N-[(1S)-2-methyl-1-({4-[(5-phenylpyrimidin-2-yl)oxy]piperidin-1-yl}carbonyl)propyl]quinoxaline-2-carboxamide

### Example 80-1

### t-Butyl 4-[(5-bromopyrimidin-2-yl)oxy]piperidine-1-carboxylate

Analogously to Example 64-1, t-butyl 4-[(5-bromopyrimidin-2-yl)oxy]piperidine-1-carboxylate (2.10 g, yield 44%) was obtained from 5-bromo-2-chloropyrimidine (2.84 g, 13.3 mmol) and t-butyl 4-hydroxypiperidine-1-carboxylate (2.68 g, 13.3 mmol) as a white solid. ¹H-NMR (CDCl₃, 400 MHz) δ: 8.48 (2H, s), 5.10-5.06 (1H, m), 3.76-3.75 (2H, m), 3.29-3.23 (2H, m), 1.94-1.94 (2H, brm), 1.77-1.74 (2H, m), 1.42 (9H, s).
MS (EI, m/z): 355 (M)⁺.

### Example 80-2

### t-Butyl 4-[(5-phenylpyrimidin-2-yl)oxy]piperidine-1-carboxylate

Potassium carbonate (1.73 g, 12.5 mmol) and tetrakis(triphenylphosphine)palladium (242 mg, 0.0209 mmol) were added sequentially to a 1,2-dimethoxyethane-water mixed solution (5.0 ml, 4:1, v/v) of t-butyl 4-[(5-bromopyrimidin-2-yl)oxy]piperidine-1-carboxylate (750 mg, 2.09 mmol) and phenylboronic acid (382 mg, 3.14 mmol), under nitrogen stream, and stirring was carried out overnight under heating to reflux. After completion of the reaction, the reaction solution was cooled to room temperature, and water was added, followed by extraction with ethyl acetate. The extract was washed sequentially with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., 40+M) to afford t-butyl 4-[(5-phenylpyrimidin-2-yl)oxy]piperidine-1-carboxylate (584 mg, yield 79%) as a white solid. ¹H-NMR (CDCl₃, 400 MHz) δ: 8.68 (2H, s), 7.45-7.40 (5H, m), 5.23-5.18 (1H, m), 3.80-3.79 (2H, m), 3.33-3.26 (2H, m), 2.01-1.99 (2H, m), 1.83-1.80 (2H, m), 1.41 (9H, s).
MS (FAB, m/z): 455 (M+H)⁺.

### Example 80-3

### t-Butyl [(1S)-2-methyl-1-({4-[(5-phenylpyrimidin-2-yl)oxy]piperidin-1-yl}carbonyl)propyl]carbamate

Analogously to Example 23-2, 5-phenyl-2-[(piperidin-4-yl)oxy]pyrimidine hydrochloride (0.476 mg) was obtained from t-butyl 4-[(5-phenylpyrimidin-2-yl)oxy]piperidine-1-carboxylate (580 mg, 1.63 mmol) as a colorless solid. The resulting compound (476 mg, 1.63 mmol) was condensed with N-(t-butoxycarbonyl)-L-valine (532 mg, 2.45 mmol) to afford t-butyl [(1S)-2-methyl-1-({4-[(5-phenylpyrimidin-2-yl)oxy]piperidin-1-yl}carbonyl)propyl]carbamate (740 mg, yield 100%) as a colorless amorphous substance.
¹H-NMR (CDCl₃, 400 MHz) δ: 8.68 (2H, s), 7.48-7.40 (5H, m), 5.32 (2H, s), 4.49 (1H, dd, J=7.2 Hz, 3.6 Hz), 3.76-3.62 (4H, m), 2.02-1.89 (5H, m), 1.41 (9H, s), 0.94 (3H, d, J=6.8 Hz), 0.86 (3H, d, J=6.8 Hz).
MS (FAB, m/z): 397 (M+H)⁺.

### Example 80-4

### 3-Hydroxy-N-[(1S)-2-methyl-1-({4-[(5-phenylpyrimidin-2-yl)oxy]piperidin-1-yl}carbonyl)propyl]quinoxaline-2-carboxamide

Analogously to Example 1-3, (2S)-3-methyl-1-oxo-1-{4-[(5-phenylpyrimidin-2-yl)oxy]piperidin-1-yl}butan-2-amine hydrochloride (0.636 mg) was obtained from t-butyl [(1S)-2-methyl-1-({4-[(5-phenylpyrimidin-2-yl)oxy]piperidin-1-yl}carbonyl)propyl]carbamate (740 mg, 1.63 mmol) as a white solid. The resulting compound (200 mg, 0.512 mmol) was condensed with 3-hydroxyquinoxaline-2-carboxylic acid (136 mg, 0.784 mmol) to afford the desired title compound (130 mg, yield 48%) as a pale yellow solid.
¹H-NMR (DMSO-d₆, 500 MHz) δ: 12.86 (1H, brs), 9.60 (1H, brs), 8.97 (2H, d, J=4.4 Hz), 7.90 (1H, d, J=7.6 Hz), 7.76 (2H, d, J=7.6 Hz), 7.66 (1H, t, J=7.6 Hz), 7.54 (2H, t, J=7.6 Hz), 7.45-7.40 (3H, m), 5.34-5.32 (1H, br m), 4.96 (1H, q, J=7.6 Hz), 4.11-3.99 (2H, m), 3.65-3.28 (2H, m), 2.06-1.75 (5H, m), 1.07 (3H, d, J=6.8 Hz), 0.99 (3H, d, J=6.8 Hz).
IR (KBr) cm⁻¹: 2960, 1685, 1635, 1525, 1440, 1370.
MS (ESI, m/z): 527 (M+H)⁺.
HRMS (ESI, m/z): 527.2422 (Calcd for C₂₉H₃₁N₆O₄: 527.2406).

### Example 81

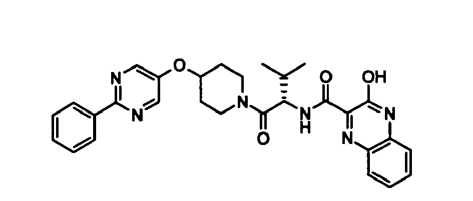

### 3-Hydroxy-N-[(1S)-2-methyl-1-({4-[(2-phenylpyrimidin-5-yl)oxy]piperidin-1-yl}carbonyl)propyl]quinoxaline-2-carboxamide

### Example 81-1

### t-Butyl [(1S)-1-({4-[(2-chloropyrimidin-5-yl)oxy]piperidin-1-yl}carbonyl)-2-methylpropyl]carbamate

Analogously to Example 64-1, t-butyl [(1S)-1-({4-[(2-chloropyrimidin-5-yl)oxy]piperidin-1-yl}carbonyl)-2-methylpropyl]carbamate (575 mg, yield 42%) was obtained from 2-chloro-5-fluoropyrimidine (452 mg, 3.66 mmol) and t-butyl 4-hydroxypiperidine-1-carboxylate (1.00 g, 3.32 mmol) as a white solid. ¹H-NMR (CDCl₃, 400 MHz) δ: 8.29 (2H, s), 5.33 (1H, d, J=9.2 Hz), 4.65-4.60 (1H, m), 4.45 (1H, dd, J=9.2 Hz, 5.6 Hz), 3.75-3.57 (4H, m), 1.96-1.88 (5H, m), 1.40 (9H, s), 0.94 (3H, d, J=6.8 Hz), 0.87 (3H, d, J=6.8 Hz).
MS (FAB, m/z): 413 (M+H)⁺.

### Example 81-2

### t-Butyl [(1S)-2-methyl-1-({4-[(2-phenylpyrimidin-5-yl)oxy]piperidin-1-yl}carbonyl)propyl]carbamate

Analogously to Example 80-2, t-butyl [(1S)-2-methyl-1-({4-[(2-phenylpiperidin-5-yl)oxy]piperidin-1-yl}carbonyl)propyl]carbamate (297 mg, yield 94%) was obtained from t-butyl [(1S)-1-({4-[(2-chloropyrimidin-5-yl)oxy]piperidin-1-yl}carbonyl)-2-methylpropyl]carbamate (280 mg, 8.92 mmol) and phenylboronic acid (163 mg, 1.34 mmol) as a white solid.
¹H-NMR (CDCl₃, 400 MHz) δ: 8.47 (2H, s), 8.34-8.32 (2H, m), 7.47-7.45 (3H, m), 5.32 (1H, d, J=9.2 Hz), 4.70-4.64 (1H, m), 4.48 (1H, dd, J=9.2 Hz, 5.6 Hz), 3.89-3.47 (4H, m), 2.09-1.87 (5H, m), 1.43 (9H, s), 0.97 (3H, d, J=6.8 Hz), 0.88 (3H, d, J=6.8 Hz).
MS (FAB, m/z): 455 (M+H)⁺.

### Example 81-3

### 3-Hydroxy-N-[(1S)-2-methyl-1-({4-[(2-phenylpyrimidin-5-yl)oxy]piperidin-1-yl}carbonyl)propyl]quinoxaline-2-carboxamide

Analogously to Example 1-3, (2S)-3-methyl-1-oxo-1-{4-[(2-phenylpyrimidin-5-yl)oxy]piperidin-1-yl}butan-2-amine hydrochloride (255 mg) was obtained from t-butyl [(1S)-2-methyl-1-({4-[(2-phenylpiperidin-5-yl)oxy]piperidin-1-yl}carbonyl)propyl]carbamate (297 mg, 0.653 mmol) as a white solid.
The resulting compound (127 mg, 0.325 mmol) was condensed with 3-hydroxyquinoxaline-2-carboxylic acid (93.0 mg, 0.487 mmol) under nitrogen stream to afford the desired title compound (80.0 mg, yield 47%) as a pale yellow solid.
¹H-NMR (DMSO-d₆, 400 MHz) δ: 12.86 (1H, brs), 9.66 (1H, brs), 8.75 (2H, d, J=5.6 Hz), 8.34 (2H, d, J=7.6 Hz), 7.88 (1H, t, J=6.8 Hz), 7.66 (1H, t, J=7.6 Hz), 7.53-7.52 (3H, m), 7.41-7.39 (2H, m), 4.96-4.93 (2H, m), 4.05-3.95 (2H, m), 3.63-3.27 (2H, m), 2.11-2.07 (3H, m), 1.79-1.63 (2H, m), 0.99-0.98 (6H, m).
IR (KBr) cm⁻¹: 2965, 1690, 1645, 1525, 1435, 1270.
MS (ESI, m/z): 527 (M+H)⁺.
HRMS (ESI, m/z): 527.2406 (Calcd for C₂₉H₃₁N₆O₄: 527.2406).

### Example 82

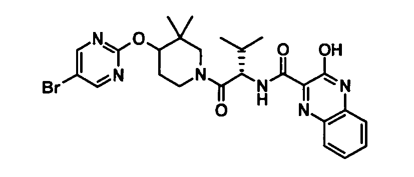

### N-[(1S)-1-({4-[(5-Bromopyrimidin-2-yl)oxy]-3,3-dimethylpiperidin-1-yl}carbonyl)-2-methylpropyl]-3-hydroxyquinoxaline-2-carboxamide

### Example 82-1

### t-Butyl 3,3-dimethyl-4-oxopiperidine-1-carboxylate

Methyl iodide (2.75 ml, 44.2 mmol) and 63% sodium hydride (2.29 g, 60.2 mmol) were added to a tetrahydrofuran solution (80 ml) of t-butyl 4-oxopiperidine-1-carboxylate (4.00 g, 20.1 mmol), at 0°C, under nitrogen stream,and stirring was carried out at 60°C for 5 hours. Water was added to the reaction solution under ice cooling, followed by extraction with ethyl acetate and sequential washing with water, a saturated aqueous sodium hydrogencarbonate solution, water and saline, and then the resulting organic layer was dried over anhydrous sodium sulfate. The organic layer was concentrated and the resulting residue was purified by a medium-pressure preparative liquid chromatograph (manufactured by Biotage, Inc., Si, 40+M) to afford t-butyl 3,3-dimethyl-4-oxopiperidine-1-carboxylate (2.17 g, yield 48%) as a white solid.
¹H-NMR (CDCl₃, 500 MHz) δ: 3.76-3.74 (2H, brm), 3.47-3.45 (2H, brm), 2.52 (2H, t, J=6.4 Hz), 1.53 (9H, s), 1.14 (6H, s).
MS (FAB, m/z): 228 (M+H)⁺.

### Example 82-2

### t-Butyl 4-[(5-bromopyrimidin-2-yl)oxy]-3,3-dimethylpiperidine-1-carboxylate

Sodium borohydride (433 mg, 11.5 mmol) was added to a methanol solution (50 ml) of t-butyl 3,3-dimethyl-4-oxopiperidine-1-carboxylate (2.17 g, 9.55 mmol), at 0°C, under nitrogen stream, and stirring was carried out at room temperature for 3 hours. Water was added to the reaction solution under ice cooling, followed by extraction with ethyl acetate and sequential washing with water, a saturated aqueous sodium hydrogencarbonate solution, water and saline, and then the resulting organic layer was dried over anhydrous sodium sulfate. The organic layer was concentrated to afford t-butyl 4-hydroxy-3,3-dimethylpiperidine-1-carboxylate (2.19 g) as a colorless oil.

Analogously to Example 64-1, t-butyl 4-[(5-bromopyrimidin-2-yl)oxy]-3,3-dimethylpiperidine-1-carboxylate (1.41 g, yield 67%) was obtained from the resulting compound (1.25 g, 5.45 mmol) and 5-bromo-2-chloropyrimidine (1.58 g, 8.18 mmol) as a white solid.
¹H-NMR (CDCl₃, 400 MHz) δ: 8.54 (2H, s), 4.90 (1H, dd, J=7.6 Hz, 4.0 Hz), 3.78 (1H, brs), 3.56 (1H, brs), 3.36-3.31 (1H, m), 3.05 (1H, d, J=13.6 Hz), 1.98 (1H, brs), 1.78 (1H, brs), 1.49 (9H, s), 1.07 (3H, s), 1.03 (3H, s).
MS (FAB, m/z): 411 (M+H)⁺.

### Example 82-3

### t-Butyl [(1S)-1-({4-[(5-bromopyrimidin-2-yl)oxy]-3,3-dimethylpiperidin-1-yl}carbonyl)-2-methylpropyl]carbamate

Analogously to Example 23-2, 5-bromo-2-[(3,3-dimethylpiperidin-4-yl)oxy]pyrimidine hydrochloride (0.251 mg) was obtained from t-butyl 4-[(5-bromopyrimidin-2-yl)oxy]-3,3-dimethylpiperidine-1-carboxylate (300 mg, 0.777 mmol) as a white solid. The resulting compound (251 mg, 0.777 mmol) was condensed with N-(t-butoxycarbonyl)-L-valine (253 mg, 1.16 mmol) to afford t-butyl [(1S)-1-({4-[(5-bromopyrimidin-2-yl)oxy]-3,3-dimethylpiperidin-1-yl}carbonyl)-2-methylpropyl]carbamate (322 mg, yield 85%) as a colorless amorphous substance.
¹H-NMR (CDCl₃, 400 MHz) δ: 8.49 (2H, s), 5.31-5.23 (1H, m), 4.90-4.85 (1H, m), 4.51-4.45 (1H, m), 4.09-4.05 (1H, m), 3.54-3.21 (3H, m), 2.09-1.74 (3H, m), 1.40 (9H, s), 1.05-0.85 (12H, m);
MS (FAB, m/z): 485 (M+H)⁺.

### Example 82-4

### N-[(1S)-1-({4-[(5-Bromopyrimidin-2-yl)oxy]-3,3-dimethylpiperidin-1-yl}carbonyl)-2-methylpropyl]-3-hydroxyquinoxaline-2-carboxamide

Analogously to Example 1-3, (2S)-1-{4-[(5-bromopyrimidin-2-yl)oxy]-3,3-dimethylpiperidin-1-yl}-3-methyl-1-oxobutan-2-amine hydrochloride (280 mg) was obtained from t-butyl [(1S)-1-({4-[(5-bromopyrimidin-2-yl)oxy]-3,3-dimethylpiperidin-1-yl}carbonyl)-2-methylpropyl]carbamate (322 mg, 0.663 mmol) as a white solid. The resulting compound (140 mg, 0.332 mmol) was condensed with 3-hydroxyquinoxaline-2-carboxylic acid (95.0 mg, 0.497 mmol) to afford the desired title compound (70.0 mg, yield 38%) as a pale yellow solid.
¹H-NMR (DMSO-d₆, 400 MHz) δ: 12.80 (1H, brs), 9.50 (1H, brs), 8.72 (2H, s), 7.83-7.82 (1H, m), 7.60 (1H, t, J=7.6 Hz), 7.35-7.34 (2H, m), 4.92-4.88 (2H, m), 3.73-3.01 (4H, m), 2.07-1.99 (3H, m), 0.95-0.90 (12H, m).
IR (KBr) cm⁻¹: 2965, 1690, 1640, 1540, 1425, 1325.
MS (ESI, m/z): 579 (M+Na)⁺.
HRMS (ESI, m/z): 579.1329 (Calcd for C₂₅H₂₉BrN₆NaO₄: 579.1331).

### Example 83

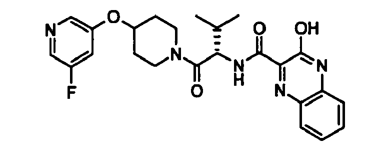

### N-[(1S)-1-({4-[(5-Fluoropyridin-3-yl)oxy]piperidin-1-yl}carbonyl)-2-methylpropyl]-3-hydroxyquinoxaline-2-carboxamide

### Example 83-1

### t-Butyl 4-[(5-fluoropyridin-3-yl)oxy]piperidine-1-carboxylate

t-Butyl 4-hydroxypiperidine-1-carboxylate (1.04 g, 5.00 mmol) was added to a N,N-dimethylformamide suspension (5.0 ml) of 60% sodium hydride (229 mg, 6.00 mmol), at 0°C, and stirring was carried out at room temperature for 30 minutes. Further, at 0°C, 3,5-difluoropyridine (645 mg, 5.50 mmol) was added, and stirring was carried out for 16.5 hours. Water was added to the reaction solution, followed by extraction with ethyl acetate. The extract was washed sequentially with water and saline, and then dried over anhydrous sodium sulfate. The organic layer was concentrated and the resulting residue was purified by silica gel column chromatography to afford t-butyl 4-[(5-fluoropyridin-3-yl)oxy]piperidine-1-carboxylate (1.48 g, yield 99%) as a colorless oil.
¹H-NMR (CDCl₃, 400 MHz) δ: 8.16-8.11 (2H, m), 6.96 (1H, dt, J=10.2, 2.3 Hz), 4.52-4.47 (1H, m), 3.73-3.67 (2H, m), 3.39-3.33 (2H, m), 1.99-1.73 (4H, m), 1.47 (9H, s).
MS (ESI, m/z): 297 (M+H)⁺.

### Example 83-2

### t-Butyl [(1S)-1-({4-[(5-fluoropyridin-3-yl)oxy]piperidin-1-yl}carbonyl)-2-methylpropyl]carbamate

Concentrated hydrochloric acid (5.0 ml) was added to an ethanol solution (10 ml) of t-butyl 4-[(5-fluoropyridin-3-yl)oxy]piperidine-1-carboxylate (1.48 g, 4.99 mmol), at 0°C, and stirring was carried out under room temperature for 16 hours. The reaction solution was concentrated to afford 3-fluoro-5-(piperidin-4-yloxy)pyridine hydrochloride. This product was used as it is in the next reaction without purification.

1-Ethyl-3-(3-dimethylaminopropyl)carbodiimide hydrochloride (1.44 g, 7.49 mmol) was added to a methylene chloride solution (50 ml) of the resulting compound, N-(t-butoxycarbonyl)-L-valine (1.09 g, 4.99 mmol), 1-hydroxybenzotriazole monohydrate (810 mg, 5.99 mmol) and N-methylmorpholine (2.75 ml, 25.0 mmol), at room temperature, and stirring was carried out at room temperature overnight. Water was added to the reaction solution, followed by extraction with methylene chloride, sequential washing with a saturated aqueous sodium hydrogencarbonate solution, water and saline, and drying over anhydrous sodium sulfate. The organic layer was concentrated and the resulting residue was purified by silica gel column chromatography to afford t-butyl [(1S)-1-({4-[(5-fluoropyridin-3-yl)oxy]piperidin-1-yl}carbonyl)-2-methylpropyl]carbamate (1.89 g, yield 96%) as a colorless amorphous substance.
¹H-NMR (CDCl₃, 400 MHz) δ: 8.16 (1H, s), 8.13 (1H, d, J=2.3 Hz), 6.96 (1H, dt, J=10.2, 2.3 Hz), 5.34-5.30 (1H, m), 4.64-4.55 (1H, m), 4.51-4.47 (1H, m), 3.87-3.48 (4H, m), 2.04-1.81 (5H, m), 1.44 (9H, s), 0.98 (3H, d, J=7.0 Hz), 0.90 (3H, d, J=6.6 Hz).
MS (ESI, m/z): 396 (M+H)⁺.

### Example 83-3

### (2S)-1-{4-[(5-Fluoropyridin-3-yl)oxy]piperidin-1-yl}-3-methyl-1-oxobutan-2-amine dihydrochloride

Concentrated hydrochloric acid (4.8 ml) was added to an ethanol solution (9.6 ml) of t-butyl [(1S)-1-({4-[(5-fluoropyridin-3-yl)oxy]piperidin-1-yl}carbonyl)-2-methylpropyl]carbamate (1.89 g, 4.78 mmol), at 0°C, and stirring was carried out at room temperature for 2 days. The reaction solution was concentrated to afford (2S)-1-{4-[(5-fluoropyridin-3-yl)oxy]piperidin-1-yl}-3-methyl-1-oxobutan-2-amine dihydrochloride (1.85 g, yield 100%) as a colorless amorphous substance.
¹H-NMR (CD₃OD, 400 MHz) δ: 8.65-8.63 (2H, m), 8.34 (1H, dt, J=10.3, 2.2 Hz), 5.06-4.94 (1H, m), 4.40 (1H, d, J=5.1 Hz), 4.13-3.45 (4H, m), 2.23-1.80 (5H, m), 1.13-1.02 (6H, m).
MS (ESI, m/z): 296 (M+H)⁺.

### Example 83-4

### N-[(1S)-1-({4-[(5-Fluoropyridin-3-yl)oxy]piperidin-1-yl}carbonyl)-2-methylpropyl]-3-hydroxyquinoxaline-2-carboxamide

1-Ethyl-3-(3-dimethylaminopropyl)carbodiimide hydrochloride (156 mg, 0.815 mmol) was added to a methylene chloride solution (5.4 ml) of (2S)-1-{4-[(5-fluoropyridin-3-yl)oxy]piperidin-1-yl}-3-methyl-1-oxobutan-2-amine dihydrochloride (200 mg, 0.543 mmol), 3-hydroxyquinoxaline-2-carboxylic acid (106 mg, 0.543 mmol), 1-hydroxybenzotriazole monohydrate (88.1 mg, 0.652 mmol) and N-methylmorpholine (0.299 ml, 2.72 mmol), at room temperature, and stirring was carried out at room temperature overnight. Water was added to the reaction solution, followed by extraction with methylene chloride, and the extract was washed sequentially with a saturated aqueous sodium hydrogencarbonate solution, water and saline, and dried over anhydrous sodium sulfate. After the organic layer was concentrated and the resulting residue was purified by silica gel column chromatography, the resulting residue was suspended in a mixed solvent of ethanol-diethyl ether, and the solid substance was collected by filtration to afford the desired title compound (160 mg, yield 63%) as a yellow solid.
¹H-NMR (CDCl₃, 400 MHz) δ: 12.56 (1H, brs), 10.12 (1H, brs), 8.20-7.99 (3H, m), 7.66-7.31 (3H, m), 7.02-6.96 (1H, m), 5.10-5.05 (1H, m), 4.70-4.61 (1H, m), 4.03-3.67 (4H, m), 2.39-1.89 (5H, m), 1.15-1.10 (6H, m).
IR (KBr) cm⁻¹: 2960, 1690, 1640, 1530, 1430.
MS (ESI, m/z): 468 (M+H)⁺.
HRMS (ESI, m/z): 490.1857 (Calcd for C₂₄H₂₆FN₅NaO₄: 490.1867).
Anal. Calcd for C₂₄H₂₆FN₅O₄: C, 61.66; H, 5.61; N, 14.98; F, 4.06. Found: C, 61.44; H, 5.71; N, 14.87; F, 4.21.

### Example 84

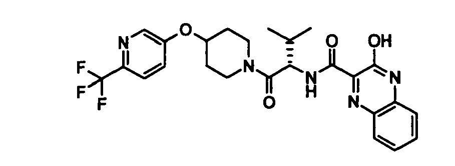

### 3-Hydroxy-N-{(1S)-2-methyl-1-[(4-{[6-(trifluoromethyl)pyridin-3-yl]oxy}piperidin-1-yl)carbonyl]propyl}quinoxaline-2-carboxamide

### Example 84-1

### t-Butyl {(1S)-2-methyl-1-[(4-{[6-(trifluoromethyl)pyridin-3-yl]oxy}piperidin-1-yl)carbonyl]propyl}carbamate

A 40% toluene solution of diisopropyl azodicarboxylate (2.10 ml, 3.99 mmol) was added to a toluene solution (15 ml) of t-butyl {(1S)-1-[(4-hydroxypiperidin-1-yl)carbonyl]-2-methylpropyl}carbamate (1.00 g, 3.33 mmol) and 2-(trifluoromethyl)pyridin-5-ol (597 mg, 3.33 mmol) and triphenylphosphine (1.05 g, 3.99 mmol), at room temperature, and stirring was carried out at the same temperature for 2 days. The reaction solution was concentrated and the resulting residue was diluted with ethyl acetate, followed by sequential washing with a 1N aqueous sodium hydroxide solution, water and saline, and the resulting organic layer was dried over anhydrous sodium sulfate. The organic layer was concentrated and the resulting residue was purified by silica gel column chromatography to afford t-butyl {(1S)-2-methyl-1-[(4-{[6-(trifluoromethyl)pyridin-3-yl]oxy}piperidin-1-yl)carbonyl]propyl}carbamate (1.11 g, yield 75%) as a colorless amorphous substance.
¹H-NMR (CDCl₃, 400 MHz) δ: 8.39 (1H, s), 7.63 (1H, d, J=8.6 Hz), 7.30 (1H, dd, J=8.6 Hz, 2.7 Hz), 5.31 (1H, d, J=8.2 Hz), 4.74-4.66 (1H, m), 4.51-4.48 (1H, m), 3.86-3.54 (4H, m), 2.06-1.84 (5H, m), 1.44 (9H, s), 0.98 (3H, d, J=6.6 Hz), 0.90 (3H, d, J=6.6 Hz).
MS (ESI, m/z): 346 (M+H)⁺.

### Example 84-2

### (2S)-3-Methyl-1-oxo-1-(4-{[6-(trifluoromethyl)pyridin-3-yl]oxy}piperidin-1-yl)butan-2-amine dihydrochloride

Concentrated hydrochloric acid (2.5 ml) was added to an ethanol solution (5.0 ml) of t-butyl {(1S)-2-methyl-1-[(4-{[6-(trifluoromethyl)pyridin-3-yl]oxy}piperidin-1-yl)carbonyl]propyl}carbamate (1.11 g, 2.49 mmol), at 0°C, and stirring was carried out at room temperature overnight. The reaction solution was concentrated to afford (2S)-3-methyl-1-oxo-1-(4-{[6-(trifluoromethyl)pyridin-3-yl]oxy}piperidin-1-yl)butan-2-amine dihydrochloride (970 mg, yield 100%) as a colorless amorphous substance.
¹H-NMR (CD₃OD, 400 MHz) δ: 8.39 (1H, dd, J=9.0 Hz, 2.7 Hz), 7.76 (1H, d, J=9.0 Hz), 7.61 (1H, d, J=8.6 Hz), 4.35 (1H, d, J=4.7 Hz), 4.09-3.46 (5H, m), 2.21-1.77 (5H, m), 1.11 (3H, d, J=7.0 Hz), 1.02 (3H, dd, J=6.8 Hz, 1.4 Hz).
MS (ESI, m/z): 346 (M+H)⁺.

### Example 84-3

### 3-Hydroxy-N-{(1S)-2-methyl-1-[(4-{[6-(trifluoromethyl)pyridin-3-yl]oxy}piperidin-1-yl)carbonyl]propyl}quinoxaline-2-carboxamide

1-Ethyl-3-(3-dimethylaminopropyl)carbodiimide hydrochloride (137 mg, 0.717 mmol) was added to a methylene chloride solution (4.8 ml) of (2S)-3-methyl-1-oxo-1-(4-{[6-(trifluoromethyl)pyridin-3-yl]oxy}piperidin-1-yl)butan-2-amine dihydrochloride (200 mg, 0.478 mmol), 3-hydroxyquinoxaline-2-carboxylic acid (93.7 mg, 0.478 mmol), 1-hydroxybenzotriazole monohydrate (77.5 mg, 0. 574 mmol) and N-methylmorpholine (0.263 ml, 2.39 mmol), at room temperature, and stirring was carried out at room temperature overnight. Water was added to the reaction solution, followed by extraction with methylene chloride, and the extract was washed sequentially with a saturated aqueous sodium hydrogencarbonate solution, water and saline, and dried over anhydrous sodium sulfate. After the organic layer was concentrated and the resulting residue was purified by silica gel column chromatography, the resulting residue was suspended in a mixed solvent of ethanol-diethyl ether, and then the solid substance was collected by filtration to afford the desired title compound (145 mg, yield 59%) as a yellow solid.
¹H-NMR (CDCl₃, 400 MHz) δ: 12.08 (1H, brs), 10.15 (1H, brs), 8.41 (1H, dd, J=10.6 Hz, 2.7 Hz), 8.02 (1H, brs), 7.66-7.30 (5H, m), 5.09-5.04 (1H, m), 4.78-4.70 (1H, m), 4.10-3.64 (4H, m), 2.35-1.95 (5H, m), 1.14-1.09 (6H, m).
IR (KBr) cm⁻¹: 2965, 1685, 1640, 1530, 1340.
MS (ESI, m/z): 518 (M+H)⁺.
HRMS (ESI, m/z): 518.2016 (Calcd for C₂₅H₂₇F₃N₅O₄: 518.2015).
Anal. Calcd for C₂₅H₂₆F₃N₅O₄·0.5H₂O: C, 57.03; H, 5.17; N, 13.30; F, 10.83. Found: C, 56.82; H, 4.98; N, 13.03; F, 10.89.

### Example 85

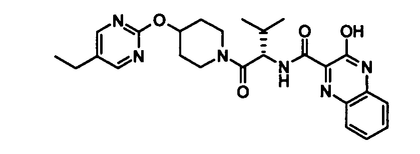

### N-[(1S)-1-({4-[(5-Ethylpyrimidin-2-yl)oxy]piperidin-1-yl}carbonyl)-2-methylpropyl]-3-hydroxyquinoxaline-2-carboxamide

### Example 85-1

### t-Butyl 4-[(5-ethylpyrimidin-2-yl)oxy]piperidine-1-carboxylate

t-Butyl 4-hydroxypiperidine-1-carboxylate (1.04 g, 5.00 mmol) was added to a N,N-dimethylformamide suspension (5.0 ml) of 60% sodium hydride (229 mg, 6.00 mmol), at 0°C, and stirring was carried out at room temperature for 30 minutes. 2-chloro-5-ethylpyrimidine (0.689 ml, 5.50 mmol) was added to the reaction solution, at 0°C, and stirring was carried out for a further 1 hour. Water was added to the reaction solution, followed by extraction with ethyl acetate, and the extract was washed sequentially with water and saline, and dried over anhydrous sodium sulfate. The organic layer was concentrated and the resulting residue was purified by silica gel column chromatography to afford t-butyl 4-[(5-ethylpyrimidin-2-yl)oxy]piperidine-1-carboxylate (1.03 g, yield 67%) as a white solid.
¹H-NMR (CDCl₃, 400 MHz) δ: 8.33 (2H, s), 5.18-5.12 (1H, m), 3.83-3.77 (2H, m), 3.34-3.27 (2H, m), 2.58 (2H, q, J=7.6 Hz), 2.02-1.97 (2H, m), 1.84-1.76 (2H, m), 1.47 (9H, s), 1.25 (3H, t, J=7.6 Hz).
MS (ESI, m/z): 308 (M+H)⁺, 252 (M+H-tBu)⁺, 208 (M+H-BOC)⁺.

### Example 85-2

### t-Butyl [(1S)-1-({4-[(5-ethylpyrimidin-2-yl)oxy]piperidin-1-yl}carbonyl)-2-methylpropyl]carbamate

Concentrated hydrochloric acid (3.4 ml) was added to an ethanol solution (6.8 ml) of t-butyl 4-[(5-ethylpyrimidin-2-yl)oxy]piperidine-1-carboxylate (1.03 g, 3.35 mmol), at 0°C, and stirring was carried out at room temperature overnight. The reaction solution was concentrated to afford 5-ethyl-2-(piperidin-4-yloxy)pyrimidine dihydrochloride. This product was used as it is in the next reaction without purification.
1-Ethyl-3-(3-dimethylaminopropyl)carbodiimide hydrochloride (964 mg, 5.03 mmol) was added to a methylene chloride solution (34 ml) of the resulting compound, N-(t-butoxycarbonyl)-L-valine (728 mg, 3.35 mmol), 1-hydroxybenzotriazole monohydrate (543 mg, 4.02 mmol) and N-methylmorpholine (1.11 ml, 10.1 mmol), at room temperature, and stirring was carried out at room temperature for 6 days. Water was added to the reaction solution, followed by extraction with ethyl acetate, and the extract was washed sequentially with a saturated aqueous sodium hydrogencarbonate solution, water and saline, and dried over anhydrous sodium sulfate. The organic layer was concentrated and the resulting residue was purified by silica gel column chromatography to afford t-butyl [(1S)-1-({4-[(5-ethylpyrimidin-2-yl)oxy]piperidin-1-yl}carbonyl)-2-methylpropyl]carbamate (1.20 g, yield 88%) as a colorless amorphous substance.
¹H-NMR (CDCl₃, 400 MHz) δ: 8.34 (2H, s), 5.38-5.35 (1H, m), 5.29-5.22 (1H, m), 4.53-4.49 (1H, m), 4.00-3.44 (4H, m), 2.59 (2H, q, J=7.6 Hz), 2.10-1.84 (5H, m), 1.44 (9H, s), 1.25 (3H, t, J=7.6 Hz), 0.97 (3H, dd, J=6.8 Hz, 2.2 Hz), 0.90-0.88 (3H, m).
MS (ESI, m/z): 407 (M+H)⁺.

### Example 85-3

### (2S)-1-{4-[(5-Ethylpyrimidin-2-yl)oxy]piperidin-1-yl}-3-methyl-1-oxobutan-2-amine dihydrochloride

Concentrated hydrochloric acid (3.0 ml) was added to an ethanol solution (6.0 ml) of t-butyl [(1S)-1-({4-[(5-ethylpyrimidin-2-yl)oxy]piperidin-1-yl}carbonyl)-2-methylpropyl]carbamate (1.20 g, 2.95 mmol), at 0°C, and stirring was carried out at room temperature overnight. The reaction solution was concentrated to afford (2S)-1-{4-[(5-ethylpyrimidin-2-yl)oxy]piperidin-1-yl}-3-methyl-1-oxobutan-2-amine dihydrochloride (1.20 g, yield 100%) as a colorless amorphous substance.
¹H-NMR (CD₃OD, 400 MHz) δ: 8.75-8.69 (2H, m), 5.54-5.47 (1H, m), 4.38 (1H, d, J=4.7 Hz), 4.07-3.48 (4H, m), 2.75-2.69 (2H, m), 2.21-1.86 (5H, m), 1.29 (3H, t, J=7.4 Hz), 1.12 (3H, d, J=7.0 Hz), 1.03 (3H, d, J=6.6 Hz).
MS (ESI, m/z): 307 (M+H)⁺.

### Example 85-4

### N-[(1S)-1-({4-[(5-Ethylpyrimidin-2-yl)oxy]piperidin-1-yl}carbonyl)-2-methylpropyl]-3-hydroxyquinoxaline-2-carboxamide

1-Ethyl-3-(3-dimethylaminopropyl)carbodiimide hydrochloride (152 mg, 0.633 mmol) was added to a methylene chloride solution (5.3 ml) of (2S)-1-{4-[(5-ethylpyrimidin-2-yl)oxy]piperidin-1-yl}-3-methyl-1-oxobutan-2-amine dihydrochloride (200 mg, 0.527 mmol), 3-hydroxyquinoxaline-2-carboxylic acid (103 mg, 0.527 mmol), 1-hydroxybenzotriazole monohydrate (85.5 mg, 0.633 mmol) and N-methylmorpholine (0.174 ml, 2.11 mmol), at room temperature, and stirring was carried out at room temperature overnight. Water was added to the reaction solution, followed by extraction with methylene chloride, and the extract was washed sequentially with a saturated aqueous sodium hydrogencarbonate solution, water and saline, and dried over anhydrous sodium sulfate. After the organic layer was concentrated and the resulting residue was purified by silica gel column chromatography, the resulting residue was suspended in a mixed solvent of ethanol-diethyl ether, and subsequently the solid substance was collected by filtration to afford the desired title compound (206 mg, yield 82%) as a yellow solid.
¹H-NMR (CDCl₃, 400 MHz) δ: 12.82-12.64 (1H, m), 10.19 (1H, brs), 8.36 (2H, d, J=6.3 Hz), 8.02 (1H, t, J=9.2 Hz), 7.62-7.27 (3H, m), 5.32-5.07 (2H, m), 4.02-3.68 (4H, m), 2.63-2.56 (2H, m), 2.34-1.96 (5H, m), 1.29-1.24 (3H, m), 1.14-1.09 (6H, m).
IR (KBr) cm⁻¹: 2965, 1690, 1630, 1525, 1435.
MS (ESI, m/z): 479 (M+H)⁺.
HRMS (ESI, m/z): 501.2226 (Calcd for C₂₅H₃₀N₆ONaO₄: 501.2219).
Anal. Calcd for C₂₅H₃₀N₆O₄: C, 62.75; H, 6.32; N, 17.56. Found: C, 62.78; H, 6.30; N, 17.45.

### Example 86

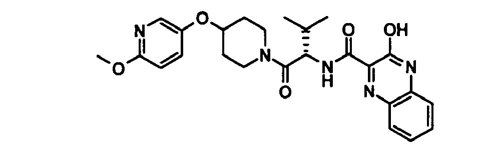

### 3-Hydroxy-N-[(1S)-1-({4-[(6-methoxypyridin-3-yl)oxy]piperidin-1-yl}carbonyl)-2-methylpropyl]quinoxaline-2-carboxamide

### Example 86-1

### t-Butyl [(1S)-1-({4-[(6-methoxypyridin-3-yl)oxy]piperidin-1-yl}carbonyl)-2-methylpropyl]carbamate

A 40% toluene solution of diisopropyl azodicarboxylate (0.690 ml, 1.31 mmol) was added to a toluene solution (5.0 ml) of t-butyl {(1S)-1-[(4-hydroxypiperidin-1-yl)carbonyl]-2-methylpropyl}carbamate (329 mg, 1.09 mmol), 6-methoxypyridin-3-ol (137 mg, 1.09 mmol) and triphenylphosphine (345 mg, 1.31 mmol), at room temperature, and stirring was carried out at room temperature for 6 days. The reaction solution was concentrated and the resulting residue was diluted with ethyl acetate, followed by sequential washing with a 1N aqueous sodium hydroxide solution, water and saline, and drying over anhydrous sodium sulfate. The organic layer was concentrated and the resulting residue was purified by silica gel column chromatography to afford t-butyl [(1S)-1-({4-[(6-methoxypyridin-3-yl)oxy]piperidin-1-yl}carbonyl)-2-methylpropyl]carbamate (273 mg, yield 61%) as a colorless amorphous substance.
¹H-NMR (CDCl₃, 400 MHz) δ: 7.84 (1H, d, J=2.7 Hz), 7.23 (1H, dd, J=9.0, 2.7 Hz), 6.70 (1H, d, J=9.0 Hz), 5.34 (1H, d, J=9.0 Hz), 4.51-4.38 (2H, m), 3.90 (3H, s), 3.88-3.42 (4H, m), 2.02-1.78 (5H, m), 1.44 (9H, s), 0.98-0.88 (6H, m).
MS (ESI, m/z): 408 (M+H)⁺.

### Example 86-2

### (2S)-1-{4-[(6-Methoxypyridin-3-yl)oxy]piperidin-1-yl}-3-methyl-1-oxobutan-2-amine dihydrochloride

Concentrated hydrochloric acid (1.0 ml) was added to an ethanol solution (2.0 ml) of t-butyl [(1S)-1-({4-[(6-methoxypyridin-3-yl)oxy]piperidin-1-yl}carbonyl)-2-methylpropyl]carbamate (273 mg, 0.669 mmol), at 0°C, and stirring was carried out at room temperature overnight. The reaction solution was concentrated to afford (2S)-1-{4-[(6-methoxypyridin-3-yl)oxy]piperidin-1-yl}-3-methyl-1-oxobutan-2-amine dihydrochloride (270 mg, yield 100%) as a colorless amorphous substance.
¹H-NMR (CD₃OD, 400 MHz) δ: 8.19-8.13 (2H, m), 7.51-7.48 (1H, m), 4.82-4.73 (1H, m), 4.37 (1H, d, J=5.1 Hz), 4.16 (3H, s), 4.09-3.43 (4H, m), 2.21-1.75 (5H, m), 1.11 (3H, d, J=7.0 Hz), 1.02 (3H, d, J=6.6 Hz).
MS (ESI, m/z): 308 (M+H)⁺.

### Example 86-3

### 3-Hydroxy-N-[(1S)-1-({4-[(6-methoxypyridin-3-yl)oxy]piperidin-1-yl}carbonyl)-2-methylpropyl]quinoxaline-2-carboxamide

1-Ethyl-3-(3-dimethylaminopropyl)carbodiimide hydrochloride (193 mg, 1.00 mmol) was added to a methylene chloride solution (6.7 ml) of (2S)-1-{4-[(6-methoxypyridin-3-yl)oxy]piperidin-1-yl}-3-methyl-1-oxobutan-2-amine dihydrochloride (255 mg, 0.669 mmol), 3-hydroxyquinoxaline-2-carboxylic acid (131 mg, 0.669 mmol), 1-hydroxybenzotriazole monohydrate (109 mg, 0.803 mmol) and N-methylmorpholine (0.221 ml, 2.68 mmol), at room temperature, and stirring was carried out at room temperature for 3 days. Water was added to the reaction solution, followed by extraction with methylene chloride, and the extract was washed sequentially with a saturated aqueous sodium hydrogencarbonate solution, water and saline, and dried over anhydrous sodium sulfate. After the organic layer was concentrated and the resulting residue was purified by silica gel column chromatography, the resulting residue was suspended in a mixed solvent of ethanol-diethyl ether, and subsequently the solid substance was collected by filtration to afford the desired title compound (253 mg, yield 79%) as a yellow solid.
¹H-NMR (CDCl₃, 400 MHz) δ: 12.54 (1H, brs), 10.16 (1H, brs), 8.02-7.98 (1H, m), 7.86 (1H, dd, J=7.4 Hz, 2.7 Hz), 7.61-7.22 (4H, m), 6.71 (1H, dd, J=8.8 Hz, 5.3 Hz), 5.10-5.05 (1H, m), 4.49-4.44 (1H, m), 3.91-3.74 (7H, m), 2.36-1.90 (5H, m), 1.26-1.09 (6H, m).
IR (KBr) cm⁻¹: 3430, 2945, 1685, 1640, 1490.
MS (ESI, m/z): 480 (M+H)⁺.
HRMS (ESI, m/z): 502.2078 (Calcd for C₂₅H₂₉N₅NaO₅: 502.2066).
Anal. Calcd for C₂₅H₂₉N₅O₅: C, 62.62; H, 6.10; N, 14.60. Found: C, 62.42; H, 6.07; N, 14.58.

### Example 87

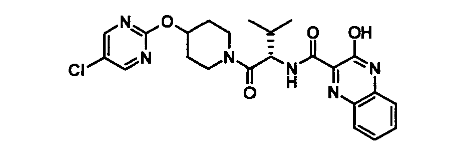

### N-[(1S)-1-({4-[(5-Chloropyrimidin-2-yl)oxy]piperidin-1-yl}carbonyl)-2-methylpropyl]-3-hydroxyquinoxaline-2-carboxamide

### Example 87-1

### t-Butyl [(1S)-1-({4-[(5-chloropyrimidin-2-yl)oxy]piperidin-1-yl}carbonyl)-2-methylpropyl]carbamate

A 40% toluene solution of diisopropyl azodicarboxylate (2.10 ml, 3.99 mmol) was added to a toluene solution (15 ml) of t-butyl {(1S)-1-[(4-hydroxypiperidin-1-yl)carbonyl]-2-methylpropyl}carbamate (1.00 g, 3.33 mmol), 5-chloro-2-hydroxypyrimidine hydrochloride (612 mg, 3.66 mmol) and triphenylphosphine (1.05 g, 3.99 mmol), at room temperature, and stirring was carried out at room temperature for 2 days. The reaction solution was concentrated and the resulting residue was diluted with ethyl acetate, and then the organic layer was washed sequentially with a 1N aqueous sodium hydroxide solution, water and saline, and dried over anhydrous sodium sulfate. The organic layer was concentrated and the resulting residue was purified by silica gel column chromatography to afford t-butyl [(1S)-1-({4-[(5-chloropyrimidin-2-yl)oxy]piperidin-1-yl}carbonyl)-2-methylpropyl]carbamate (517 mg, yield 38%) as a colorless amorphous substance.
¹H-NMR (CDCl₃, 400 MHz) δ: 8.45 (2H, s), 5.36-5.18 (2H, m), 4.52-4.48 (1H, m), 3.98-3.50 (4H, m), 2.10-1.83 (5H, m), 1.44 (9H, s), 0.97 (3H, d, J=6.3 Hz), 0.89 (3H, dd, J=6.6 Hz, 2.7 Hz).
MS (ESI, m/z): 413 (M+H)⁺.

### Example 87-2

### (2S)-1-{4-[(5-Chloropyrimidin-2-yl)oxy]piperidin-1-yl}-3-methyl-1-oxobutan-2-amine dihydrochloride

Concentrated hydrochloric acid (1.3 ml) was added to an ethanol solution (2.6 ml) of t-butyl [(1S)-1-({4-[(5-chloropyrimidin-2-yl)oxy]piperidin-1-yl}carbonyl)-2-methylpropyl]carbamate (517 mg, 1.25 mmol), at 0°C, and stirring was carried out at room temperature overnight. The reaction solution was concentrated to afford (2S)-1-{4-[(5-chloropyrimidin-2-yl)oxy]piperidin-1-yl}-3-methyl-1-oxobutan-2-amine dihydrochloride (456 mg, yield 94%) as a colorless amorphous substance.
¹H-NMR (CD₃OD, 400 MHz) δ: 8.59 (2H, d, J=1.6 Hz), 5.36-5.28 (1H, m), 4.35 (1H, d, J=5.1 Hz), 4.07-3.46 (4H, m), 2.23-1.77 (5H, m), 1.11 (3H, d, J=7.0 Hz), 1.02 (3H, d, J=7.0 Hz).
MS (ESI, m/z): 313 (M+H)⁺.

### Example 87-3

### N-[(1S)-1-({4-[(5-Chloropyrimidin-2-yl)oxy]piperidin-1-yl}carbonyl)-2-methylpropyl]-3-hydroxyquinoxaline-2-carboxamide

1-Ethyl-3-(3-dimethylaminopropyl)carbodiimide hydrochloride (149 mg, 0.778 mmol) was added to a methylene chloride solution (5.2 ml) of (2S)-1-{4-[(5-chloropyrimidin-2-yl)oxy]piperidin-1-yl}-3-methyl-1-oxobutan-2-amine dihydrochloride (200 mg, 0.519 mmol), 3-hydroxyquinoxaline-2-carboxylic acid (102 mg, 0.519 mmol), 1-hydroxybenzotriazole monohydrate (84.1 mg, 0.622 mmol) and N-methylmorpholine (0.285 ml, 2.59 mmol), at room temperature, and stirring was carried out at room temperature overnight. Water was added to the reaction solution, followed by extraction with methylene chloride, and the extract was washed sequentially with a saturated aqueous sodium hydrogencarbonate solution, water and saline, and dried over anhydrous sodium sulfate. After the organic layer was concentrated and the resulting residue was purified by silica gel column chromatography, the resulting residue was suspended in a mixed solvent of ethanol-diethyl ether, and then the solid substance was collected by filtration to afford the desired title compound (198 mg, yield 79%) as a yellow solid.
¹H-NMR (CDCl₃, 400 MHz) δ: 12.52 (1H, brs), 10.17 (1H, brs), 8.47-8.46 (2H, m), 8.05-8.00 (1H, m), 7.63-7.26 (3H, m), 5.30-5.06 (2H, m), 4.08-3.63 (4H, m), 2.35-1.91 (5H, m), 1.14-1.09 (6H, m).
IR (KBr) cm⁻¹: 2965, 1690, 1630, 1425.
MS (ESI, m/z): 485 (M+H)⁺.
HRMS (ESI, m/z): 507.1532 (Calcd for C₂₃H₂₅ClN₆NaO₄: 507.1524).
Anal. Calcd for C₂₃H₂₅ClN₆O₄·0.1H₂O: C, 56.76; H, 5.22; N, 17.27; F, 7.28. Found: C, 56.56; H, 5.07; N, 17.20; F, 7.65.

### Example 88

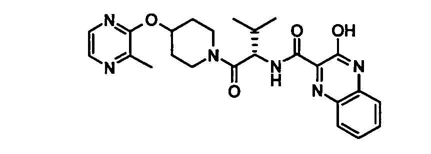

### 3-Hydroxy-N-[(1S)-2-methyl-1-({4-[(3-methylpyrazin-2-yl)oxy]piperidin-1-yl}carbonyl)propyl]quinoxaline-2-carboxamide

### Example 88-1

### t-Butyl [(1S)-2-methyl-1-({4-[(3-methylpyrazin-2-yl)oxy]piperidin-1-yl}carbonyl)propyl]carbamate

A 40% toluene solution of diisopropyl azodicarboxylate (2.10 ml, 3.99 mmol) was added to a toluene solution (15 ml) of t-butyl {(1S)-1-[(4-hydroxypiperidin-1-yl)carbonyl]-2-methylpropyl}carbamate (1.00 g, 3.33 mmol), 3-methylpyrazin-2-ol (403 mg, 3.66 mmol) and triphenylphosphine (1.05 g, 3.99 mmol), at room temperature, and stirring was carried out at the same temperature overnight. After the reaction solution was concentrated and the resulting residue was diluted with ethyl acetate, the organic layer was washed sequentially with a 1N aqueous sodium hydroxide solution, water and saline, and dried over anhydrous sodium sulfate. The organic layer was concentrated and the resulting residue was purified by silica gel column chromatography to afford t-butyl [(1S)-2-methyl-1-({4-[(3-methylpyrazin-2-yl)oxy]piperidin-1-yl}carbonyl)propyl]carbamate (933 mg, yield 71%) as a colorless amorphous substance.
¹H-NMR (CDCl₃, 400 MHz) δ: 8.00 (1H, d, J=2.7 Hz), 7.90-7.88 (1H, m), 5.37-5.30 (2H, m), 4.54-4.50 (1H, m), 3.93-3.48 (4H, m), 2.47 (3H, d, J=6.3 Hz), 2.11-1.79 (5H, m), 1.44 (9H, s), 0.99 (3H, d, J=6.6 Hz), 0.90 (3H, d, J=6.6 Hz).
MS (ESI, m/z): 293 (M+H)⁺.

### Example 88-2

### (2S)-3-Methyl-1-{4-[(3-methylpyrazin-2-yl)oxy]piperidin-1-yl}-1-oxobutan-2-amine dihydrochloride

Concentrated hydrochloric acid (2.4 ml) was added to an ethanol solution (4.8 ml) of t-butyl [(1S)-2-methyl-1-({4-[(3-methylpyrazin-2-yl)oxy]piperidin-1-yl}carbonyl)propyl]carbamate (933 mg, 2.38 mmol), at 0°C, and stirring was carried out at room temperature overnight. The reaction solution was concentrated to afford (2S)-3-methyl-1-{4-[(3-methylpyrazin-2-yl)oxy]piperidin-1-yl}-1-oxobutan-2-amine dihydrochloride (920 mg, yield 100%) as a colorless amorphous substance.
¹H-NMR (CD₃OD, 400 MHz) δ: 8.49-8.47 (1H, m), 8.18-8.16 (1H, m), 5.58-5.49 (1H, m), 4.39 (1H, d, J=4.7 Hz), 4.08-3.53 (4H, m), 2.66 and 2.63 (3H, s), 2.26-1.84 (5H, m), 1.12 (3H, d, J=7.0 Hz), 1.03 (3H, dd, J=7.0, 1.2 Hz).
MS (ESI, m/z): 293 (M+H)⁺.

### Example 88-3

### 3-Hydroxy-N-[(1S)-2-methyl-1-({4-[(3-methylpyrazin-2-yl)oxy]piperidin-1-yl}carbonyl)propyl]quinoxaline-2-carboxamide

1-Ethyl-3-(3-dimethylaminopropyl)carbodiimide hydrochloride (157 mg, 0.821 mmol) was added to a methylene chloride solution (5.5 ml) of (2S)-3-methyl-1-{4-[(3-methylpyrazin-2-yl)oxy]piperidin-1-yl}-1-oxobutan-2-amine dihydrochloride (200 mg, 0.547 mmol), 3-hydroxyquinoxaline-2-carboxylic acid (107 mg, 0.547 mmol), 1-hydroxybenzotriazole monohydrate (88.8 mg, 0.657 mmol) and N-methylmorpholine (0.301 ml, 2.74 mmol), at room temperature, and stirring was carried out at room temperature overnight. Water was added to the reaction solution, followed by extraction with methylene chloride, and the extract was washed sequentially with a saturated aqueous sodium hydrogencarbonate solution, water and saline, and dried over anhydrous sodium sulfate. After the organic layer was concentrated and the resulting residue was purified by silica gel column chromatography, the resulting residue was suspended in a mixed solvent of ethanol-diethyl ether, and subsequently the solid substance was collected by filtration to afford the desired title compound (181 mg, yield 71%) as a yellow solid.
¹H-NMR (CDCl₃, 400 MHz) δ: 12.64 (1H, brs), 10.17 (1H, brs), 8.02-8.00 (2H, m), 7.91 (1H, dd, J=6.3 Hz, 2.7 Hz), 7.63-7.38 (3H, m), 5.41-5.34 (1H, m), 5.13-5.08 (1H, m), 4.06-3.68 (4H, m), 2.51 and 2.47 (3H, s), 2.36-1.92 (5H, m), 1.16-1.11 (6H, m).
IR (KBr) cm⁻¹: 2960, 1690, 1640, 1540, 1415.
MS (ESI, m/z): 465 (M+H)⁺.
HRMS (ESI, m/z): 465.2251 (Calcd for C₂₄H₂₉N₆O₄: 465.2250).
Anal. Calcd for C₂₄H₂₈N₆O₄: C, 62.06; H, 6.08; N, 18.09. Found: C, 61.70; H, 6.05; N, 17.90.

### Example 89

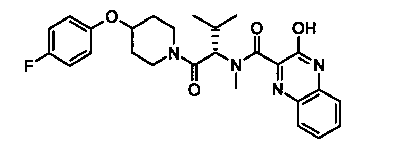

### N-[(1S)-1-{[4-(4-Fluorophenoxy)piperidin-1-yl]carbonyl}-2-methylpropyl]-3-hydroxy-N-methylquinoxaline-2-carboxamide

### Example 89-1

### t-Butyl [(1S)-1-{[4-(4-fluorophenoxy)piperidin-1-yl]carbonyl}-2-methylpropyl]methylcarbamate

Analogously to Example 2-1, t-butyl [(1S)-1-{[4-(4-fluorophenoxy)piperidin-1-yl]carbonyl}-2-methylpropyl]methylcarbamate (530 mg, yield 100%) was obtained from 4-(4-fluorophenoxy)piperidine hydrochloride (300 mg, 1.29 mmol) and N-(t-butoxycarbonyl)-N-methyl-L-valine (300 mg, 1.29 mmol) as a colorless oil.
¹H-NMR (CDCl₃, 400 MHz) δ: 7.01-6.95 (2H, m), 6.88-6.83 (2H, m), 4.62 (1H, dd, J=10.8 Hz, 4.4 Hz), 4.52-4.35 (1H, m), 4.08-3.92 (1H, m), 3.84-3.75 (1H, m), 3.71-3.60 (1H, m), 3.55-3.31 (1H, m), 2.77 and 2.75 (3H, d, J=2.4 Hz), 2 42-2 31 (1H, m), 1.95-1.61 (4H, m), 1.48 and 1.46 (9H, s), 0.91-0.84 (6H, m).
MS (ESI, m/z): 409 (M+H)⁺.

### Example 89-2

### N-[(1S)-1-{[4-(4-Fluorophenoxy)piperidin-1-yl]carbonyl}-2-methylpropyl]-3-hydroxy-N-methylquinoxaline-2-carboxamide

Analogously to Example 2-2, (2S)-1-[4-(4-fluorophenoxy)piperidin-1-yl]-N,3-dimethyl-1-oxobutan-2-amine hydrochloride (400 mg, 100%) was obtained from t-butyl [(1S)-1-{[4-(4-fluorophenoxy)piperidin-1-yl]carbonyl}-2-methylpropyl]methylcarbamate (530 mg, 1.29 mmol) as a colorless oil. The resulting compound (100 mg, 0.290 mmol) was condensed with 3-hydroxyquinoxaline-2-carboxylic acid (60.0 mg, 0.290 mmol) to afford N-[(1S)-1-{[4-(4-fluorophenoxy)piperidin-1-yl]carbonyl}-2-methylpropyl]-3-hydroxy-N-methylquinoxaline-2-carboxamide (88.0 mg, yield 63%) as a pale yellow solid.
¹H-NMR (DMSO-d₆, 400 MHz) δ: 12.74 (1H, brs), 7.81 (1H, d, J=7.8 Hz), 7.67 (1H, d, J=7.3 Hz), 7.61 (1H, d, J=11.2 Hz, 7.3 Hz), 7.38-7.32 (1H, m), 7.15 -7.10 (2H, m), 7.05-7.02 (2H, m), 5.08 (1H, d, J=11.5 Hz), 4.65-4.55 (1H, m), 4.12-4.06 (2H, m), 3.96-3.79 (2H, m), 2.77 and 2.76 (3H, s), 2.07-1.24 (5H, m), 0.97 (3H, dd, J=6.4 Hz, 2.4 Hz), 0.92 (3H, dd, J=6.4 Hz, 3.2 Hz).
IR (ATR) cm⁻¹, 3460, 2960, 1685, 1640, 1505, 1455, 1205, 1055.
MS (ESI, m/z): 503 (M+Na)⁺.
Anal. Calcd for C₂₆H₂₉FN₄O₄: C, 64.99; H, 6.08; F, 3.95; N, 11.66. Found: C, 64.69; H, 6.16; F, 4.14; N, 11.38.

### Example 90

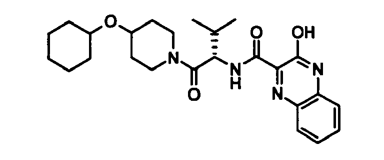

### N-[(1S)-1-{[4-(Cyclohexyloxy)piperidin-1-yl]carbonyl}-2-methylpropyl]-3-hydroxyquinoxaline-2-carboxamide

### Example 90-1

### t-Butyl 4-(2-cyclohexen-1-yloxy)piperidine-1-carboxylate

Silver oxide (1.48 g) was added to a tetrahydrofuran solution (10 ml) of t-butyl 4-hydroxypiperidine-1-carboxylate (1.00 g, 4.97 mmol) and 3-bromocyclohexene (300 mg, 1.29 mmol), at room temperature, and stirring was carried out at 40°C overnight. After the catalyst was filtered out, the mother liquor was concentrated and the resulting residual material was purified by a medium-pressure preparative liquid chromatograph (manufactured by Yamazen Corporation, W-Prep 2XY) to afford the title compound (467 mg, yield 36%) as a colorless oil. ¹H-NMR (CDCl₃, 400 MHz) δ: 5.86-5.82 (1H, m), 5.70 (1H, d, J=7.8 Hz), 4.00-3.95 (1H, m), 3.84-3.76 (2H, m), 3.62-3.56 (1H, m), 3.09-3.02 (2H, m), 2.07-1.50 (10H, m), 1.46 and 1.45 (9H, s).
MS (ESI, m/z): 262 (M+H)⁺.

### Example 90-2

### t-Butyl 4-(cyclohexyloxy)piperidine-1-carboxylate

5% Palladium carbon catalyst (60.0 mg) was added to an ethanol solution (10 ml) of t-butyl 4-(2-cyclohexen-1-yloxy)piperidine-1-carboxylate (360 mg, 1.29 mmol), at room temperature, and stirring was carried out at room temperature for 5 hours under hydrogen atmosphere. After the catalyst was filtered out, the mother liquor was concentrated, and the resulting residual material was purified by a medium-pressure preparative liquid chromatograph (manufactured by Yamazen Corporation, W-Prep 2XY) to afford t-butyl 4-(cyclohexyloxy)piperidine-1-carboxylate (211 mg, yield 58%) as a colorless oil.
¹H-NMR (CDCl₃, 400 MHz) δ: 3.82-3.75 (2H, m), 3.58-3.52 (1H, m), 3.36-3.30 (1H, m), 3.07-3.01 (2H, m), 1.88-1.70 (8H, m), 1.46 and 1.45 (9H, s), 1.32-1.15 (6H, m).
MS (ESI, m/z): 306 (M+Na)⁺.

### Example 90-3

### t-Butyl [(1S)-1-{[4-(cyclohexyloxy)piperidin-1-yl]carbonyl}-2-methylpropyl] carbamate

Analogously to Example 23-2, 4-(cyclohexyloxy)piperidine hydrochloride (160 mg, 100%) was obtained from t-butyl 4-(cyclohexyloxy)piperidine-1- (210 mg, 0.740 mmol) as a colorless oil. The resulting compound (160 mg, 0.730 mmol) was condensed with N-(t-butoxycarbonyl)-L-valine (160 mg, 0.730 mmol) to afford t-butyl [(1S)-1-{[4-(cyclohexyloxy)piperidin-1-yl]carbonyl}-2-methylpropyl]carbamate (280 mg, yield 100%) as a colorless amorphous substance.
¹H-NMR (CDCl₃, 400 MHz) δ: 5.36(1H, t, J=9.1 Hz), 4.50-4.46 (1H, m), 3.98-3.89 (1H, m), 3.80-3.71 (1H, m), 3.69-3.63 (1H, m), 3.34-3.28 (3H, m), 1.94-1.71 (7H, m), 1.63 -1.48 (4H, s), 1.43 (9H, s), 1.32-1.18 (4H, m), 0.96 (3H, dd, J=6.4 Hz, 2.4 Hz), 0.86 (3H, dd, J=6.4 Hz, 2.4 Hz).
IR (ATR) cm⁻¹: 2930, 1690, 1420, 1365, 1275, 1230, 1170, 1080.
MS (ESI, m/z): 383 (M+H)⁺.

### Example 90-4

### N-[(1S)-1-{[4-(Cyclohexyloxy)piperidin-1-yl]carbonyl}-2-methylpropyl]-3-hydroxyquinoxaline-2-carboxamide

Analogously to Example 2-2, (2S)-1-[4-(cyclohexyloxy)piperidin-1-yl]-3-methyl-1-oxobutan-2-amine hydrochloride (194 mg, 83%) was obtained from t-butyl [(1S)-1-{[4-(cyclohexyloxy)piperidin-1-yl]carbonyl}-2-methylpropyl]carbamate (280 mg, 0.730 mmol) as an amorphous substance. The resulting compound (190 mg, 0.600 mmol) was condensed with 3-hydroxyquinoxaline-2-carboxylic acid (110 mg, 0.600 mmol) to afford N-[(1S)-1-{[4-(cyclohexyloxy)piperidin-1-yl]carbonyl}-2-methylpropyl]-3-hydroxyquinoxaline-2-carboxamide (219 mg, yield 81%) as a pale yellow solid.
¹H-NMR (DMSO-d₆, 400 MHz) δ: 12.81 (1H, brs), 9.45 (1H, t, J=8.3 Hz), 7.86 (1H, d, J=8.3 Hz), 7.63 (1H, t, J=7.5 Hz), 7.39-7.36 (1H, m), 4.86 (1H, dd, J=16.1 Hz, 9.3 Hz), 3.95-3.88 (2H, m), 3.70-3.63 (1H, m), 3.39-3.27 (3H, m), 3.19-3.06 (1H, m), 2.51-2.49 (4H, m), 1.92-1.11 (11H, m), 0.95-0.92 (6H, m).
IR (ATR) cm⁻¹: 2930, 1690, 1640, 1530, 1475, 1450, 1090.
MS (ESI, m/z): 455 (M+H)⁺.
Anal. Calcd for C₂₅H₃₄N₄O₄: C, 66.06; H, 7.54; N, 12.33. Found: C, 65.77; H, 7.47; N, 12.33.

### Example 91

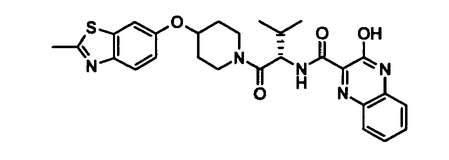

### 3-Hydroxy-N-[(1S)-2-methyl-1-({4-[(2-methyl-1,3-benzothiazol-6-yl)oxy]piperidin-1-yl}carbonyl)propyl]quinoxaline-2-carboxamide

### Example 91-1

### 2-Methyl-1,3-benzothiazol-6-ol

A 1N boron bromide methylene chloride solution (8.4 ml, 8.40 mmol) was added to a methylene chloride solution (10 ml) of 6-methoxy-2-methylbenzothiazole (500 mg, 2.79 mmol), at -78°C, under nitrogen stream, and stirring was carried out at 0°C for 1 hour. Water was added to the reaction solution, followed by extraction with methylene chloride. The extract was washed sequentially with a saturated aqueous sodium hydrogencarbonate solution and water, and then dried over anhydrous sodium sulfate. The organic layer was concentrated and the resulting residue was purified by a medium-pressure preparative liquid chromatograph (manufactured by Yamazen Corporation, W-Prep 2XY) to afford 2-methyl-1,3-benzothiazol-6-ol (103 mg, yield 22%) as a colorless oil.
¹H-NMR (CDCl₃, 400 MHz) δ: 7.66 (1H, d, J=8.8 Hz), 7.23 (1H, d, J=2.4 Hz), 6.94 (1H, dd, J=8.8 Hz, 2.4 Hz), 2.74 (3H, s).
MS (ESI, m/z): 166 (M+H)⁺.

### Example 91-2

t-Butyl [(1S)-2-methyl-1-({4-[(2-methyl-1,3-benzothiazol-6-yl)oxy]piperidin-1-yl}carbonyl)propyl]carbamate

Analogously to Example 1-2, t-butyl [(1S)-2-methyl-1-({4-[(2-methyl-1,3-benzothiazol-6-yl)oxy]piperidin-1-yl}carbonyl)propyl]carbamate (214 mg, yield 79%) was obtained from 2-methyl-1,3-benzothiazol-6-ol (100 mg, 0.610 mmol) and t-butyl {(1S)-1-[(4-hydroxypiperidin-1-yl)carbonyl]-2-methylpropyl}carbamate (200 mg, 0.670 mmol) as a colorless amorphous substance.
¹H-NMR (CDCl₃, 400 MHz) δ: 7.83 (1H, d, J=8.8 Hz), 7.32 (1H, d, J=2.0 Hz), 7.05 (1H, dd, J=8.8 Hz, 2.0 Hz), 5.40-5.36 (1H, m), 4.61-4.50 (2H, m), 3.81-3.67 (2H, m), 3.59-3.47 (2H, m), 2.80 (3H, s), 1.99-1.86 (5H, m), 1.44 (9H, s), 0.98 (3H, d, J=6.4 Hz), 0.90 (3H, dd, J=6.4 Hz, 1.5 Hz).
IR (ATR) cm⁻¹: 2965, 1705, 1635, 1450, 1365, 1210, 1165.
MS (ESI, m/z): 448 (M+H)⁺.

### Example 91-3

### 3-Hydroxy-N-[(1S)-2-methyl-1-({4-[(2-methyl-1,3-benzothiazol-6-yl)oxy]piperidin-1-yl}carbonyl)propyl]quinoxaline-2-carboxamide

Analogously to Example 2-2, (2S)-3-methyl-1-{4-[(2-methyl-1,3-benzothiazol-6-yl)oxy]piperidin-1-yl}-1-oxobutan-2-amine hydrochloride (180 mg, 100%) was obtained from t-butyl [(1S)-2-methyl-1-({4-[(2-methyl-1,3-benzothiazol-6-yl)oxy]piperidin-1-yl}carbonyl)propyl]carbamate (210 mg, 0.470 mmol) as a colorless oil. The resulting compound (180 mg, 0.470 mmol) was condensed with 3-hydroxyquinoxaline-2-carboxylic acid (90.0 mg, 0.470 mmol) to afford 3-hydroxy-N-[(1S)-2-methyl-1-({4-[(2-methyl-1,3-benzothiazol-6-yl)oxy]piperidin-1-yl}carbonyl)propyl]quinoxaline-2-carboxamide (181 mg, yield 74%) as a yellow solid.
¹H-NMR (DMSO-d₆, 400 MHz) δ: 12.78 (1H, brs), 10.20 (1H, brs), 7.99 (1H, s), 7.85 (1H, dd, J=8.8 Hz, 5.9 Hz), 7.61 (1H, brs), 7.51 (1H, brs), 7.34 (2H, dd, J=5.9 Hz, 2.0 Hz), 7.07 (1H, dt, J=9.1 Hz, 2.0 Hz), 5.09 (1H, t, J=7.3 Hz), 4.68-4.61 (1H, m), 4.02-3.73 (4H, m), 2.81 (3H, d, J=2.0 Hz), 2.40-2.17 (2H, m), 2.07-1.91 (3H, m), 1.16-1.09 (6H, m).
IR (KBr) cm⁻¹: 2960, 1690, 1640, 1530, 1455, 1210.
MS (ESI, m/z): 520 (M+H)⁺.
Anal. Calcd for C₂₇H₂₉N₅O₄S: C, 62.41; H, 5.63; N, 13.48. Found: C, 62.17; H, 5.79; N, 13.17.

### Example 92

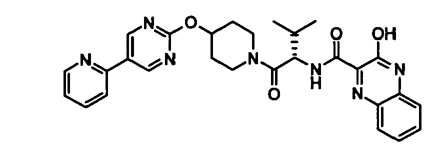

### 3-Hydroxy-N-[(1S)-2-methyl-1-({4-[(5-pyridin-2-ylpyrimidin-2-yl)oxy]piperidin-1-yl}carbonyl)propyl]quinoxaline-2-carboxamide

### Example 92-1

### t-Butyl 4-[(5-pyridin-2-ylpyrimidin-2-yl)oxy]piperidine-1-carboxylate

Tetrakis(triphenylphosphine)palladium (116 mg, 0.100 mmol) was added to a toluene solution (6.0 ml) of t-butyl 4-[(5-bromopyrimidin-2-yl)oxy]piperidine-1-carboxylate (360 mg, 1.00 mmol) and 2-(tributylstannyl)pyridine (555 mg, 1.51 mmol), under nitrogen stream, and stirring was carried out for 7 hours under heating to reflux. After completion of the reaction, the reaction solution was cooled to room temperature, and poured into ice water, followed by extraction with ethyl acetate. The extract was washed sequentially with water and saline, and then dried over anhydrous sodium sulfate. The organic layer was concentrated and the resulting residue was purified by a medium-pressure preparative liquid chromatograph (manufactured by Biotage, Inc., 25+M) to afford t-butyl 4-[(5-pyridin-2-ylpyrimidin-2-yl)oxy]piperidine-1-carboxylate (183 mg, yield 52%) as a white solid. ¹H-NMR (CDCl₃, 400 MHz) δ: 9.07 (2H, s), 8.67-8.66 (1H, m), 7.77-7.74 (1H, m), 7.63 (1H, d, J=7.2 Hz), 7.27-7.25 (1H, m), 5.27-5.21 (1H, m), 3.80-3.78 (2H, m), 3.33-3.27 (2H, m), 2.01-1.98 (2H, m), 1.83-1.80 (2H, m), 1.45 (9H, s).
MS (FAB, m/z): 357 (M+H)⁺.

### Example 92-2

### t-Butyl [(1S)-2-methyl-1-({4-[(5-pyridin-2-ylpyrimidin-2-yl)oxy]piperidin-1-yl}carbonyl)propyl]carbamate

Analogously to Example 23-2, 2-(piperidin-4-yloxy)-5-pyridin-2-ylpyrimidine dihydrochloride (169 mg) was obtained from t-butyl 4-[(5-pyridin-2-ylpyrimidin-2-yl)oxy]piperidine-1-carboxylate (183 mg, 0.513 mmol) as a white solid. The resulting compound (169 mg, 0.513 mmol) was condensed with N-(t-butoxycarbonyl)-L-valine (167 mg, 0.770 mmol) to afford t-butyl [(1S)-2-methyl-1-({4-[(5-pyridin-2-ylpyrimidin-2-yl)oxy]piperidin-1-yl}carbonyl)propyl]carbamate (212 mg, yield 91%) as a colorless amorphous substance.
¹H-NMR (CDCl₃, 500 MHz) δ: 9.15 (2H, s), 8.74 (1H, d, J=4.8 Hz), 7.85-7.82 (1H, m), 7.71 (1H, d, J=7.6 Hz), 7.34-7.32 (1H, m), 5.42-5.39 (2H, m), 4.56-4.54 (1H, m), 4.06-3.51 (4H, m), 2.19-1.91 (5H, m), 1.47 (9H, s), 1.01 (3H, d, J=6.8 Hz), 0.93 (3H, d, J=6.8 Hz).
MS (FAB, m/z): 456 (M+H)⁺.

### Example 92-3

### 3-Hydroxy-N-[(1S)-2-methyl-1-({4-[(5-pyridin-2-ylpyrimidin-2-yl)oxy]piperidin-1-yl}carbonyl)propyl]quinoxaline-2-carboxamide

Analogously to Example 23-3, (2S)-3-methyl-1-oxy-1-{4-[(5-pyridin-2-ylpyrimidin-2-yl)oxylpiperidin-1-yl}butan-2-amine dihydrochloride (200 mg) was obtained from t-butyl [(1S)-2-methyl-1-({4-[(5-pyridin-2-ylpyrimidin-2-yl)oxy]piperidin-1-yl}carbonyl)propyl]carbamate (212 mg, 0.465 mmol) as a white solid.

The resulting compound (100 mg, 0.247 mmol) was condensed with 3-hydroxyquinoxaline-2-carboxylic acid (71.0 mg, 0.371 mmol) under nitrogen stream to afford the desired title compound (70.0 mg, yield 54%) as a pale yellow solid.
¹H-NMR (DMSO-d₆, 500 MHz) δ: 12.86 (1H, br s), 9.56 (1H, br s), 9.29 (2H, d, J=4.0 Hz), 8.72 (1H, d, J=4.4 Hz), 8.07 (1H, d, J=7.6 Hz), 7.96 (1H, t, J=7.6 Hz), 7.90 (1H, dd, J=8.0 Hz, 2.0 Hz), 7.67 (1H, t, J=7.6 Hz), 7.45-7.42 (3H, m), 5.37-5.35 (1H, m), 4.96 (1H, q, J=7.6 Hz), 4.08-4.00 (2H, m), 3.66-3.29 (2H, m), 2.09-1.75 (5H, m), 1.01-0.97 (6H, m).
IR (KBr) cm⁻¹: 2965, 1685, 1630, 1425, 1335.
MS (ESI, m/z): 528 (M+H)⁺.
HRMS (ESI, m/z) : 528.2371 (Calcd for C₂₈H₃₀N₇O₄: 528.2359).

### Example 93

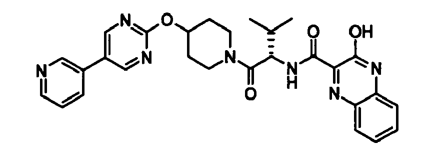

### 3-Hydroxy-N-[(1S)-2-methyl-1-({4-[(5-pyridin-3-ylpyrimidin-2-yl)oxy]piperidin-1-yl}carbonyl)propyl]quinoxaline-2-carboxamide

### Example 93-1

### t-Butyl 4-[(5-pyridin-3-ylpyrimidin-2-yl)oxy]piperidine-1-carboxylate

Analogously to Example 80-2, t-butyl 4-[(5-pyridin-3-ylpyrimidin-2-yl)oxy]piperidine-1-carboxylate (243 mg, yield 68%) was obtained from t-butyl [4-[(5-bromopyrimidin-2-yl)oxy]piperidine-1-carboxylate (360 mg, 1.00 mmol) and 2-pyridineboronic acid (186 mg, 1.51 mmol) as a white solid.
¹H-NMR (CDCl₃, 400 MHz) δ: 8.77 (1H, d, J=1.6 Hz), 8.69 (2H, s), 8.65-8.62 (1H, m), 7.81-7.78 (1H, m), 7.40-7.38 (1H, m), 5.22-5.21 (1H, m), 3.79-3.77 (2H, m), 3.33-3.26 (2H, m), 2.02-1.99 (2H, m), 1.86-1.80 (2H, m), 1.45 (9H, s).

### Example 93-2

### t-Butyl [(1S)-2-methyl-1-({4-[(5-pyridin-3-ylpyrimidin-2-yl)oxy]piperidin-1-yl}carbonyl)propyl]carbamate

Analogously to Example 23-2, 2-(piperidin-4-yloxy)-5-pyridin-3-ylpyrimidine dihydrochloride (225 mg) was obtained from t-butyl 4-[(5-pyridin-3-ylpyrimidin-2-yl)oxy]piperidine-1-carboxylate (243 mg, 0.682 mmol) as a white solid. The resulting compound (225 mg, 0.682 mmol) was condensed with N-(t-butoxycarbonyl)-L-valine (222 mg, 1.02 mmol) to afford t-butyl [(1S)-2-methyl-1-({4-[(5-pyridin-3-ylpyrimidin-2-yl)oxy]piperidin-1-yl}carbonyl)propyl]carbamate (227 mg, yield 73%) as a colorless amorphous substance.
¹H-NMR (CDCl₃, 400 MHz) δ: 8.79 (1H, d, J=2.4 Hz), 8.72 (2H, s), 8.66 (1H, d, J=4.8 Hz), 7.83 (1H, dt, J=7.6 Hz, 2.0 Hz), 7.42 (1H, dd, J=8.0 Hz, 4.8 Hz), 5.38-5.29 (2H, m), 4.50 (1H, dd, J=8.8 Hz, 5.8 Hz), 4.03-3.43 (4H, m), 2.13-1.86 (5H, m), 1.42 (9H, s), 0.96 (3H, d, J=6.8 Hz), 0.88 (3H, d, J=6.8 Hz).
MS (FAB, m/z): 456 (M+H)⁺.

### Example 93-3

### 3-Hydroxy-N-[(1S)-2-methyl-1-({4-[(5-pyridin-3-ylpyrimidin-2-yl)oxy]piperidin-1-yl}carbonyl)propyl]quinoxaline-2-carboxamide

Analogously to Example 23-3, (2S)-3-methyl-1-oxy-1-{4-[(5-pyridin-3-ylpyrimidin-2-yl)oxylpiperidin-1-yl}butan-2-amine dihydrochloride (213 mg) was obtained from t-butyl [(1S)-2-methyl-1-({4-[(5-pyridin-3-ylpyrimidin-2-yl)oxy]piperidin-1-yl}carbonyl)propyl]carbamate (227 mg, 0.498 mmol) as a white solid. The resulting compound (106 mg, 0.249 mmol) was condensed with 3-hydroxyquinoxaline-2-carboxylic acid (72.0 mg, 0.374 mmol) to afford the desired title compound (55.0 mg, yield 42%) as a pale yellow solid.
¹H-NMR (DMSO-d₆, 500 MHz) δ: 12.83 (1H, brs), 9.58 (1H, brs), 9.02 (2H, d, J=4.4 Hz), 8.96 (1H, s), 8.62 (1H, d, J=4.4 Hz), 8.17 (1H, dd, J=8.0 Hz, 2.0 Hz), 7.86 (1H, d, J=8.0 Hz), 7.63 (1H, t, J=7.6 Hz), 7.53 (1H, dd, J=7.6 Hz, 4.8 Hz), 7.37 (2H, t, J=7.6 Hz), 5.30-5.29 (1H, m), 4.93-4.90 (1H, m), 4.06-3.97 (2H, m), 3.62-3.25 (2H, m), 1.98-1.82 (5H, m), 0.98-0.92 (6H, m).
IR (KBr) cm⁻¹: 2965, 1690, 1640, 1525, 1440, 1335.
MS (ESI, m/z): 550 (M+Na)⁺.
HRMS (ESI, m/z): 550.2174 (Calcd for C₂₈H₂₉N₇NaO₄: 550.2179).

### Example 94

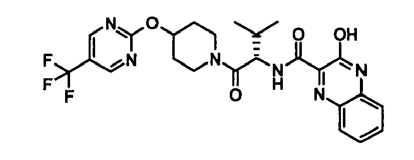

### 3-Hydroxy-N-{(1S)-2-methyl-1-[(4-{[5-(trifluoromethyl)pyrimidin-2-yl]oxy}piperidin-1-yl)carbonyl]propyl}quinoxaline-2-carboxamide

### Example 94-1

### t-Butyl 4-[(5-iodopyrimidin-2-yl)oxy]piperidine-1-carboxylate

Sodium iodide (336 mg, 2.24 mmol), copper iodide (213 mg, 1.12 mmol) and N,N-dimethylethylenediamine (0.241 ml, 2.24 mmol) were added sequentially to a 1,4-dioxane solution (8.0 ml) of t-butyl 4-[(5-bromopyrimidin-2-yl)oxy]piperidine-1-carboxylate (400 mg, 1.12 mmol), under nitrogen stream, and stirring was carried out for 5 hours under heating to reflux. After completion of the reaction, the reaction solution was cooled to room temperature, and poured into ice water, followed by extraction with ethyl acetate. The extract was washed sequentially with water and saline, and then dried over anhydrous sodium sulfate. The resulting organic layer was concentrated and the resulting residue was purified by a medium-pressure preparative liquid chromatograph (manufactured by Biotage, Inc., 25+M) to afford t-butyl 4-[(5-iodopyrimidin-2-yl)oxy]piperidine-1-carboxylate (212 mg, yield 47%) as a white solid.
¹H-NMR (CDCl₃, 400 MHz) δ: 8.59 (2H, s), 5.10-5.06 (1H, m), 3.76-3.75 (2H, m), 3.29-3.23 (2H, m), 1.94-1.94 (2H, m), 1.77-1.74 (2H, m), 1.43 (9H, s).
MS (FAB, m/z): 406 (M+H)⁺.

### Example 94-2

### t-Butyl 4-{[5-(trifluoromethyl)pyrimidin-2-yl]oxy}piperidine-1-carboxylate

Potassium fluoride (86.0 mg, 1.48 mmol), copper iodide (282 mg, 1.48 mmol) and (trifluoromethyl)trimethylsilane (0.219 ml, 1.48 mmol) were added sequentially to a N,N'-dimethylformamide solution (2.0 ml) of t-butyl 4-[(5-iodopyrimidin-2-yl)oxy]piperidine-1-carboxylate (120 mg, 0.296 mmol), under nitrogen stream, and stirring was carried out at 60°C overnight. After completion of the reaction, the reaction solution was cooled to room temperature, and poured into ice water, followed by extraction with ethyl acetate. The extract was washed sequentially with water and saline, and then dried over anhydrous sodium sulfate. The resulting organic layer was concentrated and the resulting residue was purified by a medium-pressure preparative liquid chromatograph (manufactured by Biotage, Inc., 25+M) to afford t-butyl 4-{[5-(trifluoromethyl)pyrimidin-2-yl]oxy}piperidine-1-carboxylate (60.0 mg, yield 54%) as a white solid.
¹H-NMR (CDCl₃, 400 MHz) δ: 8.74 (2H, s), 5.29-5.21 (1H, m), 3.80-3.77 (2H, m), 3.31-3.27 (2H, m), 2.03-2.00 (2H, m), 1.82-1.77 (2H, m), 1.46 (9H, s).
MS (FAB, m/z): 347 (M+H)⁺.

### Example 94-3

### t-Butyl {(1S)-2-methyl-1-[(4-{[5-(trifluoromethyl)pyrimidin-2-yl]oxy}piperidin-1-yl)carbonyl]propyl}carbamate

Analogously to Example 23-2, 2-(piperidin-4-yloxy)-5-(trifluoromethyl)pyrimidine hydrochloride (159 mg) was obtained from t-butyl 4-{[5-(trifluoromethyl)pyrimidin-2-yl]oxy}piperidine-1-carboxylate (195 mg, 0.561 mmol) as a white solid. The resulting compound (159 mg, 0.561 mmol) was condensed with N-(t-butoxycarbonyl)-L-valine (183 mg, 0.842 mmol) to afford t-butyl {(1S)-2-methyl-1-[(4-{[5-(trifluoromethyl)pyrimidin-2-yl]oxy}piperidin-1-yl)carbonyl]propyl}carbamate (217 mg, yield 87%) as a colorless amorphous substance.
¹H-NMR (CDCl₃, 400 MHz) δ: 8.73 (2H, s), 5.35-5.32 (2H, m), 4.47 (1H, dd, J=9.0 Hz, 5.6 Hz), 4.00-3.42 (4H, m), 2.03-1.88 (5H, m), 1.40 (9H, s), 0.94 (3H, d, J=6.8 Hz), 0.86 (3H, d, J=6.8 Hz).
MS (FAB, m/z): 447 (M+H)⁺.

### Example 94-4

### 3-Hydroxy-N-{(1S)-2-methyl-1-[(4-{[5-(trifluoromethyl)pyrimidin-2-yl]oxy}piperidin-1-yl)carbonyl]propyl}quinoxaline-2-carboxamide

Analogously to Example 23-3, (2S)-3-methyl-1-oxo-1-(4-{[5-(trifluoromethyl)pyrimidin-2-yl]oxy}piperidin-1-yl)butan-2-amine hydrochloride (186 mg) was obtained from t-butyl [{(1S)-2-methyl-1-[(4-{[5-(trifluoromethyl)pyrimidin-2-yl]oxy}piperidin-1-yl)carbonyl]propyl}carbamate (217 mg, 0.486 mmol) as a white solid. The resulting compound (93.0 mg, 0.243 mmol) was condensed with 3-hydroxyquinoxaline-2-carboxylic acid (70.0 mg, 0.365 mmol) to afford the desired title compound (60.0 mg, yield 48%) as a pale yellow solid.
¹H-NMR (DMSO-d₆, 400 MHz) δ: 12.78 (1H, brs), 10.21 (1H, brs), 8.78 (2H, d, J=7.6 Hz), 8.01 (1H, t, J=9.2 Hz), 7.63-7.54 (2H, m), 7.38 (1H, brs), 5.44-5.38 (1H, m), 5.12-5.08 (1H, m), 4.01-3.69 (4H, m), 2.26-2.09 (5H, m), 1.14-1.11 (6H, m).
IR (KBr) cm⁻¹: 2965, 1690, 1630, 1455, 1320, 1135.
MS (ESI, m/z): 541 (M+Na)⁺.
HRMS (ESI, m/z): 541.1786 (Calcd for C₂₄H₂₅F₃N₆NaO₄: 541.1787).

### Example 95

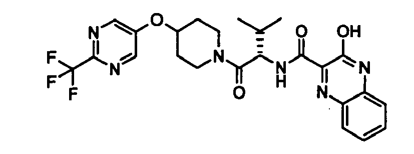

### 3-Hydroxy-N-{(1S)-2-methyl-1-[(4-{[2-(trifluoromethyl)pyrimidin-5-yl]oxy}piperidin-1-yl)carbonyl]propyl}quinoxaline-2-carboxamide

### Example 95-1

### 5-Bromo-2-(trifluoromethyl)pyrimidine

Sodium iodide (3.75 g, 25.0 mmol) and a 55 wt% aqueous hydriodic acid solution (1.48 g) were added sequentially to a chloroform solution (8.0 ml) of 5-bromo-2-chloropyrimidine (2.90 g, 15.0 mmol), at 0°C, and stirring was carried out at room temperature for 20 hours. After completion of the reaction, the reaction solution was poured into ice water, followed by extraction with ethyl acetate. The extract was washed sequentially with water and saline, and then dried over anhydrous sodium sulfate. The resulting organic layer was concentrated to afford 5-bromo-2-iodopyrimidine (3.00 g) as a colorless amorphous substance. This product was used in the next reaction without purification.

Potassium fluoride (1.22 g, 21.1 mmol), copper iodide (4.01 mg, 21.1 mmol) and (trifluoromethyl)trimethylsilane (3.13 ml, 21.1 mmol) were added sequentially to a N,N-dimethylformamide solution (20 ml) of the resulting compound (3.00 g, 10.5 mmol), and stirring was carried out at 70°C for 2 hours. After completion of the reaction, the reaction solution was cooled to room temperature, and poured into ice water, followed by extraction with ethyl acetate. The extract was washed sequentially with water and saline, and then dried over anhydrous sodium sulfate. The organic layer was concentrated and the resulting residue was purified by a medium-pressure preparative liquid chromatograph (manufactured by Biotage, Inc., 25+M) to afford 5-bromo-2-(trifluoromethyl)pyrimidine (278 mg, yield 12%) as a colorless amorphous substance.
¹H-NMR (CDCl₃, 400 MHz) δ: 8.94 (2H, s).
MS (EI, m/z): 226 (M⁺).

### Example 95-2

### 2-(Trifluoromethyl)pyrimidin-5-ol

palladium acetate (27.0 mg, 0.122 mmol), bis(pinacolato)diboron (342 mg, 1.35 mmol) and potassium acetate (359 mg, 3.66 mmol) were added sequentially to a N,N-dimethylformamide solution (3.0 ml) of 5-bromo-2-(trifluoromethyl)pyrimidine (278 mg, 1.22 mmol), under nitrogen stream, and stirring was carried out at 90°C for 3 hours. After completion of the reaction, the reaction solution was cooled to room temperature, and poured into ice water, followed by extraction with ethyl acetate. The extract was washed sequentially with water and saline, and then the resulting organic layer was dried over anhydrous sodium sulfate. The organic layer was concentrated and the resulting residue was dissolved in a mixed solution of tetrahydrofuran-water (8.0 ml, 1:1, v/v), followed by addition of sodium perborate tetrahydrate and stirring overnight. After completion of the reaction, the reaction solution was cooled to room temperature, and poured into ice water, followed by extraction with ethyl acetate. The extract was washed sequentially with water and saline, and then dried over anhydrous sodium sulfate. The organic layer was concentrated and the resulting residue was purified by a medium-pressure preparative liquid chromatograph (manufactured by Biotage, Inc., 25+M) to afford 2-(trifluoromethyl)pyrimidin-5-ol (220 mg, 100%) as a colorless amorphous substance.
¹H-NMR (CDCl₃, 400 MHz) δ: 8.46 (2H, s).
MS (EI, m/z) : 164 (M⁺).

### Example 95-3

### t-Butyl {(1S)-2-methyl-1-[(4-{[2-(trifluoromethyl)pyrimidin-5-yl]oxy}piperidin-1-yl)carbonyl]propyl}carbamate

Analogously to Example 1-2, t-butyl {(1S)-2-methyl-1-[(4-{[2-(trifluoromethyl)pyrimidin-5-yl]oxy}piperidin-1-yl)carbonyl]propyl}carbamate (320 mg, 49%) was obtained from 2-(trifluoromethyl)pyrimidin-5-ol (240 mg, 1.46 mmol) and t-butyl {(1S)-1-[(4-hydroxypiperidin-1-yl)carbonyl]-2-methylpropyl}carbamate (527 mg, 1.76 mmol) as a colorless amorphous substance.
¹H-NMR (CDCl₃, 400 MHz) δ: 8.50 (2H, s), 5.35 (1H, d, J=9.2 Hz), 4.80-4.72 (1H, m), 4.47 (1H, dd, J=9.2 Hz, 6.0 Hz), 3.91-3.50 (4H, m), 2.12-1.84 (5H, m), 1.41 (9H, s), 0.95 (3H, d, J=6.8 Hz), 0.89 (3H, d, J=6.8 Hz).
MS (FAB, m/z): 447 (M+H)⁺.

### Example 95-4

### 3-Hydroxy-N-{(1S)-2-methyl-1-[(4-{[2-(trifluoromethyl)pyrimidin-5-yl]oxy}piperidin-1-yl)carbonyl]propyl}quinoxaline-2-carboxamide

Analogously to Example 23-3, (2S)-3-methyl-1-oxo-1-(4-{[2-(trifluoromethyl)pyrimidin-5-yl]oxy}piperidin-1-yl)butan-2-amine hydrochloride (274 mg) was obtained from t-butyl {(1S)-2-methyl-1-[(4-{[2-(trifluoromethyl)pyrimidin-5-yl]oxy}piperidin-1-yl)carbonyl]propyl}carbamate (320 mg, 0.717 mmol) as a white solid.

The resulting compound (137 mg, 0.360 mmol) was condensed with 3-hydroxyquinoxaline-2-carboxylic acid (103 mg, 0.538 mmol) under nitrogen stream to afford the desired title compound (70.0 mg, yield 38%) as a pale yellow solid.
¹H-NMR (DMSO-d₆, 400 MHz) δ: 12.84 (1H, brs), 9.55 (1H, brs), 8.85 (2H, d, J=3.6 Hz), 7.86 (1H, t, J=6.8 Hz), 7.64 (1H, t, J=8.0 Hz), 7.39-7.38 (2H, m), 5.04-5.02 (1H, m), 4.93-4.89 (1H, m), 4.01-3.99 (2H, m), 3.44-3.27 (2H, m), 2.08-2.04 (3H, m), 1.61 (2H, s), 0.96-0.95 (6H, m). IR (KBr) cm⁻¹: 2965, 1685, 1640, 1535, 1350.
MS (ESI, m/z): 519 (M+H)⁺.
HRMS (ESI, m/z): 519.1966 (Calcd for C₂₄H₂₆F₃N₆O₄: 519.1968).

### Example 96

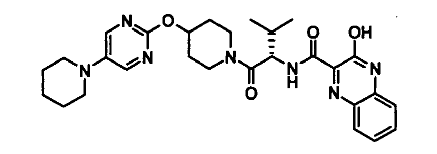

### 3-Hydroxy-N-[(1S)-2-methyl-1-({4-[(5-piperidin-1-ylpyrimidin-2-yl)oxy]piperidin-1-yl}carbonyl)propyl]quinoxaline-2-carboxamide

### Example 96-1

### t-Butyl 4-[(5-piperidin-1-ylpyrimidin-2-yl)oxy]piperidine-1-carboxylate

Tris(dibenzylideneacetone)dipalladium (96.0 mg, 0.167 mmol), 1,1'-bi-2-naphthol (312 mg, 0.501 mmol), piperidine (0.332 ml, 3.35 mmol) and sodium t-butoxide (324 mg, 3.35 mmol) were added sequentially to a toluene solution (10 ml) of t-butyl 4-[(5-bromopyrimidin-2-yl)oxy]piperidine-1-carboxylate (600 mg, 1.67 mmol), under nitrogen stream, and stirring was carried out for 5 hours under heating to reflux. After completion of the reaction, the reaction solution was cooled to room temperature, and poured into ice water, followed by extraction with ethyl acetate. The extract was washed sequentially with water and saline, and then dried over anhydrous sodium sulfate. The resulting organic layer was concentrated and the resulting residue was purified by a medium-pressure preparative liquid chromatograph (manufactured by Biotage, Inc., 25+M) to afford t-butyl 4-[(5-piperidin-1-ylpyrimidin-2-yl)oxy]piperidine-1-carboxylate (600 mg, yield 98%) as a white solid.
¹H-NMR (CDCl₃, 400 MHz) δ: 8.14 (2H, s), 5.06-5.01 (1H, m), 3.77-3.73 (2H, m), 3.29-3.23 (2H, m), 3.02-3.01 (4H, m), 1.94 (2H, brs), 1.79-1.67 (6H, m), 1.57-1.51 (2H, m), 1.43 (9H, s).
MS (FAB, m/z): 363 (M+H)⁺.

### Example 96-2

### t-Butyl [(1S)-2-methyl-1-({4-[(5-piperidin-1-ylpyrimidin-2-yl)oxy]piperidin-1-yl}carbonyl)propyl]carbamate

Analogously to Example 23-2, 5-piperidin-1-yl-2-(piperidin-4-yloxy)pyrimidine dihydrochloride (556 mg) was obtained from t-butyl 4-[(5-piperidin-1-ylpyrimidin-2-yl)oxy]piperidine-1-carboxylate (600 mg, 1.66 mmol) as a white solid. The resulting compound (556 mg, 1.66 mmol) was condensed with N-(t-butoxycarbonyl)-L-valine (539 mg, 2.48 mmol) to afford t-butyl [(1S)-2-methyl-1-({4-[(5-piperidin-1-ylpyrimidin-2-yl)oxylpiperidin-1-yl}carbonyl)propyl]carbamate (567 mg, yield 74%) as a colorless amorphous substance.
¹H-NMR (CDCl₃, 400 MHz) δ: 8.15 (2H, s), 5.35-5.33 (1H, m), 5.15-5.11 (1H, m), 4.47 (1H, dd, J=8.8 Hz, 5.6 Hz), 3.91-3.40 (4H, m), 3.03 (4H, t, J=5.6 Hz), 2.04-1.78 (5H, m), 1.73-1.67 (4H, m), 1.57-1.51 (2H, m), 1.40 (9H, s), 0.93 (3H, dd, J=6.8 Hz, 2.4 Hz), 0.85 (3H, dd, J=6.8 Hz, 4.0 Hz).
MS (FAB, m/z): 462 (M+H)⁺.

### Example 96-3

### 3-Hydroxy-N-[(1S)-2-methyl-1-({4-[(5-piperidin-1-ylpyrimidin-2-yl)oxy]piperidin-1-yl}carbonyl)propyl]quinoxaline-2-carboxamide

Analogously to Example 23-3, (2S)-3-methyl-1-oxo-1-{4-[(5-piperidin-1-ylpyrimidin-2-yl)oxylpiperidin-1-yl}butan-2-amine dihydrochloride (534 mg) was obtained from t-butyl [(1S)-2-methyl-1-({4-[(5-piperidin-1-ylpyrimidin-2-yl)oxy]piperidin-1-yl}carbonyl)propyl]carbamate (567 mg, 1.23 mmol) as a white solid. The resulting compound (283 mg, 0.614 mmol) was condensed with 3-hydroxyquinoxaline-2-carboxylic acid (175 mg, 0.921 mmol) to afford the desired title compound (105 mg, yield 32%) as a pale yellow solid. ¹H-NMR (DMSO-d₆, 400 MHz) δ: 12.84 (1H, brs), 9.56 (1H, brs), 8.32 (2H, d, J=3.6 Hz), 7.87 (1H, d, J=8.0 Hz), 7.64 (1H, t, J=8.0 Hz), 7.39-7.37 (2H, m), 5.10-5.09 (1H, m), 4.95-4.92 (1H, m), 4.00-3.95 (2H, m), 3.55-3.23 (2H, m), 3.07 (4H, t, J=5.2 Hz), 2.06-2.04 (3H, m), 1.62-1.52 (8H, m), 0.96-0.94 (6H, m).
IR (KBr) cm⁻¹: 2940, 1690, 1640, 1525, 1440, 1320.
MS (ESI, m/z): 556 (M+Na)⁺.
HRMS (ESI, m/z): 556.2641 (Calcd for C₂₈H₃₅N₇NaO₄: 556.2648).

### Example 97

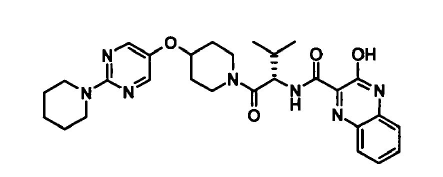

### 3-Hydroxy-N-[(1S)-2-methyl-1-({4-[(2-piperidin-1-ylpyrimidin-5-yl)oxy]piperidin-1-yl}carbonyl)propyl]quinoxaline-2-carboxamide

### Example 97-1

### t-Butyl [(1S)-2-methyl-1-({4-[(2-piperidin-1-ylpyrimidin-5-yl)oxy]piperidin-1-yl}carbonyl)propyl]carbamate

Piperidine (0.284 ml, 2.83 mmol) and potassium carbonate (330 mg, 4.78 mmol) were added sequentially to a N,N-dimethylformamide solution (4.0 ml) of t-butyl (1S)-1-({4-[(2-chloropyrimidin-5-yl)oxy]piperidin-1-yl}carbonyl)-2-methylpropyl]carbamate (300 mg, 0.956 mmol), under nitrogen stream, and stirring was carried out at 100°C for 3 hours. After completion of the reaction, the reaction solution was cooled to room temperature, and poured into ice water, followed by extraction with ethyl acetate. The extract was washed sequentially with water and saline, and then dried over anhydrous sodium sulfate. The organic layer was concentrated and the resulting residue was purified by a medium-pressure preparative liquid chromatograph (manufactured by Biotage, Inc., 25+M) to afford t-butyl [(1S)-2-methyl-1-({4-[(2-piperidin-1-ylpyrimidin-5-yl)oxy]piperidin-1-yl}carbonyl)propyl]carbamate (400 mg, yield 91%) as a colorless amorphous substance.
¹H-NMR (CDCl₃, 400 MHz) δ: 8.04 (2H, s), 5.32 (1H, d, J=9.2 Hz), 4.46-4.44 (1H, m), 4.26-4.18 (1H, m), 3.89-3.59 (6H, m), 3.49-3.34 (2H, m), 1.97-1.49 (11H, m), 1.40 (9H, s), 0.93 (3H, dd, J=6.8 Hz, 5.2 Hz), 0.85 (3H, dd, J=6.8 Hz, 2.8 Hz).

### Example 97-2

### 3-Hydroxy-N-[(1S)-2-methyl-1-({4-[(2-piperidin-1-ylpyrimidin-5-yl)oxy]piperidin-1-yl}carbonyl)propyl]quinoxaline-2-carboxamide

Analogously to Example 23-3, (2S)-3-methyl-1-oxo-1-{4-[(2-piperidin-1-ylpyrimidin-5-yl)oxy]piperidin-1-yl}butan-2-amine dihydrochloride (376 mg) was obtained from t-butyl [(1S)-2-methyl-1-({4-[(2-piperidin-1-ylpyrimidin-5-yl)oxy]piperidin-1-yl}carbonyl)propyl]carbamate (400 mg, 0.867 mmol) as a white solid. The resulting compound (188 mg, 0.433 mmol) was condensed with 3-hydroxyquinoxaline-2-carboxylic acid (125 mg, 0.650 mmol) to afford the desired title compound (70.0 mg, yield 30%) as a pale yellow solid.
¹H-NMR (DMSO-d₆, 400 MHz) δ: 12.84 (1H, brs), 9.52 (1H, brs), 8.23 (2H, d, J=6.4 Hz), 7.87 (1H, d, J=7.6 Hz), 7.64 (1H, t, J=7.6 Hz), 7.39-7.38 (2H, m), 4.92-4.89 (1H, m), 4.45-4.43 (1H, m), 3.93-3.88 (2H, m), 3.66 (4H, t, J=4.8 Hz), 3.47-3.22 (2H, m), 2.02-1.92 (3H, m), 1.60-1.51 (8H, m), 0.95 (6H, t, J=6.8 Hz).
IR (KBr) cm⁻¹: 2935, 1690, 1635, 1495, 1445, 1255.
MS (ESI, m/z): 556 (M+Na)⁺.
HRMS (ESI, m/z): 556.2651 (Calcd for C₂₈H₃₅N₇NaO₄: 556.2648).

### Example 98

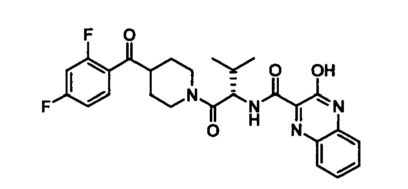

### N-[(1S)-1-{[4-(2,4-Difluorobenzoyl)piperidin-1-yl]carbonyl}-2-methylpropyl]-3-hydroxyquinoxaline-2-carboxamide

### Example 98-1

### t-Butyl [(1S)-1-{[4-(2,4-difluorobenzoyl)piperidin-1-yl]carbonyl}-2-methylpropyl] carbamate

Analogously to Example 2-1, 4-(2,4-difluorobenzoyl)piperidine hydrochloride (1.00 g, 3.82 mmol) was condensed with N-(t-butoxycarbonyl)-L-valine (1.25 g, 5.73 mmol) to afford N-[(1S)-1-{[4-(2,4-difluorobenzoyl)piperidin-1-yl]carbonyl}-2-methylpropyl]-3,4-difluoro-2-hydroxybenzamide (1.45 g, yield 90%) as a colorless amorphous substance.
¹H-NMR (CDCl₃, 400 MHz) δ: 7.87-7.84 (1H, m), 6.97 (1H, t, J=8.0 Hz), 6.91-6.84 (1H, m), 5.33 (1H, t, J=8.0 Hz), 4.53-4.48 (2H, m), 4.02-3.98 (1H, m), 3.37-3.32 (1H, m), 3.26-3.16 (1H, m), 2.87-2.83 (1H, m), 2.01-1.85 (3H, m), 1.75-1.51 (2H, m), 1.41 (9H, s), 0.94 (3H, t, J=6.8 Hz), 0.86 (3H, d, J=6.8 Hz).
MS (FAB, m/z): 425 (M+H)⁺.

### Example 98-2

### N-[(1S)-1-{[4-(2,4-Difluorobenzoyl)piperidin-1-yl]carbonyl}-2-methylpropyl]-3-hydroxyquinoxaline-2-carboxamide

Analogously to Example 23-3, (2,4-difluorophenyl)(1-L-valylpiperidin-4-yl)methanone hydrochloride (1.29 g) was obtained from t-butyl [(1S)-1-{[4-(2,4-difluorobenzoyl)piperidin-1-yl]carbonyl}-2-methylpropyl]carbamate (1.45 g, 3.44 mmol) as a white solid. The resulting compound (300 mg, 0.831 mmol) was condensed with 3-hydroxyquinoxaline-2-carboxylic acid (238 mg, 1.25 mmol) to afford the desired title compound (362 mg, yield 88%) as a pale yellow solid. ¹H-NMR (DMSO-d₆, 500 MHz) δ: 12.82 (1H, brs), 9.51 (1H, brs), 7.95-7.79 (2H, m), 7.63 (1H, t, J=8.0 Hz), 7.47-7.33 (3H, m), 7.28-7.24 (1H, m), 4.92-4.86 (1H, m), 4.44-4.40 (1H, m), 4.18-4.16 (1H, m), 3.39-3.26 (2H, m), 2.84-2.81 (1H, m), 2.00-1.83 (3H, m), 1.61-1.31 (2H, m), 0.95-0.93 (6H, m).
IR (KBr) cm⁻¹: 2965, 1685, 1610, 1530, 1270.
MS (ESI, m/z): 519 (M+Na)⁺.
HRMS (ESI, m/z): 519.1812 (Calcd for C₂₆H₂₆F₂N₄NaO₄: 519.1820).

### Example 99

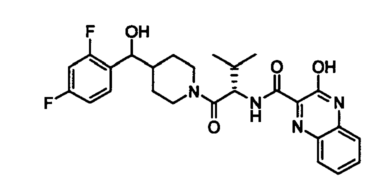

### N-[(1S)-1-({4-[(2,4-Difluorophenyl)(hydroxy)methyl]piperidin-1-yl}carbonyl)-2-methylpropyl]-3-hydroxyquinoxaline-2-carboxamide

Sodium borohydride (183 mg, 0.483 mmol) was added to a methanol solution (2.0 ml) of N-[(1S)-1-{[4-(2,4-difluorobenzoyl)piperidin-1-yl]carbonyl}-2-methylpropyl]-3-hydroxyquinoxaline-2-carboxamide (218 mg, 0.439 mmol), at 0°C, and stirring was carried out at the same temperature for 1 hour. After completion of the reaction, the reaction solution was poured into ice water, followed by extraction with ethyl acetate. The extract was washed sequentially with water and saline, and then dried over anhydrous sodium sulfate. The resulting organic layer was concentrated and the resulting residue was purified by a medium-pressure preparative liquid chromatograph (manufactured by Biotage, Inc., 25+M) to afford the desired title compound (85.0 mg, yield 39%) as a pale yellow solid.
¹H-NMR (DMSO-d₆, 500 MHz) δ: 12.83 (1H, brs), 9.55 (1H, brs), 7.87-7.85 (1H, m), 7.63 (1H, q, J=8.0 Hz), 7.50-7.47 (1H, m), 7.38-7.36 (2H, m), 7.15-7.09 (2H, m), 5.44-5.42 (1H, m), 4.92-4.85 (1H, m), 4.60-4.55 (1H, m), 4.45-4.42 (1H, m), 4.16-4.01 (1H, m), 3.38-3.33 (1H, m), 3.03-3.01 (1H, m), 2.02-2.00 (1H, m), 1.86-1.79 (2H, m), 1.44-1.06 (2H, m), 0.93-0.89 (6H, m).
IR (KBr) cm⁻¹: 3425, 2965, 1685, 1620, 1525, 1455.
MS (ESI, m/z): 521 (M+Na)⁺.
HRMS (ESI, m/z) : 521.1965 (Calcd for C₂₆H₂₈F₂N₄NaO₄: 521.1976).

### Example 100

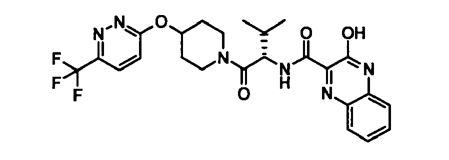

### 3-Hydroxy-N-{(1S)-2-methyl-1-[(4-{[6-(trifluoromethyl)pyridazin-3-yl]oxy}piperidin-1-yl)carbonyl]propyl}quinoxaline-2-carboxamide

### Example 100-1

### 3-Chloro-6-(trifluoromethyl)pyridazine

Sodium iodide (1.31 g, 8.72 mmol) and a 55 wt% aqueous hydriodic acid solution (1.25 g) were added sequentially to a chloroform solution (4.0 ml) of 3,6-dichloropyridazine (1.00 g, 6.71 mmol), at 0°C, and stirring was carried out at 40°C for 5 hours. After completion of the reaction, at 0°C, 2N sodium hydroxide was added for neutralization, followed by extraction with ethyl acetate. The extract was washed sequentially with water and saline, and then dried over anhydrous sodium sulfate. The resulting organic layer was concentrated to afford 3-chloro-6-iodopyridazine (1.23 g) as a white solid. This product was used as it is in the next reaction without purification.

Potassium fluoride (889 mg, 15.3 mmol), copper iodide (2.92 g, 15.3 mmol) and (trifluoromethyl)trimethylsilane (2.27 ml, 15.3 mmol) were added sequentially to a N,N-dimethylformamide solution (10 ml) of the resulting compound (1.23 g, 5.11 mmol), and stirring was carried out at 60°C for 2.5 hours. After completion of the reaction, the reaction solution was cooled to room temperature, and poured into ice water, followed by extraction with ethyl acetate. The extract was washed sequentially with water and saline, and then dried over anhydrous sodium sulfate. The organic layer was concentrated and the resulting residue was purified by a medium-pressure preparative liquid chromatograph (manufactured by Biotage, Inc., 25+M) to afford 3-chloro-6-(trifluoromethyl)pyridazine (350 mg, yield 38%) as a white solid.
¹H-NMR (CDCl₃, 400 MHz) δ: 7.83 (1H, d, J=9.2 Hz), 7.76 (1H, d, J=9.2 Hz).
MS (EI, m/z) : 182 (M⁺).

### Example 100-2

### t-Butyl {(1S)-2-methyl-1-[(4-{[6-(trifluoromethyl)pyridazin-3-yl]oxy}piperidin-1-yl)carbonyl]propyl}carbamate

Analogously to Example 64-1, t-butyl {(1S)-2-methyl-1-[(4-{[6-(trifluoromethyl)pyridazin-3-yl]oxy}piperidin-1-yl)carbonyl]propyl}carbamate (380 mg, yield 44%) was obtained from 3-chloro-6-(trifluoromethyl)pyridazine (350 mg, 1.92 mmol) and t-butyl {(1S)-1-[(4-hydroxypiperidin-1-yl)carbonyl]-2-methylpropyl}carbamate (634 mg, 2.11 mmol) as a colorless amorphous substance.
¹H-NMR (CDCl₃, 500 MHz) δ: 7.71 (1H, d, J=9.2 Hz), 7.11 (1H, dd, J=9.2 Hz, 3.2 Hz), 5.68-5.65 (1H, m), 5.34 (1H, t, J=9.2 Hz), 4.52-4.50 (1H, m), 4.13-3.79 (2H, m), 3.61-3.40 (2H, m), 2.21-2.13 (2H, m), 1.94-1.84 (3H, m), 1.44 (9H, s), 0.98 (3H, d, J=6.8 Hz), 0.90 (3H, t, J=6.0 Hz). MS (FAB, m/z): 447 (M+H)⁺.

### Example 100-3

### 3-Hydroxy-N-{(1S)-2-methyl-1-[(4-{[6-(trifluoromethyl)pyridazin-3-yl]oxy}piperidin-1-yl)carbonyl]propyl}quinoxaline-2-carboxamide

Analogously to Example 23-3, (2S)-3-methyl-1-oxo-1-{4-{[6-(trifluoromethyl)pyridazin-3-yl]oxy}piperidin-1-yl}butan-2-amine hydrochloride (299 mg) was obtained from t-butyl {(1S)-2-methyl-1-[(4-{[6-(trifluoromethyl)pyridazin-3-yl]oxy}piperidin-1-yl)carbonyl]propyl}carbamate (350 mg, 0.784 mmol) as a white solid. The resulting compound (150 mg, 0.392 mmol) was condensed with 3-hydroxyquinoxaline-2-carboxylic acid (112 mg, 0.588 mmol) to afford the desired title compound (85.0 mg, yield 42%) as a pale yellow solid.
¹H-NMR (DMSO-d₆, 400 MHz) δ: 12.79 (1H, brs), 9.57 (1H, brs), 8.17 (1H, dd, J=9.2 Hz, 4.0 Hz), 7.87-7.86 (1H, m), 7.66-7.62 (1H, m), 7.51 (1H, dd, J=9.2 Hz, 5.6 Hz), 7.39-7.37 (2H, m), 5.60-5.59 (1H, m), 4.95-4.93 (1H, m), 4.03-3.95 (2H, m), 3.63-3.32 (2H, m), 2.07-1.77 (5H, m), 0.96 (6H, t, J=6.0 Hz).
IR (KBr) cm⁻¹: 2960, 1690, 1630, 1525, 1450, 1300.
MS (ESI, m/z): 541 (M+Na)⁺.
HRMS (ESI, m/z): 541.1788 (Calcd for C₂₄H₂₅F₃N₆NaO₄: 541.1787).

### [Preparation Examples]

### Preparation Example 1

### Injection Solution

1.5% by weight of the compound of Example 8 is stirred in 10% by volume of propylene glycol followed by adjusting to a fixed volume with water for injection and sterilizing to obtain an injection solution.

### Preparation Example 2

### Hard Capsule

50 mg of the compound of Example 12 in powder form, 128.7 mg of lactose, 70 mg of cellulose and 1.3 mg of magnesium stearate are mixed and filtered with a 60 mesh sieve followed by filling the powder into a 250 mg No. 3 gelatin capsule to obtain a capsule.

### Preparation Example 3

### Tablet

50 mg of the compound of Example 16 in powder form, 124 mg of lactose, 25 mg of cellulose and 1 mg of magnesium stearate are mixed and tableted with a tablet making machine to obtain a 200 mg tablet. This tablet can be sugar-coated as necessary.

### [Test Examples]

### (Test Example 1) Measurement of Inhibitory Action on γ-Glutamyl Carboxylation Reaction

Cells were used that expressed Gas6 excreted outside the cells when subjected to γ-glutamyl carboxylation, and inhibitory action on γ-glutamyl carboxylation reaction was assessed by measuring the secreted amount of extracellular Gas6 by ELISA method.

### (A) Preparation for Assay Plate Containing Solid-Phase Anti-human Gas6 Antibody

Goat anti-human Gas6 antibody (R&D Systems, Inc.) was diluted to 1 µg/ml with Dulbecco's phosphate-buffered saline, and 40 µl aliquots thereof were dispensed into each well of a 384-well microplate. After immobilizing the antibody to the solid phase by allowing it to stand at 4°C overnight or at room temperature for 2 hours or more, each well was washed three times with wash buffer (10 mM Tris-HCl pH 7.4, 150 mM sodium chloride, 0.05% Tween20). After removing the supernatant, 80 µl aliquots of a 1% aqueous solution of Block Ace (Dainippon Sumitomo Pharma) were dispensed into each well and allowed to react at room temperature for 1 hour or more.

### (B) Exposure of Test Compounds to Cells Stably Expressing Human Gas6

Cells that stably express human Gas6 (Gas6-HEK293), which were prepared by inserting human Gas6 gene (inserted between SalI and NotI within multiple cloning sites of pCIneo expression vector) into HEK293 cells, were grown and sub-cultured using Dulbecco's modified Eagle's medium (DMEM) containing 10% fetal calf serum (FCS). On the day before ELISA measurement, the Gas6-HEK293 cells were detached with trypsin-EDTA, dispersed in DMEM containing 10% FCS, and disseminated into each well of a 384-well, collagen type I-coated plate (Becton-Dickinson) in 30 µl aliquots containing 1.0 x 10⁴ cells each, followed by culturing for 3 to 4 hours at 5% CO₂ and 37°C. 30 µL aliquots of DMEM containing test compound solutions, prepared at various concentrations by dissolving in DMSO, and vitamin K₂ at 0.1% and 100 nM, respectively (indicating final concentrations in both cases) were added to each well followed by additionally culturing for 18 to 20 hours.

### (C) Quantification of γ-Glutamyl Carboxylated Gas6 by ELISA and Calculation of 50% Inhibitory Concentrations of γ-Glutamyl Carboxylation Reaction

After removing the 1% Block Ace aqueous solution from the preliminarily prepared assay plates containing solid phase anti-human Gas6 antibody, the culture supernatant of Gas6-HEK293 cells exposed to the test compounds was collected and 40 µl aliquots were dispensed into each well followed by allowing to react at room temperature for 2 hours (Step 1). The culture supernatant was removed and each well was washed three times with wash buffer. Thereafter, the same washing procedure was carried out between Steps 2 and 3 and Steps 3 and 4. Subsequently, 40 µl aliquots of mouse anti-human γ-glutamyl carboxylase antibody (American Diagnostica inc.), adjusted to 0.5 µg/ml with immunoreaction buffer (100 mM Tris-HCl pH 7.4, 0.05% Tween20, 0.1% Block Ace), were dispensed into each well and allowed to react at room temperature for 1 hour (Step 2). Next, 40 µl aliquots of alkaline phosphatase-conjugated anti-mouse IgG antibody adjusted to 1.0 µg/ml with immunoreaction buffer were dispensed into each well and allowed to react at room temperature for 1 hour (Step 3). 40 µl aliquots of BluePhos Microwell (KPL) were dispensed into each well, and after incubating at room temperature for 10 minutes (Step 4), 20 µl aliquots of 5% EDTA were added to each well to stop the color development reaction, followed by measurement of absorbance at 620 nm (Step 5). The γ-glutamyl carboxylation reaction inhibition rate of each test compound was calculated by assigning a value of 100% for the γ-glutamyl carboxylation reaction to the absorbance of wells in which the cells were only exposed to vitamin K₂ and DMSO in the absence of test compound, and assigning a value of 0% for the γ-glutamyl carboxylation reaction to the absorbance of wells in which cells were exposed to 10 µM warfarin in the absence of vitamin K₂. 50% inhibitory concentration (IC₅₀) values for the γ-glutamyl carboxylation reaction were calculated from the γ-glutamyl carboxylation reaction inhibition rates of the test compounds at each concentration.

The results are shown in Table 1.

**(Table 1)**

| Example No. of Test Compound | IC₅₀ (nM) |
|---|---|
| Example 1 | 8.4 |
| Example 2 | 6.6 |
| Example 3 | 3.5 |
| Example 4 | 4.7 |
| Example 6 | 74 |
| Example 7 | 1.7 |
| Example 8 | 1.8 |
| Example 9 | <0.6 |
| Example 10 | <0.6 |
| Example 11 | 6.5 |
| Example 12 | 4.2 |
| Example 13 | 5.8 |
| Example 14 | 25 |
| Example 15 | 18 |
| Example 16 | 7.0 |
| Example 18 | 2.9 |
| Example 19 | 4.7 |
| Example 21 | 0.89 |
| Example 23 | <0.6 |
| Example 29 | 7.2 |
| Example 30 | 1.7 |
| Example 34 | 12 |
| Example 35 | <0.6 |
| Example 36 | 1.8 |
| Example 37 | 5.4 |
| Example 45 | 1.3 |
| Example 49 | 4.3 |
| Example 61 | 5.1 |
| Example 62 | 3.6 |
| Example 65 | 20 |
| Example 67 | 0.75 |
| Example 69 | 3.4 |
| Example 74 | 5.5 |
| Example 80 | 7.1 |
| Example 81 | 4.6 |
| Example 82 | 4.6 |
| Example 84 | 5.5 |
| Example 86 | 0.7 |
| Example 91 | 3.8 |
| Example 94 | 2.7 |
| Example 96 | 9.6 |
| Example 97 | 4.5 |
| Example 98 | 3.5 |
| Example 99 | 4.9 |
| Example 100 | 10.4 |

### (Test Example 2) Measurement of Ex Vivo Anticoagulatory Activity

### (A) Dosing and Collection of Blood Samples

Dosing solutions (1 mg/ml), in which the test compounds were dissolved or suspended in 0.5% methyl cellulose (MC), were orally administered to rats (3 mg/kg). 18 hours after administration, 0.5 ml of blood (containing aqueous citric acid solution) were sampled from the jugular vein using a syringe injected with 50 µl of a 3.13% (w/v) aqueous trisodium citrate dihydrate solution. The citrated blood was centrifuged for 10 minutes at 1500 x g to separate the plasma. Blood was also sampled from rats not dosed with test compounds and plasma was obtained therefrom for use as a control.

### (B) Measurement of Prothrombin Time and Calculation of Prolongation Rate

Prothrombin time (PT) was measured using Amelung KC-10A Micro Coagulometer (MC Medical), a coagulation time measurement apparatus. 50 µl of saline were added to 50 µl of plasma followed by pre-incubating at 37°C for 1 minute. The coagulation reaction was started by adding 100 µl of Thromboplastin C Plus (Dade Behring), which was adjusted to 0.5 U/ml by suspending in water for injection, followed by measurement of PT. The PT values of rats not dosed with test compounds were used as a control, and PT prolongation rates of rats dosed with test compounds were expressed as a percentage.

The results are shown in Table 2.

**(Table 2)**

| Example No. of Test Compound | PT (%) |
|---|---|
| Example 1 | 731 |
| Example 3 | 616 |
| Example 4 | 790 |
| Example 6 | 557 |
| Example 7 | 732 |
| Example 8 | 576 |
| Example 9 | 751 |
| Example 10 | 802 |
| Example 12 | 725 |
| Example 13 | 580 |
| Example 15 | 663 |
| Example 16 | 720 |
| Example 18 | 709 |
| Example 21 | 637 |
| Example 23 | 681 |
| Example 29 | 707 |
| Example 36 | 649 |
| Example 37 | 644 |
| Example 45 | 609 |
| Example 49 | 750 |
| Example 65 | 568 |
| Example 69 | 798 |
| Example 74 | 688 |
| Example 80 | 616 |
| Example 81 | 706 |
| Example 86 | 582 |
| Example 94 | 574 |
| Example 98 | 623 |
| Example 99 | 747 |

## Claims

1. A compound represented by the general formula (i): 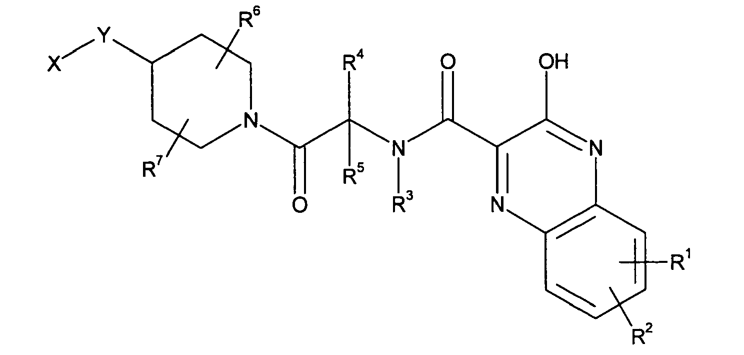 wherein,
each of R¹ and R² independently represents a hydrogen atom, a halogen atom, a cyano group, a C₁₋₄ alkyl group or a C₁₋₄ alkoxy group,
R³ represents a hydrogen atom or a C₁₋₄ alkyl group,
each of R⁴ and R⁵ independently represents a hydrogen atom, a halogen atom, a C₁₋₄ alkyl group, a C₂₋₄ alkenyl group, a C₂₋₄ alkynyl group, a C₃₋₅ cycloalkyl group or a hydroxy C₁₋₄ alkyl group,
each of R⁶ and R⁷ independently represents a hydrogen atom or a C₁₋₄ alkyl group,
X represents a monocyclic or bicyclic C₃₋₁₀ cycloalkyl group, a monocyclic or bicyclic C₆₋₁₀ aryl group or a monocyclic or bicyclic 5-to 10-membered heterocyclic group (in which the heterocyclic group includes aromatic heterocycles and non-aromatic heterocycles and contains 1 to 3 atoms selected from the group consisting of a nitrogen atom, a sulfur atom and an oxygen atom), which may be substituted with 1 to 3 substituents selected from substituent group α,
Y represents -CHRa-, -C(OH)Ra-, -CO-, -O-, -NRa-, -S-, -SO- or - SO₂-,
Ra represents a hydrogen atom, a C₁₋₄ alkyl group, a halogeno C₁₋₄ alkyl group or a C₁₋₄ alkanoyl group,or
in the case where Ra in Y is a C₁₋₄ alkyl group, a halogeno C₁₋₄ alkyl group or a C₁₋₄ alkanoyl group, Ra may form a 5- or 6-membered ring by bonding with an atom that is a component of X,
substituent group α represents:
a halogen atom, a cyano group, a nitro group, a hydroxy group;
a C₁₋₄ alkyl group, a C₂₋₄ alkenyl group, a C₂₋₄ alkynyl group, a C₃₋₆ cycloalkyl group, a C₁₋₄ alkoxy group or a C₃₋₆ cycloalkoxy group, which may be substituted with substituent(s) selected from substituent group β;
-OCORb, -CORb, -COORb, -CONRbRc, -NRbRc, -NRbCORc, -NRbSORc, - NRbSO₂Rc, -SRb, -SORb, -SO₂Rb;
a phenyl group, and
a 5- or 6-membered heterocyclic group, which may be substituted with a C₁₋₄ alkyl group(s) (in which the heterocyclic group includes aromatic heterocycles and non-aromatic heterocycles and contains 1 to 3 atoms selected from the group consisting of a nitrogen atom, a sulfur atom and an oxygen atom),
each of Rb and Rc independently represents a hydrogen atom, a C₁₋₄ alkyl group or a halogeno C₁₋₄ alkyl group, and
substituent group β represents a halogen atom, a hydroxy group, a C₁₋₄ alkoxy group and a C₁₋₄ alkanoyloxy group,
or a pharmacologically acceptable salt thereof.

2. A compound or a pharmacologically acceptable salt thereof, according to claim 1, wherein each of R¹ and R² independently represents a hydrogen atom or a halogen atom.

3. A compound or a pharmacologically acceptable salt thereof, according to claim 1, wherein each of R¹ and R² independently represents a hydrogen atom or a fluorine atom.

4. A compound or a pharmacologically acceptable salt thereof, according to claim 1, wherein R¹ and R² both represent a hydrogen atom.

5. A compound or a pharmacologically acceptable salt thereof, according to any one of claims 1 to 4, wherein R³ represents a hydrogen atom or a methyl group.

6. A compound or a pharmacologically acceptable salt thereof, according to any one of claims 1 to 4, wherein R³ represents a hydrogen atom.

7. A compound or a pharmacologically acceptable salt thereof, according to any one of claims 1 to 6, wherein R⁴ represents a hydrogen atom, a fluorine atom, a C₁₋₄ alkyl group, a C₂₋₄ alkenyl group, a C₂₋₄ alkynyl group, a C₃₋₅ cycloalkyl group or a hydroxy C₁₋₄ alkyl group, and R⁵ represents a hydrogen atom.

8. A compound or a pharmacologically acceptable salt thereof, according to any one of claims 1 to 6, wherein R⁴ represents a methyl group, an ethyl group, an isopropyl group, an isobutyl group, a t-butyl group, a cyclopropyl group or a 1-hydroxy-1-methylethyl group, and R⁵ represents a hydrogen atom.

9. A compound or a pharmacologically acceptable salt thereof, according to any one of claims 1 to 6, wherein R⁴ represents a methyl group, an ethyl group, an isopropyl group, a t-butyl group, a cyclopropyl group or a 1-hydroxy-1-methylethyl group, and R⁵ represents a hydrogen atom.

10. A compound or a pharmacologically acceptable salt thereof, according to any one of claims 1 to 6, wherein R⁴ represents an ethyl group, an isopropyl group, a cyclopropyl group or a 1-hydroxy-1-methylethyl group, and R⁵ represents a hydrogen atom.

11. A compound or a pharmacologically acceptable salt thereof, according to any one of claims 1 to 6, wherein each of R⁴ and R⁵ independently represents a hydrogen atom, a halogen atom, a C₁₋₄ alkyl group, a C₂₋₄ alkenyl group, a C₂₋₄ alkynyl group or a C₃₋₅ cycloalkyl group.

12. A compound or a pharmacologically acceptable salt thereof, according to any one of claims 1 to 6, wherein R⁴ represents a hydrogen atom, a methyl group, an ethyl group, an isopropyl group, an isobutyl group, a t-butyl group or a cyclopropyl group, and R⁵ represents a hydrogen atom.

13. A compound or a pharmacologically acceptable salt thereof, according to any one of claims 1 to 12, wherein each of R⁶ and R⁷ independently represents a hydrogen atom or a methyl group.

14. A compound or or pharmacologically acceptable salt thereof, according to any one of claims 1 to 12, wherein R⁶ and R⁷ both represent a hydrogen atom.

15. A compound or a pharmacologically acceptable salt thereof, according to any one of claims 1 to 14, wherein X represents a phenyl group, a naphthyl group, a pyridyl group, a pyridazinyl group, a pyrimidinyl group, a pyrazinyl group, a quinolyl group, an isoquinolyl group, a benzimidazolyl group, a benzoxazolyl group or a benzothiazolyl group, which may be substituted with 1 or 2 substituents selected from substituent group α, and
substituent group α represents a halogen atom, a cyano group, a nitro group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, an acetoxy group, a trifluoromethoxy group, an acetyl group, a methoxycarbonyl group, an ethoxycarbonyl group, a dimethylcarbamoyl group, a piperidyl group, a pyrrolidyl group, a morpholino group, a pyrazolyl group, a methylpyrazolyl group, an oxadiazolyl group, a methyloxadiazolyl group, a phenyl group and a pyridyl group.

16. A compound or a pharmacologically acceptable salt thereof, according to any one of claims 1 to 14, wherein X represents a phenyl group, a naphthyl group, a pyridyl group, a pyridazinyl group, a pyrimidinyl group, a pyrazinyl group, a quinolyl group or an isoquinolyl group, which may be substituted with 1 or 2 substituents selected from substituent group α, and
substituent group α represents a fluorine atom, a chlorine atom, a bromine atom, a cyano group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, a trifluoromethoxy group, a piperidyl group, a pyrrolidyl group, a phenyl group and a pyridyl group.

17. A compound or a pharmacologically acceptable salt thereof, according to any one of claims 1 to 14, wherein X represents a phenyl group, a 2-pyridyl group, a 3-pyridyl group, a 3-pyridazinyl group, a 2-pyrimidinyl group or a 5-pyrimidinyl group, which may be substituted with 1 or 2 substituents selected from substituent group α, and
substituent group α represents a fluorine atom, a chlorine atom, a bromine atom, a cyano group, a hydroxy group, a methyl group, an ethyl group, a t-butyl group, a trifluoromethyl group, a methoxy group, an ethoxy group, a trifluoromethoxy group, a phenyl group and a pyridyl group.

18. A compound or a pharmacologically acceptable salt thereof, according to any one of claims 1 to 14, wherein X represents a phenyl group or a 2-pyridyl group, which may be substituted with 1 or 2 substituents selected from substituent group α, and
substituent group α represents a fluorine atom, a chlorine atom, a trifluoromethyl group, a methoxy group and a trifluoromethoxy group.

19. A compound or a pharmacologically acceptable salt thereof, according to any one of claims 1 to 14, wherein X represents 4-fluorophenyl, 2,4-difluorophenyl, 3,4-difluorophenyl, 4-chlorophenyl, 2,4-dichlorophenyl, 4-trifluoromethylphenyl, 4-trifluoromethoxyphenyl, 5-fluoropyridin-2-yl, 5-chloropyridin-2-yl, 5-trifluoromethylpyridin-2-yl or 6-methoxypyridin-3-yl.

20. A compound or a pharmacologically acceptable salt thereof, according to any one of claims 1 to 14, wherein X represents a phenyl group, a naphthyl group, a pyridyl group, a pyridazinyl group, a pyrimidinyl group, a pyrazinyl group, a quinolyl group or an isoquinolyl group, which may be substituted with 1 or 2 substituents selected from substituent group α, and
substituent group α represents a fluorine atom, a chlorine atom, a bromine atom, a cyano group, a hydroxy group, a methyl group, an ethyl group, a propyl group, an isopropyl group, a t-butyl group, a hydroxymethyl group, a fluoromethyl group, a difluoromethyl group, a trifluoromethyl group, a methoxy group, an ethoxy group, a propoxy group, an isopropoxy group, a trifluoromethoxy group, a phenyl group and a pyridyl group.

21. A compound or a pharmacologically acceptable salt thereof, according to any one of claims 1 to 14, wherein X represents a phenyl group, a naphthyl group, a pyridyl group, a pyridazinyl group or a pyrimidinyl group, which may be substituted with 1 or 2 substituents selected from substituent group α, and
substituent group α represents a fluorine atom, a chlorine atom, a bromine atom, a cyano group, a hydroxy group, a methyl group, a trifluoromethyl group, a methoxy group, a trifluoromethoxy group and a phenyl group.

22. A compound or a pharmacologically acceptable salt thereof, according to any one of claims 1 to 14, wherein X represents a phenyl group or a pyridyl group, which may be substituted with 1 or 2 substituents selected from substituent group α, and
substituent group α represents a fluorine atom, a chlorine atom, a cyano group, a trifluoromethyl group and a trifluoromethoxy group.

23. A compound or a pharmacologically acceptable salt thereof, according to any one of claims 1 to 22, wherein Y represents -CO-, -O- or -NRa-, Ra represents a hydrogen atom, a methyl group or an ethyl group, and Ra may form a 5-membered ring by bonding with an atom that is a component of X in the case where Ra is an ethyl group in Y.

24. A compound or a pharmacologically acceptable salt thereof, according to any one of claims 1 to 22, wherein Y represents -O- or - NRa-, and Ra represents a hydrogen atom, a methyl group or an ethyl group.

25. A compound or a pharmacologically acceptable salt thereof, according to any one of claims 1 to 22, wherein Y represents -O-.

26. A compound or a pharmacologically acceptable salt thereof, according to claim 1, which is selected from the following:
N-{(1S)-2-[4-(2-fluorophenoxy)piperidin-1-yl]-1-methyl-2-oxoethyl}-3-hydroxyquinoxaline-2-carboxamide,
N-{(1S)-2-[4-(3-fluorophenoxy)piperidin-1-yl]-1-methyl-2-oxoethyl}-3-hydroxyquinoxaline-2-carboxamide,
N-{(1S)-2-[4-(4-fluorophenoxy)piperidin-1-yl]-1-methyl-2-oxoethyl}-3-hydroxyquinoxaline-2-carboxamide,
N-{(1S)-2-[4-(4-chlorophenoxy)piperidin-1-yl]-1-methyl-2-oxoethyl}-3-hydroxyquinoxaline-2-carboxamide,
3-hydroxy-N-[(1S)-1-methyl-2-oxo-2-{4-[4-(trifluoromethoxy)phenoxy]piperidin-1-yl}ethyl]quinoxaline-2-carboxamide,
N-{(1S)-2-[4-(4-cyanophenoxy)piperidin-1-yl]-1-methyl-2-oxoethyl}-3-hydroxyquinoxaline-2-carboxamide,
N-{(1S)-2-[4-(2,4-difluorophenoxy)piperidin-1-yl]-1-methyl-2-oxoethyl}-3-hydroxyquinoxaline-2-carboxamide,
N-{(1S)-2-[4-(3,4-difluorophenoxy)piperidin-1-yl]-1-methyl-2-oxoethyl}-3-hydroxyquinoxaline-2-carboxamide,
N-[(1S)-2-{4-[(5-chloropyridin-2-yl)oxy]piperidin-1-yl}-1-methyl-2-oxoethyl]-3-hydroxyquinoxaline-2-carboxamide,
N-[(1S)-2-{4-[(5-fluoropyridin-2-yl)oxy]piperidin-1-yl}-1-methyl-2-oxoethyl]-3-hydroxyquinoxaline-2-carboxamide,
3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
N-[(1S)-1-{[4-(2-fluorophenoxy)piperidin-1-yl]carbonyl}propyl]-3-hydroxyquinoxaline-2-carboxamide,
N-[(1S)-1-{[4-(3-fluorophenoxy)piperidin-1-yl]carbonyl}propyl]-3-hydroxyquinoxaline-2-carboxamide,
N-[(1S)-1-{[4-(4-fluorophenoxy)piperidin-1-yl]carbonyl}propyl]-3-hydroxyquinoxaline-2-carboxamide,
N-[(1S)-1-{[4-(4-chlorophenoxy)piperidin-1-yl]carbonyl}propyl]-3-hydroxyquinoxaline-2-carboxamide,
3-hydroxy-N-[(1S)-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}propyl]-3-hydroxyquinoxaline-2-carboxamide,
N-[(1S)-1-{[4-(2,4-difluorophenoxy)piperidin-1-yl]carbonyl}propyl]-3-hydroxyquinoxaline-2-carboxamide,
N-[(1S)-1-{[4-(3,4-difluorophenoxy)piperidin-1-yl]carbonyl}propyl]-3-hydroxyquinoxaline-2-carboxamide,
N-[(1S)-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)propyl]-3-hydroxyquinoxaline-2-carboxamide,
3-hydroxy-N-{(1S)-1-[(4-{[5-(trifluoromethyl)pyridin-2-yl]oxy}piperidin-1-yl)carbonyl]propyl}quinoxaline-2-carboxamide,
N-[(1S)-1-{[4-(2-fluorophenoxy)piperidin-1-yl]carbonyl}-2-methylpropyl]-3-hydroxyquinoxaline-2-carboxamide,
N-[(1S)-1-{[4-(3-fluorophenoxy)piperidin-1-yl]carbonyl}-2-methylpropyl]-3-hydroxyquinoxaline-2-carboxamide,
N-[(1S)-1-{[4-(4-fluorophenoxy)piperidin-1-yl]carbonyl}-2-methylpropyl]-3-hydroxyquinoxaline-2-carboxamide,
N-[(1S)-1-{[4-(4-chlorophenoxy)piperidin-1-yl]carbonyl}-2-methylpropyl]-3-hydroxyquinoxaline-2-carboxamide,
3-hydroxy-N-[(1S)-2-methyl-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}-2-methylpropyl]-3-hydroxyquinoxaline-2-carboxamide,
N-[(1S)-1-{[4-(2,4-difluorophenoxy)piperidin-1-yl]carbonyl}-2-methylpropyl]-3-hydroxyquinoxaline-2-carboxamide,
N-[(1S)-1-{[4-(3,4-difluorophenoxy)piperidin-1-yl]carbonyl}-2-methylpropyl]-3-hydroxyquinoxaline-2-carboxamide,
N-[(1S)-1-({4-[(5-chloropyridin-2-yl)oxy]piperidin-1-yl}carbonyl)-2-methylpropyl]-3-hydroxyquinoxaline-2-carboxamide,
N-[(1S)-1-({4-[(5-fluoropyridin-2-yl)oxy]piperidin-1-yl}carbonyl)-2-methylpropyl]-3-hydroxyquinoxaline-2-carboxamide,
3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide,
3-hydroxy-N-[(1S)-1-({4-[(6-methoxypyridin-3-yl)oxy]piperidin-1-yl}carbonyl)-2-methylpropyl]quinoxaline-2-carboxamide,
N-[(1S)-1-{[4-(2,4-difluorobenzoyl)piperidin-1-yl]carbonyl}-2-methylpropyl]-3-hydroxyquinoxaline-2-carboxamide,
N-[(1S)-1-({4-[(2,4-difluorophenyl)(hydroxy)methyl]piperidin-1-yl}carbonyl)-2-methylpropyl]-3-hydroxyquinoxaline-2-carboxamide,
N-[(1S)-1-{[4-(4-fluorophenoxy)piperidin-1-yl]carbonyl}-2-hydroxy-2-methylpropyl]-3-hydroxyquinoxaline-2-carboxamide,
N-{(1S)-1-cyclopropyl-2-[4-(2-fluorophenoxy)piperidin-1-yl]-2-oxoethyl}-3-hydroxyquinoxaline-2-carboxamide,
N-{(1S)-1-cyclopropyl-2-[4-(3-fluorophenoxy)piperidin-1-yl]-2-oxoethyl}-3-hydroxyquinoxaline-2-carboxamide,
N-{(1S)-1-cyclopropyl-2-[4-(4-fluorophenoxy)piperidin-1-yl]-2-oxoethyl}-3-hydroxyquinoxaline-2-carboxamide,
N-{(1S)-2-[4-(4-chlorophenoxy)piperidin-1-yl]-1-cyclopropyl-2-oxoethyl}-3-hydroxyquinoxaline-2-carboxamide,
N-[(1S)-1-cyclopropyl-2-oxo-2-{4-[4-(trifluoromethoxy)phenoxy]piperidin-1-yl}ethyl]-3-hydroxyquinoxaline-2-carboxamide,
N-{(1S)-2-[4-(4-cyanophenoxy)piperidin-1-yl]-1-cyclopropyl-2-oxoethyl}-3-hydroxyquinoxaline-2-carboxamide,
N-{(1S)-1-cyclopropyl-2-[4-(2,4-difluorophenoxy)piperidin-1-yl]-2-oxoethyl}-3-hydroxyquinoxaline-2-carboxamide,
N-{(1S)-1-cyclopropyl-2-[4-(3,4-difluorophenoxy)piperidin-1-yl]-2-oxoethyl}-3-hydroxyquinoxaline-2-carboxamide,
N-[(1S)-1-cyclopropyl-2-{4-[(5-fluoropyridin-2-yl)oxy]piperidin-1-yl}-2-oxoethyl]-3-hydroxyquinoxaline-2-carboxamide,
N-[(1S)-2-{4-[(5-chloropyridin-2-yl)oxy]piperidin-1-yl}-1-cyclopropyl-2-oxoethyl]-3-hydroxyquinoxaline-2-carboxamide,
N-[(1S)-1-cyclopropyl-2-oxo-2-(4-{[5-(trifluoromethyl)pyridin-2-yl]oxy}piperidin-1-yl)ethyl]-3-hydroxyquinoxaline-2-carboxamide,
N-[(1S)-2,2-dimethyl-1-{[4-(2-fluorophenoxy)piperidin-1-yl]carbonyl}propyl]-3-hydroxyquinoxaline-2-carboxamide,
N-[(1S)-2,2-dimethyl-1-{[4-(3-fluorophenoxy)piperidin-1-yl]carbonyl}propyl]-3-hydroxyquinoxaline-2-carboxamide,
N-[(1S)-2,2-dimethyl-1-{[4-(4-fluorophenoxy)piperidin-1-yl]carbonyl}propyl]-3-hydroxyquinoxaline-2-carboxamide,
N-[(1S)-1-{[4-(4-chlorophenoxy)piperidin-1-yl]carbonyl}-2,2-dimethylpropyl]-3-hydroxyquinoxaline-2-carboxamide,
N-[(1S)-2,2-dimethyl-1-({4-[4-(trifluoromethoxy)phenoxy]piperidin-1-yl}carbonyl)propyl]-3-hydroxyquinoxaline-2-carboxamide,
N-[(1S)-1-{[4-(4-cyanophenoxy)piperidin-1-yl]carbonyl}-2,2-dimethylpropyl]-3-hydroxyquinoxaline-2-carboxamide,
N-[(1S)-1-{[4-(2,4-difluorophenoxy)piperidin-1-yl]carbonyl}-2,2-dimethylpropyl]-3-hydroxyquinoxaline-2-carboxamide,
N-[(1S)-1-{[4-(3,4-difluorophenoxy)piperidin-1-yl]carbonyl}-2,2-dimethylpropyl]-3-hydroxyquinoxaline-2-carboxamide,
N-[(1S)-2,2-dimethyl-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)-2,2-dimethylpropyl]-3-hydroxyquinoxaline-2-carboxamide, and
N-{(1S)-2,2-dimethyl-1-[(4-{[5-(trifluoromethyl)pyridin-2-yl]oxy}piperidin-1-yl)carbonyl]propyl}-3-hydroxyquinoxaline-2-carboxamide.

27. A compound or a pharmacologically acceptable salt thereof, according to claim 1, which is selected from the following:
N-{(1S)-2-[4-(2-fluorophenoxy)piperidin-1-yl]-1-methyl-2-oxoethyl}-3-hydroxyquinoxaline-2-carboxamide,
N-{(1S)-2-[4-(3-fluorophenoxy)piperidin-1-yl]-1-methyl-2-oxoethyl}-3-hydroxyquinoxaline-2-carboxamide,
N-{(1S)-2-[4-(4-fluorophenoxy)piperidin-1-yl]-1-methyl-2-oxoethyl}-3-hydroxyquinoxaline-2-carboxamide,
N-{(1S)-2-[4-(4-chlorophenoxy)piperidin-1-yl]-1-methyl-2-oxoethyl}-3-hydroxyquinoxaline-2-carboxamide,
3-hydroxy-N-[(1S)-1-methyl-2-oxo-2-{4-[4-(trifluoromethoxy)phenoxy]piperidin-1-yl}ethyl]quinoxaline-2-carboxamide,
N-{(1S)-2-[4-(4-cyanophenoxy)piperidin-1-yl]-1-methyl-2-oxoethyl}-3-hydroxyquinoxaline-2-carboxamide,
N-{(1S)-2-[4-(2,4-difluorophenoxy)piperidin-1-yl]-1-methyl-2-oxoethyl}-3-hydroxyquinoxaline-2-carboxamide,
N-{(1S)-2-[4-(3,4-difluorophenoxy)piperidin-1-yl]-1-methyl-2-oxoethyl}-3-hydroxyquinoxaline-2-carboxamide,
N-[(1S)-2-{4-[(5-chloropyridin-2-yl)oxy]piperidin-1-yl}-1-methyl-2-oxoethyl]-3-hydroxyquinoxaline-2-carboxamide,
N-[(1S)-2-{4-[(5-fluoropyridin-2-yl)oxy]piperidin-1-yl}-1-methyl-2-oxoethyl]-3-hydroxyquinoxaline-2-carboxamide,
3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
N-[(1S)-1-{[4-(2-fluorophenoxy)piperidin-1-yl]carbonyl}propyl]-3-hydroxyquinoxaline-2-carboxamide,
N-[(1S)-1-{[4-(3-fluorophenoxy)piperidin-1-yl]carbonyl}propyl]-3-hydroxyquinoxaline-2-carboxamide,
N-[(1S)-1-{[4-(4-fluorophenoxy)piperidin-1-yl]carbonyl}propyl]-3-hydroxyquinoxaline-2-carboxamide,
N-[(1S)-1-{[4-(4-chlorophenoxy)piperidin-1-yl]carbonyl}propyl]-3-hydroxyquinoxaline-2-carboxamide,
3-hydroxy-N-[(1S)-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}propyl]-3-hydroxyquinoxaline-2-carboxamide,
N-[(1S)-1-{[4-(2,4-difluorophenoxy)piperidin-1-yl]carbonyl}propyl]-3-hydroxyquinoxaline-2-carboxamide,
N-[(1S)-1-{[4-(3,4-difluorophenoxy)piperidin-1-yl]carbonyl}propyl]-3-hydroxyquinoxaline-2-carboxamide,
N-[(1S)-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)propyl]-3-hydroxyquinoxaline-2-carboxamide,
3-hydroxy-N-{(1S)-1-[(4-{[5-(trifluoromethyl)pyridin-2-yl]oxy}piperidin-1-yl)carbonyl]propyl}quinoxaline-2-carboxamide,
N-[(1S)-1-{[4-(2-fluorophenoxy)piperidin-1-yl]carbonyl}-2-methylpropyl]-3-hydroxyquinoxaline-2-carboxamide,
N-[(1S)-1-{[4-(3-fluorophenoxy)piperidin-1-yl]carbonyl}-2-methylpropyl]-3-hydroxyquinoxaline-2-carboxamide,
N-[(1S)-1-{[4-(4-fluorophenoxy)piperidin-1-yl]carbonyl}-2-methylpropyl]-3-hydroxyquinoxaline-2-carboxamide,
N-[(1S)-1-{[4-(4-chlorophenoxy)piperidin-1-yl]carbonyl}-2-methylpropyl]-3-hydroxyquinoxaline-2-carboxamide,
3-hydroxy-N-[(1S)-2-methyl-1-({4-[4-(trifluoromethoxy)phenoxy]piperidin-1-yl}carbonyl)propyl]quinoxaline-2-carboxamide,
N-[(1S)-1-{[4-(4-cyanophenoxy)piperidin-1-yl]carbonyl}-2-methylpropyl]-3-hydroxyquinoxaline-2-carboxamide,
N-[(1S)-1-{[4-(2,4-difluorophenoxy)piperidin-1-yl]carbonyl}-2-methylpropyl]-3-hydroxyquinoxaline-2-carboxamide,
N-[(1S)-1-{[4-(3,4-difluorophenoxy)piperidin-1-yl]carbonyl}-2-methylpropyl]-3-hydroxyquinoxaline-2-carboxamide,
N-[(1S)-1-({4-[(5-chloropyridin-2-yl)oxy]piperidin-1-yl}carbonyl)-2-methylpropyl]-3-hydroxyquinoxaline-2-carboxamide,
N-[(1S)-1-({4-[(5-fluoropyridin-2-yl)oxy]piperidin-1-yl}carbonyl)-2-methylpropyl]-3-hydroxyquinoxaline-2-carboxamide,
3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide,
N-{(1S)-1-cyclopropyl-2-[4-(2-fluorophenoxy)piperidin-1-yl]-2-oxoethyl}-3-hydroxyquinoxaline-2-carboxamide,
N-{(1S)-1-cyclopropyl-2-[4-(3-fluorophenoxy)piperidin-1-yl]-2-oxoethyl}-3-hydroxyquinoxaline-2-carboxamide,
N-{(1S)-1-cyclopropyl-2-[4-(4-fluorophenoxy)piperidin-1-yl]-2-oxoethyl}-3-hydroxyquinoxaline-2-carboxamide,
N-{(1S)-2-[4-(4-chlorophenoxy)piperidin-1-yl]-1-cyclopropyl-2-oxoethyl}-3-hydroxyquinoxaline-2-carboxamide,
N-[(1S)-1-cyclopropyl-2-oxo-2-{4-[4-(trifluoromethoxy)phenoxy]piperidin-1-yl}ethyl]-3-hydroxyquinoxaline-2-carboxamide,
N-{(1S)-2-[4-(4-cyanophenoxy)piperidin-1-yl]-1-cyclopropyl-2-oxoethyl}-3-hydroxyquinoxaline-2-carboxamide,
N-{(1S)-1-cyclopropyl-2-[4-(2,4-difluorophenoxy)piperidin-1-yl]-2-oxoethyl}-3-hydroxyquinoxaline-2-carboxamide,
N-{(1S)-1-cyclopropyl-2-[4-(3,4-difluorophenoxy)piperidin-1-yl]-2-oxoethyl}-3-hydroxyquinoxaline-2-carboxamide,
N-[(1S)-1-cyclopropyl-2-{4-[(5-fluoropyridin-2-yl)oxy]piperidin-1-yl}-2-oxoethyl]-3-hydroxyquinoxaline-2-carboxamide,
N-[(1S)-2-{4-[(5-chloropyridin-2-yl)oxy]piperidin-1-yl}-1-cyclopropyl-2-oxoethyl]-3-hydroxyquinoxaline-2-carboxamide,
N-[(1S)-1-cyclopropyl-2-oxo-2-(4-{[5-(trifluoromethyl)pyridin-2-yl]oxy}piperidin-1-yl)ethyl]-3-hydroxyquinoxaline-2-carboxamide,
N-[(1S)-2,2-dimethyl-1-{[4-(2-fluorophenoxy)piperidin-1-yl]carbonyl}propyl]-3-hydroxyquinoxaline-2-carboxamide,
N-[(1S)-2,2-dimethyl-1-{[4-(3-fluorophenoxy)piperidin-1-yl]carbonyl}propyl]-3-hydroxyquinoxaline-2-carboxamide,
N-[(1S)-2,2-dimethyl-1-{[4-(4-fluorophenoxy)piperidin-1-yl]carbonyl}propyl]-3-hydroxyquinoxaline-2-carboxamide,
N-[(1S)-1-{[4-(4-chlorophenoxy)piperidin-1-yl]carbonyl}-2,2-dimethylpropyl]-3-hydroxyquinoxaline-2-carboxamide,
N-[(1S)-2,2-dimethyl-1-({4-[4-(trifluoromethoxy)phenoxy]piperidin-1-yl}carbonyl)propyl]-3-hydroxyquinoxaline-2-carboxamide,
N-[(1S)-1-{[4-(4-cyanophenoxy)piperidin-1-yl]carbonyl}-2,2-dimethylpropyl]-3-hydroxyquinoxaline-2-carboxamide,
N-[(1S)-1-{[4-(2,4-difluorophenoxy)piperidin-1-yl]carbonyl}-2,2-dimethylpropyl]-3-hydroxyquinoxaline-2-carboxamide,
N-[(1S)-1-{[4-(3,4-difluorophenoxy)piperidin-1-yl]carbonyl}-2,2-dimethylpropyl]-3-hydroxyquinoxaline-2-carboxamide,
N-[(1S)-2,2-dimethyl-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)-2,2-dimethylpropyl]-3-hydroxyquinoxaline-2-carboxamide, and
N-{(1S)-2,2-dimethyl-1-[(4-{[5-(trifluoromethyl)pyridin-2-yl]oxy}piperidin-1-yl)carbonyl]propyl}-3-hydroxyquinoxaline-2-carboxamide.

28. A compound or a pharmacologically acceptable salt thereof, according to claim 1, which is selected from the following:
N-{(1S)-2-[4-(4-chlorophenoxy)piperidin-1-yl]-1-methyl-2-oxoethyl}-3-hydroxyquinoxaline-2-carboxamide,
N-{(1S)-2-[4-(2,4-difluorophenoxy)piperidin-1-yl]-1-methyl-2-oxoethyl}-3-hydroxyquinoxaline-2-carboxamide,
N-{(1S)-2-[4-(3,4-difluorophenoxy)piperidin-1-yl]-1-methyl-2-oxoethyl}-3-hydroxyquinoxaline-2-carboxamide,
N-[(1S)-2-{4-[(5-chloropyridin-2-yl)oxy]piperidin-1-yl}-1-methyl-2-oxoethyl]-3-hydroxyquinoxaline-2-carboxamide,
N-[(1S)-2-{4-[(5-fluoropyridin-2-yl)oxy]piperidin-1-yl}-1-methyl-2-oxoethyl]-3-hydroxyquinoxaline-2-carboxamide,
3-hydroxy-N-[(1S)-1-methyl-2-oxo-2-(4-{[5-(trifluoromethyl)pyridin-2-yl]oxy}piperidin-1-yl)ethyl]quinoxaline-2-carboxamide,
N-[(1S)-1-{[4-(4-chlorophenoxy)piperidin-1-yl]carbonyl}propyl]-3-hydroxyquinoxaline-2-carboxamide,
N-[(1S)-1-{[4-(4-fluorophenoxy)piperidin-1-yl]carbonyl}propyl]-3-hydroxyquinoxaline-2-carboxamide,
N-[(1S)-1-({4-[(5-fluoropyridin-2-yl)oxy]piperidin-1-yl}carbonyl)propyl]-3-hydroxyquinoxaline-2-carboxamide,
N-[(1S)-1-({4-[(5-chloropyridin-2-yl)oxy]piperidin-1-yl}carbonyl)propyl]-3-hydroxyquinoxaline-2-carboxamide,
3-hydroxy-N-{(1S)-1-[(4-{[5-(trifluoromethyl)pyridin-2-yl]oxy}piperidin-1-yl)carbonyl]propyl}quinoxaline-2-carboxamide,
N-[(1S)-1-{[4-(4-fluorophenoxy)piperidin-1-yl]carbonyl}-2-methylpropyl]-3-hydroxyquinoxaline-2-carboxamide,
3-hydroxy-N-[(1S)-2-methyl-1-({4-[4-(trifluoromethoxy)phenoxy]piperidin-1-yl}carbonyl)propyl]quinoxaline-2-carboxamide,
N-[(1S)-1-({4-[(5-fluoropyridin-2-yl)oxy]piperidin-1-yl}carbonyl)-2-methylpropyl]-3-hydroxyquinoxaline-2-carboxamide,
N-[(1S)-1-({4-[(5-chloropyridin-2-yl)oxy]piperidin-1-yl}carbonyl)-2-methylpropyl]-3-hydroxyquinoxaline-2-carboxamide,
3-hydroxy-N-{(1S)-2-methyl-1-[(4-{[5-(trifluoromethyl)pyridin-2-yl]oxy}piperidin-1-yl)carbonyl]propyl}quinoxaline-2-carboxamide, and
N-{(1S)-1-cyclopropyl-2-[4-(4-fluorophenoxy)piperidin-1-yl]-2-oxoethyl}-3-hydroxyquinoxaline-2-carboxamide.

29. A pharmaceutical composition comprising as an active ingredient a compound or a pharmacologically acceptable salt thereof according to any one of claims 1 to 28.

30. A pharmaceutical composition for preventing and/or treating a blood coagulation disorder, comprising as an active ingredient a compound or a pharmacologically acceptable salt thereof according to any one of claims 1 to 28.

31. A pharmaceutical composition for preventing and/or treating thromboembolism, congenital anticoagulant factor deficiency or plasminogen abnormality, comprising as an active ingredient a compound or a pharmacologically acceptable salt thereof according to any one of claims 1 to 28.

32. A pharmaceutical composition for preventing thrombogenesis following artificial valve replacement surgery, or thrombogenesis caused by nonvalvular atrial fibrillation or atrial fibrillation accompanying valvular heart disease, comprising as an active ingredient a compound or a pharmacologically acceptable salt thereof according to any one of claims 1 to 28.
